# EUROPEAN PATENT SPECIFICATION

(11) **EP 4 025 253 B1**
(45) Date of publication and mention of the grant of the patent: **20.03.2024**
(21) Application number: 20764406.3
(22) Date of filing: 04.09.2020
(51) Int. Cl.: A61K 47/68, A61K 31/351, C07D 309/10

(54) **DRUG ANTIBODY CONJUGATES**
ARZNEIMITTEL-ANTIKÖRPER-KONJUGATE
CONJUGUÉS ANTICORPS-MÉDICAMENTS

(30) Priority: 05.09.2019 EP 19382765
(43) Date of publication of application: 13.07.2022
(73) Proprietor: Pharma Mar, S.A., 28770 Madrid (ES)
(72) Inventor: LATORRE LOZANO, Alfonso, 28770 Madrid (ES); GUTIÉRREZ LORIENTE, Agustín, 28770 Madrid (ES); CUEVAS MARCHANTE, Maria Del Carmen, 28770 Madrid (ES)
(74) Representative: Marks & Clerk LLP
(86) International application number: PCT/EP2020/074695
(87) International publication number: WO 2021/043951

(56) References cited:
- WO-A1-2013/016120
- WO-A1-2018/167270
- ZBIGNIEW J. WITCZAK ET AL: "Mycalamides, Pederin and Psymberin as Natural Carbohydrates and Potential Antitumor Agents: Past and Future Perspectives", MINI REVIEWS IN MEDICINAL CHEMISTRY, vol. 12, no. 14, 1 November 2012 (2012-11-01), pages 1520-1532, XP055742352, NL ISSN: 1389-5575, DOI: 10.2174/138955712803832744
- SCOTT C. JEFFREY ET AL: "Expanded Utility of the &bgr;-Glucuronide Linker: ADCs That Deliver Phenolic Cytotoxic Agents", ACS MEDICINAL CHEMISTRY LETTERS, vol. 1, no. 6, 9 September 2010 (2010-09-09), pages 277-280, XP055225196, US ISSN: 1948-5875, DOI: 10.1021/ml100039h

## Description

### Field of the Invention

The present invention relates to novel drug conjugates, drug linker compounds, to methods for their preparation, pharmaceutical compositions containing said drug conjugates and their use as antitumoral agents.

### Background to the Invention

Patent application publication WO2013016120 describes a total synthesis of pederin and analogues thereof of formula: 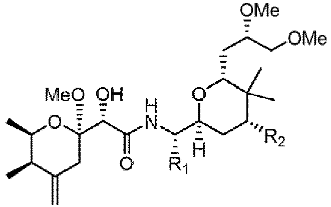 wherein at least one of R₁ or R₂ includes a linker that includes a reactive functional group that can bind to a targeting moiety.

International patent application publication WO2018167270 is directed to pederin-like compounds isolated from a free-living marine alphaproteobacteria and analogues thereof with high cytotoxic activity.

The treatment of cancer has progressed significantly in recent years with the development of pharmaceutical entities that target and kill cancer cells more efficiently. Researchers have taken advantage of cell-surface receptors and antigens selectively expressed by target cells such as cancer cells to develop pharmaceutical entities based on antibodies that bind, in the example of tumors, the tumor-specific or tumor-associated antigens. In order to achieve this, cytotoxic molecules such as chemotherapeutic drugs, bacteria, plant toxins and radionuclides have been chemically linked to monoclonal antibodies that bind tumor-specific or tumor-associated cell surface antigens.

Antibody-drug-conjugates (ADCs) represent a targeted strategy to deliver a cytotoxic molecule to a cancer cell. However, given the complex payload, linker and antibody structure, ADCs represent a challenging area of development, and there remains a need for further ADCs to be developed.

### Summary of the Invention

There is a need for novel active drug conjugates. The present invention addresses this need. It further provides novel drug linker compounds for use in the preparation of drug conjugates of the present invention, processes for the preparation of the novel drug conjugates of the present invention, pharmaceutical compositions containing said drug conjugates and their use as antitumoral agents, as well as a kit comprising the drug conjugate of the present invention for use in the treatment of cancer.

In a first aspect of the present invention there is provided a drug conjugate comprising a drug moiety covalently attached to the rest of the drug conjugate, the compound having formula [D-(X)_{b}-(AA)_{w}-(T)_{g}-(L)-]ₙ-Ab wherein:
D is a drug moiety having the following formula (I) or a pharmaceutically acceptable salt or ester thereof, 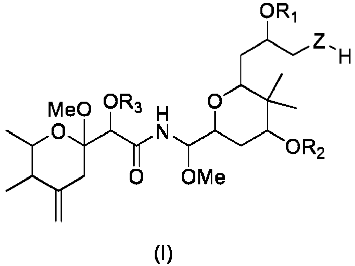 wherein:
D is covalently attached via a hydroxy group at OR_{1,} OR₃ or ZH or via a thiol group at ZH, to (X)_{b} if any, or (AA)_{w} if any, or to (T)_{g} if any, or (L);
R₁ is selected from hydrogen, substituted or unsubstituted C₁-C₁₂ alkyl, substituted or unsubstituted C₂-C₁₂ alkenyl, substituted or unsubstituted C₂-C₁₂ alkynyl, -C(=O)Rₐ, - C(=O)OR_{b} and -C(=O)NR_{c}R_{d};
R₂ is hydrogen;
R₃ is selected from hydrogen, -C(=O)Rₐ, -C(=O)OR_{b}, and -C(=O)NR_{c}R_{d};
Z is selected from -O- and -S-;
Rₐ is selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group;
R_{b} is selected from substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group;
R_{c} and R_{d} are independently selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl and substituted or unsubstituted heterocyclic group;
X and T are extending groups that may be the same or different;
each AA is independently an amino acid unit;
L is a linker group;
w is an integer ranging from 0 to 12;
b is an integer of 0 or 1;
g is an integer of 0 or 1;
Ab is a moiety comprising at least one antigen binding site; and
n is the ratio of the group [D-(X)_{b}-(AA)_{w}-(T)_{g}-(L)-] to the moiety comprising at least one antigen binding site and is in the range from 1 to 20.

In a further aspect of the present invention there is provided a drug conjugate comprising a drug moiety covalently attached to the rest of the drug conjugate, the compound having formula [D-(X)_{b}-(AA)_{w}-(T)_{g}-(L)-]ₙ-Ab wherein:
D is a drug moiety having the following formula (II) or a pharmaceutically acceptable salt or ester thereof, 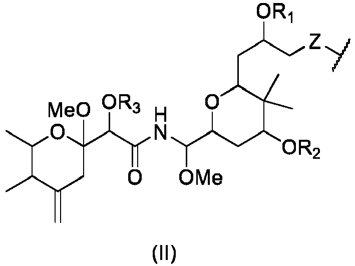 wherein:
the wavy line indicates the point of covalent attachment to (X)_{b} if any, or (AA)_{w} if any, or to (T)_{g} if any, or to (L);
R₁ is hydrogen, substituted or unsubstituted C₁-C₁₂ alkyl, substituted or unsubstituted C₂-C₁₂ alkenyl, substituted or unsubstituted C₂-C₁₂ alkynyl, -C(=O)Rₐ, -C(=O)OR_{b} and -C(=O)NR_{c}R_{d}; wherein the optional substituents are one or more substituents Rₓ;
R₂ is hydrogen;
R₃ is selected from hydrogen, -C(=O)Rₐ, -C(=O)OR_{b}, and --C(=O)NR_{c}R_{d};
Z is selected from -O- and -S-;
Rₐ is selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group; wherein the optional substituents are one or more substituents Rₓ;
R_{b} is selected from substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group; wherein the optional substituents are one or more substituents Rₓ;
R_{c} and R_{d} are independently selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl and substituted or unsubstituted heterocyclic group; wherein the optional substituents are one or more substituents Rₓ;
substituents Rₓ are selected from the group consisting of C₁-C₁₂ alkyl groups which may be optionally substituted with at least one group R_{y}, C₂-C₁₂ alkenyl groups which may be optionally substituted with at least one group R_{y}, C₂-C₁₂ alkynyl groups which may be optionally substituted with at least one group R_{y}, halogen atoms, oxo groups, thio groups, cyano groups, nitro groups, OR_{y}, OCOR_{y}, OCOOR_{y}, COR_{y}, COOR_{y}, OCONR_{y}R_{z}, CONR_{y}R_{z}, SR_{y}, S(=O)R_{y}, SO₂R_{y}, SSR_{y}, P(O)(R_{y})OR_{z}, NR_{y}R_{z}, NR_{y}COR_{z}, NR_{y}C(=O)NR_{y}R_{z}, NR_{y}C(=NR_{y})NR_{y}R_{z}, aryl groups having from 6 to 18 carbon atoms in one or more rings which may optionally be substituted with one or more substituents which may be the same or different selected from the group consisting of R_{y}, OR_{y}, OCOR_{y}, OCOOR_{y}, NR_{y}R_{z}, NR_{y}COR_{z}, and NR_{y}C(=NR_{y})NR_{y}R_{z}, aralkyl groups comprising an alkyl group having from 1 to 12 carbon atoms substituted with an optionally substituted aryl group as defined above, aralkyloxy groups comprising an alkoxy group having from 1 to 12 carbon atoms substituted with an optionally substituted aryl group as defined above, and a 5- to 14- membered saturated or unsaturated heterocyclic group having one or more rings and comprising at least one oxygen, nitrogen or sulphur atom in said ring(s), said heterocyclic group optionally being substituted with one or more substituents R_{y}, and where there is more than one optional substituents on any given group the optional substituents R_{y} may be the same or different;
each R_{y} and R_{z} is independently selected from the group consisting of hydrogen, C₁-C₁₂ alkyl groups, C₁-C₁₂ alkyl groups that are substituted with at least one halogen atom, aralkyl groups comprising a C₁-C₁₂ alkyl group that is substituted with an aryl group having from 6 to 18 carbon atoms in one or more rings and heterocycloalkyl groups comprising a C₁-C₁₂ alkyl group that is substituted with a 5- to 14- membered saturated or unsaturated heterocyclic group having one or more rings and comprising at least one oxygen, nitrogen or sulphur atom in said ring(s);
X and T are extending groups that may be the same or different;
each AA is independently an amino acid unit;
L is a linker group;
w is an integer ranging from 0 to 12;
b is an integer of 0 or 1;
g is an integer of 0 or 1;
where b+g+w is optionally not 0;
Ab is a moiety comprising at least one antigen binding site; and
n is the ratio of the group [D-(X)_{b}-(AA)_{w}-(T)_{g}-(L)-] to the moiety comprising at least one antigen binding site and is in the range from 1 to 20.

As we shall explain and exemplify in greater detail below, the drug conjugates of formula [D-(X)_{b}-(AA)_{w}-(T)_{g}-(L)-]ₙ-Ab of the present invention represent a breakthrough in addressing the problems outlined above of requiring further drug conjugates in addition to those based on the three main families of cytotoxic drugs that have been used as payloads to date, that show excellent antitumor activity.
In a further aspect of the present invention, there is provided a compound of formula D-(X)_{b}-(AA)_{w}-(T)_{g}-L₁ or of formula D-(X)_{b}-(AA)_{w}-(T)_{g}-H, wherein:
L₁ is a linker selected from the group of formulas consisting of: 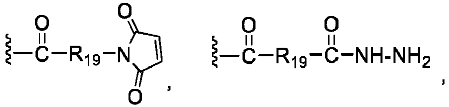 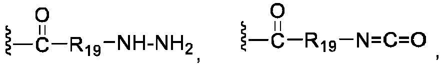 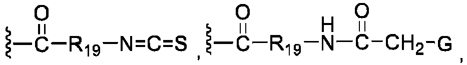 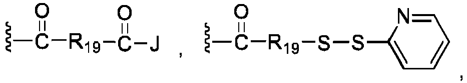 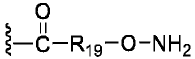
each of the the wavy lines indicates the point of covalent attachment to (T)_{g} if any, or (AA)_{w} if any, or to (X)_{b} if any, or to D;
G is selected from halo, -O-mesyl and -O-tosyl;
J is selected from halo, hydroxy, -N-succinimidoxy, -G-(4-nitrophenyl), -O-pentafluorophenyl, - O-tetrafluorophenyl and -O-C(O)-OR₂₀;
R₁₉ is selected from -C₁₋C₁₂ alkylene-, -C₃-C₈ carbocyclo-, -O-(C₁-C₁₂ alkylene)-, -C₆-C₁₈ arylene- in one or more rings which may optionally be substituted with one or more substituents Rₓ, -C₁₋C₁₂ alkylene-C₆-C₁₈ arylene- wherein the arylene group is in one or more rings which may optionally be substituted with one or more substituents Rₓ, -C₆-C₁₈ arylene-C₁-C₁₂ alkylene- wherein the arylene group is in one or more rings which may optionally be substituted with one or more substituents Rₓ, -C₁₋C₁₂ alkylene-(C₃-C₈ carbocyclo)-, -(C₃-C₈ carbocyclo)-C₁-C₁₂ alkylene-, -C₅-C₁₄ heterocyclo- wherein said heterocyclo group may be a saturated or unsaturated group having one or more rings and comprising at least one oxygen, nitrogen or sulphur atom in said ring(s), said group optionally being substituted with one or more substituents Rₓ, -C₁₋C₁₂ alkylene-(C₅-C₁₄ heterocyclo)- wherein said heterocyclo group may be a saturated or unsaturated group having one or more rings and comprising at least one oxygen, nitrogen or sulphur atom in said ring(s), said group optionally being substituted with one or more substituents Rₓ, -(C₅-C₁₄ heterocyclo)-C₁-C₁₂ alkylene-, wherein said heterocyclo group may be a saturated or unsaturated group having one or more rings and comprising at least one oxygen, nitrogen or sulphur atom in said ring(s), said group optionally being substituted with one or more substituents Rₓ, -(OCH₂CH₂)ᵣ- and -CH₂-(OCH₂CH₂)ᵣ-, wherein each of the above alkylene substituents whether alone or attached to another moiety in the carbon chain may optionally be substituted by one or more substituents Rₓ;
R₂₀ is a C₁C₁₂ alkyl or an aryl group having from 6 to 18 carbon atoms in one or more aromatic rings, said aryl groups optionally being substituted with one or more substituents Rₓ;
r is an integer ranging from 1-10;
g is an integer of 0 or 1;
b is an integer of 0 or 1;
w is an integer ranging from 0 to 12; and
each of D, Rₓ, X, T, and AA is as defined herein;
wherein for compounds of formula D-(X)_{b}-(AA)_{w}-(T)_{g}-H, b+g+w is not 0.

In preferred embodiments of the present invention, b+g+w is not 0. In further embodiments, b+w is not 0. In yet further embodiments, when w is not 0, then b is 1.

In a further aspect of the present invention, there is provided a compound of formula D-(X)_{b}-(AA)_{w}-(T)_{g}-L₁ or of formula D-(X)_{b}-(AA)_{w}-(T)_{g}-H, or a pharmaceutically acceptable salt or ester thereof; wherein each of D, X, AA, T, L₁, b, g and w are as defined herein; but further wherein if the compound is a compound of formula D-(X)_{b}-(AA)_{w}-(T)_{g}-H then b+w+g≠0.

In a further aspect of the present invention, there is provided a drug moiety D for use as a payload in an antibody drug conjugate. In a further aspect of the present invention, there is provided the use of a drug moiety D as described herein, in the manufacture of an antibody drug conjugate.

In a further aspect of the present invention, there is provided a pharmaceutical composition comprising a drug conjugate according to the present invention and a pharmaceutically acceptable carrier.

In a further aspect of the present invention, there is provided a drug conjugate according to the present invention, for use as a medicament.

In a further aspect of the present invention, there is provided a drug conjugate according to the present invention for use in the treatment of cancer.

In a further aspect of of the present invention, there is provided a kit comprising a therapeutically effective amount of a drug conjugate according to the present invention and a pharmaceutically acceptable carrier. The kit is for use in the treatment of cancer.

A kit according to the present invention may comprise a therapeutically effective amount of a drug conjugate according to the present invention and, optionally, instructions for use of the drug conjugate in the treatment of cancer.

In the above aspects of the present invention, the cancer may be selected from lung cancer, including NSCLC, gastric cancer, colorectal cancer, breast cancer, pancreas carcinoma, endometrial cancer, bladder cancer, cervical cancer, esophageal cancer, gallbladder cancer, uterine cancer, salivary duct cancer, ovarian cancer, kidney cancer, leukaemia, multiple myeloma, and lymphoma. In a preferred embodiment, the cancer is a HER2 positive cancer. Preferred HER2 positive cancers include HER2 positive lung cancer including HER2 positive NSCLC, HER2 positive gastric cancer, HER2 positive colorectal cancer, HER2 positive breast cancer, HER2 positive pancreas carcinoma, HER2 positive endometrial cancer, HER2 positive bladder cancer, HER2 positive cervical cancer, HER2 positive esophageal cancer, HER2 positive gallbladder cancer, HER2 positive uterine cancer, HER2 positive salivary duct cancer and HER2 positive ovarian cancer. More preferred cancers are HER2 positive breast cancer, HER2 positive ovarian cancer and HER2 positive gastric cancer. Most preferred cancer is HER2 positive breast cancer.

In a further aspect of the present invention there is provided a process for the preparation of a drug conjugate according to the present invention comprising conjugating a moiety Ab comprising at least one antigen binding site and a drug D, Ab and D being as defined herein.

### Detailed Description of Preferred Embodiments

The following apply to all aspects of the present invention:
In the compounds of the present invention, the alkyl groups may be branched or unbranched, and preferably have from 1 to about 12 carbon atoms. One more preferred class of alkyl groups has from 1 to about 6 carbon atoms. Even more preferred are alkyl groups having 1, 2, 3 or 4 carbon atoms. Methyl, ethyl, *n*-propyl, isopropyl and butyl, including *n-*butyl, isobutyl, *sec*-butyl and *tert*-butyl are particularly preferred alkyl groups in the compounds of the present invention.

In the compounds of the present invention, the alkenyl groups may be branched or unbranched, have one or more double bonds and from 2 to about 12 carbon atoms. One more preferred class of alkenyl groups has from 2 to about 6 carbon atoms. Even more preferred are alkenyl groups having 2, 3 or 4 carbon atoms. Ethenyl, 1-propenyl, 2-propenyl, 1-methylethenyl, 1-butenyl, 2-butenyl, and 3-butenyl are particularly preferred alkenyl groups in the compounds of the present invention.

In the compounds of the present invention, the alkynyl groups may be branched or unbranched, have one or more triple bonds and from 2 to about 12 carbon atoms. One more preferred class of alkynyl groups has from 2 to about 6 carbon atoms. Even more preferred are alkynyl groups having 2, 3 or 4 carbon atoms.

Suitable aryl groups in the compounds of the present invention include single and multiple ring compounds, including multiple ring compounds that contain separate and/or fused aryl groups. Typical aryl groups contain from 1 to 3 separated and/or fused rings and from 6 to about 18 carbon ring atoms. Preferably aryl groups contain from 6 to about 10 carbon ring atoms. Specially preferred aryl groups included substituted or unsubstituted phenyl, substituted or unsubstituted naphthyl, substituted or unsubstituted biphenyl, substituted or unsubstituted phenanthryl and substituted or unsubstituted anthryl.

Suitable heterocyclic groups include heteroaromatic and heteroalicyclic groups containing from 1 to 3 separated and/or fused rings and from 5 to about 18 ring atoms. Preferably heteroaromatic and heteroalicyclic groups contain from 5 to about 10 ring atoms, most preferably 5, 6, or 7 ring atoms. Suitable heteroaromatic groups in the compounds of the present invention contain one, two or three heteroatoms selected from N, O or S atoms and include, e.g., coumarinyl including 8-coumarinyl, quinolyl including 8-quinolyl, isoquinolyl, pyridyl, pyrazinyl, pyrazolyl, pyrimidinyl, furyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridyl, purinyl, oxadiazolyl, thiadiazolyl, furazanyl, pyridazinyl, triazinyl, cinnolinyl, benzimidazolyl, benzofuranyl, benzofurazanyl, benzothiophenyl, benzothiazolyl, benzoxazolyl, quinazolinyl, quinoxalinyl, naphthyridinyl and furopyridyl. Suitable heteroalicyclic groups in the compounds of the present invention contain one, two or three heteroatoms selected from N, O or S and include, e.g., pyrrolidinyl, tetrahydrofuranyl, tetrahydrothienyl, tetrahydrothiopyranyl, piperidyl, morpholinyl, thiomorpholinyl, thioxanyl, piperazinyl, azetidinyl, oxetanyl, thietanyl, homopiperidyl, oxepanyl, thiepanyl, oxazepinyl, diazepinyl, thiazepinyl, 1,2,3,6-tetrahydropyridyl, 2-pirrolinyl, 3-pyrrolinyl, indolinyl, 2*H*-pyranyl, 4*H*-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexyl, 3-azabicyclo[4.1.0]heptyl, 3*H*-indolyl, and quinolizinyl.The groups above mentioned may be substituted at one or more available positions by one or more suitable groups such as OR', =O, SR', SOR', SO₂R', NO₂, NHR', NR'R', =N-R', NHCOR', N(COR')₂, NHSO₂R', NR'C(=NR')NR'R', CN, halogen, COR', COOR', OCOR', OCONHR', OCONR'R', CONHR', CONR'R', protected OH, protected amino, protected SH, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group, where each of the R' groups is independently selected from the group consisting of hydrogen, OH, NO₂, NH₂, SH, CN, halogen, COH, COalkyl, CO₂H, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group. Where such groups are themselves substituted, the substituents may be chosen from the foregoing list. In addition, where there are more than one R' groups on a substituent, each R' may be the same or different.

In the compounds for the present invention, the halogen substituents include F, C!, Br, and I.

More particularly, in the compounds of the present invention, the alkyl groups in the definitions of R₁, R₂₀, Rₐ, R_{b}, R_{c}, R_{d}, Rₓ, R_{y}, R_{z} and R' may be straight chain or branched alkyl chain groups having from 1 to 12 carbon atoms, and they are preferably an alkyl group having from 1 to 6 carbon atoms, more preferably a methyl group, an ethyl group or an *i*-propyl group, and most preferably a methyl group. In the definitions of M and Q, they may be straight chain or branched alkyl chain groups having from 1 to 6 carbon atoms. Methyl, ethyl, *n*-propyl, isopropyl and butyl, including *n*-butyl, isobutyl, *sec*-butyl and *tert*-butyl are particularly preferred alkyl groups in the compounds of the present invention.

In the compounds of the present invention, the alkenyl groups in the definitions of R₁, Rₐ, R_{b}, R_{c}, R_{d}, Rₓ and R' are branched or unbranched, and may have one or more double bonds and from 2 to 12 carbon atoms. Preferably, they have from 2 to 6 carbon atoms, and more preferably they are branched or unbranched alkenyl groups having 2, 3 or 4 carbon atoms. Ethenyl, 1-propenyl, 2-propenyl, 1-methylethenyl, 1-butenyl, 2-butenyl, and 3-butenyl are particularly preferred alkenyl groups in the compounds of the present invention.

In the compounds of the present invention, the alkynyl group in the definitions of R₁, Rₐ, R_{b}, R_{c}, R_{d}, Rₓ and R' are branched or unbranched, and may have one or more triple bonds and from 2 to 12 carbon atoms. Preferably, they have from 2 to 6 carbon atoms, and more preferably they are branched or unbranched alkynyl groups having 2, 3 or 4 carbon atoms.

In the compounds of the present invention, the halogen substituents in the definitions of Rₓ, R_{y} and R_{z} include F, CI, Br and I, preferably Cl.

In the compounds of the present invention, the heterocyclic group in the definitions of Rₐ, R_{b}, R_{c}, R_{d}, Rₓ and R' is a 5- to 14-membered saturated or unsaturated heterocyclic group having one or more rings, comprising at least one oxygen, nitrogen or sulphur atom in said ring(s). The heterocyclic group is a group which may be a heteroaromatic group or a heteroalicyclic group, the latter of which may be partially unsaturated, both the aromatic and the alicyclic heterocyclic group containing from 1 to 3 separated or fused rings. Preferably the heteroaromatic and heteroalicyclic group contain from 5 to 10 ring atoms. Suitable heteroaromatic groups in the compounds of the present invention contain one, two or three heteroatoms selected from N, O and S atoms and include, for example, quinolyl including 8-quinolyl, isoquinolyl, coumarinyl including 8-coumarinyl, pyridyl, pyrazinyl, pyrazolyl, pyrimidinyl, furyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuranyl, benzofurazanyl, benzothiophenyl, benzothiazolyl, benzoxazolyl, quinazolinyl, quinoxalinyl, naphthyridinyl and furopyridyl. Suitable heteroalicyclic groups in the compounds of the present invention contain one, two or three heteroatoms selected from N, O and S atoms and include, for example, pyrrolidinyl, tetrahydrofuranyl, dihydrofuranyl, tetrahydrothienyl, tetrahydrothiopyranyl, piperidyl, morpholinyl, thiomorpholinyl, thioxanyl, piperazinyl, azetidinyl, oxetanyl, thietanyl, homopiperidyl, oxepanyl, thiepanyl, oxazepinyl, diazepinyl, thiazepinyl, 1,2,3,6-tetrahydropyridyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexyl, 3-azabicyclo[4.1.0]heptyl, 3H-indolyl, and quinolizinyl.

In the compounds of the present invention, the aryl group in the definition of Rₐ, R_{b}, R_{c}, R_{d}, Rₓ, R₂₀, and R' is a single or multiple ring compound that contain separate and/or fused aryl groups and has from 6 to 18 ring atoms and is optionally substituted. Typical aryl groups contain from 1 to 3 separated or fused rings. Preferably aryl groups contain from 6 to 12 carbon ring atoms. Particularly preferred aryl groups include substituted or unsubstituted phenyl, substituted or unsubstituted naphthyl, substituted or unsubstituted biphenyl, substituted or unsubstituted phenanthryl and substituted or unsubstituted anthryl, and most preferred substituted or unsubstituted phenyl, wherein the substituents are as indicated above.

In the compounds of the present invention, the aralkyl groups in the definitions of Rₓ, R_{y} and R_{z} comprise an alkyl group as defined and exemplified above which is substituted with one or more aryl groups as defined and exemplified above. Preferred examples include optionally substituted benzyl, optionally substituted phenylethyl and optionally substituted naphthylmethyl.

In the compounds of the present invention, the aralkyloxy groups in the definitions of Rₓ comprise an alkoxy group having from 1 to 12 carbon atoms which is substituted with one or more aryl groups as defined and exemplified above. Preferably, the alkoxy moiety has from 1 to 6 carbon atoms and the aryl group contains from 6 to about 12 carbon ring atoms, and most preferably the aralkyloxy group is optionally substituted benzyloxy, optionally substituted phenylethoxy and optionally substituted naphthylmethoxy.

In the compounds of the present invention, the heterocycloalkyl groups in the definitions of R_{y} and R_{z} comprise an alkyl group as defined and exemplified above which is substituted with one or more heterocyclic groups as defined and exemplified above. Preferably, the heterocycloalkyl groups comprise an alkyl group having from 1 to 6 carbon atoms which is substituted with a heterocyclic group having from 5 to 10 ring atoms in 1 or 2 ring atoms and can be aromatic, partially saturated or fully saturated. More preferably, the heterocycloalkyl groups comprise a methyl or ethyl group which is substituted with a heterocyclic group selected from the group consisting of pyrrolidinyl, imidazolidinyl, piperidinyl, piperazinyl, morpholinyl, tetrahydrofuranyl, oxanyl, thianyl, 8-quinolyl, isoquinolyl, pyridyl, pyrazinyl, pyrazolyl, pyrimidinyl, furyl, pyrrolyl, thienyl, thiazolyl, isothiazolyl, triazolyl, tetrazolyl, isoxazolyl, oxazolyl and benzimidazole.

In the compounds of the present invention, the alkylene groups in the definition of R₁₉ are straight or branched alkylene groups having from 1 to 12 carbon atoms and the alkylene groups in the definitions of M, X, T, and R₃₀ are straight or branched alkylene groups having from 1 to 6 carbon atoms. Preferably, the alkylene groups in the definition of R₁₉ are straight or branched alkylene groups having from 1 to 8 carbon atoms, more preferably straight or branched alkylene groups having from 1 to 6 carbon atoms. For M, preferred are straight or branched alkylene groups having from 1 to 3 carbon atoms. In the definition of X, the alkylene groups in the definition of X are preferably straight or branched alkylene groups having from 2 to 4 carbon atoms. For T, preferred are straight or branched alkylene groups having from 2 to 4 carbon atoms. In the definition of R₃₀, preferred are straight or branched alkylene groups having from 2 to 4 carbon atoms, being most preferred a straight alkylene group having 3 carbon atoms. For the avoidance of doubt, the term "alkylene" is used to refer to alkanediyl groups.

In the compounds of the present invention, the carbocyclo groups in the definitions of R₁₉ and M are cycloalkyl groups having from 3 to 8 carbon atoms which have two covalent bonds at any position on the cycloalkyl ring connecting said cycloalkyl group to the remainder of the drug conjugate. Preferably, the carbocyclo groups in the definitions of R₁₉ and M are cycloalkyl groups having from 3 to 7 carbon atoms, and more preferably carbocyclo groups having from 5 to 7 carbon atoms.

In the compounds of the present invention, the arylene groups in the definition of R₁₉ are aryl groups having from 6 to 18 carbon atoms in one or more rings which have two covalent bonds at any position on the aromatic ring system connecting said arylene groups to the remainder of the drug conjugate. Preferably, the arylene groups in the definition of R₁₉ are aryl groups having from 6 to 12 carbon atoms in one or more rings which have two covalent bonds at any position on the aromatic ring system, and most preferably they are phenylene groups.

In the compounds of the present invention, the heterocyclo groups in the definition of R₁₉ are heterocyclic groups containing from 1 to 3 separated or fused rings having from 5 to 14 ring atoms and comprising at least one oxygen, nitrogen or sulphur atom in said ring(s), wherein there are two covalent bonds at any position on the ring system of said heterocyclic groups. The heterocyclic groups are groups which may be heteroaromatic groups or heteroalicyclic groups (the latter may be partially unsaturated). Preferably, the heterocyclo groups in the definition of R₁₉ are heterocyclic groups containing from 1 to 3 separated or fused rings having from 5 to 12 ring atoms and comprising at least one oxygen, nitrogen or sulphur atom in said ring(s), wherein there are two covalent bonds at any position on the ring system of said heterocyclic groups.

Where there are more than one group Rₐ, R_{b}, R_{c} or R_{d} in the same drug moiety, each group Rₐ may be the same or different, R_{b} may be the same or different, R_{c} may be the same or different and R_{d} may be the same or different.

Where there are more than one optional substituyents Rₓ, R_{y}, R_{z}, or R' on a substituent, each substituent Rₓ may be the same or different, each substituent R_{y} may be the same or different, each substituent R_{z} may be the same or different and each substituent R' may be the same or different.

In an embodiment, D may be a drug moiety of formula (Ia) or a pharmaceutically acceptable salt or ester thereof: 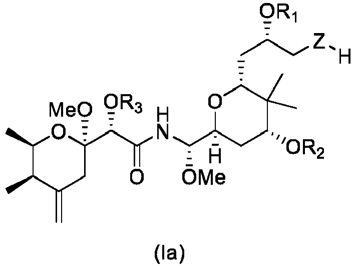 wherein D is covalently attached via a hydroxy group at OR₁, OR₃ or ZH, or via a thiol group at ZH, to (X)_{b} if any, or (AA)_{w} if any, or to (T)_{g} if any, or (L); and R₁, R₂, R₃ and Z are as defined above for formula (I).

In another embodiment, D may be a drug moiety of formula (Ila) or a pharmaceutically acceptable salt or ester thereof: 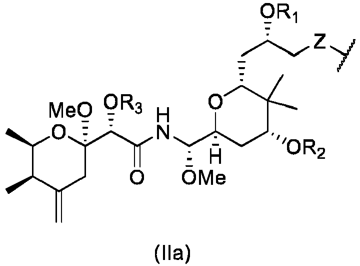 wherein the wavy line indicates the point of covalent attachment to (X)_{b} if any, or (AA)_{w} if any, or to (T)_{g} if any, or to (L); and R₁, R₂, R₃ and Z are as defined above for formula (I) or (II).

Preferred drug moieties are drug moieties of general formula (I), (Ia), (II) and (Ila) wherein:
R₁ is selected from hydrogen and substituted or unsubstituted C₁-C₁₂ alkyl;
and R₂, R₃ and Z are as defined as above.

Further preferred drug moieties are drug moieties of general formula (I), (Ia), (II) and (Ila) wherein:
R₃ is selected from hydrogen and -C(=O)Rₐ, wherein Rₐ is substituted or unsubstituted C₁-C₁₂ alkyl;
and R₁, R₂ and Z are as defined as above.

Preferred drug moieties are drug moieties of general formula (I), (Ia), (II) and (Ila) wherein:
Z is -O-;
and R₁, R₂, and R₃ are as defined as above.

Further preferred drug moieties are drug moieties of general formula (I), (Ia), (II) and (Ila) wherein:
R₁ is selected from hydrogen and substituted or unsubstituted C₁-C₁₂ alkyl;
R₂ is hydrogen;
and R₃ and Z are as defined as above.

Further preferred drug moieties are drug moieties of general formula (I), (Ia), (II) and (Ila) wherein:
R₁ is selected from hydrogen and substituted or unsubstituted C₁-C₁₂ alkyl;
R₃ is selected from hydrogen and -C(=O)Rₐ, wherein Rₐ is substituted or unsubstituted C₁-C₁₂ alkyl;
and R₂ and Z are as defined as above.

Further preferred drug moieties are drug moieties of general formula (I), (Ia), (II) and (Ila) wherein:
R₂ is hydrogen;
R₃ is selected from hydrogen and -C(=O)Rₐ, wherein Rₐ is substituted or unsubstituted C₁-C₁₂ alkyl;
and R₁ and Z are as defined as above.

Further preferred drug moieties are drug moieties of general formula (I), (Ia), (II) and (Ila) wherein:
R₁ is selected from hydrogen and substituted or unsubstituted C₁-C₁₂ alkyl;
R₂ is hydrogen;
R₃ is selected from hydrogen and -C(=O)Rₐ, wherein Rₐ is substituted or unsubstituted C₁-C₁₂ alkyl; and
Z is as defined as above.

Further preferred drug moieties are drug moieties of general formula (I), (Ia), (II) and (Ila) wherein:
R₁ is selected from hydrogen and substituted or unsubstituted C₁-C₁₂ alkyl;
R₂ is hydrogen;
R₃ is selected from hydrogen and -C(=O)Rₐ, wherein Rₐ is substituted or unsubstituted C₁-C₁₂ alkyl; and
Z is -O-.

The following preferred substituents apply to drug moieties of formula (I), (Ia), (II) and (IIa):
Particularly preferred R₁ is hydrogen and substituted or unsubstituted C₁-C₆ alkyl; more preferably, R₁ is selected from hydrogen, methyl, ethyl, *n*-propyl, isopropyl, *n*-butyl, *tert-*butyl, *sec*-butyl, and isobutyl. Most preferred R₁ are hydrogen and methyl.

Particularly preferred R₃ is hydrogen and -C(=O)Rₐ wherein Rₐ is substituted or unsubstituted C₁-C₆ alkyl; more preferably Rₐ is selected from methyl, ethyl, *n*-propyl, isopropyl, *n*-butyl, *tert*-butyl, *sec*-butyl, and isobutyl. Most preferred R₃ is hydrogen.

Particularly preferred Z is -O-.

Preferred drug moieties in the compounds according to the present invention include:
- Drug moieties of general formula (I), (Ia), (II) and (Ila) wherein:
   R₁ is selected from hydrogen and substituted or unsubstituted C₁-C₆ alkyl;
   and R₂, R₃ and Z are as defined as above.
- Drug moieties of general formula (I), (Ia), (II) and (Ila) wherein:
   R₃ is selected from hydrogen and a -C(=O)Rₐ, wherein Rₐ is substituted or
   unsubstituted C₁-C₆ alkyl;
   and R₁, R₂ and Z are as defined as above.
- Drug moieties of general formula (I), (Ia), (II) and (Ila) wherein:
   Z is -O-;
   and R₁, R₂ and R₃ are as defined as above.
- Drug moieties of general formula (I), (Ia), (II) and (Ila) wherein:
   R₁ is selected from hydrogen and substituted or unsubstituted C₁-C₆ alkyl;
   R₃ is selected from hydrogen and -C(=O)Rₐ, wherein Rₐ is substituted or
   unsubstituted C₁-C₆ alkyl;
   and R₂ and Z are as defined as above.
- Drug moieties of general formula (I), (Ia), (II) and (Ila) wherein:
   R₁ is selected from hydrogen and substituted or unsubstituted C₁-C₆ alkyl;
   R₂ is hydrogen;
   R₃ is selected from hydrogen and -C(=O)Rₐ, wherein Rₐ is substituted or unsubstituted C₁-C₆ alkyl; and
   Z is -O-.

Preferred drug moieties in the compounds according to the present invention include:
- Drug moieties of general formula (I), (Ia), (II) and (Ila) wherein:
   R₁ is selected from hydrogen and methyl;
   and R₂, R₃ and Z are as defined as above.
- Drug moieties of general formula (I), (Ia), (II) and (Ila) wherein:
   R₃ is hydrogen;
   and R₁, R₂ and Z are as defined as above.
- Drug moieties of general formula (I), (Ia), (II) and (Ila) wherein:
   Z is -O-;
   and R₁, R₂ and R₃ are as defined as above.

Further drug moieties in the compounds according to the present invention include:
- Drug moieties of general formula (I), (Ia), (II) and (Ila) wherein:
   R₁ is selected from hydrogen and methyl;
   R₃ is hydrogen;
   and R₂ and Z are as defined as above.
- Drug moieties of general formula (I), (Ia), (II) and (Ila) wherein:
   R₁ is selected from hydrogen and methyl;
   R₂ is hydrogen;
   R₃ is hydrogen; and
   Z is -O-.

Even more preferred drug moieties in the compounds according to the present invention are selected from: 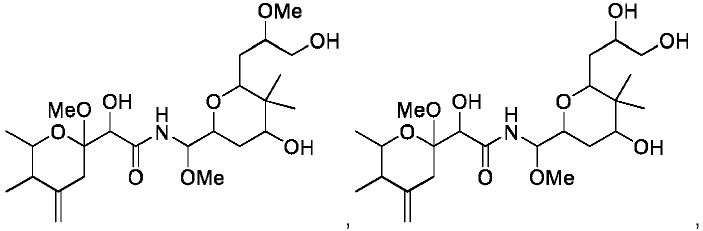 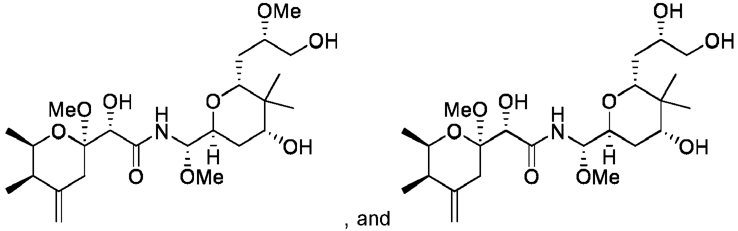 wherein D is covalently attached via a hydroxy group at OR₁, OR₃ or ZH to (X)_{b} if any, or (AA)_{w} if any, or to (T)_{g} if any, or (L).

Most preferred drug moieties in the compounds according to the present invention are selected from: 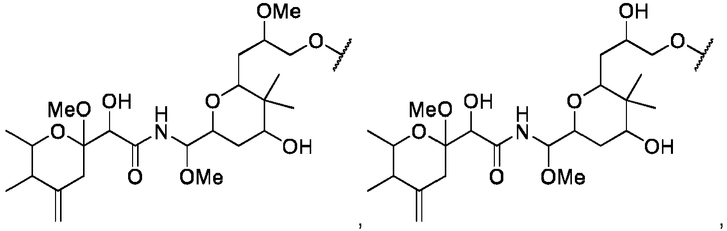 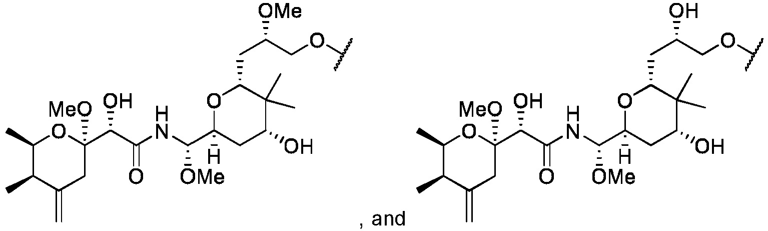 wherein the wavy line represent the point of covalent attachment to (X)_{b} if any, or (AA)_{w} if any, or to (T)_{g} if any, or to (L).

In additional preferred embodiments, the preferences described above for the different substituents are combined. The present invention is also directed to such combinations of preferred substitutions (where allowed by possible substituent groups) in compounds having drug moieties of formula (I), (Ia), (II) or (Ila) according to the present invention.

For the avoidance of doubt, the drug moieties of formula (I) and (Ia) are covalently attached via a hydroxy group at OR₁, OR₃ or ZH, or via a thiol group at ZH, to (X)_{b} if any, or (AA)_{w} if any, or to (T)_{g} if any, or (L). Thus, when conjugated, a covalent bond replaces a proton on a hydroxy group or on a thiol group on the compound.

Preferred drug conjugates according to the the present invention are given below. The preferred definitions of (X)_{b}, (AA)_{w}, (T)_{g}, and (L) as set out below are applicable to all of the drug moiety D compounds described above. Preferred drug conjugates according to the the present invention include:
- a drug conjugate of formula [D-(X)_{b}-(AA)_{w}-(T)_{g}-(L)-]ₙ-Ab according to the present invention wherein L is a linker group selected from the group consisting of: 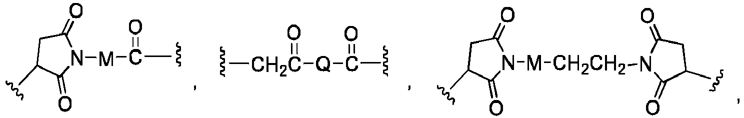 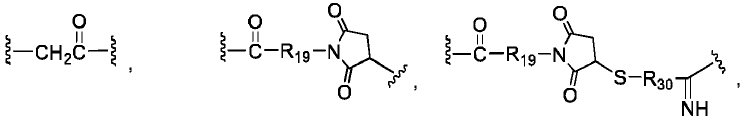 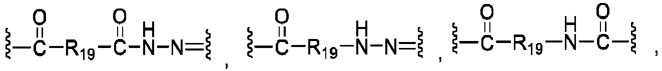 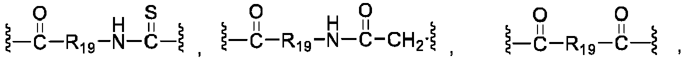 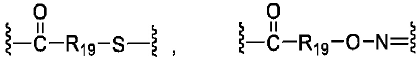 wherein
   the wavy lines indicate the point of covalent attachments to an Ab (the wavy line to the right) and to (T)_{g} if any, or (AA)_{w} if any, or to (X)_{b} if any, or to D (the wavy line to the left);
   R₁₉ is selected from -C₁₋C₁₂ alkylene-, -C₃-C₈ carbocyclo-, -O-(C₁-C₁₂ alkylene)-, - C₆-C₁₈ arylene- in one or more rings which may optionally be substituted with one or more substituents Rₓ, -C₁₋C₁₂ alkylene-C₆-C₁₈ arylene- wherein the arylene group is in one or more rings which may optionally be substituted with one or more substituents Rₓ, -C₆-C₁₈ arylene-C₁₋C₁₂ alkylene- wherein the arylene group is in one or more rings which may optionally be substituted with one or more substituents Rₓ, -C₁₋C₁₂ alkylene-(C₃-C₈ carbocyclo)-, -(C₃-C₈ carbocyclo)-C₁-C₁₂ alkylene-, -C₅-C₁₄ heterocyclo- wherein said heterocyclo group may be a saturated or unsaturated group having one or more rings and comprising at least one oxygen, nitrogen or sulphur atom in said ring(s), said group optionally being substituted with one or more substituents Rₓ, -C₁₋C₁₂ alkylene-(C₅-C₁₄ heterocyclo)- wherein said heterocyclo group may be a saturated or unsaturated group having one or more rings and comprising at least one oxygen, nitrogen or sulphur atom in said ring(s), said group optionally being substituted with one or more substituents Rₓ, -(C₅-C₁₄ heterocyclo)-C₁-C₁₂ alkylene- wherein said heterocyclo group may be a saturated or unsaturated group having one or more rings and comprising at least one oxygen, nitrogen or sulphur atom in said ring(s), said group optionally being substituted with one or more substituents Rₓ, - (OCH₂CH₂)ᵣ-, and -CH₂-(OCH₂CH₂)ᵣ-, wherein each of the above alkylene substituents whether alone or attached to another moiety in the carbon chain may optionally be substituted by one or more substituents Rₓ;
   R₃₀ is a -C₁₋C₆ alkylene- group;
   M is selected from the group consisting of -C₁₋C₆ alkylene-, -C₁₋C₆ alkylene-(C₃-C₈ carbocyclo)-, -(CH₂CH₂O)ₛ-, -C₁₋C₆ alkylene-(C₃-C₈ carbocyclo)-CON(H or C₁-C₆ alkyl)-C₁₋C₆ alkylene-, -phenylene- which may optionally be substituted with one or more substituents Rₓ, -phenylene-C₁-C₆ alkylene- wherein the phenylene moiety may optionally be substituted with one or more substituents Rₓ and -C₁₋C₆ alkylene-CON(H or C₁-C₆ alkyl)C₁-C₆ alkylene-;
   Q is selected from the group consisting of -N(H or C₁-C₆ alkyl)phenylene- and -N(H or C₁-C₆ alkyl)-(CH₂)ₛ;
   r is an integer ranging from 1 to 10; and
   s is an integer ranging from 1 to 10.
- a drug conjugate of formula [D-(X)_{b}-(AA)_{w}-(T)_{g}-(L)-]ₙ-Ab according to the present invention wherein L is selected from the group consisting of: 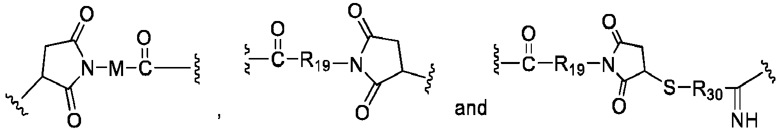 wherein:
   the wavy lines indicate the point of covalent attachments to an Ab (the wavy line to the right) and to (T)_{g} if any, or (AA)_{w} if any, or (X)_{b} if any, or to D (the wavy line to the left);
   R₁₉ is selected from -C₁₋C₁₂ alkylene-, -O-(C₁-C₁₂ alkylene)-, -C₆-C₁₂ arylene- in one or more rings which may optionally be substituted with one or more substituents Rₓ, -C₁₋C₁₂ alkylene-C₆-C₁₂ arylene- wherein the arylene group is in one or more rings which may optionally be substituted with one or more substituents Rₓ, -C₆-C₁₂ arylene-C₁₋C₁₂ alkylene- wherein the arylene group is in one or more rings which may optionally be substituted with one or more substituents Rₓ, -C₅-C₁₂ heterocyclo- wherein said heterocyclo group may be a saturated or unsaturated group having one or more rings and comprising at least one oxygen, nitrogen or sulphur atom in said ring(s), said group optionally being substituted with one or more substituents Rₓ, -C₁₋C₁₂ alkylene-(C₅-C₁₂ heterocyclo)- wherein said heterocyclo group may be a saturated or unsaturated group having one or more rings and comprising at least one oxygen, nitrogen or sulphur atom in said ring(s), said group optionally being substituted with one or more substituents Rₓ, -(C₅-C₁₂ heterocyclo)-C₁-C₁₂ alkylene- wherein said heterocyclo group may be a saturated or unsaturated group having one or more rings and comprising at least one oxygen, nitrogen or sulphur atom in said ring(s), said group optionally being substituted with one or more substituents Rₓ, - (OCH₂CH₂)ᵣ-, and -CH₂-(OCH₂CH₂)ᵣ- wherein each of the above alkylene substituents whether alone or attached to another moiety in the carbon chain may optionally be substituted by one or more substituents Rₓ;
   R₃₀ is a -C₁₋C₆ alkylene- group;
   M is selected from the group consisting of -C₁₋C₆ alkylene-, -C₁₋C₆ alkylene-(C₃-C₈ carbocyclo)- and -phenylene- which may optionally be substituted with one or more substituents Rₓ; and
   r is an integer ranging from 1-6.
- a drug conjugate of formula [D-(X)_{b}-(AA)_{w}-(T)_{g}-(L)-]ₙ-Ab according to the present invention selected from formulas (V), (VI) and (VII): 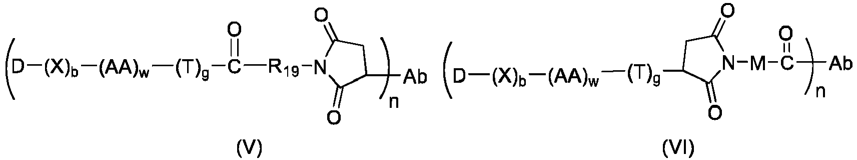 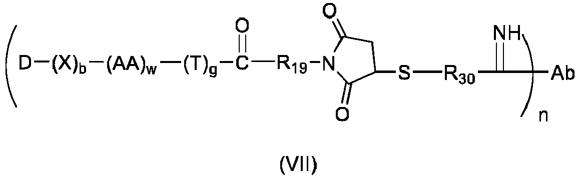 wherein:
   X and T are extending groups that may be the same or different;
   each AA is independently an amino acid unit as defined herein;
   w is an integer ranging from 0 to 12;
   b is an integer of 0 or 1;
   g is an integer of 0 or 1;
   where b+g+w is optionally not 0;
   D is a drug moiety;
   Ab is a moiety comprising at least one antigen binding site;
   n is the ratio of the group [D-(X)_{b}-(AA)_{w}-(T)_{g}-(L)-] wherein L is as defined in formula (V), (VI) or (VII) to the moiety comprising at least one antigen binding site and is in the range from 1 to 20;
   R₁₉ is selected from -C₁-C₈ alkylene-, -O-(C₁-C₈ alkylene)-, -C₁-C₈ alkylene-C₆-C₁₂ arylene- wherein the arylene group is in one or more rings which may optionally be substituted with one or more substituents Rₓ, and -C₆-C₁₂ arylene-C₁₋C₈ alkylene-wherein the arylene group is in one or more rings which may optionally be substituted with one or more substituents Rₓ, wherein each of the above alkylene substituents whether alone or attached to another moiety in the carbon chain may optionally be substituted by one or more substituents Rₓ;
   R₃₀ is a -C₂-C₄ alkylene- group; and
   M is selected from the group consisting of -C₁₋C₃ alkylene- and -C₁₋C₃ alkylene-(C₅-C₇ carbocyclo)-.
- a drug conjugate of formula [D-(X)_{b}-(AA)_{w}-(T)_{g}-(L)-]ₙ-Ab according to the present invention, selected from formulas (V), (VI) and (VII): 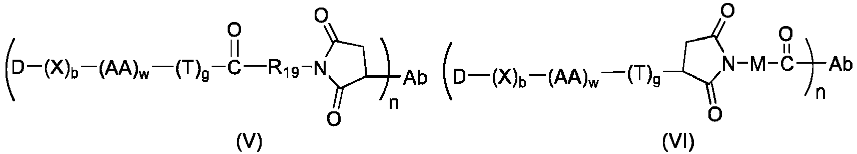 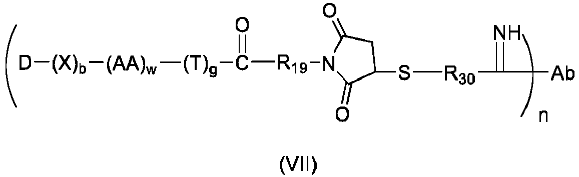 wherein:
   X and T are extending groups that may be the same or different;
   each AA is independently an amino acid unit;
   w is an integer ranging from 0 to 12;
   b is an integer of 0 or 1;
   g is an integer of 0 or 1;
   where b+g+w is optionally not 0;
   D is a drug moiety;
   Ab is a moiety comprising at least one antigen binding site;
   n is the ratio of the group [D-(X)_{b}-(AA)_{w}-(T)_{g}-(L)-] wherein L is as defined in formulas (V), (VI) or (VII) to the moiety comprising at least one antigen binding site and is in the range from 1 to 20;
   R₁₉ is selected from -C₁₋C₆ alkylene-, -phenylene-C₁-C₆ alkylene- wherein the phenylene group may optionally be substituted with one or more substituents Rₓ selected from the group consisting of alkyl groups having from 1 to 6 carbon atoms, alkoxy groups having from 1 to 6 carbon atoms, halogen atoms, nitro groups and cyano groups, wherein each of the above alkylene substituents whether alone or attached to another moiety in the carbon chain may optionally be substituted by one or more substituents Rₓ selected from the group consisting of alkyl groups having from 1 to 6 carbon atoms, alkoxy groups having from 1 to 6 carbon atoms, aryl groups having from 6 to 12 carbon atoms, halogen atoms, nitro groups and cyano groups, and preferably R₁₉ is a -C₁₋C₆ alkylene- group;
   R₃₀ is a -C₂-C₄ alkylene- group; and
   M is -C₁₋C₃ alkylene-(C₅-C₇ carbocyclo)-.
- It is preferred that in the definition of the drug conjugate of formula [D-(X)_{b}-(AA)_{w}-(T)_{g}-(L)-]ₙ-Ab, L is as defined in the preferred definitions for said group above and (AA)_{w} is of formula (III): 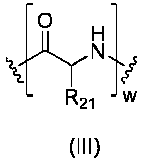
   wherein the wavy lines indicate the point of covalent attachments to (X)_{b} if any, or to the drug moiety (the wavy line to the left) and to (T)_{g} if any, or to the linker (the wavy line to the right); and
   R₂₁ is, at each occurrence, selected from the group consisting of hydrogen, methyl, isopropyl, isobutyl, *sec*-butyl, benzyl, *p*-hydroxybenzyl, -CH₂OH, - CH(OH)CH₃, -CH₂CH₂SCH₃, -CH₂CONH₂, -CH₂COOH, -CH₂CH₂CONH₂, - CH₂CH₂COOH, -(CH₂)₃NHC(=NH)NH₂, -(CH₂)₃NH₂, -(CH₂)₃NHCOCH₃, - (CH₂)₃NHCHO, -(CH₂)₄NHC(=NH)NH₂, -(CH₂)₄NH₂, -(CH₂)₄NHCOCH₃, - (CH₂)₄NHCHO, -(CH₂)₃NHCONH₂, -(CH₂)₄NHCONH₂, -CH₂CH₂CH(OH)CH₂NH₂, 2-pyridylmethyl-, 3-pyridylmethyl-, 4-pyridylmethyl-, phenyl, cyclohexyl, 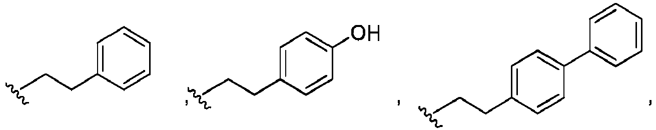 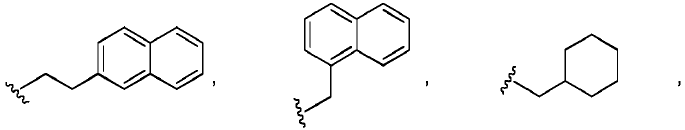 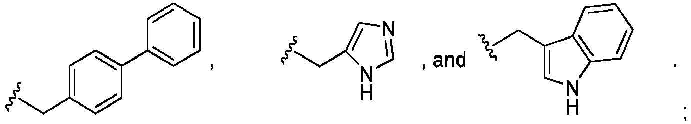
   and w is an integer ranging from 0 to 12.
- a drug conjugate of formula [D-(X)_{b}-(AA)_{w}-(T)_{g}-(L)-]ₙ-Ab according to the first aspect of the present invention, wherein L is as defined in the preferred definitions for said group above and (AA)_{w} is of formula (III) wherein:
   R₂₁ is selected, at each occurrence, from the group consisting of hydrogen, methyl, isopropyl, sec-butyl, benzyl, indolylmethyl, -(CH₂)₃NHCONH₂, -(CH₂)₄NH₂, -(CH₂)₃NHC(=NH)NH₂ and -(CH₂)₄NHC(=NH)NH₂; and
   w is an integer ranging from 0 to 6.
- a drug conjugate of formula [D-(X)_{b}-(AA)_{w}-(T)_{g}-(L)-]ₙ-Ab according to the first aspect of the present invention, wherein L is as defined in the preferred definitions for said group above, wherein w is 0 or 2, and when w is 2, then (AA)_{w} is of formula (IV) wherein: 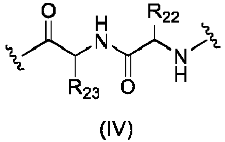
   the wavy lines indicate the point of covalent attachments to (X)_{b} if any, or to the drug moiety (the wavy line to the left) and to (T)_{g} if any, or to the linker (the wavy line to the right);
   R₂₂ is selected from methyl, benzyl, isopropyl, sec-butyl and indolylmethyl; and
   R₂₃ is selected from methyl, -(CH₂)₄NH₂, -(CH₂)₃NHCONH₂ and - (CH₂)₃NHC(=NH)NH₂.
- In embodiments of the present invention b+g+w is not 0. In further embodiments, b+w is not 0. In yet further embodiments, when w is not 0, then b is 1.Further, it is preferred that in the definition of the drug conjugate of formula [D-(X)_{b}-(AA)_{w}-(T)_{g}-(L)-]ₙ-Ab, L and (AA)_{w} are as defined in the preferred definitions for said groups above and X is an extending group selected from:
   -CONH-(C₁-C₆ alkylene)NH-;
   -COO-CH₂-(phenylene which may optionally be substituted with one or more substituents Rₓ)-NH-;
   -CONH-(C₁-C₆ alkylene)NH-COO-CH₂-(phenylene which may optionally be substituted with one or more substituents Rₓ)-NH-;
   -COCH₂NH-COCH₂-NH-;
   -COCH₂NH-;
   -CONH-(C₁-C₆ alkylene)S-;
   -CONH-(C₁-C₆ alkylene)NHCO(C₁-C₆, alkylene)S-; and
   b is 0 or 1, preferably 1.
- a drug conjugate of formula [D-(X)_{b}-(AA)_{w}-(T)_{g}-(L)-]ₙ-Ab according to the present invention, wherein L and (AA)_{w} are as defined in the preferred definitions for said groups above and X is an extending group selected from the group consisting of:
   -CONH-(C₂-C₄ alkylene)NH-;
   -COO-CH₂-phenylene-NH-, wherein said phenylene group may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups;
   -CONH-(C₂-C₄ alkylene)NH-COO-CH₂-(phenylene which may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups)-NH-;
   -COCH₂NH-COCH₂-NH-;
   -CONH-(C₂-C₄ alkylene)S-;
   -CONH-(C₂-C₄ alkylene)NHCO(C₁-C₃ alkylene)S-; and
   b is 0 or 1, preferably 1.
- a drug conjugate of formula [D-(X)_{b}-(AA)_{w}-(T)_{g}-(L)-]ₙ-Ab according to the present invention, wherein L and (AA)_{w} are as defined in the preferred definitions for said groups above and X is an extending group selected from the group consisting of:
   - COO-CH₂-phenylene-NH-;
   - CONH(CH₂)₃NHCOOCH₂-phenylene-NH-;
   - CONH(CH₂)₃NH-;
   - CONH(CH₂)₃-S-;
   - CONH(CH₂)₃NHCO(CH₂)₂S-; and
      b is 0 or 1, preferably 1.
- a drug conjugate of formula [D-(X)_{b}-(AA)_{w}-(T)_{g}-(L)-]ₙ-Ab according to the present invention, wherein L, (AA)_{w}, and (X)_{b} are as defined in the preferred definitions for said groups above and T is an extending group selected from the group consisting of:
   -CO-(C₁-C₆ alkylene)-NH-;
   -CO-(C₁-C₆ alkylene)-[O-(C₂-C₆ alkylene)]ⱼ-NH-;
   -COO-(C₁-C₆ alkylene)-[O-(C₂-C₆ alkylene)]ⱼ-NH-;
   where j is an integer from 1 to 25, and
      g is 0 or 1, preferably 0.
- A drug conjugate of formula [D-(X)_{b}-(AA)_{w}-(T)_{g}-(L)-]ₙ-Ab according to the present invention, wherein L, (AA)_{w}, and (X)_{b} are as defined in the preferred definitions for said groups above and T is an extending group selected from the group consisting of:
   -CO-(C₁-C₄ alkylene)NH-;
   -CO-(C₁-C₄ alkylene)-[O-(C₂-C₄ alkylene)]ⱼ-NH-;
   -COO-(C₁-C₄ alkylene)-[O-(C₂-C₄ alkylene)]ⱼ-NH-;
   where j is an integer from 1 to 10; and
      g is 0 or 1, preferably 0.
- A drug conjugate of formula [D-(X)_{b}-(AA)_{w}-(T)_{g}-(L)-]ₙ-Ab according to the present invention, wherein L, (AA)_{w}, and (X)_{b} are as defined in the preferred definitions for said groups above and T is an extending group selected from the group consisting of:
   -CO-(C₁-C₄ alkylene)NH-;
   -CO-(C₁-C₄ alkylene)-[O-(C₂-C₄ alkylene)]ⱼ-NH-;
   -COO-(C₁-C₄ alkylene)-[O-(C₂-C₄ alkylene)]ⱼ-NH-;
   where j is an integer from 1 to 5; and
      g is 0 or 1, preferably 0.
- A preferred drug conjugate of formula [D-(X)_{b}-(AA)_{w}-(T)_{g}-(L)-]ₙ-Ab according to the present invention is one wherein L, (AA)_{w}, (X)_{b}, and (T)_{g} are as defined above and wherein D is a compound of formula (I), (Ia), (II) or (Ila) wherein R₁ is hydrogen or a substituted or unsubstituted C₁-C₆ alkyl; wherein the optional substituents are one or more substituents Rₓ and more preferably R₁ is hydrogen or methyl.
- Another preferred drug conjugate of formula [D-(X)_{b}-(AA)_{w}-(T)_{g}-(L)-]ₙ-Ab according to the present invention is one wherein L, (AA)_{w}, (X)_{b}, and (T)_{g} are as defined above and wherein D is a compound of formula (I), (Ia), (II) or (Ila) wherein R₃ is hydrogen or - C(=O)Rₐ, wherein Rₐ is substituted or unsubstituted C₁-C₆ alkyl, wherein the optional substituents are one or more substituents Rₓ, and more preferably R₃ is hydrogen.
- Another preferred drug conjugate of formula [D-(X)_{b}-(AA)_{w}-(T)_{g}-(L)-]ₙ-Ab according to the present invention is one wherein L, (AA)_{w}, (X)_{b}, and (T)_{g} are as defined above and wherein D is a compound of formula (I), (Ia), (II) or (Ila) wherein Z is -O-.
- A further preferred drug conjugate of formula [D-(X)_{b}-(AA)_{w}-(T)_{g}-(L)-]ₙ-Ab according to the present invention is one wherein L, (AA)_{w}, (X)_{b}, and (T)_{g} are as defined above and wherein D is a compound of formula (I), (Ia), (II) or (Ila), or a pharmaceutically acceptable salt or ester thereof, wherein:
   R₁ is hydrogen or substituted or unsubstituted C₁-C₆ alkyl, wherein the optional substituents are one or more substituents Rₓ;
   R₂ is hydrogen;
   R₃ is hydrogen or -C(=O)Rₐ, wherein Rₐ is substituted or unsubstituted C₁-C₆ alkyl, wherein the optional substituents are one or more substituents Rₓ; and Z is -O-.
- A further preferred drug conjugate of formula [D-(X)_{b}-(AA)_{w}-(T)_{g}-(L)-]ₙ-Ab according to the present invention is one wherein L, (AA)_{w}, (X)_{b}, and (T)_{g} are as defined above and wherein D is a compound of formula (I), (Ia), (II) or (IIa), or a pharmaceutically acceptable salt or ester thereof, wherein:
   R₁ is hydrogen or methyl;
   R₂ is hydrogen;
   R₃ is hydrogen; and
   Z is -O-.
- A further preferred drug conjugate of formula [D-(X)_{b}-(AA)_{w}-(T)_{g}-(L)-]ₙ-Ab according to the present invention is one wherein L, (AA)_{w}, (X)_{b}, and (T)_{g} are as defined above and wherein D is a compound of formula (I), (Ia), (II) or (IIa), or a pharmaceutically acceptable salt or ester thereof, wherein:
   R₁ is methyl;
   R₂ is hydrogen;
   R₃ is hydrogen; and
   Z is -O-.
- A further preferred drug conjugate of formula [D-(X)_{b}-(AA)_{w}-(T)_{g}-(L)-]ₙ-Ab according to the present invention is one wherein L, (AA)_{w}, (X)_{b}, and (T)_{g} are as defined above and wherein D is selected from: 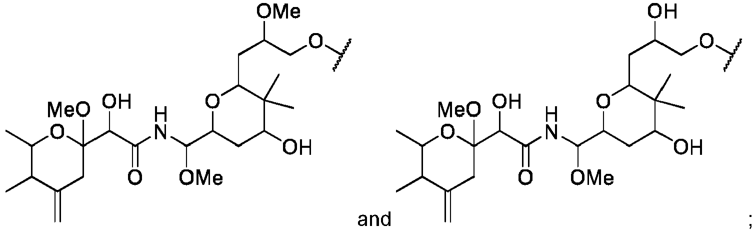 or a pharmaceutically acceptable salt or ester thereof; wherein the wavy lines indicate the point of covalent attachment to (X)_{b} if any, or (AA)_{w} if any, or to (T)_{g} if any or to (L).
- A further preferred drug conjugate of formula [D-(X)_{b}-(AA)_{w}-(T)_{g}-(L)-]ₙ-Ab according to the present invention is one wherein L, (AA)_{w}, (X)_{b}, and (T)_{g} are as defined above and wherein D is selected from: 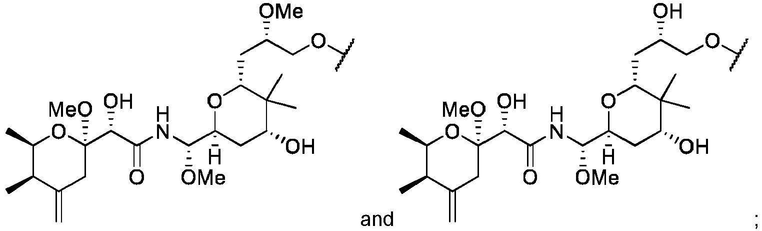 or a pharmaceutically acceptable salt or ester thereof; wherein the wavy lines indicate the point of covalent attachment to (X)_{b} if any, or (AA)_{w} if any, or to (T)_{g} if any or to (L).
- A further preferred drug conjugate of formula [D-(X)_{b}-(AA)_{w}-(T)_{g}-(L)-]ₙ-Ab according to the present invention is one wherein L, (AA)_{w}, (X)_{b}, (T)_{g} and D are as defined above and wherein the moiety Ab comprising at least one antigen binding site is an antigen-binding peptide.
- A further preferred drug conjugate of formula [D-(X)_{b}-(AA)_{w}-(T)_{g}-(L)-]ₙ-Ab according to the present invention is one wherein L, (AA)_{w}, (X)_{b}, (T)_{g} and D are as defined above and the moiety Ab comprising at least one antigen binding site is an antibody, a single domain antibody or an antigen-binding fragment thereof.
- A further preferred drug conjugate of formula [D-(X)_{b}-(AA)_{w}-(T)_{g}-(L)-]ₙ-Ab according to the present invention is one wherein L, (AA)_{w}, (X)_{b}, (T)_{g} and D are as defined above and the moiety Ab comprising at least one antigen binding site is a monoclonal, polyclonal antibody or bispecific antibody and wherein the antibody or antigen-binding fragment thereof is derived from any species, preferably a human, mouse or rabbit.
- A further preferred drug conjugate of formula [D-(X)_{b}-(AA)_{w}-(T)_{g}-(L)-]ₙ-Ab according to the present invention is one wherein L, (AA)_{w}, (X)_{b}, (T)_{g} and D are as defined above and the moiety Ab comprising at least one antigen binding site is an antibody or antigen-binding fragment thereof which is selected from the group consisting of a human antibody, an antigen-binding fragment of a human antibody, a humanized antibody, an antigen-binding fragment of a humanized antibody, a chimeric antibody, an antigen-binding fragment of a chimeric antibody, a glycosylated antibody and a glycosylated antigen binding fragment.
- A further preferred drug conjugate of formula [D-(X)_{b}-(AA)_{w}-(T)_{g}-(L)-]ₙ-Ab according to the present invention is one wherein L, (AA)_{w}, (X)_{b}, (T)_{g} and D are as defined above and the moiety Ab comprising at least one antigen binding site is an antibody or antigen-binding fragment thereof, wherein the antibody or antigen-binding fragment thereof is an antigen-binding fragment selected from the group consisting of an Fab fragment, an Fab' fragment, an F(ab')2 fragment and an Fv fragment.
- A further preferred drug conjugate of formula [D-(X)_{b}-(AA)_{w}-(T)_{g}-(L)-]ₙ-Ab according to the present invention is one wherein L, (AA)_{w}, (X)_{b}, (T)_{g} and D are as defined above and the moiety Ab comprising at least one antigen binding site is an antibody or antigen-binding fragment thereof, wherein the antibody or antigen-binding fragment thereof is a monoclonal antibody which immunospecifically binds to cancer cell antigens, viral antigens, antigens of cells that produce autoimmune antibodies associated with autoimmune disease, microbial antigens, and preferably a monoclonal antibody which immunospecifically binds to cancer cell antigens, as defined herein.
- A further preferred drug conjugated of formula [D-(X)_{b}-(AA)_{w}-(T)_{g}-(L)-]ₙ-Ab according to the the present invention is one wherein L, (AA)_{w}, (X)_{b}, (T)_{g} and D are as defined herein and the moiety Ab comprising at least one antigen binding site is an anti-HER2 antibody.
- A further preferred drug conjugate of formula [D-(X)_{b}-(AA)_{w}-(T)_{g}-(L)-]ₙ-Ab according to the the present invention is one wherein L, (AA)_{w}, (X)_{b}, (T)_{g} and D are as defined herein and the moiety Ab comprising at least one antigen binding site is an antibody selected from the group consisting of Abciximab, Alemtuzumab, Anetumab, Atezolizumab, Avelumab, Basiliximab, Bevacizumab, Blinatomumab, Brentuximab, Catumaxomab, Cetuximab, Coltuximab, Daclizumab, Daratumumab, Denintuzumab, Denosumab, Depatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labetuzumab, Ladiratuzumab, Laprituximab, Lifastuzumab, Lorvotuzumab, Milatuzumab, Mirvetuximab, Naratuximab, Necitumumab, Nimotuzumab, Nivolumab, Obinutuzumab, Ofatumumab, Olaratumab, Omalizumab, Palivizumab, Panitumumab, Pembrolizumab, Pertuzumab, Pinatuzumab, Polatuzumab, Ramucirumab, Rovalpituzumab, Sacituzumab, Siltuximab, Sirtratumab, Sofituzumab, Vadastuximab, Vorsetuzumab, an anti-HER2 antobody such as Trastuzumab, an anti-CD4 antibody, an anti-CD5 antibody, and an anti-CD30 antibody, or an antigen-binding fragment or an immunologicallly active portion thereof, wherein preferably the antibody is selected from Abciximab, Alemtuzumab, Anetumab, Atezolizumab, Avelumab, Basiliximab, Bevacizumab, Blinatomumab, Brentuximab, Catumaxomab, Cetuximab, Daclizumab, Daratumumab, Denintuzumab, Denosumab, Depatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labetuzumab, Ladiratuzumab, Laprituximab, Mirvetuximab, Naratuximab, Necitumumab, Nimotuzumab, Nivolumab, Obinutuzumab, Ofatumumab, Olaratumab, Omalizumab, Palivizumab, Panitumumab, Pembrolizumab, Pertuzumab, Polatuzumab, Ramucirumab, Rovalpituzumab, Sacituzumab, Siltuximab, Sirtratumab, Vadastuximab, Vorsetuzumab, an anti-HER2 antibody such as Trastuzumab, an anti-CD4 antibody, an anti-CD5 antibody, and an anti-CD30 antibody, or an antigen-binding fragment or an immunologicallly active portion thereof, and yet more preferably Abciximab, Alemtuzumab, Atezolizumab, Avelumab, Basiliximab, Bevacizumab, Blinatomumab, Brentuximab, Catumaxomab, Cetuximab, Daclizumab, Daratumumab, Denosumab, Dinutuximab, Durvalumab, Elotuzumab, Gemtuzumab, Ibritumomab, Inotuzumab, Ipilimumab, Labetuzumab, Necitumumab, Nimotuzumab, Nivolumab, Obinutuzumab, Ofatumumab, Olaratumab, Omalizumab, Palivizumab, Panitumumab, Pembrolizumab, Pertuzumab, Ramucirumab, Rovalpituzumab, Siltuximab, an anti-HER2 antibody such as Trastuzumab, an anti-CD4 antibody, an anti-CD5 antibody, and an anti-CD30 antibody, or an antigen-binding fragment or an immunologically active portion thereof. Of these, particularly preferred are Brentuximab, Gemtuzumab, Inozutumab, Rovalpituzumab, an anti-HER2 antibody such as Trastuzumab, an anti-CD4 antibody, an anti-CD5 antibody, and an anti-CD30 antibody, or an antigen-binding fragment or an immunologicallly active portion thereof; or the antibody is an anti-HER2 antibody such as Trastuzumab or an antigen-binding fragment or an immunologically active portion thereof, particularly Trastuzumab or an antigen-binding fragment or an immunologicallly active portion thereof.
- Particularly preferred drug conjugates of formula [D-(X)_{b}-(AA)_{w}-(T)_{g}-(L)-]ₙ-Ab according to the present invention include the following:
   (a) a drug conjugate according to the present invention wherein:
      L is selected from the group consisting of: 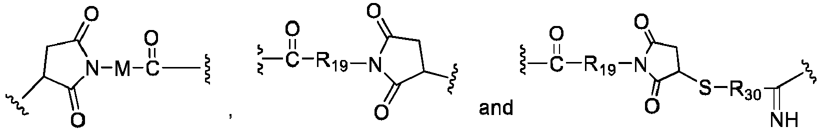 wherein:
      the wavy lines indicate the point of covalent attachments to an Ab (the wavy line to the right) and to (T)_{g} if any, or (AA)_{w} if any, or to (X)_{b} if any, or to (D) (the wavy line to the left);
      R₁₉ is selected from -C₁₋C₁₂ alkylene-, -O-(C₁-C₁₂ alkylene)-, -C₆-C₁₂ arylene- in one or more rings which may optionally be substituted with one or more substituents Rₓ, -C₁₋C₁₂ alkylene-C₆-C₁₂ arylene- wherein the arylene group is in one or more rings which may optionally be substituted with one or more substituents Rₓ, -C₆-C₁₂ arylene-C₁₋C₁₂ alkylene- wherein the arylene group is in one or more rings which may optionally be substituted with one or more substituents Rₓ, -C₅-C₁₂ heterocyclo- wherein said heterocyclo group may be a saturated or unsaturated group having one or more rings and comprising at least one oxygen, nitrogen or sulphur atom in said ring(s), said group optionally being substituted with one or more substituents Rₓ, -C₁₋C₁₂ alkylene-(C₅-C₁₂ heterocyclo)- wherein said heterocyclo group may be a saturated or unsaturated group having one or more rings and comprising at least one oxygen, nitrogen or sulphur atom in said ring(s), said group optionally being substituted with one or more substituents Rₓ, -(C₅-C₁₂ heterocyclo)-C₁-C₁₂ alkylene- wherein said heterocyclo group may be a saturated or unsaturated group having one or more rings and comprising at least one oxygen, nitrogen or sulphur atom in said ring(s), said group optionally being substituted with one or more substituents Rₓ, -(OCH₂CH₂)ᵣ- and -CH₂-(OCH₂CH₂)ᵣ-, wherein each of the above alkylene substituents whether alone or attached to another moiety in the carbon chain may optionally be substituted by one or more substituents Rₓ;
      R₃₀ is a -C₁₋C₆ alkylene- group;
      M is selected from the group consisting of -C₁₋C₆ alkylene-, -C₁₋C₆ alkylene-(C₃-C₈ carbocyclo)- and -phenylene- which may optionally be substituted with one or more substituents Rₓ;
      r is an integer ranging from 1-6;
      (AA)_{w} is of formula (III): 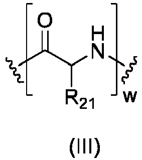
      wherein the wavy lines indicate the point of covalent attachments to (X)_{b} if any, or to the drug moiety (the wavy line to the left) and to (T)_{g} if any, or to the linker (the wavy line to the right);
      R₂₁ is, at each occurrence, selected from the group consisting of hydrogen, methyl, isopropyl, isobutyl, *sec*-butyl, benzyl, *p*-hydroxybenzyl, -CH₂OH, -CH(OH)CH₃, -CH₂CH₂SCH₃, - CH₂CONH₂, -CH₂COOH, -CH₂CH₂CONH₂, -CH₂CH₂COOH, -(CH₂)₃NHC(=NH)NH₂, - (CH₂)₃NH₂, -(CH₂)₃NHCOCH₃, -(CH₂)₃NHCHO, -(CH₂)₄NHC(=NH)NH₂, -(CH₂)₄NH₂, - (CH₂)₄NHCOCH₃, -(CH₂)₄NHCHO, -(CH₂)₃NHCONH₂, -(CH₂)₄NHCONH₂, - CH₂CH₂CH(OH)CH₂NH₂, 2-pyridylmethyl-, 3-pyridylmethyl-, 4-pyridylmethyl-, phenyl, cyclohexyl, 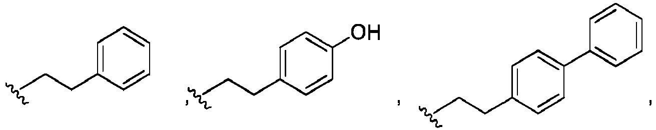 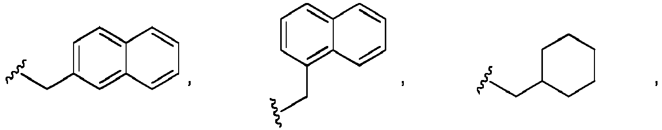 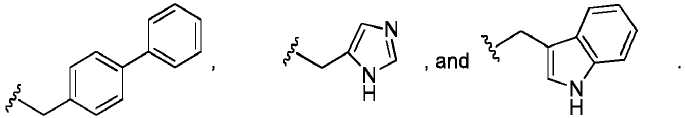
      w is an integer ranging from 0 to 12;
      wherein X is an extending group selected from:
         -CONH-(C₁-C₆ alkylene)NH-, -COO-CH₂-(phenylene which may optionally be substituted with one or more substituents Rₓ)-NH-, -CONH-(C₁-C₆ alkylene)NH-COO-CH₂-(phenylene which may optionally be substituted with one or more substituents Rₓ)-NH-, -COCH₂NH-COCH₂-NH-, -COCH₂NH-, -CONH-(C₁-C₆ alkylene)S-, and -CONH-(C₁-C₆ alkylene)NHCO(C₁-C₆ alkylene)S-;
         b is 0 or 1, preferably 1;
         wherein T is an extending group selected from -CO-(C₁-C₆ alkylene)-NH-, -CO-(C₁-C₆ alkylene)-[O-(C₂-C₆ alkylene)]ⱼ-NH-, and -COO-(C₁-C₆ alkylene)-[O-(C₂-C₆ alkylene)]ⱼ-NH-, where j is an integer from 1 to 25;
         g is 0 or 1, preferably 0;
         D is a drug moiety of formula (I), (Ia), (II) or (Ila), or a pharmaceutically acceptable salt or ester thereof wherein:
            R₁ is hydrogen or substituted or unsubstituted C₁-C₆ alkyl, wherein the optional substituents are one or more substituents Rₓ;
            R₂ is hydrogen;
            R₃ is hydrogen or -C(=O)Rₐ, wherein Rₐ is substituted or unsubstituted C₁-C₆ alkyl, wherein the optional substituents are one or more substituents Rₓ;
            Z is -O-;
            the moiety Ab comprising at least one antigen binding site is an antibody or an antigen-binding fragment thereof and it is selected from the group consisting of a human antibody, an antigen-binding fragment of a human antibody, a humanized antibody, an antigen-binding fragment of a humanized antibody, a chimeric antibody, an antigen-binding fragment of a chimeric antibody, a glycosylated antibody and a glycosylated antigen binding fragment; and
            n is the ratio of the group [D-(X)_{b}-(AA)_{w}-(T)_{g}-(L)-] to the moiety Ab comprising at least one antigen binding site and is in the range from 1 to 12.
   (b) a drug conjugate according to the present invention selected from the formulas (V), (VI) and (VII): 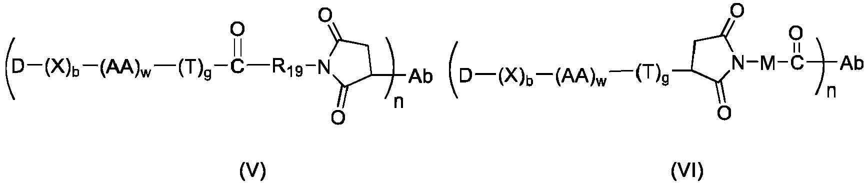 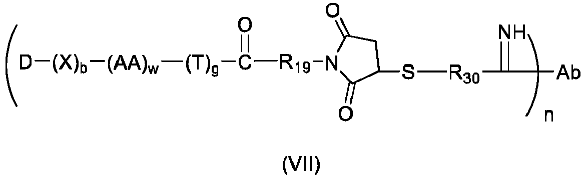 wherein:
      R₁₉ is selected from -C₁-C₈ alkylene-, -O-(C₁-C₈ alkylene)-, -C₁-C₈ alkylene-C₆-C₁₂ arylene-wherein the arylene group is in one or more rings which may optionally be substituted with one or more substituents Rₓ and -C₆-C₁₂ arylene-C₁₋C₈ alkylene- wherein the arylene group is in one or more rings which may optionally be substituted with one or more substituents Rₓ, wherein each of the above alkylene substituents whether alone or attached to another moiety in the carbon chain may optionally be substituted by one or more substituents Rₓ;
      R₃₀ is a -C₂-C₄ alkylene- group;
      M is selected from the group consisting of -C₁₋C₃ alkylene- and -C₁₋C₃ alkylene-(C₅-C₇ carbocyclo)-;
      (AA)_{w} is of formula (III): 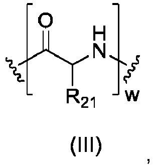 wherein:
      the wavy lines indicate the point of covalent attachments to (X)_{b} if any, or to the drug moiety (the wavy line to the left) and to (T)_{g} if any, or to the linker (the wavy line to the right);
      R₂₁ is, at each occurrence, selected from the group consisting of hydrogen, methyl, isopropyl, sec-butyl, benzyl, indolylmethyl, -(CH₂)₃NHCONH₂, -(CH₂)₄NH₂, -(CH₂)₃NHC(=NH)NH₂ and - (CH₂)₄NHC(=NH)NH₂;
      w is an integer from 0 to 6;
      X is an extending group selected from the group consisting of
         -CONH-(C₂-C₄ alkylene)NH-, -COO-CH₂-phenylene-NH-, wherein said phenylene group may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups, -CONH-(C₂-C₄ alkylene)NH-COO-CH₂-(phenylene which may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups)-NH- , -COCH₂NH-COCH₂-NH-, -CONH-(C₂-C₄ alkylene)S-, and -CONH-(C₂-C₄ alkylene)NHCO(C₁-C₃ alkylene)S-;
         b is 0 or 1, preferably 1;
         wherein T is an extending group selected from -CO-(C₁-C₄ alkylene)-NH-, -CO-(C₁-C₄ alkylene)-[O-(C₂-C₄ alkylene)]ⱼ-NH-, and -COO-(C₁-C₄ alkylene)-[O-(C₂-C₄ alkylene)]ⱼ-NH-, where j is an integer from 1 to 10;
         g is 0 or 1, preferably 0;
         D is a drug moiety of formula (I), (Ia), (II) or (Ila), or a pharmaceutically acceptable salt or ester thereof wherein:
            R₁ is hydrogen or methyl, more preferably methyl;
            R₂ is hydrogen;
            R₃ is hydrogen;
            Z is -O-;
            the moiety Ab comprising at least one antigen binding site is an antibody or an antigen-binding fragment thereof, wherein the antibody or antigen-binding fragment is a monoclonal antibody which immunospecifically binds to cancer cell antigens, viral antigens, antigens of cells that produce autoimmune antibodies associated with autoimmune disease, microbial antigens, and preferably a monoclonal antibody which immunospecifically binds to cancer cell antigens; and
            n is the ratio of the group [D-(X)_{b}-(AA)_{w}-(T)_{g}-(L)-] wherein L is as defined in formulas (V), (VI) or (VII) to the moiety Ab comprising at least one antigen binding site and is in the range from 3 to 8.
   (c) a drug conjugate according to the present invention selected from the formulas (V), (VI) and (VII): 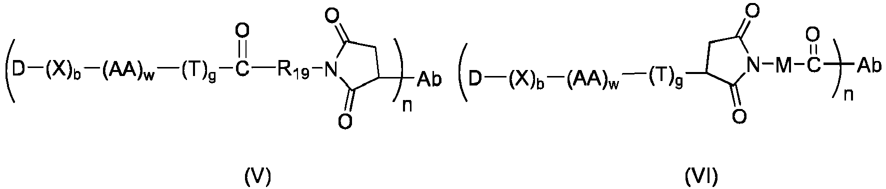 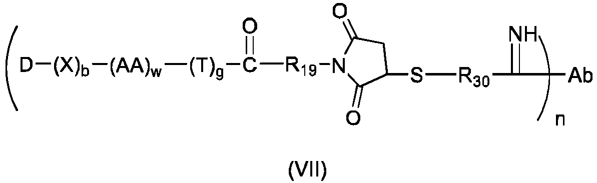 wherein:
      R₁₉ is selected from -C₁₋C₆ alkylene-, -phenylene-C₁-C₆ alkylene- wherein the phenylene group may optionally be substituted with one or more substituents Rₓ selected from the group consisting of alkyl groups having from 1 to 6 carbon atoms, alkoxy groups having from 1 to 6 carbon atoms, halogen atoms, nitro groups and cyano groups, wherein each of the above alkylene substituents whether alone or attached to another moiety in the carbon chain may optionally be substituted by one or more substituents Rₓ selected from the group consisting of alkyl groups having from 1 to 6 carbon atoms, alkoxy groups having from 1 to 6 carbon atoms, aryl groups having from 6 to 12 carbon atoms, halogen atoms, nitro groups and cyano groups, and preferably R₁₉ is a -C₁₋C₆ alkylene- group;
      R₃₀ is a -C₂-C₄ alkylene- group;
      M is -C₁₋C₃ alkylene-(C₅-C₇ carbocyclo)-;
      w is 0 or 2, and where w is 2, then (AA)_{w} is of formula (IV): 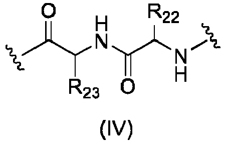
      wherein the wavy lines indicate the point of covalent attachments to (X)_{b} if any, or to the drug moiety (the wavy line to the left) and to (T)_{g} if any, or to the linker (the wavy line to the right);
      R₂₂is selected from methyl, benzyl, isopropyl, sec-butyl and indolylmethyl;
      R₂₃ is selected from methyl, -(CH₂)₄NH₂, -(CH2)₃NHCONH₂and -(CH₂)₃NHC(=NH)NH₂;
      X is an extending group selected from the group consisting of -CONH-(C₂-C₄ alkylene)NH-, - COO-CH₂-phenylene-NH-, wherein said phenylene group may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups, -CONH-(C₂-C₄ alkylene)NH-COO-CH₂-(phenylene which may optionally be substituted with from one to four substituents Rₓ selected from the group consisting of alkyl groups having from 1 to 6 carbon atoms, alkoxy groups having from 1 to 6 carbon atoms, halogen atoms, nitro groups or cyano groups)-NH-, -COCH₂NH-COCH₂-NH-,- CONH-(C₂-C₄ alkylene)S-, and -CONH-(C₂-C₄ alkylene)NHCO(C₁-C₃ alkylene)S-;
      b is 0 or 1, preferably 1;
      T is an extending group selected from -CO-(C₁-C₄ alkylene)-NH-, -CO-(C₁-C₄ alkylene)-[O-(C₂-C₄ alkylene)]ⱼ-NH-, and -COO-(C₁-C₄ alkylene)-[O-(C₂-C₄ alkylene)]ⱼ-NH-, where j is an integer from 1 to 5;
      g is 0 or 1; preferably 0;
      D is a drug moiety of formula (I), (Ia), (ii) or (Ila), or a pharmaceutically acceptable salt or ester thereof wherein:
         R₁ is hydrogen or methyl, more preferably methyl;
         R₂ is hydrogen;
         R₃ is hydrogen;
         Z is -O-;
         the moiety Ab comprising at least one antigen binding site is a monoclonal antibody selected from the group consisting of Abciximab, Alemtuzumab, Anetumab, Atezolizumab, Avelumab, Basiliximab, Bevacizumab, Blinatomumab, Brentuximab, Catumaxomab, Cetuximab, Coltuximab, Daclizumab, Daratumumab, Denintuzumab, Denosumab, Depatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labetuzumab, Ladiratuzumab, Laprituximab, Lifastuzumab, Lorvotuzumab, Milatuzumab, Mirvetuximab, Naratuximab, Necitumumab, Nimotuzumab, Nivolumab, Obinutuzumab, Ofatumumab, Olaratumab, Omalizumab, Palivizumab, Panitumumab, Pembrolizumab, Pertuzumab, Pinatuzumab, Polatuzumab, Ramucirumab, Rovalpituzumab, Sacituzumab, Siltuximab, Sirtratumab, Sofituzumab, Vadastuximab, Vorsetuzumab, an anti-HER2 antibody such as Trastuzumab, an anti-CD4 antibody, an anti-CD5 antibody, and an anti-CD30 antibody, or an antigen-binding fragment or an immunologicallly active portion thereof, wherein preferably the antibody is selected from Abciximab, Alemtuzumab, Anetumab, Atezolizumab, Avelumab, Basiliximab, Bevacizumab, Blinatomumab, Brentuximab, Catumaxomab, Cetuximab, Daclizumab, Daratumumab, Denintuzumab, Denosumab, Depatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labetuzumab, Ladiratuzumab, Laprituximab, Mirvetuximab, Naratuximab, Necitumumab, Nimotuzumab, Nivolumab, Obinutuzumab, Ofatumumab, Olaratumab, Omalizumab, Palivizumab, Panitumumab, Pembrolizumab, Pertuzumab, Polatuzumab, Ramucirumab, Rovalpituzumab, Sacituzumab, Siltuximab, Sirtratumab, Vadastuximab, Vorsetuzumab, an anti-HER2 antibody such as Trastuzumab, an anti-CD4 antibody, an anti-CD5 antibody, and an anti-CD30 antibody, or an antigen-binding fragment or an immunologicallly active portion thereof, and yet more preferably Abciximab, Alemtuzumab, Atezolizumab, Avelumab, Basiliximab, Bevacizumab, Blinatomumab, Brentuximab, Catumaxomab, Cetuximab, Daclizumab, Daratumumab, Denosumab, Dinutuximab, Durvalumab, Elotuzumab, Gemtuzumab, Ibritumomab, Inotuzumab, Ipilimumab, Labetuzumab, Necitumumab, Nimotuzumab, Nivolumab, Obinutuzumab, Ofatumumab, Olaratumab, Omalizumab, Palivizumab, Panitumumab, Pembrolizumab, Pertuzumab, Ramucirumab, Rovalpituzumab, Siltuximab, an anti-HER2 antibody such as Trastuzumab, an anti-CD4 antibody, an anti-CD5 antibody, and an anti-CD30 antibody, or an antigen-binding fragment or an immunologically active portion thereof. Of these, particularly preferred are Brentuximab, Gemtuzumab, Inozutumab, Rovalpituzumab, an anti-HER2 antibody such as Trastuzumab, an anti-CD4 antibody, an anti-CD5 antibody, and an anti-CD30 antibody, or an antigen-binding fragment or an immunologicallly active portion thereof; or the antibody is an anti-HER2 antibody such as Trastuzumab or an antigen-binding fragment or an immunologically active portion thereof, particularly Trastuzumab or an antigen-binding fragment or an immunologicallly active portion thereof; and n is the ratio of the group [D-(X)_{b}-(AA)_{w}-(T)_{g}-(L)-] wherein L is as defined in formulas (V), (VI) or (VII) to the moiety Ab comprising at least one antigen binding site and is in the range from 3 to 5.
   (d) A drug conjugate according to the present invention selected from the formulas (V), (VI) and (VII): 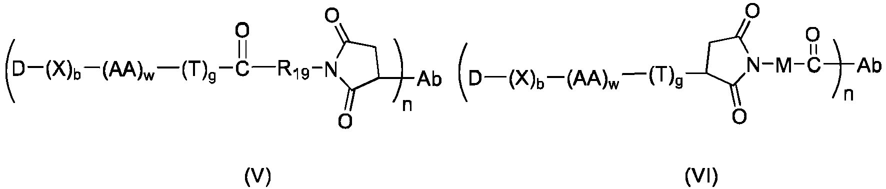 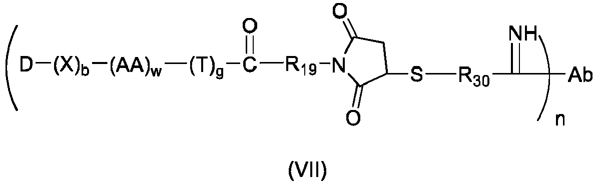 wherein:
      R₁₉ is -C₂-C₆ alkylene-;
      R₃₀ is a -C₂-C₄ alkylene- ;
      M is -C₁₋C₃ alkylene-(C₅-C₇ carbocyclo)-;
      w is 0 or 2, and where w is 2, then (AA)_{w} is of formula (IV): 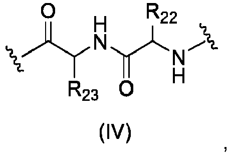
      wherein R₂₂ is isopropyl, R₂₃ is selected from methyl and -(CH₂)₃NHCONH₂, wherein the wavy lines indicate the point of covalent attachments to (X)_{b} if any, or to the drug moiety (the wavy line to the left) and to (T)_{g} if any, or to the linker (the wavy line to the right);
      X is an extending group selected from the group consisting of -CONH-(C₂-C₄ alkylene)NH-, - COO-CH₂-phenylene-NH-, wherein said phenylene group may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups, -CONH-(C₂-C₄ alkylene)NH-COO-CH₂-(phenylene which may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups)-NH-, -COCH₂NH-COCH₂-NH-, -CONH-(C₂-C₄ alkylene)S-, and -CONH-(C₂-C₄ alkylene)NHCO(C₁-C₃ alkylene)S-;
      b is 0 or 1, preferably 1;
      T is an extending group selected from -CO-(C₁-C₄ alkylene)-NH-, -CO-(C₁₋C₄ alkylene)-[O-(C₂-C₄ alkylene)]ⱼ-NH-, and -COO-(C₁-C₄ alkylene)-[O-(C₂-C₄ alkylene)]ⱼ-NH-, where j is an integer from 1 to 5;
      g is 0 or 1; preferably 0;
      D is a drug moiety selected from: 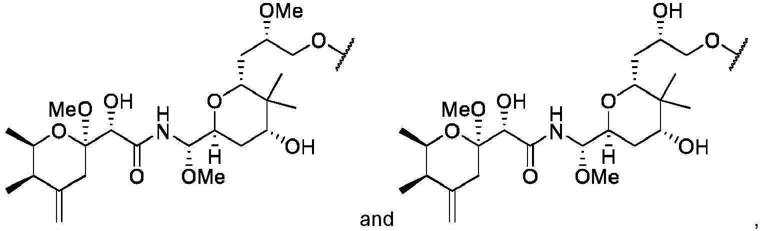
      or a pharmaceutically acceptable salt or ester thereof; wherein the wavy line indicates the point of covalent attachment to (X)_{b} if any, or (AA)_{w} if any, or to (T)_{g} if any, or to (L);
      the moiety Ab comprising at least one antigen binding site is selected from Brentuximab, Gemtuzumab, Inozutumab, Rovalpituzumab, an anti-HER2 antibody such as Trastuzumab, an anti-CD4 antibody, an anti-CD5 antibody, and an anti-CD30 antibody, or an antigen-binding fragment or an immunologicallly active portion thereof, and more preferably is an anti-HER2 antibody such as Trastuzumab or an antigen-binding fragment or an immunologically active portion thereof, particularly Trastuzumab or an antigen-binding fragment or an immunologicallly active portion thereof; and
      n is the ratio of the group [D-(X)_{b}-(AA)_{w}-(T)_{g}-(L)-] wherein L is as defined in formulas (V), (VI) or (VII) to the moiety Ab comprising at least one antigen binding site and is in the range from 3 to 5.
   (e) A drug conjugate according to the present invention selected from the formulas (V), (VI), and (VII): 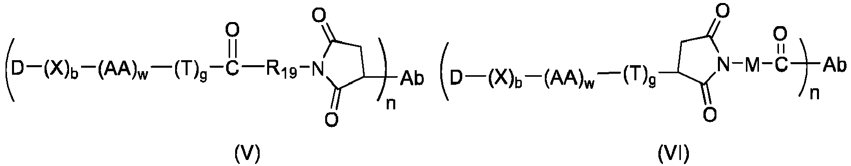 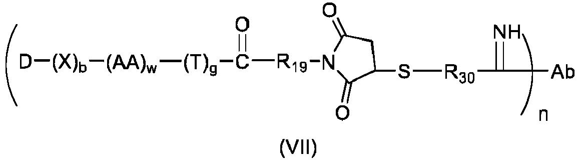 wherein:
      R₁₉ is -C₂-C₆ alkylene-;
      R₃₀ is -C₂-C₄ alkylene-;
      M is -C₁₋C₃ alkylene-(C₅-C₇ carbocyclo)-;
      w is 0 or 2, and where w is 2, then (AA)_{w} is of formula (IV): 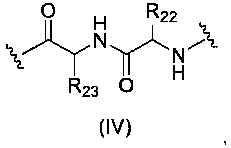
      wherein R₂₂ is isopropyl, R₂₃ is selected from methyl and -(CH₂)₃NHCONH₂, and the wavy lines indicate the point of covalent attachments to (X)_{b} if any, or to the drug moiety (the wavy line to the left) and to (T)_{g} if any, or to the linker (the wavy line to the right);
      X is an extending group selected from the group consisting of -CONH-(C₂-C₄ alkylene)NH-, - COO-CH₂-phenylene-NH-, wherein said phenylene group may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups, -CONH-(C₂-C₄ alkylene)NH-COO-CH₂-(phenylene which may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups)-NH-, -COCH₂NH-COCH₂-NH-, -CONH-(C₂-C₄ alkylene)S-, and -CONH-(C₂-C₄ alkylene)NHCO(C₁-C₃ alkylene)S-;
      b is 0 or 1, preferably 1;
      wherein T is an extending group selected from -CO-(C₁-C₄ alkylene)-NH-, -CO-(C₁-C₄ alkylene)-[O-(C₂-C₄ alkylene)]ⱼ-NH-, and -COO-(C₁-C₄ alkylene)-[O-(C₂-C₄ alkylene)]ⱼ-NH-, where j is an integer from 1 to 5;
      g is 0 or 1; preferably 0;
      D is a drug moiety selected from: 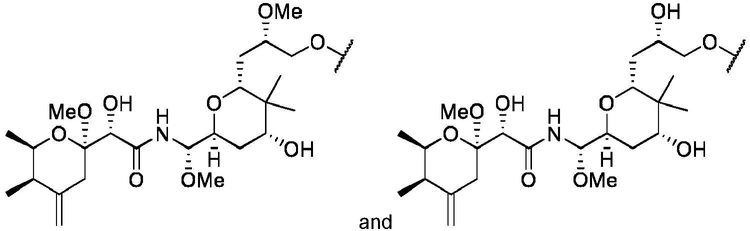
      or a pharmaceutically acceptable salt or ester thereof ; wherein the wavy line indicates the point of covalent attachment to (X)_{b} if any, or (AA)_{w} if any, or to (T)_{g} if any, or to (L);
      the moiety Ab comprising at least one antigen binding site is selected from Brentuximab, Gemtuzumab, Inozutumab, Rovalpituzumab, an anti-HER2 antibody such as Trastuzumab, an anti-CD4 antibody, an anti-CD5 antibody, and an anti-CD30 antibody, or an antigen-binding fragment or an immunologicallly active portion thereof, and more preferably is an anti-HER2 antibody such as Trastuzumab or an antigen-binding fragment or an immunologically active portion thereof, particularly Trastuzumab or an antigen-binding fragment or an immunologicallly active portion thereof; and
      n is the ratio of the group [D-(X)_{b}-(AA)_{w}-(T)_{g}-(L)-] wherein L is as defined in formulas (V), (VI) or (VII) to the moiety comprising at least one antigen binding site and is in the range from 3 to 5.
   (f) A drug conjugate according to the present invention of formula (V): 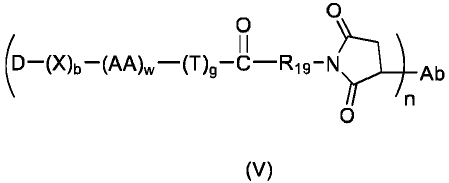 wherein:
      R₁₉ is -C₂-C₅ alkylene-;
      w is 0 or 2, and where w is 2, then (AA)_{w} is of formula (IV): 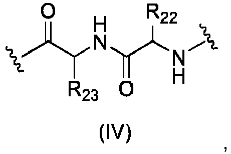
      wherein R₂₂ is isopropyl, R₂₃ is selected from methyl and -(CH₂)₃NHCONH₂, and the wavy lines indicate the point of covalent attachments to (X)_{b} (the wavy line to the left) and to (T)_{g} if any, or to the linker (the wavy line to the right);X is -CONH-(C₃ alkylene)NH- or -CONH-(C₃ alkylene)NH-COO-CH₂-phenylene-NH-;
      b is 1;
      T is an extending group of formula -CO-(C₁-C₄ alkylene)-[O-(C₂-C₄ alkylene)]₄-NH-;
      g is 0 or 1; preferably 0;
      or of formula (VI) 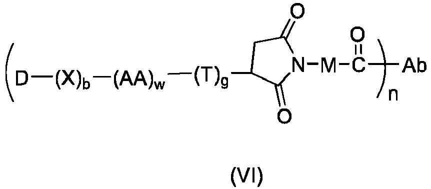
      wherein M is -methyl-cyclohexylene-;
      b is 1;
      w is 0;
      X is an extending group selected from -CONH(CH₂)₃S- and -CONH(CH₂)₃NHCO(CH₂)₂S-g is 0;
      or of formula (VII) 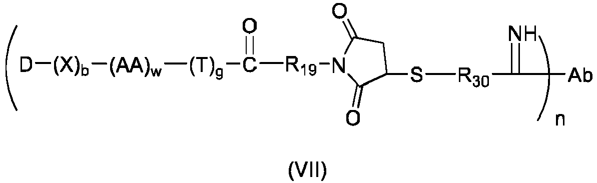
      wherein R₁₉ is -C₂-C₅ alkylene-;
      R₃₀ is -C₃ alkylene-;
      w is 0 or 2, and where w is 2, then (AA)_{w} is of formula (IV): 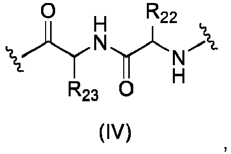
      wherein R₂₂ is isopropyl, R₂₃ is selected from methyl and -(CH₂)₃NHCONH₂, and the wavy lines indicate the point of covalent attachments to (X)_{b} (the wavy line to the left) and to (T)_{g} if any, or to the linker (the wavy line to the right); and
      X is -CONH-(C₃ alkylene)NH- or -CONH-(C₃ alkylene)NH-COO-CH₂-phenylene-NH-;
      b is 1;
      T is an extending group of formula -CO-(C₁-C₄ alkylene)-[O-(C₂-C₄ alkylene)]₄-NH-;
      g is 0 or 1; preferably 0;
      D is a drug moiety selected from: 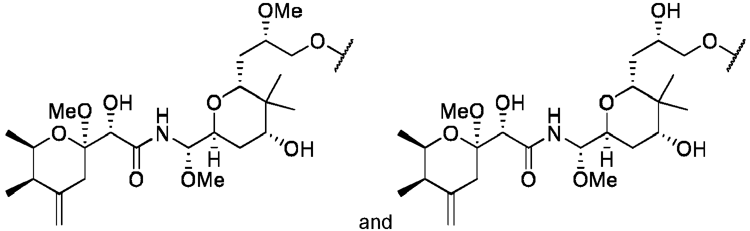
      or a pharmaceutically acceptable salt or ester thereof; wherein the wavy line indicates the point of covalent attachment to (X)_{b};
      the moiety Ab comprising at least one antigen binding site is Brentuximab, Gemtuzumab, Inozutumab, Rovalpituzumab, an anti-HER2 antibody such as Trastuzumab, an anti-CD4 antibody, an anti-CD5 antibody, and an anti-CD30 antibody, or an antigen-binding fragment or an immunologicallly active portion thereof, and more preferably its is an anti-HER2 antibody such as Trastuzumab or an antigen-binding fragment or an immunologically active portion thereof, particularly Trastuzumab or an antigen-binding fragment or an immunologicallly active portion thereof; and
      n is the ratio of the group [D-(X)_{b}-(AA)_{w}-(T)_{g}-(L)-] wherein L is as defined in formula (V), (VI) or (VII) to the moiety Ab comprising at least one antigen binding site and is in the range from 3 to 5, and preferably 4.
   g) an antibody drug conjugate according according to the present invention, selected from the group consisting of: 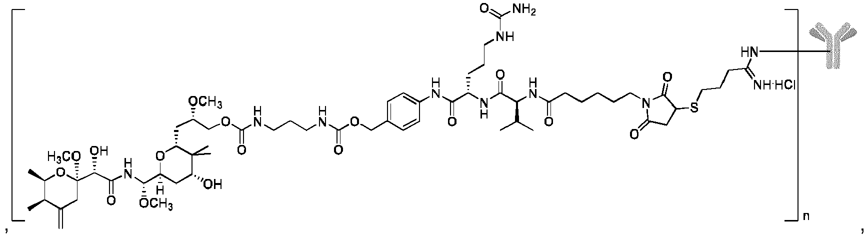 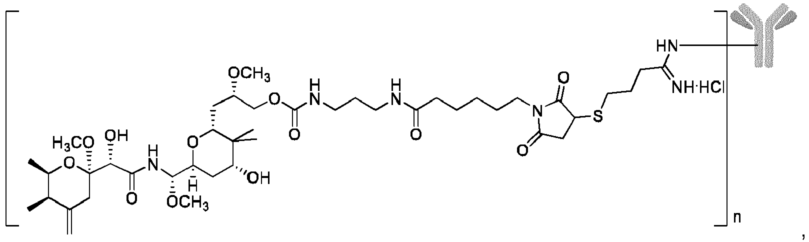 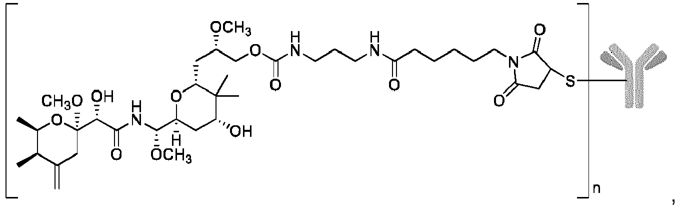 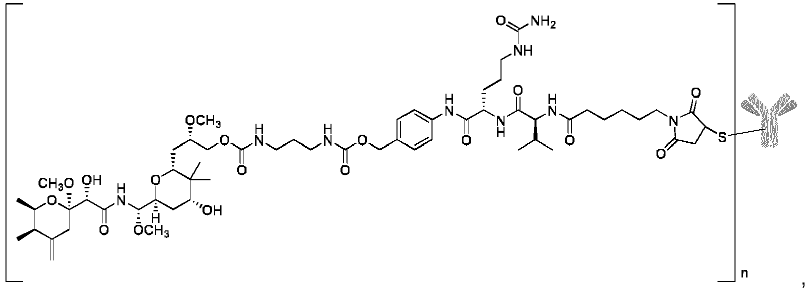 and 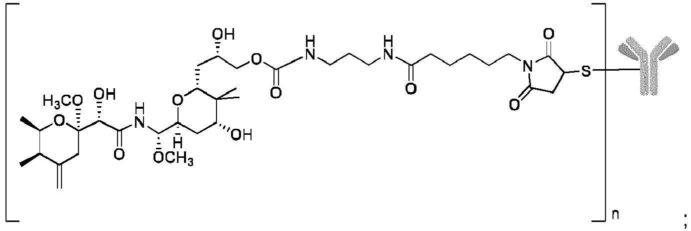 wherein n is from 2 to 6, more preferably 3, 4, or 5 and each 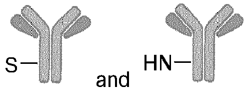 is independently selected from Brentuximab, Gemtuzumab, Inozutumab, Rovalpituzumab, an anti-HER2 antibody such as Trastuzumab, an anti-CD4 antibody, an anti-CD5 antibody, and an anti-CD30 antibody, or an antigen-binding fragment or an immunologically active portion thereof, and more preferably it is an anti-HER2 antibody such as Trastuzumab or an antigen-binding fragment or an immunologically active portion thereof, particularly Trastuzumab or an antigen-binding fragment or an immunologically active portion thereof.

More preferably the antibody drug conjugate is selected from the group consisting of: 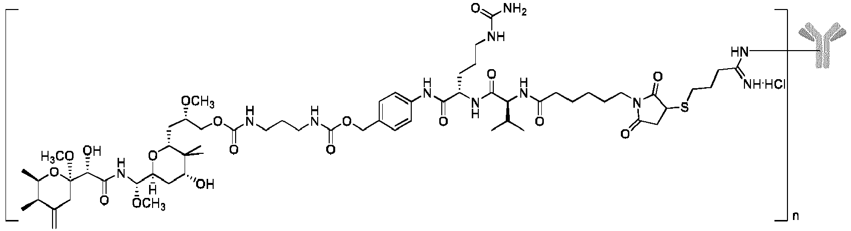
wherein n is from 2 to 6, more preferably 3, 4, or 5 and 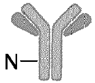 is an anti-HER2 antibody such as Trastuzumab or an antigen-binding fragment or an immunologically active portion thereof, more preferably is Trastuzumab or an antigen binding fragment or an immunologically active portion thereof, 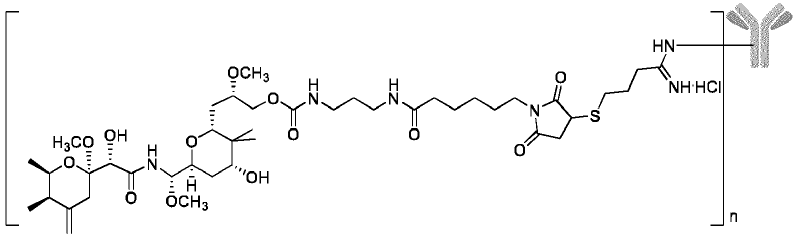
wherein n is from 2 to 6, more preferably 3, 4, or 5 and 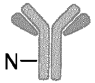 is an anti-HER2 antibody such as Trastuzumab or an antigen-binding fragment or an immunologically active portion thereof, more preferably is Trastuzumab or an antigen binding fragment or an immunologically active portion thereof, 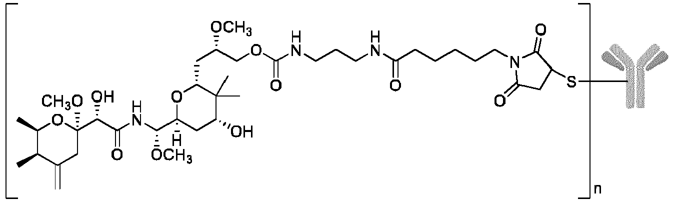
wherein n is from 2 to 6, more preferably 3, 4, or 5 and 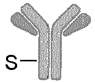 is an anti-HER2 antibody such as Trastuzumab or an antigen-binding fragment or an immunologically active portion thereof, more preferably is Trastuzumab or an antigen-binding fragment or an immunologically active portion thereof,

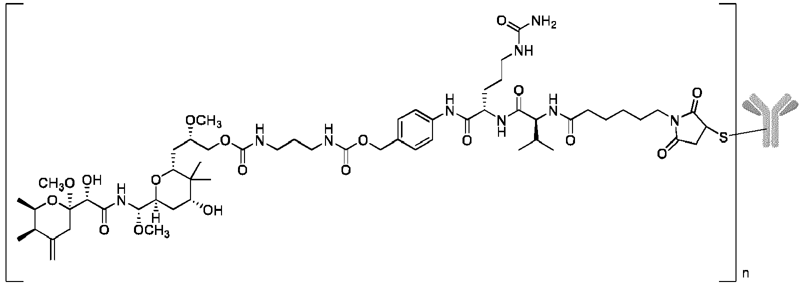

wherein n is from 2 to 6, more preferably 3, 4, or 5 and 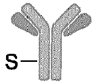 is an anti-HER2 antibody such as Trastuzumab or an antigen-binding fragment or an immunologically active portion thereof, more preferably is Trastuzumab or an antigen-binding fragment or an immunologically active portion thereof, and 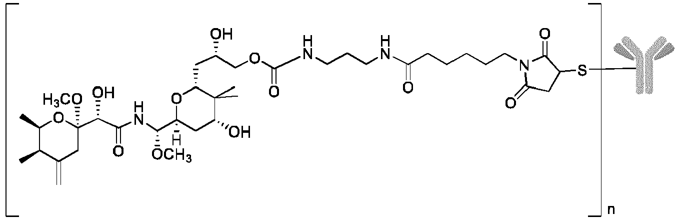
wherein n is from 2 to 6, more preferably 3, 4, or 5 and 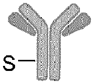 is an anti-HER2 antibody such as Trastuzumab or an antigen-binding fragment or an immunologically active portion thereof, more preferably is Trastuzumab or an antigen-binding fragment or an immunologically active portion thereof.

Particularly preferably, the antibody drug conjugates according to the present invention should be in isolated or purified form.

Preferred compounds of formula D-(X)_{b}-(AA)_{w}-(T)_{g}-L₁ or of formula D-(X)_{b}-(AA)_{w}-(T)_{g}-H according to the present invention include:
- A compound of formula D-(X)_{b}-(AA)_{w}-(T)_{g}-L₁ or of formula D-(X)_{b}-(AA)_{w}-(T)_{g}-H wherein each of D, X, AA, T, L_{1,} b, g and w are as defined herein in the present invention wherein for compounds of formula D-(X)_{b}-(AA)_{w}-(T)_{g}-H, b+w+g is not 0.
- A compound of formula D-(X)_{b}-(AA)_{w}-(T)_{g}-L₁ or of formula D-(X)_{b}-(AA)_{w}-(T)_{g}-H according to the present invention; wherein b+w+g is not 0.
- A compound of formula D-(X)_{b}-(AA)_{w}-(T)_{g}-L₁ or of formula D-(X)_{b}-(AA)_{w}-(T)_{g}-H according to the present invention wherein:
   L₁ is a linker of formula: 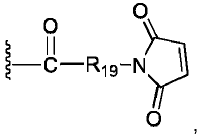 wherein:
   the wavy line indicates the point of covalent attachment to (T)_{g} if any, or (AA)_{w} if any, or to (X)_{b} if any, or to D;
   R₁₉ is selected from -C₁₋C₁₂ alkylene-, -O-(C₁-C₁₂ alkylene)-, -C₆-C₁₂ arylene- in one or more rings which may optionally be substituted with one or more substituents Rₓ, -C₁₋C₁₂ alkylene-C₆-C₁₂ arylene- wherein the arylene group is in one or more rings which may optionally be substituted with one or more substituents Rₓ, -C₆-C₁₂ arylene-C₁₋C₁₂ alkylene- wherein the arylene group is in one or more rings which may optionally be substituted with one or more substituents Rₓ, -C₅-C₁₂ heterocyclo- wherein said heterocyclo group may be a saturated or unsaturated group having one or more rings and comprising at least one oxygen, nitrogen or sulphur atom in said ring(s), said group optionally being substituted with one or more substituents Rₓ, -C₁₋C₁₂ alkylene-(C₅-C₁₂ heterocyclo)- wherein said heterocyclo group may be a saturated or unsaturated group having one or more rings and comprising at least one oxygen, nitrogen or sulphur atom in said ring(s), said group optionally being substituted with one or more substituents Rₓ, -(C₅-C₁₂ heterocyclo)-C₁-C₁₂ alkylene- wherein said heterocyclo group may be a saturated or unsaturated group having one or more rings and comprising at least one oxygen, nitrogen or sulphur atom in said ring(s), said group optionally being substituted with one or more substituents Rₓ, -(OCH₂CH₂)ᵣ- and -CH₂-(OCH₂CH₂)ᵣ-, wherein each of the above alkylene substituents whether alone or attached to another moiety in the carbon chain may optionally be substituted by one or more substituents Rₓ;
   r is an integer ranging from 1-6; and
   each of D, Rₓ, X, AA, T, b, g and w is as defined in the present invention; but wherein if the compound is a compound of formula D-(X)_{b}-(AA)_{w}-(T)_{g}-H then b+w+g≠0.
- a compound of formula D-(X)_{b}-(AA)_{w}-(T)_{g}-L₁ or of formula D-(X)_{b}-(AA)_{w}-(T)_{g}-H according to the present invention wherein:
   L₁ is linker of formula: 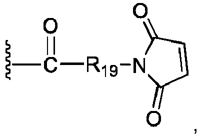 wherein:
   the wavy line indicates the point of covalent attachment to (T)_{g} if any, or (AA)_{w} if any, or to (X)_{b} if any, or to D;
   R₁₉ is selected from -C₁₋C₈ alkylene-, -O-(C₁-C₈ alkylene)-, -C₁-C₈ alkylene-C₆-C₁₂ arylene-wherein the arylene group is in one or more rings which may optionally be substituted with one or more substituents Rₓ, and -C₆-C₁₂ arylene-C₁-C₈ alkylene- wherein the arylene group is in one or more rings which may optionally be substituted with one or more substituents Rₓ, wherein each of the above alkylene substituents whether alone or attached to another moiety in the carbon chain may optionally be substituted by one or more substituents Rₓ;
   (AA)_{w} is of formula (III): 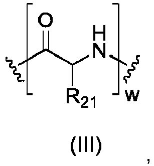
   wherein the wavy lines indicate the point of covalent attachments to (X)_{b}, if any, or to D (the wavy line to the left) and to (T)_{g} if any, or L₁ or to a hydrogen atom (the wavy line to the right);
   wherein R₂₁ is selected, at each occurrence, from the group consisting of hydrogen, methyl, isopropyl, sec-butyl, benzyl, indolylmethyl, -(CH₂)₃NHCONH₂, -(CH₂)₄NH₂, - (CH₂)₃NHC(=NH)NH₂ and -(CH₂)₄NHC-(=NH)NH₂, and w is an integer from 0 to 6;
   X is an extending group selected from the group consisting of:
      -CONH-(C₂-C₄ alkylene)NH-, -COO-CH₂-phenylene-NH-, wherein said phenylene group may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups, -CONH-(C₂-C₄ alkylene)NH-COO-CH₂-(phenylene which may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups)-NH-, -COCH₂NH-COCH₂-NH-, -CONH-(C₂-C₄ alkylene)S-, and -CONH-(C₂-C₄ alkylene)NHCO(C₁-C₃ alkylene)S-;
      T is an extending group selected from -CO-(C₁-C₄ alkylene)-NH-; -CO-(C₁-C₄ alkylene)-[O-(C₂-C₄ alkylene)]ⱼ-NH- and -COO-(C₁-C₄ alkylene)-[O-(C₂-C₄ alkylene)]ⱼ-NH-, where j is an integer from 1 to 10;
      b is 0 or 1, preferably 1;
      g is 0 or 1, preferably 0;
      wherein if the compound is a compound of formula D-(X)_{b}-(AA)_{w}-(T)_{g}-H then b+w+g≠0; and
      D is a drug moiety of formula (I), (Ia) or a pharmaceutically acceptable salt or ester thereof: 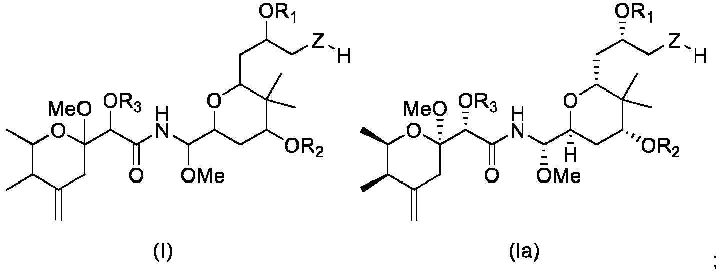
      and is covalently attached via a hydroxy group at OR_{1,} OR₃ or ZH ; or
      D is a drug moiety of formula (II) or a formula (Ila), or a pharmaceutically acceptable salt or ester thereof: 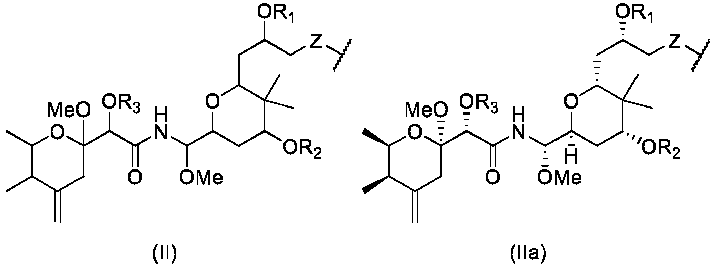
      wherein the wavy lines of (II) and (Ila) indicate the point of covalent attachment to (X)_{b} if any, or (AA)_{w} if any, or to (T)_{g} if any, or to L₁;
      R₁ is hydrogen or substituted or unsubstituted C₁-C₆ alkyl, wherein the optional substituents are one or more substituents Rₓ;
      R₂ is hydrogen;
      R₃ is hydrogen or -C(=O)Rₐ, wherein Rₐ is substituted or unsubstituted C₁-C₆ alkyl, wherein the optional substituents are one or more substituents Rₓ.
      Z is -O-.
- a compound of formula D-(X)_{b}-(AA)_{w}-(T)_{g}-L₁ or of formula D-(X)_{b}-(AA)_{w}-(T)_{g}-H according to the present invention wherein:
   L₁ is a group of formula: 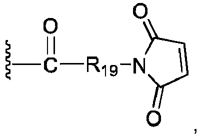 wherein:
   the wavy line indicates the point of covalent attachment to (T)_{g} if any, or (AA)_{w} if any, or to (X)_{b} if any, or to D;
   R₁₉ is selected from -C₁-C₆ alkylene-, -phenylene-C₁-C₆ alkylene- wherein the phenylene group may optionally be substituted with one or more substituents Rₓ selected from the group consisting of alkyl groups having from 1 to 6 carbon atoms, alkoxy groups having from 1 to 6 carbon atoms, halogen atoms, nitro groups and cyano groups, wherein each of the above alkylene substituents whether alone or attached to another moiety in the carbon chain may optionally be substituted by one or more substituents Rₓ selected from the group consisting of alkyl groups having from 1 to 6 carbon atoms, alkoxy groups having from 1 to 6 carbon atoms, aryl groups having from 6 to 12 carbon atoms, halogen atoms, nitro groups and cyano groups, and preferably R₁₉ is a -C₁-C₆ alkylene- group;
   w is 0 or 2, and where w is 2, then (AA)_{w} is of formula (IV): 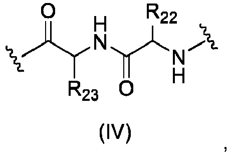
   wherein the wavy lines indicate the point of covalent attachments to (X)_{b} if any, or to D (the wavy line to the left) and to (T)_{g} if any, or L₁ or to a hydrogen atom (the wavy line to the right);
   R₂₂ is selected from methyl, benzyl, isopropyl, sec-butyl and indolylmethyl;
   R₂₃ is selected from methyl, -(CH₂)₄NH₂, -(CH₂)₃NHCONH₂ and -(CH₂)₃NHC(=NH)NH₂;
   X is an extending group selected from
      -COO-CH₂-phenylene-NH-, -CONH(CH₂)₃NHCOOCH₂-phenylene-NH-, -CONH(CH₂)₃NH-, - CONH(CH₂)₃-S-, and -CONH(CH₂)₃NHCO(CH₂)₂S-;
      wherein T is an extending group selected from -CO-(C₁-C₄ alkylene)-NH-, -CO-(C₁-C₄ alkylene)-[O-(C₂-C₄ alkylene)]ⱼ-NH-, and -COO-(C₁-C₄ alkylene)-[O-(C₂-C₄ alkylene)]ⱼ-NH-, where j is an integer from 1 to 5;
      b is 0 or 1, preferably 1;
      g is 0 or 1, preferably 0;
      wherein if the compound is a compound of formula D-(X)_{b}-(AA)_{w}-(T)_{g}-H then b+w+g≠0; and
      D is a drug moiety of formula (I) or (Ia), or a pharmaceutically acceptable salt or ester thereof and is covalently attached via a hydroxy group at OR_{1,} OR₃ or ZH; 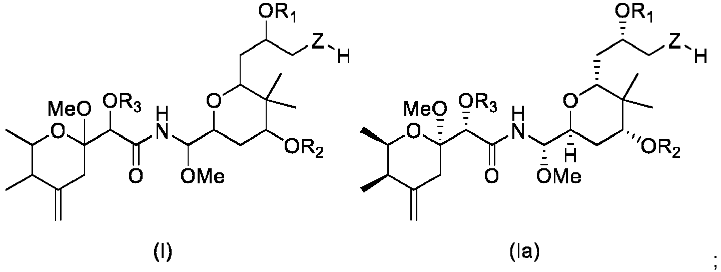
      or
   D is a drug moiety of formula (II) or a formula (Ila), or a pharmaceutically acceptable salt or ester thereof: 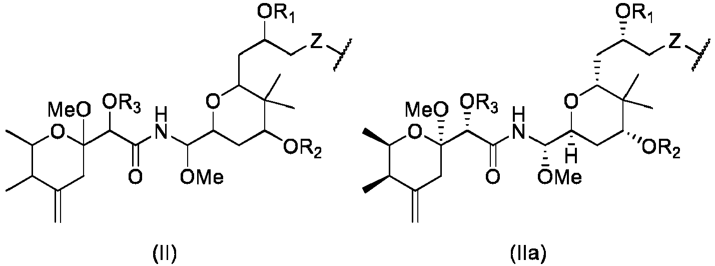
   wherein the wavy lines of (II) and (Ila) indicate the point of covalent attachment to (X)_{b} if any, or (AA)_{w} if any, or to (T)_{g} if any, or to L₁;
   R₁ is hydrogen or methyl, more preferably methyl;
   R₂ is hydrogen;
   R₃ is hydrogen; and
   Z is -O-.
- a compound of formula D-(X)_{b}-(AA)_{w}-(T)_{g}-L₁ or of formula D-(X)_{b}-(AA)_{w}-(T)_{g}-H according to the present invention wherein:
   L₁ is a linker of formula: 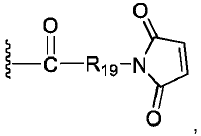 wherein:
   the wavy line indicates the point of covalent attachment to (T)_{g} if any, or (AA)_{w} if any, or to (X)_{b} if any, or to D;
   R₁₉ is -C₂-C₆ alkylene-;
   w is 0 or 2, and where w is 2, then (AA)_{w} is of formula (IV): 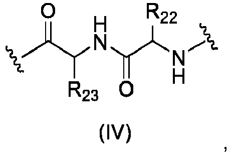
   R₂₂ is isopropyl, R₂₃ is selected from methyl and -(CH₂)₃NHCONH₂, wherein the wavy lines indicate the point of covalent attachments to (X)_{b}, if any, or D (the wavy line to the left) and to (T)_{g} if any, or L₁ or to a hydrogen atom (the wavy line to the right);
   X is an extending group selected from -COO-CH₂-phenylene-NH-, -CONH(CH₂)₃NHCOO-CH₂-phenylene-NH, -CONH-(CH₂)₃)NH-, -CONH(CH₂)₃-S-, and -CONH-(CH₂)₃NHCO-(CH₂)₂S-;
   wherein T is an extending group selected from -CO-(C₁-C₄ alkylene)-NH-, -CO-(C₁-C₄ alkylene)-[O-(C₂-C₄ alkylene)]ⱼ-NH-, and -COO-(C₁-C₄ alkylene)-[O-(C₂-C₄ alkylene)]ⱼ-NH-, where j is an integer from 1 to 5;
   b is 0 or 1, preferably 1;
   g is 0 or 1; preferably 0;
   wherein if the compound is a compound of formula D-(X)_{b}-(AA)_{w}-(T)_{g}-H then b+w+g≠0; and
   D is a drug moiety selected from: 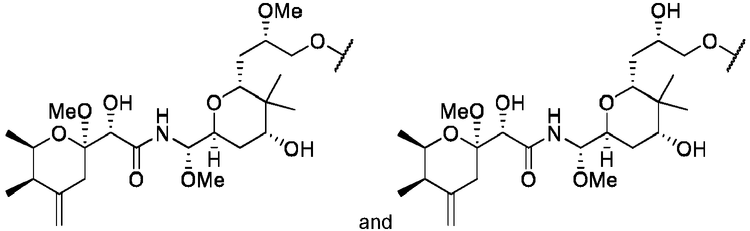
   or a pharmaceutically acceptable salt or ester thereof; wherein the wavy line indicates the point of covalent attachment to (X)_{b} if any, or (AA)_{w} if any, or to (T)_{g} if any, or to L₁.
- a compound of formula D-(X)_{b}-(AA)_{w}-(T)_{g}-L₁ or of formula D-(X)_{b}-(AA)_{w}-(T)_{g}-H according to the present invention wherein:
   L₁ is a group of formula: 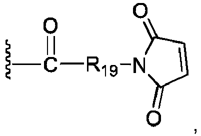 wherein:
   the wavy line indicates the point of covalent attachment to (T)_{g} if any, or (AA)_{w} if any, or to (X)_{b}, if any or to D;
   R₁₉ is a -C₂-C₅ alkylene-;
   w is 0 or 2, and where w is 2, then (AA)_{w} is of formula (IV): 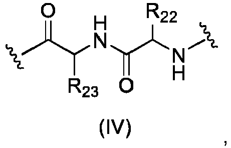
   wherein R₂₂ is isopropyl, R₂₃ is selected from methyl and -(CH₂)₃NHCONH₂, wherein the wavy lines indicate the point of covalent attachments to (X)_{b} (the wavy line to the left) and to (T)_{g} if any, or L₁ or to a hydrogen atom (the wavy line to the right);
   X is -CONH-(C₃ alkylene)NH- or -CONH-(C₃ alkylene)NH-COO-CH₂-phenylene-NH-;
   T is a -CO-(CH₂)₂-[O-(CH₂)₂]₄-NH- group;
   b is 1;
   g is 0 or 1, preferably 0;
   wherein if the compound is a compound of formula D-(X)_{b}-(AA)_{w}-(T)_{g}-H then b+w+g≠0; and D is a drug moiety selected from: 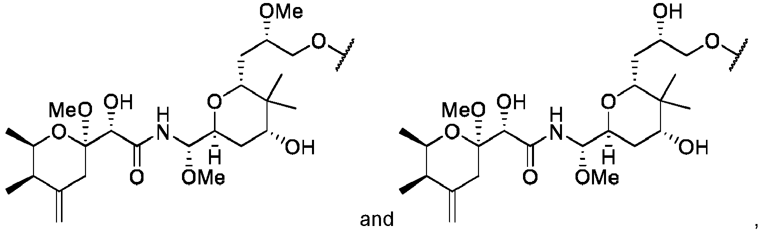
   or a pharmaceutically acceptable salt or ester thereof; wherein the wavy line indicates the point of covalent attachment to (X)_{b}.
- a compound of formula D-(X)_{b}-(AA)_{w}-(T)_{g}-L₁ selected from: 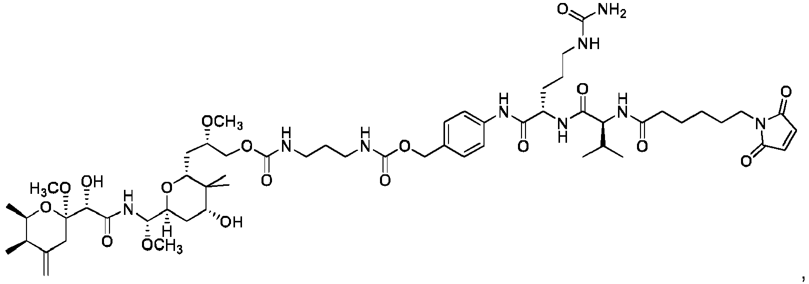 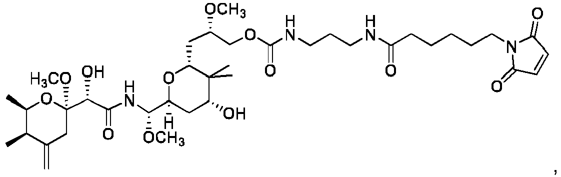 and

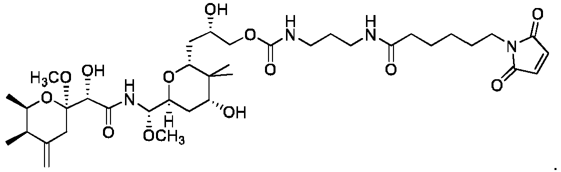

The term "pharmaceutically acceptable salts or esters" in the drug conjugates of the present invention refers to any pharmaceutically acceptable salt, ester, solvate, hydrate or stereosiomeric form or any other compound which, upon administration to the patient is capable of providing a compound as described herein, whether directly or indirectly. However, it will be appreciated that non-pharmaceutically acceptable salts also fall within the scope of the invention since those may be useful in the preparation of pharmaceutically acceptable salts. The preparation of salts, prodrugs and derivatives can be carried out by methods known in the art.

For instance, pharmaceutically acceptable salts of compounds provided herein are synthesized from the parent compound, which contains a basic or acidic moiety, by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, trifluoroacetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium and ammonium salts, and organic alkali salts such as, for example, ethylenediamine, ethanolamine, N,N-dialkylenethanolamine, triethanolamine and basic aminoacids salts.

The drug conjugates of the present invention may be in crystalline form either as free compounds or as solvates (e.g. hydrates) and it is intended that both forms are within the scope of the present invention. Methods of solvation are generally known within the art.

Any compound that is a prodrug of the drug conjugate of the present invention is within the scope and spirit of the invention. The term "prodrug" is used in its broadest sense and encompasses those derivatives that are converted *in vivo* to the compounds of the invention. Such derivatives would readily occur to those skilled in the art, and include, for example, compounds where a free hydroxy group is converted into an ester derivative. Many suitable prodrugs are well-known to the person in the art and can be found, for example, in Burger "Medicinal Chemistry and Drug Discovery 6th ed. (Donald J. Abraham ed., 2001, Wiley) and "Design and Applications of Prodrugs" (H. Bundgaard ed., 1985, Harwood Academic Publishers), the contents of which are incorporated herein by reference.

In relations to the compounds of the present invention, the pharmacologically acceptable esters are not particularly restricted, and can be selected by a person with an ordinary skill in the art. In the case of said esters, it is preferable that such esters can be cleaved by a biological process such as hydrolysis *in vivo.* The group constituting the said esters (the group shown as R when the esters thereof are expressed as -COOR) can be, for example, a C₁-C₄ alkoxy C₁-C₄ alkyl group such as methoxyethyl, 1-ethoxyethyl, 1-methyl-1-methoxyethyl, 1-(isopropoxy)ethyl, 2-methoxyethyl, 2-ethoxyethyl, 1,1-dimethyl-1-methoxymethyl, ethoxymethyl, propoxymethyl, isopropoxymethyl, butoxymethyl or t-butoxymethyl; a C₁-C₄ alkoxylated C₁-C₄ alkoxy C₁-C₄ alkyl group such as 2-methoxyethoxymethyl; a C₆-C₁₀ aryloxy C₁-C₄ alkyl group such as phenoxymethyl; a halogenated C₁-C₄ alkoxy C₁-C₄ alkyl group such as 2,2,2-trichloroethoxymethyl or bis(2-chloroethoxy)methyl; a C₁-C₄ alkoxycarbonyl C₁-C₄ alkyl group such as methoxycarbonylmethyl; a cyano C₁-C₄ alkyl group such as cyanomethyl or 2-cyanoethyl; a C₁-C₄ alkylthiomethyl group such as methylthiomethyl or ethylthiomethyl; a C₆-C₁₀ arylthiomethyl group such as phenylthiomethyl or naphthylthiomethyl; a C₁-C₄ alkylsulfonyl C₁-C₄ lower alkyl group, which may be optionally substituted with a halogen atom(s) such as 2-methanesulfonylethyl or 2-trifluoromethanesulfonylethyl; a C₆-C₁₀ arylsulfonyl C₁-C₄ alkyl group such as 2-benzenesulfonylethyl or 2-toluenesulfonylethyl; a C₁-C₇ aliphatic acyloxy C₁-C₄ alkyl group such as formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 2-formyloxyethyl, 2-acetoxyethyl, 2-propionyloxyethyl, 2-butyryloxyethyl, 2-pivaloyloxyethyl, 2-valeryloxyethyl, 2-isovaleryloxyethyl, 2-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl or 1-pivaloyloxyhexyl; a C₅-C₆ cycloalkylcarbonyloxy C₁-C₄ alkyl group such as cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl or 1-cyclohexylcarbonyloxybutyl; a C₆-C₁₀ arylcarbonyloxy C₁-C₄ alkyl group such as benzoyloxymethyl; a C₁-C₆ alkoxycarbonyloxy C₁-C₄ alkyl group such as methoxycarbonyloxymethyl, 1-(methoxycarbonyloxy)ethyl, 1-(methoxycarbonyloxy)propyl, 1-(methoxycarbonyloxy)butyl, 1-(methoxycarbonyloxy)pentyl, 1-(methoxycarbonyloxy)hexyl, ethoxycarbonyloxymethyl, 1-(ethoxycarbonyloxy)ethyl, 1-(ethoxycarbonyloxy)propyl, 1-(ethoxycarbonyloxy)butyl, 1-(ethoxycarbonyloxy)pentyl, 1-(ethoxycarbonyloxy)hexyl, propoxycarbonyloxymethyl, 1-(propoxycarbonyloxy)ethyl, 1-(propoxycarbonyloxy)propyl, 1-(propoxycarbonyloxy)butyl, isopropoxycarbonyloxymethyl, 1-(isopropoxycarbonyloxy)ethyl, 1-(isopropoxycarbonyloxy)butyl, butoxycarbonyloxymethyl, 1-(butoxycarbonyloxy)ethyl, 1-(butoxycarbonyloxy)propyl, 1-(butoxycarbonyloxy)butyl, isobutoxycarbonyloxymethyl, 1-(isobutoxycarbonyloxy)ethyl, 1-(isobutoxycarbonyloxy)propyl, 1-(isobutoxycarbonyloxy)butyl, t-butoxycarbonyloxymethyl, 1-(t-butoxycarbonyloxy)ethyl, pentyloxycarbonyloxymethyl, 1-(pentyloxycarbonyloxy)ethyl, 1-(pentyloxycarbonyloxy)propyl, hexyloxycarbonyloxymethyl, 1-(hexyloxycarbonyloxy)ethyl or 1-(hexyloxycarbonyloxy)propyl; a C₅-C₆ cycloalkyloxycarbonyloxy C₁-C₄ alkyl group such as cyclopentyloxycarbonyloxymethyl, 1-(cyclopentyloxycarbonyloxy)ethyl, 1-(cyclopentyloxycarbonyloxy)propyl, 1-(cyclopentyloxycarbonyloxy)butyl, cyclohexyloxycarbonyloxymethyl, 1-(cyclohexyloxycarbonyloxy)ethyl, 1-(cyclohexyloxycarbonyloxy)propyl or 1-(cyclohexyloxycarbonyloxy)butyl; a [5-(C₁-C₄ alkyl)-2-oxo-1,3-dioxolen-4-yl]methyl group such as (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl or (5-butyl-2-oxo-1,3-dioxolen-4-yl)methy; a [5-(phenyl, which may be optionally substituted with a C₁-C₄ alkyl, C₁-C₄ alkoxy or halogen atom(s))-2-oxo-1,3-dioxolen-4-yl]methyl group such as (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl or [5-(4-chlorophenyl)-2-oxo-1,3-dioxolen-4-yl]methyl; or a phthalidyl group, which may be optionally substituted with a C₁-C₄ alkyl or C₁-C₄ alkoxy group(s), such as phthalidyl, dimethylphthalidyl or dimethoxyphthalidyl, and is preferably a pivaloyloxymethyl group, phthalidyl group or (5-methyl-2-oxo-1,3-dioxolen-4-yl)methyl group, and more preferably a (5-methyl-2-oxo-1,3-dioxolen-4-yl)methyl group.

Any compound referred to herein is intended to represent such specific compound as well as certain variations or forms. In particular, compounds referred to herein may have asymmetric centres and therefore exist in different enantiomeric forms. All optical isomers and stereoisomers of the compounds referred to herein, and mixtures thereof, are considered within the scope of the present invention. Thus any given compound referred to herein is intended to represent any one of a racemate, one or more enantiomeric forms, one or more diastereomeric forms, one or more atropisomeric forms, and mixtures thereof. Particularly, the drug conjugates of formula [D-(X)_{b}-(AA)_{w}-(T)_{g}-(L)]ₙ-Ab and compounds of formula D-(X)_{b}-(AA)_{w}-(T)_{g}-L₁ or D-(X)_{b}-(AA)_{w}-(T)_{g}-H may include enantiomers depending on their asymmetry or diastereoisomers. Stereoisomerism about the double bond is also possible, therefore in some cases the molecule could exist as (E)-isomer or (Z)-isomer. If the molecule contains several double bonds, each double bond will have its own stereoisomerism, that could be the same or different than the stereoisomerism of the other double bonds of the molecule. The single isomers and mixtures of isomers fall within the scope of the present invention.

Furthermore, compounds referred to herein may exist as geometric isomers (i.e., *cis* and *trans* isomers), as tautomers, or as atropisomers. Specifically, the term tautomer refers to one of two or more structural isomers of a compound that exist in equilibrium and are readily converted from one isomeric form to another. Common tautomeric pairs are amine-imine, amide-imide, keto-enol, lactam-lactim, etc. Additionally, any compound referred to herein is intended to represent hydrates, solvates, and polymorphs, and mixtures thereof when such forms exist in the medium. In addition, compounds referred to herein may exist in isotopically-labelled forms. All geometric isomers, tautomers, atropisomers, hydrates, solvates, polymorphs, and isotopically labelled forms of the compounds referred to herein, and mixtures thereof, are considered within the scope of the present invention.

Protected forms of the compounds disclosed herein are considered within the scope of the present invention. Suitable protecting groups are well known for the skilled person in the art. A general review of protecting groups in organic chemistry is provided by Wuts, PGM and Greene TW in Protecting Groups in Organic Synthesis, 4th Ed. Wiley-Interscience, and by Kocienski PJ in Protecting Groups, 3rd Ed. Georg Thieme Verlag. These references provide sections on protecting groups for OH, amino and SH groups. All these references are incorporated by reference in their entirety.

Within the scope of the present invention an OH protecting group is defined to be the O-bonded moiety resulting from the protection of the OH through the formation of a suitable protected OH group. Examples of such protected OH groups include ethers, silyl ethers, esters, sulfonates, sulfenates and sulfinates, carbonates, and carbamates. In the case of ethers the protecting group for the OH can be selected from methyl, methoxymethyl, methylthiomethyl, (phenyldimethylsilyl)methoxymethyl, benzyloxymethyl, *p-*methoxybenzyloxymethyl, [(3,4-dimethoxybenzyl)oxy]methyl, *p*-nitrobenzyloxymethyl, *o-*nitrobenzyloxymethyl, [(*R*)-1-(2-nitrophenyl)ethoxy]methyl, (4-methoxyphenoxy)methyl, guaiacolmethyl, [(*p*-phenylphenyl)oxy]methyl, *t*-butoxymethyl, 4-pentenyloxymethyl, siloxymethyl, 2-methoxyethoxymethyl, 2-cyanoethoxymethyl, bis(2-chloroethoxy)methyl, 2,2,2-trichloroethoxymethyl, 2-(trimethylsilyl)ethoxymethyl, menthoxymethyl, *O*-bis(2-acetoxy-ethoxy)methyl, tetrahydropyranyl, fluorous tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, 4-methoxytetrahydrothiopyranyl S,S-dioxide, 1-[(2-chloro-4-methyl)-phenyl]-4-methoxypiperidin-4-yl, 1-(2-fluorophenyl)-4-methoxypiperidin-4-yl, 1-(4-chlorophenyl)-4-methoxypiperidin-4-yl, 1,4-dioxan-2-yl, tetrahydrofuranyl, tetrahydrothiofuranyl, 2,3,3*a*,4,5,6,7,7*a*-octahydro-7,8,8-trimethyl-4,7-methanobenzofuran-2-yl, 1-ethoxyethyl, 1-(2-chloroethoxy)ethyl, 2-hydroxyethyl, 2-bromoethyl, 1-[2-(trimethylsilyl)ethoxy]ethyl, 1-methyl-1-methoxyethyl, 1-methyl-1-benzyloxyethyl, 1-methyl-1-benzyloxy-2-fluoroethyl, 1-methyl-1-phenoxyethyl, 2,2,2-trichloroethyl, 1,1-dianisyl-2,2,2-trichloroethyl, 1,1,1,3,3,3-hexafluoro-2-phenylisopropyl, 1-(2-cyanoethoxy)ethyl, 2-trimethylsilylethyl, 2-(benzylthio)ethyl, 2-(phenylselenyl)ethyl, *t*-butyl, cyclohexyl, 1-methyl-1'-cyclopropylmethyl, allyl, prenyl, cinnamyl, 2-phenallyl, propargyl, *p*-chlorophenyl, *p-*methoxyphenyl, *p*-nitrophenyl, 2,4-dinitrophenyl, 2,3,5,6-tetrafluoro-4-(trifluoromethyl)phenyl, benzyl, *p*-methoxybenzyl, 3,4-dimethoxybenzyl, 2,6-dimethoxybenzyl, *o*-nitrobenzyl, *p-*nitrobenzyl, pentadienylnitrobenzyl, pentadienylnitropiperonyl, halobenzyl, 2,6-dichlorobenzyl, 2,4-dichlorobenzyl, 2,6-difluorobenzyl, *p*-cyanobenzyl, fluorous benzyl, 4-fluorousalkoxybenzyl, trimethylsilylxylyl, *p*-phenylbenzyl, 2-phenyl-2-propyl, *p-*acylaminobenzyl, *p*-azidobenzyl, 4-azido-3-chlorobenzyl, 2-trifluoromethylbenzyl, 4-trifluoromethylbenzyl, *p*-(methylsulfinyl)benzyl, *p*-siletanylbenzyl, 4-acetoxybenzyl, 4-(2-trimethylsilyl)ethoxymethoxybenzyl, 2-naphthylmethyl, 2-picolyl, 4-picolyl, 3-methyl-2-picolyl *N*-oxide, 2-quinolinylmethyl, 6-methoxy-2-(4-methylphenyl)-4-quinolinemethyl, 1-pyrenylmethyl, diphenylmethyl, 4-methoxydiphenylmethyl, 4-phenyldiphenylmethyl, *p,p'-*dinitrobenzhydryl, 5-dibenzosuberyl, triphenylmethyl, tris(4-*t*-butylphenyl)methyl, α-naphthyldiphenylmethyl, *p*-methoxyphenyldiphenylmethyl, di(*p*-methoxyphenyl)phenyl-methyl, tri(*p*-methoxyphenyl)methyl, 4-(4'-bromophenacyloxy)phenyldiphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 4,4'-dimethoxy-3"-[*N*-(imidazolylmethyl)]trityl, 4,4'-dimethoxy-3"-[*N*-(imidazolylethyl)carbamoyl]trityl, bis(4-methoxyphenyl)-1'-pyrenylmethyl, 4-(17-tetrabenzo[*a,c,g,i*]fluorenylmethyl)-4,4"-dimethoxytrityl, 9-anthryl, 9-(9-phenyl)xanthenyl, 9-phenylthioxanthyl, 9-(9-phenyl-10-oxo)anthryl, 1,3-benzodithiolan-2-yl, 4,5-bis(ethoxycarbonyl)-[1,3]-dioxolan-2-yl, benzisothiazolyl *S,S*-dioxide. In the case of silyl ethers the protecting group for the OH can be selected from trimethylsilyl, triethylsilyl, triisopropylsilyl, dimethylisopropylsilyl, diethylisopropylsilyl, dimethylhexylsilyl, 2-norbornyldimethylsilyl, *t*-butyldimethylsilyl, *t*-butyldiphenylsilyl, tribenzylsilyl, tri-*p*-xylylsilyl, triphenylsilyl, diphenylmethylsilyl, di-*t*-butylmethylsilyl, bis(*t*-butyl)-1-pyrenylmethoxysilyl, tris(trimethylsilyl)silyl, (2-hydroxystyryl)dimethylsilyl, (2-hydroxystyryl)diisopropylsilyl, *t-*butylmethoxyphenylsilyl, *t*-butoxydiphenylsilyl, 1,1,3,3-tetraisopropyl-3-[2-(triphenylmethoxy) ethoxy]disiloxane-1-yl, and fluorous silyl. In the case of esters the protecting group for the OH together with the oxygen atom of the unprotected OH to which it is attached form an ester that can be selected from formate, benzoylformate, acetate, chloroacetate, dichloroacetate, trichloroacetate, trichloroacetamidate, trifluoroacetate, methoxyacetate, triphenylmethoxyacetate, phenoxyacetate, *p*-chlorophenoxyacetate, phenylacetate, diphenylacetate, 3-phenylpropionate, bisfluorous chain type propanoyl, 4-pentenoate, 4-oxopentanoate, 4,4-(ethylenedithio)pentanoate, 5[3-bis(4-methoxyphenyl)hydro-xymethylphenoxy]levulinate, pivaloate, 1-adamantoate, crotonate, 4-methoxycrotonate, benzoate, *p*-phenylbenzoate, 2,4,6-trimethylbenzoate, 4-bromobenzoate, 2,5-difluorobenzoate, *p*-nitrobenzoate, picolinate, nicotinate, 2-(azidomethyl)benzoate, 4-azido-butyrate, (2-azidomethyl)phenylacetate, 2-{[(tritylthio)oxy]methyl}benzoate, 2-{[(4-methoxytritylthio)oxy]methyl}benzoate, 2-{[methyl(tritylthio)amino]methyl}benzoate, 2-{{[(4-methoxytrityl)thio]methylamino}methyl}benzoate, 2-(allyloxy)phenylacetate, 2-(prenyloxymethyl)benzoate, 6-(levulinyloxymethyl)-3-methoxy-2-nitrobenzoate, 6-(levulinyloxymethyl)-3-methoxy-4-nitrobenzoate, 4-benzyloxybutyrate, 4-trialkylsilyloxy-butyrate, 4-acetoxy-2,2-dimethylbutyrate, 2,2-dimethyl-4-pentenoate, 2-iodobenzoate, 4-nitro-4-methylpentanoate, o-(dibromomethyl)benzoate, 2-formylbenzenesulfonate, 4-(methylthio-methoxy)butyrate, 2-(methylthiomethoxymethyl)benzoate, 2-(chloroacetoxymethyl)benzoate, 2-[(2-chloroacetoxy)ethyl]benzoate, 2-[2-(benzyloxy)ethyl]benzoate, 2-[2-(4-methoxybenzyl-oxy)ethyl]benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenyl-acetate, isobutyrate, monosuccinoate, (*E*)-2-methyl-2-butenoate, *o-*(methoxycarbonyl)benzoate, α-naphthoate, nitrate, alkyl *N,N,N',N'-*tetramethylphosphorodiamidate, and 2-chlorobenzoate. In the case of sulfonates, sulfenates and sulfinates the protecting group for the OH together with the oxygen atom of the unprotected OH to which it is attached form a sulfonate, sulfenate or sulfinates that can be selected from sulfate, allylsulfonate, methanesulfonate, benzylsulfonate, tosylate, 2-[(4-nitrophenyl)ethyl]sulfonate, 2-trifluoromethylbenzenesulfonate, 4-monomethoxytritylsulfenate, alkyl 2,4-dinitrophenylsulfenate, 2,2,5,5-tetramethylpyrrolidin-3-one-1-sulfinate, and dimethylphosphinothioyl. In the case of carbonates the protecting group for the OH together with the oxygen atom of the unprotected OH to which it is attached form a carbonate that can be selected from methyl carbonate, methoxymethyl carbonate, 9-fluorenylmethyl carbonate, ethyl carbonate, bromoethyl carbonate, 2-(methylthiomethoxy)ethyl carbonate, 2,2,2-trichloroethyl carbonate, 1,1-dimethyl-2,2,2-trichloroethyl carbonate, 2-(trimethylsilyl)ethyl carbonate, 2-[dimethyl(2-naphthylmethyl)silyl]ethyl carbonate, 2-(phenylsulfonyl)ethyl carbonate, 2-(triphenylphosphonio)ethyl carbonate, *cis*-[4-[[(methoxytrityl)sulfenyl]oxy]tetrahydrofuran-3-yl]oxy carbonate, isobutyl carbonate, *t*-butyl carbonate, vinyl carbonate, allyl carbonate, cinnamyl carbonate, propargyl carbonate, *p-*chlorophenyl carbonate, *p*-nitrophenyl carbonate, 4-ethoxy-1-naphthyl carbonate, 6-bromo-7-hydroxycoumarin-4-ylmethyl carbonate, benzyl carbonate, *o*-nitrobenzyl carbonate, *p-*nitrobenzyl carbonate, *p*-methoxybenzyl carbonate, 3,4-dimethoxybenzyl carbonate, anthraquinon-2-ylmethyl carbonate, 2-dansylethyl carbonate, 2-(4-nitrophenyl)ethyl carbonate, 2-(2,4-dinitrophenyl)ethyl carbonate, 2-(2-nitrophenyl)propyl carbonate, 2-(3,4-methylenedioxy-6-nitrophenyl)propyl carbonate, 2-cyano-1-phenylethyl carbonate, 2-(2-pyridyl)amino-1-phenylethyl carbonate, 2-[*N*-methyl-*N*-(2-pyridyl)]amino-1-phenylethyl carbonate, phenacyl carbonate, 3',5'-dimethoxybenzoin carbonate, methyl dithiocarbonate, and *S*-benzyl thiocarbonate. And in the case of carbamates the protecting group for OH together with the oxygen atom of the unprotected OH to which it is attached forms a carbamate that can be selected from dimethyl thiocarbamate, *N*-phenyl carbamate, and *N-*methyl-*N*-(*o*-nitrophenyl) carbamate.

Within the scope of the present invention an amino protecting group is defined to be the *N*-bonded moiety resulting from the protection of the amino group through the formation of a suitable protected amino group. Examples of protected amino groups include carbamates, ureas, amides, heterocyclic systems, *N*-alkyl amines, *N*-alkenyl amines, *N*-alkynyl amines, *N-*aryl amines, imines, enamines, N-metal derivatives, N-N derivatives, N-P derivatives, N-Si derivatives, and N-S derivatives. In the case of carbamates the protecting group for the amino group together with the amino group to which it is attached form a carbamate that can be selected from methyl carbamate, ethyl carbamate, 9-fluorenylmethyl carbamate, 2,6-di-*t*-butyl-9-fluorenylmethyl carbamate, 2,7-bis(trimethylsilyl)fluorenylmethyl carbamate, 9-(2-sulfo)fluorenylmethyl carbamate, 9-(2,7-dibromo)fluorenylmethyl carbamate, 17-tetrabenzo[*a*,*c*,*g*,*i*]fluorenylmethyl carbamate, 2-chloro-3-indenylmethyl carbamate, benz[*f*]inden-3-ylmethyl carbamate, 1,1-dioxobenzo[b]-thiophene-2-ylmethyl carbamate, 2-methylsulfonyl-3-phenyl-1-prop-2-enyl carbamate, 2,7-di-*t*-butyl-[9,(10,10-dioxo-10,10,10,10-tetrahydrothioxanthyl)]methyl carbamate, 2,2,2-trichloroethyl carbamate, 2-trimethylsilylethyl carbamate, (2-phenyl-2-trimethylsilyl)ethyl carbamate, 2-phenylethyl carbamate, 2-chloroethyl carbamate, 1,1-dimethyl-2-haloethyl carbamate, 1,1-dimethyl-2,2-dibromoethyl carbamate, 1,1-dimethyl-2,2,2-trichloroethyl carbamate, 2-(2'-pyridyl)ethyl carbamate, 2-(4'-pyridyl)ethyl carbamate, 2,2-bis(4'-nitrophenyl)ethyl carbamate, 2-[(2-nitrophenyl)dithio]-1-phenylethyl carbamate, 2-(*N*,*N*-dicyclohexylcarboxamido)ethyl carbamate, *t*-butyl carbamate, fluorous BOC carbamate, 1-adamantyl carbamate, 2-adamantyl carbamate, 1-(1-adamantyl)-1-methylethyl carbamate, 1-methyl-1-(4-byphenylyl)ethyl carbamate, 1-(3,5-di-*t*-butylphenyl)-1-methylethyl carbamate, triisopropylsilyloxy carbamate, vinyl carbamate, allyl carbamate, prenyl carbamate, 1-isopropylallyl carbamate, cinnamyl carbamate, 4-nitrocinnamyl carbamate, 3-(3'-pyridyl)prop-2-enyl carbamate, hexadienyl carbamate, propargyl carbamate, 1,4-but-2-ynyl biscarbamate, 8-quinolyl carbamate, *N*-hydroxypiperidinyl carbamate, alkyl dithiocarbamate, benzyl carbamate, 3,5-di-*t*-butylbenzyl carbamate, *p*-methoxybenzyl carbamate, *p*-nitrobenzyl carbamate, *p*-bromobenzyl carbamate, *p*-chlorobenzyl carbamate, 2,4-dichlorobenzyl carbamate, 4-methylsulfinylbenzyl carbamate, 4-trifluoromethylbenzyl carbamate, fluorous benzyl carbamate, 2-naphthylmethyl carbamate, 9-anthrylmethyl carbamate, diphenylmethyl carbamate, 4-phenylacetoxybenzyl carbamate, 4-azidobenzyl carbamate, 4-azido-methoxybenzyl carbamate, *m*-chloro*-p*-acyloxybenzyl carbamate, *p-*(dihydroxyboryl)-benzyl carbamate, 5-benzisoxazolylmethyl carbamate, 2-(trifluoromethyl)-6-chromonylmethyl carbamate, 2-methylthioethyl carbamate, 2-methylsulfonylethyl carbamate, 2-(*p*-toluenesulfonyl)ethyl carbamate, 2-(4-nitrophenylsulfonyl)ethyl carbamate, 2-(2,4-dinitrophenylsulfonyl)ethyl carbamate, 2-(4-trifluoromethylphenylsulfonyl)ethyl carbamate, [2-(1,3-dithianyl)]methyl carbamate, 2-phosphonioethyl carbamate, 2-[phenyl(methyl)sulfonio]ethyl carbamate, 1-methyl-1-(triphenylphosphonio)ethyl carbamate, 1,1-dimethyl-2-cyanoethyl carbamate, 2-dansylethyl carbamate, 2-(4-nitrophenyl)ethyl carbamate, 4-methylthiophenyl carbamate, 2,4-dimethylthiophenyl carbamate, *m*-nitrophenyl carbamate, 3,5-dimethoxybenzyl carbamate, 1-methyl-1-(3,5-dimethoxyphenyl)ethyl carbamate, *α*-methylnitropiperonyl carbamate, *o*-nitrobenzyl carbamate, 3,4-dimethoxy-6-nitrobenzyl carbamate, phenyl(*o*-nitrophenyl)methyl carbamate, 2-nitrophenylethyl carbamate, 6-nitroveratryl carbamate, 4-methoxyphenacyl carbamate, 3',5'-dimethoxybenzoin carbamate, 9-xanthenylmethyl carbamate, *N*-methyl-*N*-(*o*-nitrophenyl) carbamate, *t*-amyl carbamate, 1-methylcyclobutyl carbamate, 1-methylcyclohexyl carbamate, 1-methyl-1-cyclopropylmethyl carbamate, cyclobutyl carbamate, cyclopentyl carbamate, cyclohexyl carbamate, isobutyl carbamate, isobornyl carbamate, cyclopropylmethyl carbamate, *p*-decyloxybenzyl carbamate, diisopropylmethyl carbamate, 2,2-dimethoxy-carbonylvinyl carbamate, *o-*(*N,N-*dimethylcarboxamido)benzyl carbamate, 1,1-dimethyl-3-(*N*,*N*-dimethyl-carboxamido)propyl carbamate, butynyl carbamate, 1,1-dimethylpropynyl carbamate, 2-iodoethyl carbamate, 1-methyl-1-(4'-pyridyl)ethyl carbamate, 1-methyl-1-(*p*-phenylazophenyl)ethyl carbamate, *p*-(*p*'-methoxyphenylazo)benzyl carbamate, *p*-(phenylazo)benzyl carbamate, 2,4,6-trimethylbenzyl carbamate, isonicotinyl carbamate, 4-(trimethyl-ammonium)benzyl carbamate, *p*-cyanobenzyl carbamate, di(2-pyridyl)methyl carbamate, 2-furanylmethyl carbamate, phenyl carbamate, 2,4,6-tri*-t*-butylphenyl carbamate, 1-methyl-1-phenylethyl carbamate, and *S*-benzyl thiocarbamate. In the case of ureas the protecting groups for the amino group can be selected from phenothiazinyl-(10)-carbonyl, *N*'-*p*-toluenesulfonylaminocarbonyl, *N'-*phenylaminothiocarbonyl, 4-hydroxyphenylaminocarbonyl, 3-hydroxytryptaminocarbonyl, and *N'*-phenylaminothiocarbonyl. In the case of amides the protecting group for the amino together with the amino group to which it is attached form an amide that can be selected from formamide, acetamide, chloroacetamide, trichloroacetamide, trifluoroacetamide, phenylacetamide, 3-phenylpropanamide, pent-4-enamide, picolinamide, 3-pyridylcarboxamide, *N*-benzoylphenylalanyl amide, benzamide, *p*-phenylbenzamide, *o-*nitrophenylacetamide, 2,2-dimethyl-2-(*o*-nitrophenyl)acetamide, *o*-nitrophenoxyacetamide, 3-(*o*-nitrophenyl)propanamide, 2-methyl-2-(*o*-nitrophenoxy)propanamide, 3-methyl-3-nitrobutanamide, *o*-nitrocinnamide, *o*-nitrobenzamide, 3-(4-*t*-butyl-2,6-dinitrophenyl)-2,2-dimethylpropanamide, *o*-(benzoyloxyme-thyl)benzamide, 2-(acetoxymethyl)benzamide, 2-[(*t-*butyldiphenylsiloxy)methyl]benzamide, 3-(3',6'-dioxo-2',4',5'-trimethylcyclohexa-1',4'-diene)-3,3-dimethylpropionamide, o-hydroxy-*trans*-cinnamide, 2-methyl-2-(*o-*phenylazophenoxy)propanamide, 4-chlorobutanamide, aceto-acetamide, 3-(*p-*hydroxyphenyl)propanamide, (*N*'-dithiobenzyloxycarbonylamino)acetamide, and *N-*acetylmethionine amide. In the case of heterocyclic systems the protecting group for the amino group together with the amino group to which it is attached form a heterocyclic system that can be selected from 4,5-diphenyl-3-oxazolin-2-one, *N*-phthalimide, *N-*dichlorophthalimide, *N*-tetrachlorophthalimide, *N*-4-nitrophthalimide, *N*-thiodiglycoloyl, *N-*dithiasuccinimide, *N*-2,3-diphenylmaleimide, *N*-2,3-dimethylmaleimide, *N*-2,5-dimethylpyrrole, *N*-2,5-bis(triisopropylsiloxy)pyrrole, *N*-1,1,4,4-tetramethyldisilylazacyclopentane adduct, *N-*1,1,3,3-tetramethyl-1,3-disilaisoindoline, *N*-diphenylsilyldiethylene, *N*-5-substituted-1,3-dimethyl-1,3,5-triazacyclohexan-2-one, *N*-5-substituted-1,3-benzyl-1,3,5-triazacyclohexan-2-one, 1-substituted 3,5-dinitro-4-pyridone, and 1,3,5-dioxazine. In the case of *N*-alkyl, *N-*alkenyl, *N*-alkynyl or *N*-aryl amines the protecting group for the amino group can be selected from *N*-methyl, *N*-t-butyl, *N*-allyl, *N*-prenyl, *N*-cinnamyl, *N*-phenylallyl, *N*-propargyl, *N-*methoxymethyl, *N*-[2-(trimethylsilyl)ethoxy]methyl, *N*-3-acetoxypropyl, *N*-cyanomethyl, *N*-2-azanorbornenes, *N*-benzyl, *N*-4-methoxybenzyl, *N*-2,4-dimethoxybenzyl, *N*-2-hydroxybenzyl, *N*-ferrocenylmethyl, *N*-2,4-dinitrophenyl, *o*-methoxyphenyl, *p*-methoxyphenyl, *N*-9-phenylfluorenyl, *N*-fluorenyl, *N*-2-picolylamine N'-oxide, N*-*7-methoxycoumar-4-ylmethyl, *N-*diphenylmethyl, *N*-bis(4-methoxyphenyl)methyl, *N*-5-dibenzosuberyl, *N*-triphenylmethyl, *N*-(4-methylphenyl)diphenylmethyl, and *N*-(4-methoxyphenyl)diphenylmethyl. In the case of imines the protecting group for the amino group can be selected from *N*-1,1-dimethylthiomethylene, N-benzylidene, *N*-*p*-methoxybenzylidene, *N*-diphenylmethylene, *N-*[2-pyridyl)mesityl]methylene, *N*-(*N*',*N*'-dimethylaminomethylene), *N-(N',N'-*dibenzylaminomethylene), *N*-(*N*'-*t*-butylaminome-thylene), *N*,*N*'-isopropylidene, *N-p-*nitrobenzylidene, *N*-salicylidene, *N*-5-chlorosalicylidene, *N*-(5-chloro-2-hydroxyphenyl)phenylmethylene, *N*-cyclohexylidene, and *N*-*t*-butylidene. In the case of enamines the protecting group for the amino group can be selected from *N*-(5,5-dimethyl-3-oxo-1-cyclohexenyl), *N*-2,7-dichloro-9-fluorenylmethylene, *N*-1-(4,4-dimethyl-2,6-dioxocyclohexylidene)ethyl, *N*-(1,3-dimethyl-2,4,6-(1*H*,3*H*,5*H*)-trioxopyrimidine-5-ylidene)-methyl, *N*-4,4,4-trifluoro-3-oxo-1-butenyl, and *N*-(1-isopropyl-4-nitro-2-oxo-3-pyrrolin-3-yl). In the case of N-metal derivatives the protecting group for the amino group can be selected from N-borane, *N*-diphenylborinic ester, *N*-diethylborinic ester, *N*-9-borabicyclononane, *N-*difluoroborinic ester, and 3,5-bis(trifluoromethyl)phenylboronic acid; and also including *N-*phenyl(pentacarbonylchromium)carbenyl, *N*-phenyl(pentacarbonyl-tungsten)carbenyl, *N-*methyl(pentacarbonylchromium)carbenyl, *N*-methyl(pentacarbonyltungsten)carbenyl, *N-*copper chelate, *N*-zinc chelate, and a 18-crown-6-derivative. In the case of N-N derivatives the protecting group for the amino group together with the amino group to which it is attached form a N-N derivative that can be selected from *N*-nitroamino, *N*-nitrosoamino, amine *N-*oxide, azide, triazene derivative, and *N*-trimethylsilylmethyl-*N*-benzylhydrazine. In the case of N-P derivatives the protected group for the amino group together with the amino group to which it is attached form a N-P derivative that can be selected from diphenylphosphinamide, dimethylthiophosphinamide, diphenylthiophosphinamide, dialkyl phosphoramidate, dibenzyl phosphoramidate, diphenyl phosphoramidate, and iminotriphenylphosphorane. In the case of N-Si derivatives the protecting group for the NH₂ can be selected from *t*-butyldiphenylsilyl and triphenylsilyl. In the case of N-S derivatives the protected amino group can be selected from N-sulfenyl or N-sulfonyl derivatives. The N-sulfenyl derivatives can be selected from benzenesulfenamide, 2-nitrobenzenesulfenamide, 2,4-dinitrobenzenesulfenamide, pentachlorobenzenesulfenamide, 2-nitro-4-methoxybenzenesulfenamide, triphenylmethylsulfe-namide, 1-(2,2,2-trifluoro-1,1-diphenyl)ethylsulfenamide, and *N*-3-nitro-2-pyridinesulfenamide. The N-sulfonyl derivatives can be selected from methanesulfonamide, trifluoromethanesulfonamide, *t*-butylsulfonamide, benzylsulfonamide, 2-(trimethylsilyl) ethanesulfonamide, *p*-toluenesulfonamide, benzenesulfonamide, o-anisylsulfonamide, 2-nitrobenzenesulfonamide, 4-nitrobenzenesulfonamide, 2,4-dinitrobenzenesulfonamide, 2-naphthalenesulfonamide, 4-(4',8'-dimethoxynaphthylmethyl)benzenesulfonamide, 2-(4-methylphenyl)-6-methoxy-4-methylsulfonamide, 9-anthracenesulfonamide, pyridine-2-sulfonamide, benzothiazole-2-sulfonamide, phenacylsulfonamide, 2,3,6-trimethyl-4-methoxybenzenesulfonamide, 2,4,6-trimethoxybenzenesulfonamide, 2,6-dimethyl-4-methoxybenzenesulfonamide, pentamethylbenzenesulfonamide, 2,3,5,6-tetramethyl-4-methoxybenzenesulfonamide, 4-methoxybenzenesulfonamide, 2,4,6-trimethylbenzenesulfonamide, 2,6-dimethoxy-4-methylbenzenesulfonamide, 3-methoxy-4-t-butylbenzenesulfonamide, and 2,2,5,7,8-pentamethylchroman-6-sulfonamide.

Within the scope of the present invention a protecting group for SH is defined to be the S-bonded moiety resulting from the protection of the SH group through the formation of a suitable a protected SH group. Examples of such protected SH groups include thioethers, disulfides, silyl thioethers, thioesters, thiocarbonates, and thiocarbamates. In the case of thioethers the protecting group for the SH can be selected from *S*-alkyl, *S*-benzyl, *S-p-*methoxybenzyl, *S-o*-hydroxybenzyl, *S-p*-hydroxybenzyl, *S-o*-acetoxybenzyl, *S-p-*acetoxybenzyl, *S-p*-nitrobenzyl, *S*-o-nitrobenzyl, *S*-2,4,6-trimethylbenzyl, *S*-2,4,6,-trimethoxybenzyl, *S*-4-picolyl, *S*-2-picolyl-*N*-oxide, *S*-2-quinolinylmethyl, *S*-9-anthrylmethyl, *S-*9-fluorenylmethyl, *S-*xanthenyl, *S*-ferrocenylmethyl, *S*-diphenylmethyl, *S*-bis(4-methoxyphenyl)methyl, *S*-5-dibenzosuberyl, *S*-triphenylmethyl, 4-methoxytrityl, *S*-diphenyl-4-pyridylmethyl, *S*-phenyl, *S*-2,4-dinitrophenyl, *S*-2-quinolyl, *S*-t-butyl, *S*-1-adamantyl, *S-*methoxymethyl, *S*-isobutoxymethyl, *S*-benzyloxymethyl, *S*-1-ethoxyethyl, *S-*2-tetrahydropyranyl, *S*-benzylthiomethyl, *S*-phenylthiomethyl, *S*-acetamidomethyl (Acm), *S-*trimethylacetamidomethyl, *S*-benzamidomethyl, *S*-allyloxycarbonylaminomethyl, *S-N*-[2,3,5,6-tetrafluoro-4-(*N*'-piperidino)-phenyl-*N*-allyloxycarbonylaminomethyl, *S-*phthalimidomethyl, *S-*phenylacetamidomethyl, *S*-acetylmethyl, *S*-carboxymethyl, *S*-cyanomethyl, *S*-(2-nitro-1-phenyl)ethyl, *S*-2-(2,4-dinitrophenyl)ethyl, *S*-2-(4'-pyridyl)ethyl, *S*-2-cyanoethyl, *S*-2-(trimethylsilyl)ethyl, *S*-2,2-bis(carboethoxy)ethyl, *S*-(1-m-nitrophenyl-2-benzoyl)ethyl, *S*-2-phenylsulfonylethyl, *S*-1-(4-methylphenylsulfonyl)-2-methylprop-2-yl, and *S-p-*hydroxyphenacyl. In the case of disulfides the protected SH group can be selected from *S-*ethyl disulfide, *S-t*-butyl disulfide, *S*-2-nitrophenyl disulfide, *S*-2,4-dinitrophenyl disulfide, *S*-2-phenylazophenyl disulfide, *S*-2-carboxyphenyl disulfide, and *S*-3-nitro-2-pyridyl disulfide. In the case of silyl thioethers the protecting group for the SH can be selected from the list of groups that was listed above for the protection of OH with silyl ethers. In the case of thioesters the protecting group for the SH can be selected from *S*-acetyl, *S*-benzoyl, *S*-2-methoxyisobutyryl, *S*-trifluoroacetyl, *S*-*N*-[[*p*-biphenylyl)-isopropyloxy]carbonyl]-*N*-methyl-γ-aminothiobutyrate, and *S*-*N*-(*t*-butoxycarbonyl)-*N*-methyl-γ-aminothiobutyrate. In the case of thiocarbonate protecting group for the SH can be selected from *S*-2,2,2-trichloroethoxycarbonyl, *S-t*-butoxycarbonyl, *S*-benzyloxycarbonyl, *S-p-*methoxybenzyloxycarbonyl, and S-fluorenylmethylcarbonyl. In the case of thiocarbamate the protected SH group can be selected from *S*-(*N*-ethylcarbamate) and *S*-(*N-*methoxymethylcarbamate).

The mention of these groups should not be interpreted as a limitation of the scope of the invention, since they have been mentioned as a mere illustration of protecting groups for OH, amino and SH groups, but further groups having said function may be known by the skilled person in the art, and they are to be understood to be also encompassed by the present invention.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

"Antibody-drug-conjugates (ADCs)" represent a targeted strategy to deliver a cytotoxic molecule to a cancer cell. (see, for example, International Patent Applications WO-A-2004/010957, WO-A-2006/060533 and WO-A-2007/024536). Such compounds are typically referred to as drug, toxin and radionuclide "conjugates". Tumor cell killing occurs upon binding of the drug conjugate to a tumor cell and release and/or activation of the cytotoxic activity of the drug moiety. The selectivity afforded by drug conjugates minimizes toxicity to normal cells, thereby enhancing tolerability of the drug in the patient. Three examples of drug antibody conjugates of this type that have received marketing approval are: Gemtuzumab ozogamicin for acute myelogenous leukemia, Brentuximab vedotin for relapsed and refractory Hodgkin lymphoma and anaplastic large cell lymphoma, and ado-Trastuzumab emtansine for breast cancer, especially HER2+.

The effectiveness of drugs for cancer chemotherapy generally relies on differences in growth rates, biochemical pathways, and physiological characteristics between cancer and normal tissues. Consequently, most standard chemotherapeutics are relatively nonspecific and exhibit dose-limiting toxicities that contribute to suboptimal therapeutic effects. One approach to selectively target malignant cells and not healthy tissues is to use specific monoclonal antibodies (mAbs) that recognize tumor-associated antigens expressed on the surface of tumor cells [Meyer, D.L. & Senter, P.D. (2003) Recent advances in antibody drug conjugates for cancer therapy. Annu. Rep. Med. Chem., 38, 229-237; Chari, R.V. (2008) Targeted cancer therapy: conferring specificity to cytotoxic drugs. Acc. Chem. Res. 41, 98-107]. More than 30 G-type immunoglobulins (IgG) and related agents have been approved over the past 25 years mainly for cancers and inflammatory diseases.

An alternative strategy is to look to chemically conjugate small anti-neoplastic molecules to mAbs, used both as carriers (increased half-life) and as targeting agents (selectivity). Considerable effort has been directed toward the use of monoclonal antibodies (mAbs) for targeted drug delivery due to their high selectivities for tumor-associated antigens, favorable pharmacokinetics, and relatively low intrinsic toxicities. The mAb-drug conjugates (ADCs) are formed by covalently linking anticancer drugs to mAbs, usually through a conditionally stable linker system. Upon binding to cell surface antigens, mAbs used for most ADCs are actively transported to lysosomes or other intracellular compartments, where enzymes, low pH, or reducing agents facilitate drug release. There are, however, currently limited ADCs in development.

Antigens must have high tumor cell selectivity to limit toxicity and off-target effects. A plethora of tumor-associated antigens have been investigated in pre-clinical models and in clinical trials including antigens over-expressed in B-cells (e.g., CD20, CD22, CD40, CD79), T-cells (CD25, CD30), carcinoma cells (HER2, EGFR, EpCAM, EphB2, PSMA), endothelial (endoglin), or stroma cells (fibroblast activated protein), to name a few [Teicher B.A. (2009). Antibody-drug conjugate targets. Curr Cancer Drug Targets 9(8):982-1004]. An important property for ADC targets is their ability to be internalized; this can be an intrinsic feature of the antigen by itself, or it can be induced by the binding of the antibody to its antigen. Indeed, ADC internalization is crucial to reduce toxicity associated with an extracellular delivery of the drug payload.

Regarding the conjugated small molecules and in contrast to the vast variety of putative antigen targets, a limited number of families of cytotoxic drugs used as payloads in ADCs are currently actively investigated in clinical trials: calicheamycin (Pfizer), duocarmycins (Synthon), pyrrolobenzodiazepines (Spirogen), irinotecan (Immunomedics), maytansinoids (DM1 and DM4; ImmunoGen + Genentech/Roche, Sanofi-Aventis, Biogen Idec, Centocor/Johnson & Johnson, Millennium/Takeda), and auristatins (MMAE and MMAF; Seattle Genetics + Genentech/Roche, Medlmmune/AstraZeneca, Bayer-Schering, Celldex, Progenics, Genmab). Calicheamycin, duocarmycins and pyrrolobenzodiazepines are DNA minor groove binders, irinotecan is a topoisomerase I inhibitor, whereas maytansinoids and auristatins are tubulin depolymerization agents.

Interestingly, a representative of three of these cytotoxic-derived ADCs has reached late stage clinical trials. Trastuzumab emtansine (T-DM1), trastuzumab linked to a maytansinoid hemi-synthetic drug by a stable linker (FDA approval on February 22, 2013 for advanced HER2 positive breast cancer); Inotuzumab ozogamicin (CMC-544), a humanized anti-CD22 mAb (G5/44, IgG4) conjugated to calicheamycin with an acid labile linker (acetylphenoxy-butanoic) (B-cell non-Hodgkin's lymphoma); Brentuximab vedotin, a humanized anti-CD30 mAb linked to monomethyl auristatin E (MMAE), via a maleimidecaproyl-valyl-citrullinyl-p-aminobenzylcarbamate linker (FDA approval on August 19, 2011 for anaplastic large cell lymphoma and Hodking's lymphoma).

Linkers represent the key component of ADC structures. Several classes of second generation linkers have been investigated, including acid-labile hydrazone linkers (lysosomes) (e.g. gemtuzumab and inotuzumab ozogamicin); disulfide-based linkers (reductive intracellular environment); non-cleavable thioether linkers (catabolic degradation in lysosomes) (e.g., trastuzumab emtansine); peptide linkers (e.g. citruline-valine) (lysosomal proteases like cathepsin-B) (e.g. brentuximab vedotin): see, for example, WO-A-2004/010957, WO-A-2006/060533 and WO-A-2007/024536. Purification of antibody-drug conjugates by size exclusion chromatography (SEC) has also been described [see, e.g., Liu et al., Proc. Natl. Acad. Sci. USA, 93: 8618-8623 (1996), and Chari et al., Cancer Research, 52: 127-131 (1992)].

Trastuzumab (Herceptin) is a monoclonal antibody that interferes with the HER2/neu receptor. Its main use is to treat certain gastric and breast cancers. The HER receptors are proteins that are embedded in the cell membrane and communicate molecular signals from outside the cell (molecules called EGFs) to inside the cell, and turn genes on and off. The HER proteins stimulate cell proliferation. In some cancers, notably certain types of breast, ovarian, gastric, lung, uterine, endometrial, salivary duct, bladder, cervical, colorectal esophageal, pancreas and gallbladder cancers, HER2 is over-expressed, and causes cancer cells to reproduce uncontrollably.

The HER2 (human epithelial growth factor receptor 2) gene is amplified in 20-30% of early-stage breast cancers, which makes it overexpress epidermal growth factor (EGF) receptors in the cell membrane. In some types of cancer, HER2 may send signals without growth factors arriving and binding to the receptor, making its effect in the cell constitutive; however, trastuzumab is not effective in this case.

The HER2 pathway promotes cell growth and division when it is functioning normally; however when it is overexpressed, cell growth accelerates beyond its normal limits. In some types of cancer the pathway is exploited to promote rapid cell growth and proliferation and hence tumor formation. In cancer cells the HER2 protein can be expressed up to 100 times more than in normal cells (2 million versus 20,000 per cell). This overexpression leads to strong and constant proliferative signaling and hence tumor formation. Overexpression of HER2 also causes deactivation of checkpoints, allowing for even greater increases in proliferation.

In the compounds of the present invention, Ab is a moiety comprising at least one antigen binding site. In an alternative embodiment, Ab can be any suitable agent that is capable of binding to a target cell, preferably an animal cell and more preferably, a human cell. Examples of such agents include lymphokines, hormones, growth factors and nutrient-transport molecules (e.g. transferrin). In another example, Ab may be an aptamer, such as a nucleic acid or a peptide aptamer, an affimer or a bicylic peptide.

Where Ab is a moiety comprising at least one antigen binding site, the moiety is preferably an antigen-binding peptide or polypeptide. In a preferred embodiment, the moiety is an antibody or an antigen-binding fragment thereof.

The term 'antibody' in the drug conjugates of the present invention refers to any immunolglobulin, preferably a full-length immunoglobulin. Preferably, the term covers monoclonal antibodies, polyclonal antibodies, multispecific antibodies, such as bispecific antibodies, and antibody fragments thereof, so long as they exhibit the desired biological activity. Antibodies may be derived from any species, but preferably are of rodent, for examples rat or mouse, human or rabbit origin. Alternatively, the antibodies, preferably monoclonal antibodies, may be humanised, chimeric or antibody fragments thereof. The term 'chimeric antibodies' may also include "primatised" antibodies comprising variable domain antigen-binding sequences derived from a non-human primate (e.g., Old World Monkey, Ape etc) and human constant region sequences. The immunoglobulins can also be of any type (e.g. IgG, IgE, IgM, IgD, and IgA), class (e.g., IgGI, IgG2, IgG3, IgG4, IgAI and IgA2) or subclass of immunoglobulin molecule.

The term 'monoclonal antibody' refers to a substantially homogenous population of antibody molecules (i.e. the individual antibodies comprising the population are identical except for possible naturally occurring mutations that may be present in minor amounts), produced by a single clone of B lineage cells, often a hybridoma. Importantly, each monoclonal has the same antigenic specificity - i.e. it is directed against a single determinant on the antigen.

The production of monoclonal antibodies can be carried out by methods known in the art. However, as an example, the monoclonal antibodies can be made by the hybridoma method (Kohler et al (1975) Nature 256:495), the human B cell hybridoma technique (Kozbor et al., 1983, Immunology Today 4: 72), or the EBV-hybridoma technique (Cole et al., 1985, Monoclonal Antibodies and Cancer Therapy, Alan R. Liss, Inc., pp. 77-96). Alternatively, the monoclonal antibody can be produced using recombinant DNA methods (see, US 4816567) or isolated from phage antibody libraries using the techniques described in Clackson et al (1991) Nature, 352:624-628; Marks et al (1991) J. Mol. Biol., 222:581-597.

Polyclonal antibodies are antibodies directed against different determinants (epitopes). This heterogenous population of antibody can be derived from the sera of immunised animals using various procedures well known in the art.

The term 'bispecific antibody' refers to an artificial antibody composed of two different monoclonal antibodies. They can be designed to bind either to two adjacent epitopes on a single antigen, thereby increasing both avidity and specificity, or bind two different antigens for numerous applications, but particularly for recruitment of cytotoxic T- and natural killer (NK) cells or retargeting of toxins, radionuclides or cytotoxic drugs for cancer treatment (Holliger & Hudson, Nature Biotechnology, 2005, 9, 23). The bispecific antibody may have a hybrid immunoglobulin heavy chain with a first binding specificity in one arm, and a hybrid immunoglobulin heavy chain-light chain pair (providing a second binding specificity) in the other arm. This asymmetric structure facilitates the separation of the desired bispecific compound from unwanted immunoglobulin chain combinations, as the presence of an immunoglobulin light chain in only one half of the bispecific molecule provides for a facile way of separation (WO 94/04690; Suresh et al., Methods in Enzymology, 1986, 121:210; Rodrigues et al., 1993, J. of Immunology 151:6954-6961; Carter et al., 1992, Bio/Technology 10:163-167; Carter et al., 1995, J. of Hematotherapy 4:463-470; Merchant et al., 1998, Nature Biotechnology 16:677-681.

Methods to prepare hybrid or bispecific antibodies are known in the art. In one method, bispecific antibodies can be produced by fusion of two hybridomas into a single 'quadroma' by chemical cross-linking or genetic fusion of two different Fab or scFv modules (Holliger & Hudson, Nature Biotechnology, 2005, 9, 23).

The term 'chimeric' antibody refers to an antibody in which different portions are derived from different animal species. For example, a chimeric antibody may derive the variable region from a mouse and the constant region from a human. In contrast, a 'humanised antibody' comes predominantly from a human, even though it contains non-human portions. Specifically, humaised antibodies are human immunoglobulins (recipient antibody) in which residues from a hypervariable region of the recipient are replaced by residues from hypervariable regions of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanised antibodies may comprise residues that are not found in the recipient antibody or in the donor antibody. These modifications are made to further refine antibody performance. In general, the humanised antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanised antibody optionally also will comprise at least a portion of an immunoglobulin constant region (Fc), typically that of a human immunoglobulin.

Recombinant antibodies such as chimeric and humanised monoclonal antibodies can be produced by recombinant DNA techniques known in the art. Completely human antibodies can be produced using transgenic mice that are incapable of expressing endogenous immunoglobulin heavy and light chains genes, but which can express human heavy and light chain genes. The transgenic mice are immunized in the normal fashion with a selected antigen. Monoclonal antibodies directed against the antigen can be obtained using conventional hybridoma technology. The human immunoglobulin transgenes harboured by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, see, for example, US Patent Nos. 5625126; 5633425; 5569825; 5661016; 5545806; each of which is incorporated herein by reference in its entirety. Other human antibodies can be obtained commercially from, for example, Abgenix, Inc. (Freemont, CA) and Genpharm (San Jose, CA).

The term 'antigen-binding fragment' in the drug conjugates of the present invention refers to a portion of a full length antibody where such antigen-binding fragments of antibodies retain the antigen-binding function of a corresponding full-length antibody. The antigen-binding fragment may comprise a portion of a variable region of an antibody, said portion comprising at least one, two, preferably three CDRs selected from CDR1, CDR2 and CDR3. The antigen-binding fragment may also comprise a portion of an immunoglobulin light and heavy chain. Examples of antibody fragments include Fab, Fab', F(ab')2, scFv, di-scFv, sdAb, and BiTE (Bi-specific T-cell engagers), Fv fragments including nanobodies, diabodies, diabody-Fc fusions, triabodies and, tetrabodies; minibodies; linear antibodies; fragments produced by a Fab expression library, anti-idiotypic (anti-Id) antibodies, CDR (complementary determining region), and epitope-binding fragments of any of the above that immunospecifically bind to a target antigen such as a cancer cell antigens, viral antigens or microbial antigens, single-chain or single-domain antibody molecules including heavy chain only antibodies, for example, camelid VHH domains and shark V-NAR; and multispecific antibodies formed from antibody fragments. For comparison, a full length antibody, termed 'antibody' is one comprising a VL and VH domains, as well as complete light and heavy chain constant domains.

The antibody may also have one or more effector functions, which refer to the biological activities attributable to the Fc region (a native sequence Fc region or amino acid sequence variant Fc region engineered according to methods in the art to alter receptor binding) of an antibody. Examples of antibody effector functions include Clq binding; complement dependent cytotoxicity; Fc receptor binding; antibody-dependent cell-mediated cytotoxicity (ADCC); phagocytosis; down regulation of cell surface receptors (e.g., B cell receptor; BCR), etc.

The antibody can also be a functionally active fragment (also referred to herein as an immunologically active portion), derivative or analog of an antibody that immunospecifically binds to a target antigen such as a cancer cell antigen, viral antigen, or microbial antigen or other antibodies bound to tumour cells. In this regard, functionally active means that the fragment, derivative or analog is able to elicit anti-idiotype antibodies that recognise the same antigen that the antibody from which the fragment, derivative or analog is derived recognised. Specifically, in an exemplary embodiment the antigenicity of the idiotype of the immunoglobulin molecule can be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art (e.g., the BIA core assay), see, for example, Kabat et al., 1991, Sequences of Proteins of Immunological Interest, Fifth Edition, National Institute of Health, Bethesda, Md; Kabat E et al., 1980, J. of Immunology 125(3):961-969).

The term 'antibody' may also include a fusion protein of an antibody, or a functionally active fragment thereof, for example in which the antibody is fused via a covalent bond (e.g., a peptide bond), at either the N-terminus or the C-terminus to an amino acid sequence of another protein (or portion thereof, such as at least 10, 20 or 50 amino acid portion of the protein) that is not the antibody. The antibody or fragment thereof may be covalently linked to the other protein at the N-terminus of the constant domain.

Furthermore, the antibody or antigen-binding fragments of the present invention may include analogs and derivatives of antibodies or antigen-binding fragments thereof that are either modified, such as by the covalent attachment of any type of molecule as long as such covalent attachment permits the antibody to retain its antigen binding immunospecificity. Examples of modifications include glycosylation, acetylation, pegylation, phosphorylation, amidation, derivatization by known protecting/blocking groups, proteolytic cleavage, linkage to a cellular antibody unit or other protein, etc. Any of numerous chemical modifications can be carried out by known techniques, including, but not limited to specific chemical cleavage, acetylation, formylation, metabolic synthesis in the presence of tunicamycin, etc. Additionally, the analog or derivative can contain one or more unnatural amino acids.

The antibodies or antigen-binding fragments of the present invention may also have modifications (e.g., substitutions, deletions or additions) in the Fc domain of the antibody. Specifically, the modifications may be in the Fc-hinge region and result in an increased binding for the FcRn receptor (WO 97/34631).

In one embodiment, the antibody in the drug conjugate of the present invention may be any antibody or antigen-binding fragment thereof, preferably a monoclonal antibody that is useful in the treatment of a disease, preferably cancer and more preferably a cancer selected from lung cancer, including NSCLC, gastric cancer, colorectal cancer, breast cancer, pancreas carcinoma, endometrial cancer, bladder cancer, cervical cancer, esophageal cancer, gallbladder cancer, uterine cancer, salivary duct cancer, ovarian cancer, kidney cancer, leukaemia, multiple myeloma, and lymphoma, wherein the cancer is preferably a HER2 positive cancer, wherein the HER2 positive cancers include HER2 positive lung cancer including HER2 positive NSCLC, HER2 positive gastric cancer, HER2 positive colorectal cancer, HER2 positive breast cancer, HER2 positive pancreas carcinoma, HER2 positive endometrial cancer, HER2 positive bladder cancer, HER2 positive cervical cancer, HER2 positive esophageal cancer, HER2 positive gallbladder cancer, HER2 positive uterine cancer, HER2 positive salivary duct cancer and HER2 positive ovarian cancer, more preferably HER2 positive breast cancer, HER2 positive ovarian cancer and HER2 positive gastric cancer, most preferably HER2 positive breast cancer.

Antibodies that may be useful in the treatment of cancer include, but are not limited to, antibodies against cancer cell antigens. As used herein, a "cancer cell antigen" may be selected from one of the following antigens: HER2 (human epithelial growth factor receptor 2), CA125 (ovarian), CA15-3 (carcinomas), CA19-9 (carcinomas), L6 (carcinomas), Lewis Y (carcinomas), Lewis X (carcinomas), alpha fetoprotein (carcinomas), CA 242 (colorectal), placental alkaline phosphatase (carcinomas), prostate specific antigen (prostate), prostatic acid phosphatase (prostate), epidermal growth factor (carcinomas) for example EGF receptor 2 protein (breast cancer), MAGE-I (carcinomas), MAGE-2 (carcinomas), MAGE-3 (carcinomas), MAGE-4 (carcinomas), anti-transferrin receptor (carcinomas), p97 (melanoma), MUCI-KLH (breast cancer), CEA (colorectal), gplOO (melanoma), MARTI (melanoma), PSA (prostate), IL-2 receptor (T-cell leukemia and lymphomas), CD20 (non-Hodgkin's lymphoma), CD52 (leukemia), CD33 (leukemia),CD22 (lymphoma), human chorionic gonadotropin (carcinoma), CD38 (multiple myeloma), CD40 (lymphoma), mucin (carcinomas), P21 (carcinomas), MPG (melanoma), and Neu oncogene product (carcinomas). In one embodiment, the cancer cell antigen is HER2. Some specific, useful antibodies include, but are not limited to, BR96 mAb (Trail, P. A., et al Science (1993) 261, 212-215), BR64 (Trail, PA, et al Cancer Research (1997) 57, 100-105, mAbs against the CD40 antigen, such as S2C6 mAb (Francisco, J. A., et al Cancer Res. (2000) 60:3225-3231), mAbs against the CD70 antigen, such as 1F6 mAb, and mAbs against the CD30 antigen, such as ACIO (Bowen, M. A., et al (1993) J. Immunol., 151:5896- 5906; Wahl et al., 2002 Cancer Res. 62(13):3736-42 ). Many other internalizing antibodies that bind to tumor associated antigens can be used and have been reviewed (Franke, A. E., et al Cancer Biother Radiopharm. (2000) 15:459-76; Murray, J. L., (2000) Semin Oncol, 27:64-70; Breitling, F., and Dubel, S., Recombinant Antibodies, John Wiley, and Sons, New York, 1998).

Other tumour-associated antigens include, but are not limited to, BMPR1B, E16, STEAP1, STEAP2, 0772P. MPF, Napi3b, Sema5b, PSCA hlg, ETBR, MSG783, TrpM4, CRIPTO, CD21, CD79b, FcRH2, HER2, NCA, MDP, IL20Rα, Brevican, EphB2R, ASLG659, PSCA, GEDA, BAFF-R, CD79A, CXCR5, HLA-DOB, P2X5, CD72, LY64, FCRH1, IRTA2 and TENB2.

In an alternative embodiment, the antibody in the drug conjugate of the present invention may be an antibody or antigen-binding fragment thereof, preferably a monoclonal antibody, that immunospecifically binds to a viral antigen, microbial antigen or an antigen of a cell that produces autoimmune antibodies associated with autoimmune disease.

The viral antigen may include, but is not limited to, any viral peptide, polypeptide or protein such as HIV gpl20, HIV nef, RSV F glycoprotein, influenza virus neuraminidase, influenza virus hemagglutinin, HTLV tax, herpes simplex virus glycoprotein (e.g., Gb, Gc, Gd, and Ge) and hepatitis B surface antigen) that is capable of eliciting an immune response.

The microbial antigen may include, but is not limited to, any microbial peptide, polypeptide, protein, saccharide, polysaccharide, or lipid molecule (e.g., a bacterial, fungi, pathogenic protozoa, or yeast polypeptide including, e.g., LPS and capsular polysaccharide) that is capable of eliciting an immune response.

In a further embodiment, the antibody or antigen-binding fragment binds to an epitope that is present on a cell, such as a tumour cell. Preferably, where the cell is a tumour cell, the tumour cell epitope is not present on non-tumour cells, or is present at a lower concentration or in a different steric configuration than in tumour cells.

In one embodiment, the antibody or antigen-binding fragment binds to an epitope present in the context of one of the following antigens: CA125, CA15-3, CA19-9 L6, Lewis Y, Lewis X, alpha fetoprotein, CA 242, placental alkaline phosphatase, prostate specific antigen, prostatic acid phosphatase, epidermal growth factor for example EGF receptor 2 protein, MAGE-I, MAGE-2, MAGE-3, MAGE-4, anti-transferrin receptor, p97, MUCI-KLH, CEA, gplOO, MART!, PSA, IL-2 receptor, CD20, CD52, CD33 ,CD22, human chorionic gonadotropin, CD38, CD40, mucin, P21, MPG, Neu oncogene product, BMPR1B, E16, STEAP1, STEAP2, 0772P. MPF, Napi3b, Sema5b, PSCA hlg, ETBR, MSG783, TrpM4, CRIPTO, CD21, CD79b, FcRH2, HER2, NCA, MDP, IL20Rα, Brevican, EphB2R, ASLG659, PSCA, GEDA, BAFF-R, CD79A, CXCR5, HLA-DOB, P2X5, CD72, LY64, FCRH1, IRTA2, TENB2, a viral antigen (such as any viral peptide, polypeptide or protein such as HIV gpl20, HIV nef, RSV F glycoprotein, influenza virus neuraminidase, influenza virus hemagglutinin, HTLV tax, herpes simplex virus glycoprotein (e.g., Gb, Gc, Gd, and Ge) and hepatitis B surface antigen) that is capable of eliciting an immune response), microbial antigen (any microbial peptide, polypeptide, protein, saccharide, polysaccharide, or lipid molecule (e.g., a bacterial, fungi, pathogenic protozoa, or yeast polypeptide including, e.g., LPS and capsular polysaccharide) that is capable of eliciting an immune response) or an antigen of a cell that produces autoimmune antibodies associated with autoimmune disease.

In one embodiment, where the antigen is ErBB2 (also known as ERBB2, CD340 or HER2; such terms may be used interchangeably), the antibody or antigen-binding fragment may bind to one or more of the following epitopes: ARHC L (SEQ ID NO: 1), QNGS (SEQ ID NO: 2) and PPFCVARC PSG (SEQ ID NO: 3). These epitopes correspond to positions 557-561, 570-573 and 593-603 respectively of the human HER2 polypetide sequence (Accession: NM_004448, Version: NM_004448.3).

In another embodiment, the antibody may be any antibody known for the treatment or prevention of viral or microbial infection - i.e. an infectious disease. Examples of such antibodies include, but are not limited to, PRO542 (Progenies) which is a CD4 fusion antibody useful for the treatment of HIV infection; OsTAVIR (Protein Design Labs, Inc., CA) which is a human antibody useful for the treatment of hepatitis B virus; PROTOVIR. (Protein Design Labs, Inc., CA) which is a humanised IgG1 antibody useful for the treatment of cytomegalovirus (CMV); and anti-LPS antibodies.

Other antibodies useful in the treatment of infectious diseases include, but are not limited to, antibodies against the antigens from pathogenic strains of bacteria (*Streptococcus pyogenes, Streptococcus pneumoniae, Neisseria gonorrheae, Neisseria meningitidis, Corynebacterium diphtheriae, Clostridium botulinum, Clostridium perfringens, Clostridium tetani, Hemophilus influenzae, Klebsiella pneumoniae, Klebsiella ozaenas, Klebsiella rhinoscleromotis, Staphylococcus aureus, Vibrio colerae, Escherichia coli, Pseudomonas aeruginosa, Campylobacter (Vibrio) fetus, Aeromonas hydrophila, Bacillus cereus, Edwardsiella tarda, Yersinia enterocolitica, Yersinia pestis, Yersinia pseudotuberculosis, Shigella dysenteriae, Shigella flexneri, Shigella sonnei, Salmonella typhimurium, Treponema pallidum, Treponema pertenue, Treponema carateneum, Borrelia vincentii, Borrelia burgdorferi, Leptospira icterohernorrhagiae, Mycobacterium tuberculosis, Pneumocystis carinii, Francisella tularensis, Brucella abortus, Brucella suis, Brucella melitensis, Mycoplasma spp., Rickettsia prowazeki, Rickettsia tsutsugumushi, Chlamydia spp.*); pathogenic fungi (*Coccidioides immitis, Aspergillus fumigatus, Candida albicans, Blastomyces dermatitidis, Cryptococcus neoformans, Histoplasma capsulatum*)*;* protozoa (*Entomoeba histolytica, Toxoplasma gondii, Trichomonas tenas, Trichomonas hominis, Trichomonas vaginalis, Tryoanosoma gambiense, Trypanosoma rhodesiense, Trypanosoma cruzi, Leishmania donovani, Leishmania tropica, Leishmania braziliensis, Pneumocystis pneumonia, Plasmodium vivax, Plasmodium falciparum, Plasmodium malaria*)*;* or Helminiths *(Enterobius vermicularis, Trichuris trichiura, Ascaris lumbricoides, Trichinella spiralis, Strongyloides stercoralis, Schistosoma japonicum, Schistosoma mansoni, Schistosoma haematobium,* and hookworms).

Other antibodies useful for the treatment of viral disease include, but are not limited to, antibodies against antigens of pathogenic viruses, including as examples and not by limitation: Poxviridae, Herpesviridae, Herpes Simplex virus 1, Herpes Simplex virus 2, Adenoviridae, Papovaviridae, Enteroviridae, Picornaviridae, Parvoviridae, Reoviridae, Retroviridae, influenza viruses, parainfluenza viruses, mumps, measles, respiratory syncytial virus, rubella, Arboviridae, Rhabdoviridae, Arenaviridae, Hepatitis A virus, Hepatitis B virus, Hepatitis C virus, Hepatitis E virus, Non-A/Non-B Hepatitis virus, Rhinoviridae, Coronaviridae, Rotoviridae, and Human Immunodeficiency Virus.

In an alternative embodiment, the antibody of the drug conjugate of the present invention may also be any antibody known for the treatment of prevention of autoimmune disorders, such as, but not limited to, Th2-lymphocyte related disorders (e. g. atopic dermatitis, atopic asthma, rhinoconjunctivitis, allergic rhinitis, Omenn's syndrome, systemic sclerosis, and graft versus host disease); Th1 lymphocyte-related disorders (e. g. rheumatoid arthritis, multiple sclerosis, psoriasis, Sjorgren's syndrome, Hashimoto's thyroiditis, Grave's disease, primary biliary cirrhosis, Wegener's granulomatosis, and tuberculosis); activated B lymphocyte-related disorders (e. g. systemic lupus erythematosus, Goodpasture's syndrome, rheumatoid arthritis, and type I diabetes); and Active Chronic Hepatitis, Addison's Disease, Allergic Alveolitis, Allergic Reaction, Allergic Rhinitis, Alport's Syndrome, Anaphlaxis, Ankylosing Spondylitis, Anti-phosholipid Syndrome, Arthritis, Ascariasis, Aspergillosis, Atopic Allergy, Atropic Dermatitis, Atropic Rhinitis, Behcet's Disease, Bird-Fancier's Lung, Bronchial Asthma, Caplan's Syndrome, Cardiomyopathy, Celiac Disease, Chagas' Disease, Chronic Glomerulonephritis, Cogan's Syndrome, Cold Agglutinin Disease, Congenital Rubella Infection, CREST Syndrome, Crohn's Disease, Cryoglobulinemia, Cushing's Syndrome, Dermatomyositis, Discoid Lupus, Dresser's Syndrome, Eaton-Lambert Syndrome, Echovirus Infection, Encephalomyelitis, Endocrine opthalmopathy, Epstein-Barr Virus Infection, Equine Heaves, Erythematosis, Evan's Syndrome, Felty's Syndrome, Fibromyalgia, Fuch's Cyclitis, Gastric Atrophy, Gastrointestinal Allergy, Giant Cell Arteritis, Glomerulonephritis, Goodpasture's Syndrome, Graft v. Host Disease, Graves' Disease, Guillain-Barre Disease, Hashimoto's Thyroiditis, Hemolytic Anemia, Henoch-Schonlein Purpura, Idiopathic Adrenal Atrophy, Idiopathic Pulmonary Fibritis, IgA Nephropathy, Inflammatory Bowel Diseases, Insulin-dependent Diabetes Mellitus, Juvenile Arthritis, Juvenile Diabetes Mellitus (Type I), Lambert-Eaton Syndrome, Laminitis, Lichen Planus, Lupoid Hepatitis, Lupus Lymphopenia, Meniere's Disease, Mixed Connective Tissue Disease, Multiple Sclerosis, Myasthenia Gravis, Pernicious Anemia, Polyglandular Syndromes, Presenile Dementia, Primary Agammaglobulinemia, Primary Biliary Cirrhosis, Psoriasis, Psoriatic Arthritis, Raynauds Phenomenon, Recurrent Abortion, Reiter's Syndrome, Rheumatic Fever, Rheumatoid Arthritis, Sampter's Syndrome, Schistosomiasis, Schmidt's Syndrome, Scleroderma, Shulman's Syndrome, Sjorgen's Syndrome, Stiff-Man Syndrome, Sympathetic Ophthahnia, Systemic Lupus Erythematosis, Takayasu's Arteritis, Temporal Arteritis, Thyroiditis, Thrombocytopenia, Thyrotoxicosis, Toxic Epidermal Necrolysis, Type B Insulin Resistance, Type I Diabetes Mellitus, Ulcerative Colitis, Uveitis, Vitiligo, Waldenstrom's Macroglobulemia and Wegener's Granulomatosis.

Antibodies immunospecific for an antigen of a cell that is responsible for producing autoimmune antibodies can be obtained by any method known to one of skill in the art such as, e.g., chemical synthesis or recombinant expression techniques. Examples of autoimmune antibodies include, but are not limited to, Anti-Nuclear Antibody; Anti ds DNA; Anti ss DNA, Anti Cardiolipin Antibody IgM, IgG; Anti Phospholipid Antibody IgM, IgG; Anti SM Antibody; Anti Mitochondrial Antibody; Thyroid Antibody; Microsomal Antibody; Thyroglobulin Antibody; Anti SCL-70; Anti- Jo; Anti-U1RNP; Anti- La/SSB; Anti SSA; Anti SSB; Anti Perital Cells Antibody; Anti Histones; Anti-RNP; C-ANCA; P-ANCA; Anti centromere; Anti-Fibrillarin, and Anti-GBM Antibody.

In another embodiment, the antibody of the drug conjugate of the present invention can be one that binds to both a receptor or a receptor complex expressed on an activated lymphocyte, such as one associated with an autoimmune disease. The receptor or receptor complex can comprise an immunoglobulin gene superfamily member, a TNF receptor superfamily member, an integrin, an interleukin, a cytokine receptor, a chemokine receptor, a major histocompatibility protein, a lectin, or a complement control protein. Non-limiting examples of suitable immunoglobulin superfamily members are CD2, CD3, CD4, CD5, CD8, CD19, CD22, CD28, CD79, CD90, CD152/CTLA-4, PD-I, and ICOS. Non-limiting examples of suitable TNF receptor superfamily members are CD27, CD40, CD95/Fas, CD134/OX40, CD137/4-1BB, TNF-RI, TNFR-2, RANK, TACI, BCMA, osteoprotegerin, Apo2/TRAEL-RI, TRAIL-R2, TRAIL-R3, TRABL-R4, and APO-3. Non-limiting examples of suitable integrins are CDI la, CDIIb, CDIIc, CD18, CD29, CD41, CD49a, CD49b, CD49c, CD49d, CD49e, CD49f, CD103, and CD104. Non-limiting examples of suitable lectins are C-type, S- type, and I-type lectin.

An antibody that binds a molecular target or an antigen of interest, e.g., ErbB2 antigen, is one capable of binding that antigen with sufficient affinity such that the antibody is useful in targeting a cell expressing the antigen. Where the antibody is one which binds ErbB2, it will usually preferentially bind ErbB2 as opposed to other ErbB receptors, and may be one which does not significantly cross-react with other proteins such as EGFR, ErbB 3 or ErbB4. In such embodiments, the extent of binding of the antibody to these non- ErbB2 proteins (e.g., cell surface binding to endogenous receptor) will be less than 10% as determined by fluorescence activated cell sorting (FACS) analysis or radioimmunoprecipitation (RIA). Sometimes, the anti- ErbB2 antibody will not significantly cross-react with the rat neu protein, e.g., as described in Schecter et al., Nature 312:513 (1984) and Drebin et al., Nature 312:545-548 (1984).

In another embodiment, the antibody of the drug conjugate or target of the present invention may be selected from an antibody or target in the below table. Such antibodies are immunospecific for a target antigen and can be obtained commercially or produced by any method known in the art such as, e.g., recombinant expression techniques.

**Table 1: Therapeutic monoclonal antibodies**

| **Name** | **Trade name** | **Target** |
|---|---|---|
| 3F8 | | GD2 ganglioside |
| 8H9 | | B7-H3 |
| Abagovomab | | CA-125 (imitation) |
| Abciximab | ReoPro | CD41 7E3 |
| Abituzumab | | CD51 |
| Abrezekimab | | Interleukin 13 |
| Abrilumab | | Integrin α4β7 |
| Actoxumab | | *Clostridium difficile* |
| Adalimumab | Humira | TNF-α |
| Adecatumumab | | EpCAM |
| Atidortoxumab | | *Staphylococcus aureus* alpha toxin |
| Aducanumab | | Beta-amyloid |
| Afasevikumab | | IL17A and IL17F |
| Afutuzumab | | CD20 |
| Alemtuzumab | Campath, Lemtrada | CD52 |
| Alirocumab | Praluent | PCSK9 |
| Altumomab | Hybri-ceaker | CEA |
| Amatuximab | | Mesothelin |
| Andecaliximab | | gelatinase B |
| Anetumab | | MSLN |
| Anifrolumab | | interferon α/β receptor |
| Anrukinzumab | | IL-13 |
| Apolizumab | | HLA-DR β-chain |
| Aprutumab | | FGFR2 |
| Ascrinvacumab | | Activin receptor-like kinase 1 |
| Aselizumab | | L-selectin (CD62L) |
| Atezolizumab | Tecentriq | PD-L1 |
| Atidortoxumab | | Staphylococcus aureus alpha toxin |
| Atinumab | | RTN4 |
| Atorolimumab | | Rhesus factor |
| Avelumab | Bavencio | PD-L1 |
| Azintuxizumab | | CD319 |
| Bapineuzumab | | beta amyloid |
| Basiliximab | Simulect | CD25 (α chain of IL-2 receptor) |
| Bavituximab | | phosphatidylserine |
| BCD-100 | | PD-1 |
| Bectumomab | LymphoScan | CD22 |
| Begelomab | | DPP4 |
| Belantamab | | BCMA |
| Belimumab | Benlysta | BAFF |
| Bemarituzumab | | FGFR2 |
| Benralizumab | Fasenra | CD125 |
| Berlimatoxumab | | *Staphylococcus aureus* bi-component leukocidin |
| Bermekimab | Xilonix | IL1A |
| Bersanlimab | | ICAM-1 |
| Bertilimumab | | CCL11 (eotaxin-1) |
| Besilesomab | Scintimun | CEA-related antigen |
| Bevacizumab | Avastin | VEGF-A |
| Bezlotoxumab | Zinplava | *Clostridium difficile* |
| Blinatomumab | Blincyto | CD19, CD3 |
| Bimagrumab | | ACVR2B |
| Bimekizumab | | IL 17A and IL17F |
| Birtamimab | | Serum amyloid A protein |
| Bivatuzumab | | CD44 v6 |
| BIVV009 | | C1s |
| Bleselumab | | CD40 |
| Blontuvetmab | Blontress | CD20 |
| Blosozumab | | SOST |
| Bococizumab | | Neural apoptosis-regulated proteinase 1 |
| Brazikumab | | IL23 |
| Brentuximab | Adcetris | CD30 (TNFRSF8) |
| Briakinumab | | IL-12, IL-23 |
| Brodalumab | Siliz | IL-17 |
| Brontictuzumab | | Notch 1 |
| Burosumab | Crysvita | FGF 23 |
| Cabiralizumab | | CSF1R |
| Camidanlumab | | CD25 |
| Camrelizumab | | Programmed cell death 1 |
| Canakinumab | Ilaris | IL-1 |
| Cantuzumab | | MUC-1 |
| Capromab | Prostascint | prostatic carcinoma cells |
| Carlumab | | MCP-1 |
| Carotuximab | | endoglin |
| Catumaxomab | Removab | EpCAM, CD3 |
| CC49 | | TAG-72 |
| cBR96 | | Lewis-Y antigen |
| Cedelizumab | | CD4 |
| Cemiplimab | | PCDC1 |
| Cergutuzumab | | IL2 |
| Cetrelimab | | Programmed cell death 1 |
| Cetuximab | Erbitux | EGFR |
| Cibisatamab | | CEACAM5 |
| Cixutumumab | | IGF-1 receptor (CD221) |
| Clazakizumab | | IL6 |
| Clenoliximab | | CD4 |
| Clivatuzumab | hPAM4-Cide | MUC1 |
| Codrituzumab | | glypican 3 |
| Cofetuzumab | | PTK7 |
| Coltuximab | | CD19 |
| Conatumumab | | TRAIL-R2 |
| Concizumab | | TFPI |
| Cosfroviximab | ZMapp | Ebolavirus glycoprotein |
| CR6261 | | Influenza A hemagglutinin |
| Crenezumab | | 1-40-β-amyloid |
| Crizanlizumab | | Selectin P |
| Crotedumab | | GCGR |
| Cusatuzumab | | CD70 |
| Dacetuzumab | | CD40 |
| Daclizumab | Zenapax | CD25 (α chain of IL-2 receptor) |
| Dalotuzumab | | IGF-1 receptor (CD221) |
| Dapirolizumab pegol | | CD154 (CD40L) |
| Daratumumab | Darzalex | CD38 |
| Dectrekumab | | IL-13 |
| Demcizumab | | DLL4 |
| Denintuzumab | | CD19 |
| Denosumab | Prolia | RANKL |
| Depatuxizumab | | EGFR |
| Derlotuximab | | Histone complex |
| Detumomab | | B-lymphoma cell |
| Dezamizumab | | Serum amyloid P component |
| Dinutuximab | Unituxin | GD2 ganglioside |
| Diridavumab | | hemagglutinin |
| Domagrozumab | | GDF-8 |
| Dostarlimab | | PCDP1 |
| Drozitumab | | DR5 |
| Duligotuzumab | | ERBB3 (HER3) |
| Dupilumab | Dupixent | IL4 |
| Durvalumab | Imfinzi | PD-L1 |
| Dusigitumab | | ILGF2 |
| Ecromeximab | | GD3 ganglioside |
| Eculizumab | Soliris | C5 |
| Edobacomab | | endotoxin |
| Edrecolomab | Panorex | EpCAM |
| Efalizumab | Raptiva | LFA-1 (CD11a) |
| Eldelumab | | interferon gamma-induced protein |
| Elezanumab | | RGMA |
| Elgemtumab | | ERBB3 (HER3) |
| Elotuzumab | Empliciti | SLAMF7 |
| Elsilimomab | | IL-6 |
| Emactuzumab | | CSF1R |
| Emapalumab | Gamifant | Interferon gamma |
| Emibetuzumab | | HHGFR |
| Emicizumab | Hemlibra | Activated F9, F10 |
| Enapotamab | | AXL |
| Enavatuzumab | | TWEAK receptor |
| Enfortumab | | nectin-4 |
| Enlimomab pegol | | ICAM-1 (CD54) |
| Enoblituzumab | | CD276 |
| Enokizumab | | IL9 |
| Enoticumab | | DLL4 |
| Ensituximab | | 5AC |
| Epitumomab | | episialin |
| Epratuzumab | | CD22 |
| Eptinezumab | | Calcitonin gene-related peptide |
| Erenumab | Aimovig | CGRP |
| Ertumaxomab | Rexomun | HER2/neu, CD3 |
| Etaracizumab | Abegrin | integrin αᵥβ₃ |
| Etigilimab | | TIGIT |
| Etrolizumab | | integrin β₇ |
| Evinacumab | | Angiopoietin 3 |
| Evolocumab | Repatha | PCSK9 |
| Exbivirumab | | hepatitis B surface antigen |
| Fanolesomab | NeutroSpec | CD15 |
| Faralimomab | | interferon receptor |
| Faricimab | | VEGF-A and Ang-2 |
| Farletuzumab | | folate receptor 1 |
| Fasinumab | | HNGF |
| FBTA05 | Lymphomun | CD20 |
| Felvizumab | | respiratory syncytial virus |
| Fezakinumab | | IL-22 |
| Fibatuzumab | | Ephrin receptor A3 |
| Ficlatuzumab | | HGF |
| Figitumumab | | IGF-1 receptor (CD221) |
| Firivumab | | Influenza A virus hemagglutinin |
| Flanvotumab | | TYRP1 (glycoprotein 75) |
| Fletikumab | | IL-20 |
| Fontolizumab | HuZAF | IFN-γ |
| Foralumab | | CD3 epsilon |
| Foravirumab | | rabies virus glycoprotein |
| Fremanezumab | | Calcitonin gene-related peptide alpha |
| Fresolimumab | | TGF-β |
| Frovocimab | | PCSK9 |
| Frunevetmab | | NGF |
| Fulranumab | | NGF |
| Futuximab | | EGFR |
| Galcanezumab | | calcitonin |
| Galiximab | | CD80 |
| Ganitumab | | 1 receptor (CD221) |
| Gantenerumab | | beta amyloid |
| Gatipotuzumab | | MUC1 |
| Gavilimomab | | CD147 (basigin) |
| Gedivumab | | Hemagglutinin HA |
| Gemtuzumab | Mylotarg | CD33 |
| Gevokizumab | | IL-1β |
| Gilvetmab | | PCDC1 |
| Gimsilumab | | CSF2 |
| Girentuximab | Rencarex | carbonic anhydrase 9 (CA-IX) |
| Glembatumumab | | GPNMB |
| Golimumab | Simponi | TNF-α |
| Gomiliximab | | CD23 (IgE receptor) |
| Gosuranemab | | tau protein |
| Guselkumab | Tremfya | IL23 |
| lanalumab | | BAFF-R |
| Ibalizumab | Trogarzo | CD4 |
| Ibritumomab | Zevalin | CD20 |
| Icrucumab | | VEGFR-1 |
| Idarucizumab | Praxbind | dabigatran |
| Ifabotuzumab | | EPHA3 |
| Iladatuzumab | | CD97B |
| IMAB362 | | CLDN18.2 |
| Imalumab | | MIF |
| Imaprelimab | | MCAM |
| Imciromab | Myoscint | cardiac myosin |
| Imgatuzumab | | EGFR |
| Inclacumab | | selectin P |
| Indatuximab | | SDC1 |
| indusatumab | | GUCY2C |
| inebilizumab | | CD19 |
| Infliximab | Remicade | TNF-α |
| Inolimomab | | CD25 (α chain of IL-2 receptor) |
| Inotuzumab | Besponsa | CD22 |
| Intetumumab | | CD51 |
| Ipilimumab | Yervoy | CD152 |
| Iomab-B | | CD45 |
| Iratumumab | | CD30 (TNFRSF8) |
| Isatuximab | | CD38 |
| Iscalimab | | CD40 |
| Istiratumab | | IGF1R, CD221 |
| Itolizumab | Alzumab | CD6 |
| Ixekizumab | Taltz | IL-17A |
| Keliximab | | CD4 |
| Labetuzumab | CEA-Cide | CEA |
| Lacnotuzumab | | CSF1, MCSF |
| Ladiratuzumab | | LIV-1 |
| Lanadelumab | | kallikrein |
| Landogrozumab | | GDF-8 |
| Laprituximab | | EGFR |
| Larcaviximab | | Ebolavirus glycoprotein |
| Lebrikizumab | | IL-13 |
| Lemalesomab | | NCA-90 (granulocyte antigen) |
| Lendalizumab | | C5 |
| Lenvervimab | | Hepatitis B surface antigen |
| Lenzilumab | | CSF2 |
| Lerdelimumab | | TGF beta 2 |
| Leronlimab | | CCR5 |
| Lesofavumab | | Hemagglutinin HA |
| Lexatumumab | | TRAIL-R2 |
| Libivirumab | | hepatitis B surface antigen |
| Lifastuzumab | | Phosphate-sodium co-transporter |
| Ligelizumab | | IGHE |
| Lilotomab | | CD37 |
| Lintuzumab | | CD33 |
| Lirilumab | | KIR2D |
| Lodelcizumab | | PCSK9 |
| Lokivetmab | Cytopoint | *Canis lupus familiaris* IL31 |
| Loncastuximab | | CD19 |
| Losatuxizumab | | EGFR, ERBB1 HER1 |
| Lorvotuzumab | | CD56 |
| Lucatumumab | | CD40 |
| Lulizumab pegol | | CD28 |
| Lumiliximab | | CD23 (IgE receptor) |
| Lumretuzumab | | ERBB3 (HER3) |
| Lupartumab | | LYPD3 |
| Lutikizumab | | Interleukin 1 alpha |
| MABp1 | Xilonix | IL1A |
| Mapatumumab | | TRAIL-R1 |
| Margetuximab | | HER2 |
| Marstacimab | | TFPI |
| Maslimomab | | T-cell receptor |
| Mavrilimumab | | GMCSF receptor α-chain |
| Matuzumab | | EGFR |
| Mepolizumab | Bosatria | IL-5 |
| Metelimumab | | TGF beta 1 |
| Milatuzumab | | CD74 |
| Minretumomab | | TAG-72 |
| Mirikizumab | | IL23A |
| Mirvetuximab | | Folate receptor alpha |
| Mitumomab | | GD3 ganglioside |
| Modotuximab | | EGFR extracellular domain III |
| Mogamulizumab | Poteligeo | CCR4 |
| Monalizumab | | NKG2A |
| Morolimumab | | Rhesus factor |
| Mosunetuzumab | | CD3E, MS4A1, CD20 |
| Motavizumab | Numax | respiratory syncytial virus |
| Moxetumomab | | CD22 |
| Muromonab-CD3 | Orthoclone OKT3 | CD3 |
| Namilumab | | CSF2 |
| Naratuximab | | CD37 |
| Narnatumab | | RON |
| Natalizumab | Tysabri | integrin α₄ |
| Navicixizumab | | DLL4 |
| Navivumab | | Influenza A virus hemagglutinin HA |
| Naxitamab | | C-Met |
| Nebacumab | | endotoxin |
| Necitumumab | Portrazza | EGFR |
| Nemolizumab | | IL31RA |
| Nerelimomab | | TNF-α |
| Nesvacumab | | angiopoietin 2 |
| Netakimab | | Interleukin 17A |
| Nimotuzumab | Theracim, Theraloc | EGFR |
| Nirsevimab | | RSVFR |
| Nivolumab | Opdivo | PD-1 |
| Obiltoxaximab | Anthim | *Bacillus anthracis* anthrax |
| Obinutuzumab | Gazyva | CD20 |
| Ocaratuzumab | | CD20 |
| Ocrelizumab | Ocrevus | CD20 |
| Odulimomab | | LFA-1 (CD11a) |
| Ofatumumab | Arzerra | CD20 |
| Olaratumab | Lartruvo | PDGF-R α |
| Oleclumab | | 5'-nucleotidase |
| Olendalizumab | | Complement C5a |
| Olokizumab | | IL6 |
| Omalizumab | Xolair | IgE Fc region |
| Omburtamab | | CD276 |
| OMS721 | | MASP-2 |
| Onartuzumab | | human scatter factor receptor kinase |
| Ontuxizumab | | TEM1 |
| Onvatilimab | | VSIR |
| Opicinumab | | LINGO-1 |
| Oregovomab | OvaRex | CA-125 |
| Orticumab | | oxLDL |
| Otelixizumab | | CD3 |
| Otilimab | | GMCSF |
| Otlertuzumab | | CD37 |
| Oxelumab | | OX-40 |
| Ozanezumab | | NOGO-A |
| Ozoralizumab | | TNF-α |
| Pagibaximab | | lipoteichoic acid |
| Palivizumab | Synagis, Abbosynagis | F protein of respiratory syncytial virus |
| Pamrevlumab | | CTGF |
| Panitumumab | Vectibix | EGFR |
| Pankomab | | Tumor specific glycosylation of MUC1 |
| Panobacumab | | *Pseudomonas aeruginosa* |
| Parsatuzumab | | EGFL7 |
| Pascolizumab | | IL-4 |
| Pasotuxizumab | | Folate hydrolase |
| Pateclizumab | | LTA |
| Patritumab | | ERBB3 (HER3) |
| Pembrolizumab | Keytruda | PD1 |
| Pemtumomab | Theragyn | MUC1 |
| Perakizumab | | IL17A |
| Pertuzumab | Omnitarg | HER2/neu |
| Pidilizumab | | PD-1 |
| Pinatuzumab | | CD22 |
| Pintumomab | | adenocarcinoma antigen |
| Placulumab | | human TNF |
| Plozalizumab | | CCR2 |
| Pogalizumab | | TNFR superfamily member 4 |
| Polatuzumab | | CD79B |
| Ponezumab | | human beta-amyloid |
| Porgaviximab | | Zaire evolavirus glycoprotein |
| Prasinezumab | | NACP |
| Prezalizumab | | ICOSL |
| Priliximab | | CD4 |
| Pritoxaximab | | *E. coli* shiga toxin type-1 |
| Pritumumab | | vimentin |
| PRO 140 | | CCR5 |
| Quilizumab | | IGHE |
| Racotumomab | Vaxira | NGNA ganglioside |
| Radretumab | | fibronectin extra domain-B |
| Rafivirumab | | rabies virus glycoprotein |
| Ralpancizumab | | Neural apoptosis-regulated proteinase 1 |
| Ramucirumab | Cyramza | VEGFR2 |
| Ranevetmab | | NGF |
| Ravagalimab | | CD40 |
| Ravulizumab | | C5 |
| Raxibacumab | | anthrax toxin, protective antigen |
| Refanezumab | | Myelin-associated glycoprotein |
| Regavirumab | | cytomegalovirus glycoprotein B |
| Relatlimab | | LAG3 |
| Remtolumab | | Interleukin 17 alpha, TNF |
| Reslizumab | Cinqair | IL-5 |
| Rilotumumab | | HGF |
| Rinucumab | | Platelet-derived growth factor receptor beta. |
| Risankizumab | | IL23A |
| Rituximab | MabThera, Rituxan | CD20 |
| Rivabazumab pegol | | *Pseudomonas aeruginosa* type III secretion system |
| Robatumumab | | IGF-1 receptor (CD221) |
| Rmab | RabiShield | Rabies virus G glycoprotein |
| Roledumab | | RHD |
| Romilkimab | | Interleukin 13 |
| Romosozumab | Evenity | sclerostin |
| Rontalizumab | | IFN-α |
| Rosmantuzumab | | Root plate-specific spondin 3 |
| Rovalpituzumab | | DLL3 |
| Rovelizumab | LeukArrest | CD11, CD18 |
| Rozanolixizumab | | FCGRT |
| Ruplizumab | Antova | CD154 (CD40L) |
| SA237 | | IL-6R |
| Sacituzumab | | TROP-2 |
| Samalizumab | | CD200 |
| Samrotamab | | LRRC15 |
| Sapelizumab | | IL6R |
| Sarilumab | Kevzara | IL6 |
| Satralizumab | | IL6 receptor |
| Satumomab | | TAG-72 |
| Secukinumab | Cosentyx | IL-17A |
| Selicrelumab | | CD40 |
| Seribantumab | | ERBB3 (HER3) |
| Setoxaximab | | *E. coli* shiga toxin type-2 |
| Setrusumab | | SOST |
| Sevirumab | | cytomegalovirus |
| Sibrotuzumab | | FAP |
| SGN-CD19A | | CD19 |
| SHP647 | | Mucosal addressin cell adhesion molecule |
| Sifalimumab | | IFN-α |
| Siltuximab | Sylvant | IL-6 |
| Simtuzumab | | LOXL2 |
| Sintilimab | | PD-1 |
| Siplizumab | | CD2 |
| Sirtratumab | | SLITRK6 |
| Sirukumab | | IL-6 |
| Sofituzumab | | CA-125 |
| Solanezumab | | beta amyloid |
| Sonepcizumab | | sphingosine-1-phosphate |
| Sontuzumab | | episialin |
| Spartalizumab | | PDCD1, CD279 |
| Stamulumab | | myostatin |
| Suptavumab | | RSVFR |
| Sutimlimab | | C1S |
| Suvizumab | | HIV-1 |
| Suvratoxumab | | *Staphylococcus aureus* alpha toxin |
| Tabalumab | | BAFF |
| Tacatuzumab | AFP-Cide | alpha-fetoprotein |
| Talacotuzumab | | CD123 |
| Talizumab | | IgE |
| Tamtuvetmab | Tactress | CD52 |
| Tanezumab | | NGF |
| Taplitumomab | | CD19 |
| Tarextumab | | Notch receptor |
| Tavolimab | | CD134 |
| Tefibazumab | Aurexis | clumping factor A |
| Telisotuzumab | | HGFR |
| Tenatumomab | | tenascin C |
| Teneliximab | | CD40 |
| Teplizumab | | CD3 |
| Tepoditamab | | Dendritic cell-associated lectin 2 |
| Teprotumumab | | IGF-1 receptor (CD221) |
| Tesidolumab | | C5 |
| Tetulomab | | CD37 |
| Tezepelumab | | TSLP |
| Tibulizumab | | BAFF |
| Tigatuzumab | | TRAIL-R2 |
| Tildrakizumab | Ilumya | IL23 |
| Timigutuzumab | | HER2 |
| Timolumab | | AOC3 |
| Tiragotumab | | TIGIT |
| Tislelizumab | | PCDC1, CD279 |
| Tisotumab | | Coagulation factor III |
| Tocilizumab | Actemra, RoActemra | IL-6 receptor |
| Tomuzotuximab | | EGFR, HER1 |
| Toralizumab | | CD154 (CD40L) |
| Tosatoxumab | | *Staphylococcus aureus* |
| Tositumomab | Bexxar | CD20 |
| Tovetumab | | CD140a |
| Tralokinumab | | IL-13 |
| Trastuzumab | Herceptin | HER2/neu |
| TRBS07 | Ektomab | GD2 ganglioside |
| Tregalizumab | | CD4 |
| Tremelimumab | | CTLA-4 |
| Trevogrumab | | Growth differentiation factor 8 |
| Tucotuzumab | | EpCAM |
| Tuvirumab | | hepatitis B virus |
| Ublituximab | | MS4A1 |
| Ulocuplumab | | CXCR4 (CD184) |
| Urelumab | | 4-1BB (CD137) |
| Urtoxazumab | | *Escherichia coli* |
| Ustekinumab | Stelara | IL-12, IL-23 |
| Utomilumab | | 4-1BB (CD137) |
| Vadastuximab | | CD33 |
| Vanalimab | | CD40 |
| Vandortuzumab | | STEAP1 |
| Vantictumab | | Frizzled receptor |
| Vanucizumab | | angiopoietin 2 |
| Vapaliximab | | AOC3 (VAP-1) |
| Varisacumab | | VEGF-A |
| Varlilumab | | CD27 |
| Vatelizumab | | ITGA2 (CD49b) |
| Vedolizumab | Entyvio | integrin α₄β₇ |
| Veltuzumab | | CD20 |
| Vepalimomab | | AOC3 (VAP-1) |
| Vesencumab | | NRP1 |
| Visilizumab | Nuvion | CD3 |
| Volociximab | | integrin α₅β₁ |
| Vonlerolizumab | | CD134 |
| Vopratelimab | | ICOS |
| Vorsetuzumab | | CD70 |
| Votumumab | HumaSPECT | tumor antigen CTAA16.88 |
| Vunakizumab | | Interleukin 17 alpha |
| Xentuzumab | | IGF1, IGF2 |
| XMAB-5574 | | CD19 |
| Zalutumumab | HuMax-EGFr | EGFR |
| Zanolimumab | HuMax-CD4 | CD4 |
| Zatuximab | | HER1 |
| Zenocutuzumab | | ERBB3, HER3 |
| Ziralimumab | | CD147 (basigin) |
| Zolbetuximab | | CLDN18 |
| Zolimomab | | CD5 |

In addition to the above, the antibody of the drug antibody conjugate of the present invention may be Vitaxin which is a humanised antibody for the treatment of sarcoma; Smart IDIO which is a humanised anti-HLA-DR antibody for the treatment of non-Hodgkin's lymphoma; Oncolym which is a radiolabeled murine anti-HLA-DrIO antibody for the treatment of non-Hodgkin's lymphoma; and Allomune which is a humanised anti-CD2 mAb for the treatment of Hodgkin's Disease or non-Hodgkin's lymphoma.

The antibody of the drug conjugate of the present invention may also be any antibody-fragment known for the treatment of any disease, preferably cancer. Again, such antibody fragments are immunospecific for a target antigen and can be obtained commercially or produced by any method known in the art such as, e.g., recombinant expression techniques. Examples of such antibodies available include any from the below table.

**Table 2: Therapeutic monoclonal antibody fragments**

| **Fragment type/format** | **Name** | **Trade name** | **Target** |
|---|---|---|---|
| Fab/chimeric | Abciximab | ReoPro | CD41 (integrin alpha-lib) |
| Fab/humanised | Abrezekimab | | Interleukin 13 |
| F(ab')₂/mouse | Afelimomab | | TNF-α |
| F(ab')₂/humanised | Alacizumab pegol | | VEGFR2 |
| Fab/mouse | Anatumomab | | TAG-72 |
| Fab/ovine | | CroFab | Snake venom |
| Fab/ovine | | DigiFab | Digoxin |
| Fab/ovine | | Digibind | Digoxin |
| Fab'/mouse | Arcitumomab | CEA-scan | CEA |
| Fab'/mouse | Bectumomab | LymphoScan | CD22 |
| Fab'/mouse | Biciromab | FibriScint | fibrin II, beta chain |
| BiTE/mouse | Blinatumomab | Blincyto | CD19 |
| scFv/humanised | Brolucizumab | | VEGFA |
| sdAb/humanised | Caplacizumab | Cablivi | VWF |
| Fab'/PEGylated humanised | Certolizumab pegol | Cimzia | TNF-α |
| Fab/humanised | Citatuzumab | | EpCAM |
| F(ab')₂/mouse | Dorlimomab | | unknown |
| scFv/chimeric humanised | Duvortuxizumab | | CD19, CD3E |
| scFv/human | Efungumab | Mycograb | Hsp90 |
| F(ab')₂/humanised | Erlizumab | | ITGB2 (CD18) |
| Di-scFy | Flotetuzumab | | IL-3 receptor |
| scFv/human | Gancotamab | | unknown |
| F(ab')₂/mouse | Igovomab | Indimacis-125 | CA-125 |
| Fab/humanised | Lampalizumab | | CFD |
| scFv/humanised | Letolizumab | | TRAP |
| Fab/mouse | Nacolomab | | C242 antigen |
| Fab/mouse | Naptumomab | | 5T4 |
| Fab/mouse | Nofetumomab | | unknown |
| scFv/humanised | Oportuzumab | Vicinium | EpCAM |
| scFv/humanised | Pexelizumab | | C5 |
| Fab/humanised | Ranibizumab | Lucentis | VEGF-A |
| BiTE/mouse | Solitomab | | EpCAM |
| Fab'/mouse | Sulesomab | LeukoScan | NCA-90 (granulocyte antigen) |
| Fab | Tadocizumab | | integrin α_{IIb}β3 |
| Fab/mouse | Telimomab | | unknown |
| scFv/humanised | Vobarilizumab | | IL6R |
| Fab/humanised | | Thromboview | D-dimer |
| Fab/PEGylated humanised | CDP791 | | VEGF |
| Fab/bispecific humanised | MDX-H210 | | Her2/Neu & CD64 (yFcR1) |
| scFv/humanised | Pexelizumab | | Complement C5 |
| (ScFv)₄ fused to streptavidin mouse | CC49 | | TAG-72 Pancarcinoma antigen |
| ScFv fused to β-lactamase human | SGN-17 | | P97 antigen |
| ScFv fused to PEG human | F5 scFv-PEG Immunoliposome | | Her2 |
| Diabody (V_{H}-V_{L})₂ human | C6.5K-A | | Her2/Neu |
| Diabody (V_{H}-V_{L})₂ human | L19 L19-γIFN | | EDB domain of fibronectin |
| Diabody (V_{L}-V_{H})₂ human | T84.66 | | CEA |
| Minibody (scFᵥ₋C_{H}3)₂ murine-human chimera (minibody) | T84.66 | | CEA |
| Minibody murine-human chimera (minibody) | 10H8 | | Her2 |
| S_{c}Fᵥ dimer Fc (S_{c}Fᵥ)₂-Fc murine-human chimera (minibody) | T84.66 | | CEA |
| Bispecific scFv (V_{L}-V_{H}-V_{H}-V_{L}) mouse | r28M | | CD28 and MAP |
| Bispecific scFv | BiTE MT103 | | CD19 and CD3 |
| (V_{L}-V_{H}-V_{H}-V_{L}) origin unknown | | | |
| Bispecific scFv (V_{L}-V_{H}-V_{H}-V_{L}) origin unknown | BiTE | | Ep-CAM and CD3 |
| Bispecific tandem diabody (VH-VL- VH -VL) (mouse) | Tandab | | CD19 & CD3 |
| VhH-β-lactamase fusion camelid | Nanobody | | CEA |
| Dab/human | Anti-TNFα dAb | | TNFα |
| VhH/camelid | Nanobody | | TNFα |
| VhH/camelid | Nanobody | | Von Willebrand factor |

| | | | |
|---|---|---|---|
| Fab fragment, antigen-binding (one arm) F(ab')₂ fragment, antigen-binding, including hinge region (both arms) Fab'fragment, antigen-binding, including hinge region (one arm) scFv single-chain variable fragment di-scFv dimeric, single-chain variable fragment (Holliger & Hudson, Nature Biotechnology, 2005, 9, 23). | | | |

In preferred embodiments, the antibody in the drug conjugates of the present invention may bind to a receptor encoded by the ErbB gene. The antibody may bind specifically to an ErbB receptor selected from EGFR, HER2, HER3 and HER4. Preferably, the antibody in the drug conjugate may specifically bind to the extracellular domain of the HER2 receptor and inhibit the growth of tumour cells which overexpress the HER2 receptor. The antibody of the drug conjugate may be a monoclonal antibody, e.g. a murine monoclonal antibody, a chimeric antibody, or a humanised antibody. Preferably, the humanised antibody may be huMAb4D5-1, huMAb4D5-2, huMAb4D5-3, huMAb4D5-4, huMAb4D5-5, huMAb4D5-6, huMAb4D5-7 or huMAb4D5-8 (Trastuzumab), particularly preferably Trastuzumab. The antibody may also be an antibody fragment, e.g. a Fab fragment.

Other preferred antibodies include:
(i) anti-CD4 antibodies. The antibody of the drug conjugate may be a monoclonal antibody, e.g. a murine monoclonal antibody, a chimeric antibody, or a humanised antibody;
(ii) anti-CD5 antibodies. The antibody of the drug conjugate may be a monoclonal antibody, e.g. a murine monoclonal antibody, a chimeric antibody, or a humanised antibody;
(iii) anti-CD20 antibodies. The antibody of the drug conjugate may be a monoclonal antibody, e.g. a murine monoclonal antibody, a chimeric antibody, or a humanised antibody. Preferably, the humanised antibody is Rituximab or an antibody fragment thereof, e.g. a Fab fragment; and
(iv) anti-CD30 antibodies. The antibody of the drug conjugate may be a monoclonal antibody, e.g. a murine monoclonal antibody, a chimeric antibody, or a humanised antibody. Preferably the humanised antibody is Brentuximab vedotin or an antibody fragment thereof.

In one embodiment of the invention, the drug antibody conjugate may demonstrate one or more of the following: (i) increased cytotoxicity (or a decrease in cell survival), (ii) increased cytostatic activity (cytostasis), (iii) increased binding affinity to the target antigen or epitope, (iv) increased internalisation of the conjugate, (v) reduction of patient side effects, and/or (vi) improved toxicity profile. Such increase may be relative to a known drug antibody conjugate in the art that binds the same or a different epitope or antigen.

### Processes for the Preparation of the Drug Antibody Conjugates

The drug antibody conjugates of the present invention can be prepared according to techniques that are well known in the art. Processes for conjugating moieties comprising at least one antigen binding site antibodies such as antibodies to a number of different drugs using different processes have been described and exemplified previously in, for example, WO-A-2004/010957, WO-A-2006/060533 and WO-A-2007/024536, the contents of which are incorporated herein by reference thereto. These involve use of a linker group that derivatises the drug, toxin or radionuclide in such a way that it can then be attached to the moiety such as an antibody. Attachment to the moiety such as an antibody is typically by one of three routes: via free thiol groups in cysteines after partial reduction of disulfide groups in the antibody; via free amino groups in lysines in the antibody; and via free hydroxyl groups in serines and/or threonines in the antibody. The attachment method varies depending upon the site of attachment on the moiety such as an antibody. Purification of antibody-drug conjugates by size exclusion chromatography (SEC) has also been described [see, e.g., Liu et al., Proc. Natl. Acad. Set (USA), 93: 8618-8623 (1996), and Chari et al., Cancer Research, 52: 127-131 (1992)].

As previously noted, the drug payloads with Z = -O- of the drug conjugates of the present invention are pederin like compounds disclosed in, or fall within the scope of, International patent application publication WO2018167270, the contents of which are incorporated herein by reference thereto. These payloads are obtained according to the processes described in such document.

Useful intermediates for the drug moieties of formula (I), (Ia), (II) or (Ila) where Z is -Scan be obtained from a compound of formula (Ib): 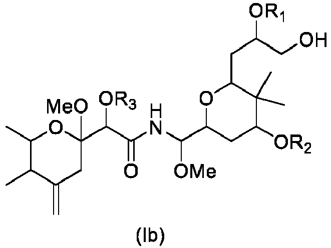 wherein
R₁ is a protecting group for OH, substituted or unsubstituted C₁-C₁₂ alkyl, substituted or unsubstituted C₂-C₁₂ alkenyl, substituted or unsubstituted C₂-C₁₂ alkynyl, -C(=O)Rₐ, - C(=O)OR_{b} and -C(=O)NR_{c}R_{d};
R₂ is hydrogen;
R₃ is selected from a protecting group for OH, -C(=O)Rₐ, -C(=O)OR_{b}, and -C(=O)NR_{c}R_{d};
Rₐ is selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group;
R_{b} is selected from substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group;
R_{c} and R_{d} are independently selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl and substituted or unsubstituted heterocyclic group;
   by reacting the compound of formula (Ib) with a sulfonyl chloride or a sulfonyl anhydride to give a sulfonyl ester and reacting such sulfohyl ester with a thiocarboxylate, particularly thioacetate, to give a thioester, followed by hydrolysis of the such thioester to give the corresponding thiol.

As noted earlier, there is provided a process for the preparation of a drug conjugate according to the present invention comprising conjugating a moiety Ab comprising at least one antigen binding site and a drug D of formula (I) or (Ia) or a drug D of formula (II)-H or (Ila)-H, Ab and D being as defined herein.

One example of a process for the preparation of a drug conjugate of the present invention involves the preparation of drug antibody conjugates of formula (G), (G') or (G") of the present invention as follows: 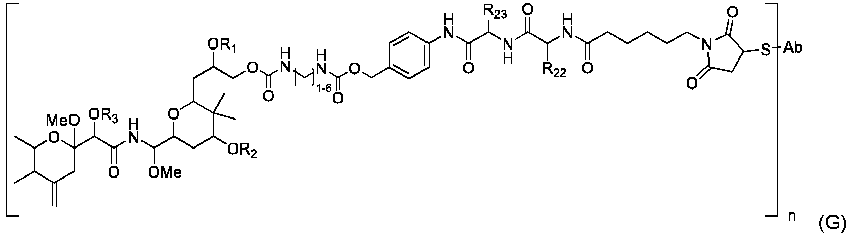 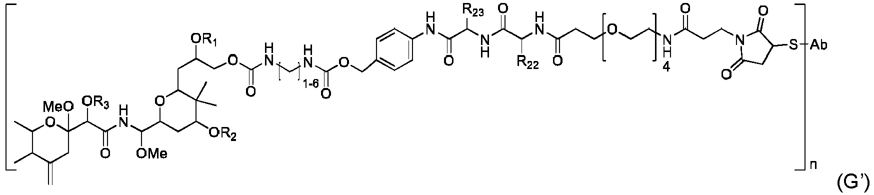 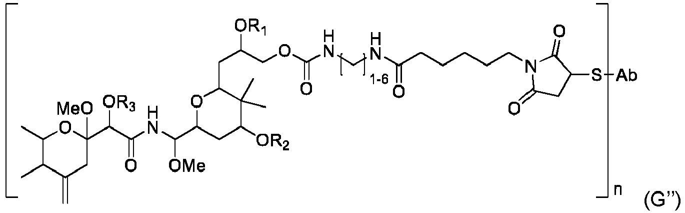 said process comprising the following steps:
(i) either:
   (a) reacting a compound of formula (Ib) as defined above with a compound of formula X₂-C(=O)-X₁ wherein X₁ and X₂ are leaving groups to give a compound of formula (B): 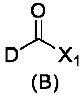 and the point of attachment of the -C(=O)X₁ moiety is the free primary -OH group of the compound of formula (Ib), or
   (b) reacting a compound of formula (Ib) as defined above with 4-nitro-phenylchloroformate to give a compound of formula (J): 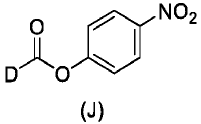 wherein the point of attachment of the (4-nitrophenyl)-O-CO- group is the same as that for the X₁(CO) moiety in (a) above; or
   (c) reacting a compound of formula (Ib) as defined above with an isocyanate of formula O=C=N-(CH₂)₁₋₆NHProt^{NH} wherein Prot^{NH} is a protecting group for amino suitable to be deprotected under basic conditions to give a compound of formula 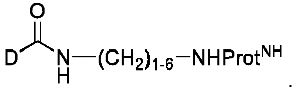
(ii) either
   (a) reacting the compound of formula (B) or (J) produced in step (i)(a) or (i)(b) with an amino compound of formula NH₂-(CH₂)₁₋₆NH₂ to give a compound of formula (C): 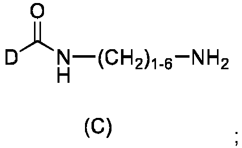 ; or
   (b)deprotecting the compound of formula 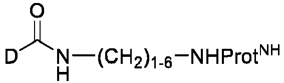 obtained in step (i)(c) to give a compound of formula (C).
(iii) reacting the compound of formula (C) with a compound of formula (D'), (E) or E': 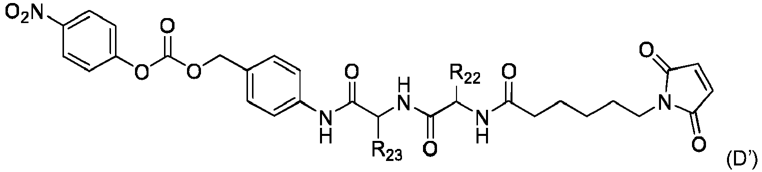 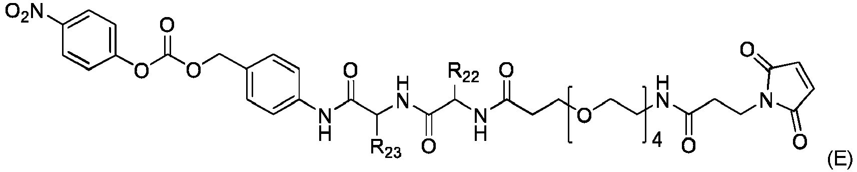 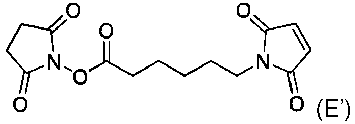
   wherein R₂₂ and R₂₃ are as defined above in the definitions of AA groups;
   to give a compound of formula (F), (F') or (F"), respectively: 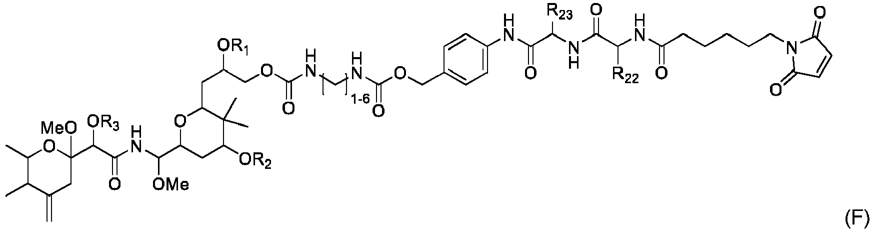 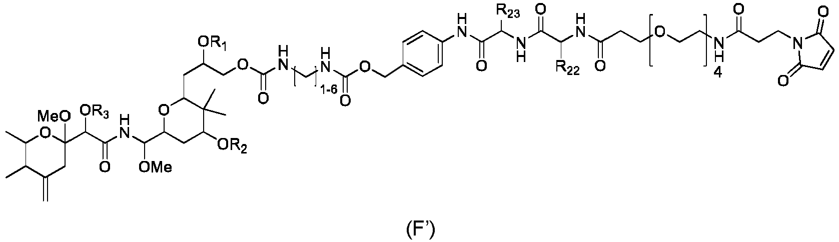 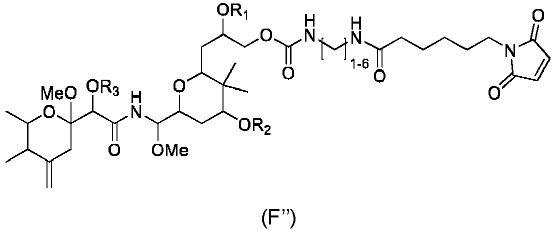
   wherein R₁, R₂ and R₃ are as defined above for the compounds of formula (Ib) and R₂₂ and R₂₃ are as defined above in the definitions of AA groups.
(iv) if protecting groups for OH are present in the compounds of formula (F), (F') or (F"), removing such protecting groups to give compounds of formula (F), (F') or (F") wherein R₁, R₂ and R₃ are as defined above for the compounds of formula (II).
(v) partial reduction of one or more disulfide bonds in the antibody to be conjugated to give a reduced antibody Ab-SH having free thiol groups: 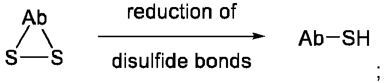 and
(vi) reacting the partially reduced antibody Ab-SH having free thiol groups with the compound of formula (F), (F') or (F") produced in step (iv) to give the desired drug antibody conjugate of formula (G), (G') or (G") respectively:

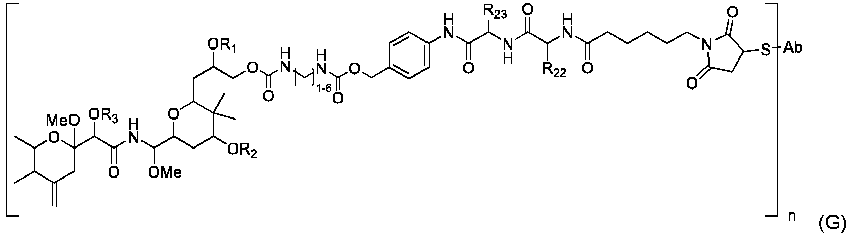

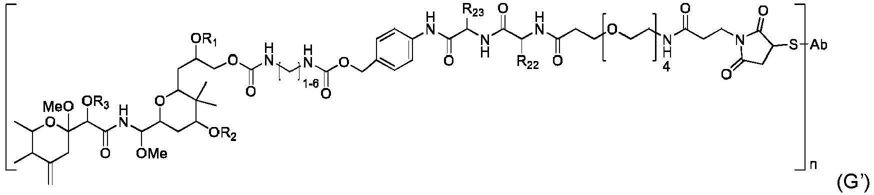

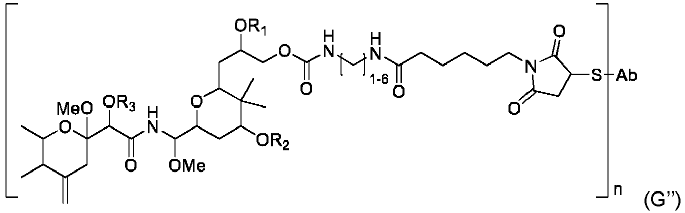

In another preferred embodiment of this process, the antibody is selected from Brentuximab, Gemtuzumab, Inozutumab, Rovalpituzumab, an anti-HER2 antibody such as Trastuzumab, an anti-CD4 antibody, an anti-CD5 antibody, and an anti-CD30 antibody, or an antigen-binding fragment or an immunologically active portion thereof, or it is an anti-HER2 antibody such as Trastuzumab or an antigen-binding fragment or an immunologically active portion thereof, and most preferably it is Trastuzumab or an antigen-binding fragment or an immunologically active portion thereof. Furthermore, the partial reduction of this monoclonal antibodody is performed using tris[2-carboxyethyl]phosphine hydrochloride (TCEP).

Another example of a process for the preparation of a drug conjugate of the present invention involves the preparation of drug antibody conjugates of formula (W), (W') or (W") of the present invention as follows: 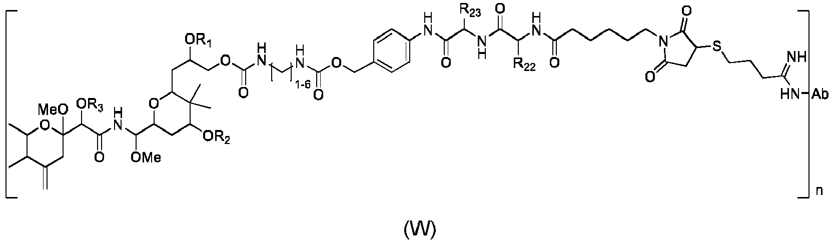 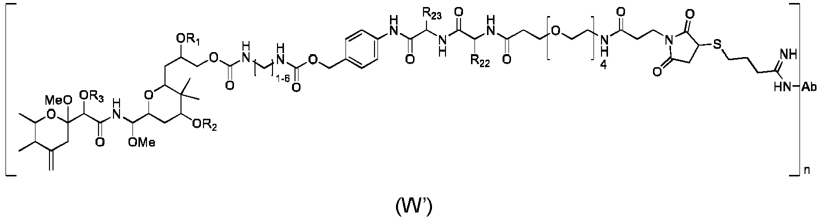 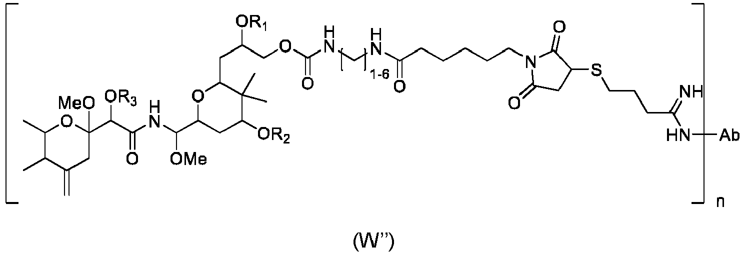 or a salt thereof, said process comprising the following steps:
(i) reacting the antibody with 2-iminothiolane hydrochloride (Traut's reagent) to give a thiol-activated antibody: 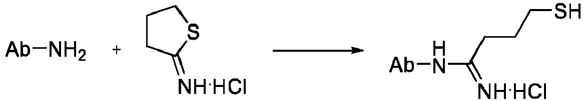
(ii) reacting the thiol-activated antibody with the compound of formula (F), (F') or (F"), to give the desired drug antibody conjugate of formula (W), (W') or (W"), respectively. 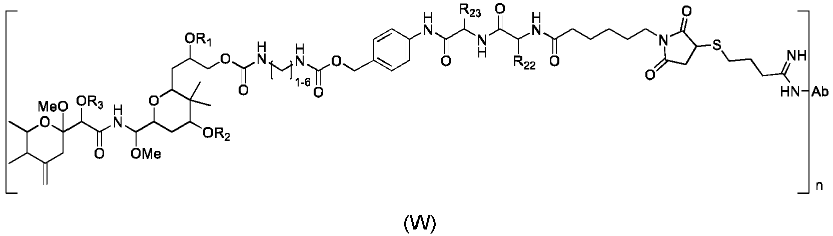 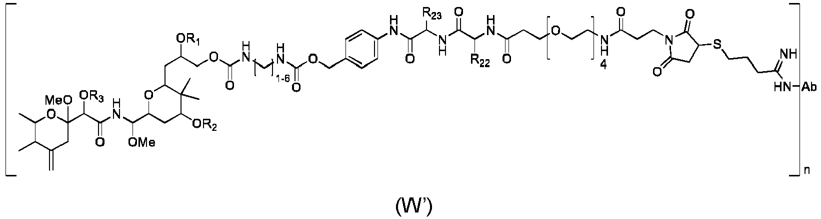 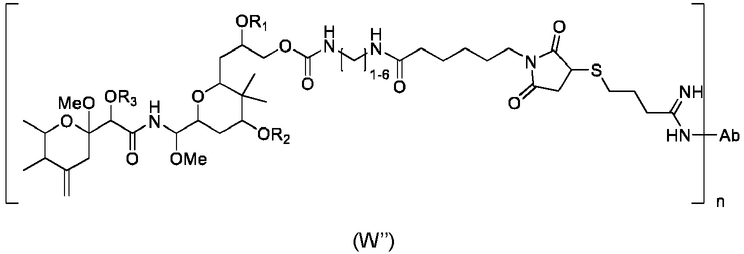 or a salt thereof, wherein R₁, R₂ and R₃ are as defined above in formula (II) and R₂₂ and R₂₃ are as defined above in the definitions of AA groups.

In another preferred embodiment of this process, the antibody is selected from Brentuximab, Gemtuzumab, Inozutumab, Rovalpituzumab, an anti-HER2 antibody such as Trastuzumab, an anti-CD4 antibody, an anti-CD5 antibody, an anti-CD30 antibody, or an antigen-binding fragment or an immunologically active portion thereof, or it is an anti-HER2 antibody such as Trastuzumab or an antigen-binding fragment or an immunologically active portion thereof, and most preferably it is Trastuzumab or an antigen-binding fragment or an immunologically active portion thereof.

Another example of a process for the preparation of a drug antibody conjugate of the present invention, involves the preparation of drug antibody conjugates of formula (O) or (P) as follows 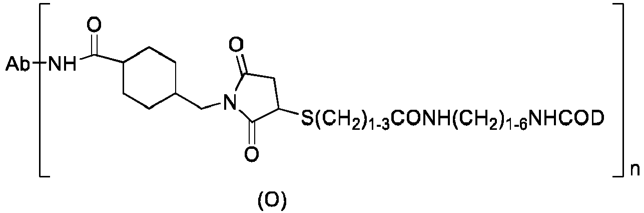 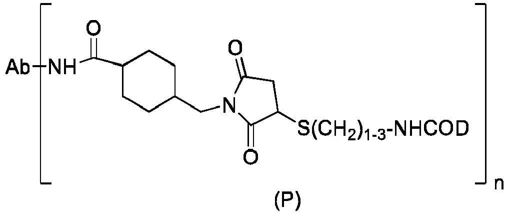 said process comprising the following steps:
(i) either
   (a) reacting the compound of formula (C) with a compound of formula Me-S-S-(CH₂)₁₋₃-CO₂H to give a compound of formula (K) 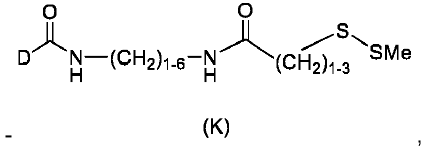 , or
   (b) reacting a compound (J) with a compound of formula NH₂-(CH₂)₁₋₃SProt^{SH} and removing the Prot^{SH} group from the coupled compound to give a compound of formula (L): 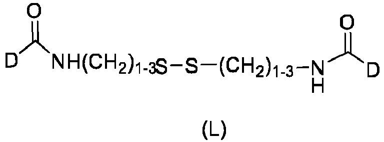
(ii) reacting (K) or (L) produced in step (i) with dithiothreitol under disulfide reducing conditions to give compounds of formula (M) and (N) respectively: 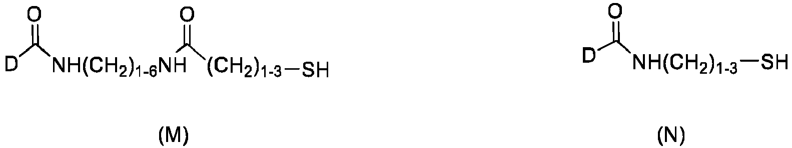
(iii) reacting the antibody to be conjugated with succininimidyl-4-(*N-*maleimidomethyl)cyclohexane-1-carboxylate to derivatise said antibody at one or more lysine groups with a succininimidyl-4-(*N*-maleimidomethyl)cyclohexane-1-carbonyl group: 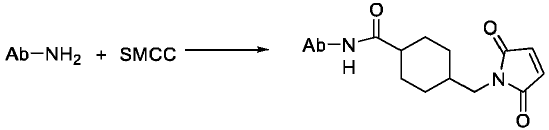
(iv) reacting the derivatised antibody produced in step (iii) with either (M) or (N) produced in step (ii) to give the desired drug antibody conjugate of formula (O) or (P):

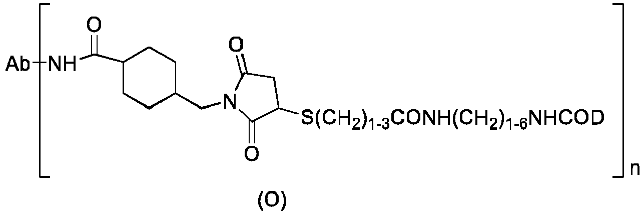

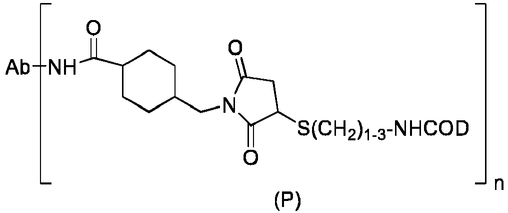

The compound of formula X₂-C(=O)-X₁ is preferably 1,1'-carbonyldiimidazole. Similarly, the amino compound reacted with the compound of formula (B) is preferably NH₂-(CH₂)₂₋₄-NH₂, and more preferably NH₂-(CH₂)₃-NH₂.

In one preferred embodiment of this invention, the compound reacted with the compound of formula (C) to give the compound of formula (K) is 3-(methyldisulfanyl)propanoic acid.

In another preferred embodiment, the compound NH₂-(CH₂)₁₋₃SProt^{SH} that is reacted with a compound of formula (J) to give a compound of formula (L) is NH₂-(CH₂)₃SProt^{SH}.

Where attachment to the drug linker moiety is via free thiol groups in cysteines after partial reduction of disulfide groups in the moiety comprising at least one antigen binding site such as a monoclonal antibody, the partial reduction is typically conducted by first diluting to a suitable concentration and buffering the solution before partial reduction of the disulfide bonds by means of the addition of a suitable reducing agent such as tris[2-carboxyethyl]phosphine hydrochloride (TCEP) or dithiothreitol (DTT). By choosing appropriate ratios of the moiety to be reduced such as a monoclonal antibody and the reducing agent, the reaction conditions and the time of the reduction it is possible to obtain a desired free thiol to moiety ratio, e.g. four free thiol groups per monoclonal antibody.

The partially reduced moiety such as the partially reduced monoclonal antibody having the free thiol groups, prepared as described above, is then reacted with drug-linker compounds of the invention of formula D-(X)_{b}-(AA)_{w}-(T)_{g}-L₁ (wherein the group L₁ in such compound is a maleimide group which is free to react with the thiol groups). The resulting drug antibody conjugates are purified by any suitable means known in the art, e.g. by size exclusion chromatography (SEC) [see, e.g., Liu et al., Proc. Natl. Acad. Sci. USA, 93: 8618-8623 (1996), and Chari et al., Cancer Research, 52: 127-131 (1992)].

In one preferred embodiment of this invention, the partially reduced monoclonal antibody is an anti-HER2 antibody such as Trastuzumab or an antigen-binding fragment or an immunologically active portion thereof, preferably Trastuzumab or an antigen-binding fragment or an immunologically active portion thereof.

In an alternative embodiment of the invention, lysines in the moiety comprising at least one antigen binding site such as a monoclonal antibody can first be reacted with succinimidyl-4-(*N*-maleimidomethyl)cyclohexane-1-carboxylate. A free amine group on an antibody can react with the N-hydroxysuccinimide ester to give a maleimide-activated antibody:

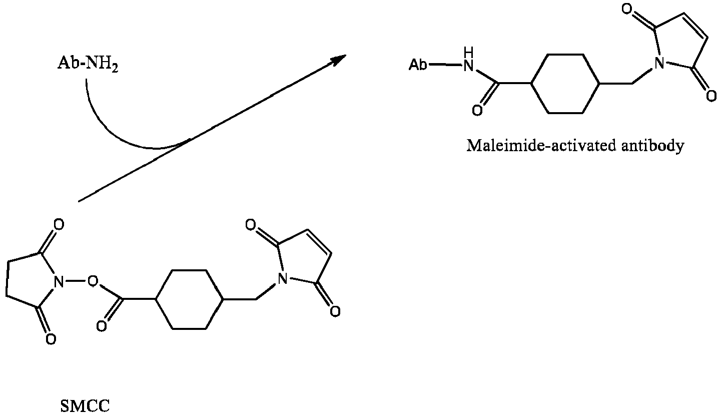

The maleimide-activated antibody can then be reacted with a compound of formula D-(X)_{b}-(AA)_{w}-(T)_{g}-H having a reactive thiol moiety.

In an alternative embodiment of the invention, lysines in the moiety comprising at least one antigen binding site such as a monoclonal antibody can first be reacted with 2-iminothiolane hydrochloride (Traut's reagent). A free amine group on an antibody can react with the imidic thiolactone to give a thiol-activated antibody.

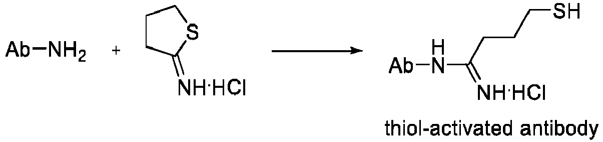

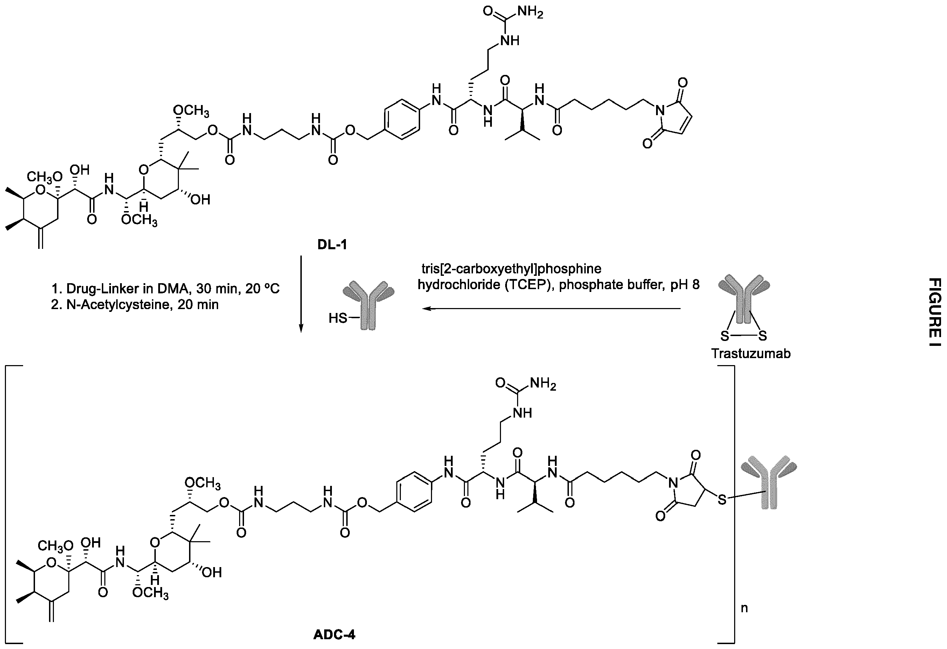

One specific example of processes for the preparation of drug antibody conjugates of formula [D-(X)_{b}-(AA)_{w}-(T)_{g}-(L)-]ₙ-Ab of the present invention by conjugation via free thiol groups in cysteines after partial reduction of disulfide groups in the antibody is shown in Figure 1.

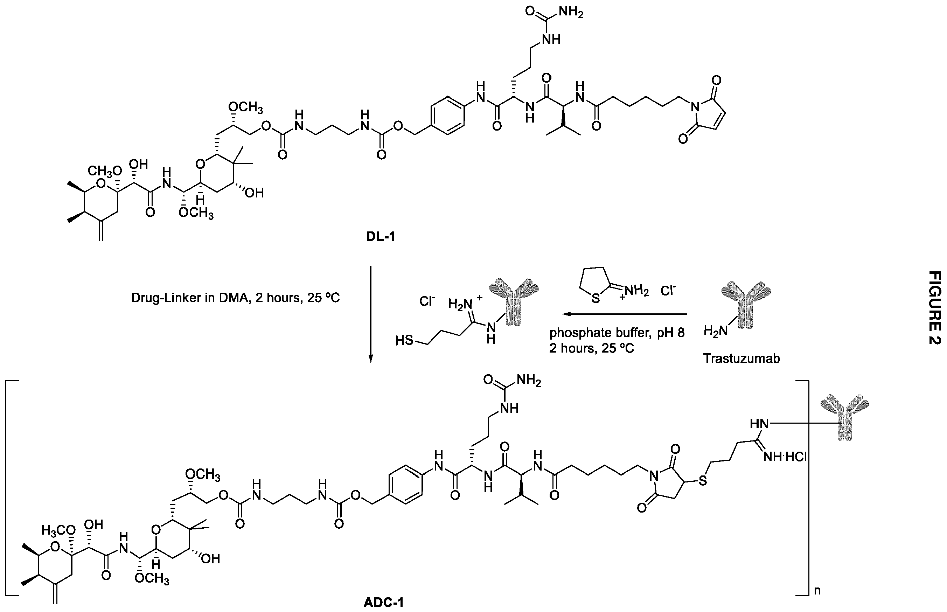

Another specific example of processes for the preparation of drug antibody conjugates of formula [D-(X)_{b}-(AA)_{w}-(T)_{g}-(L)-]ₙ-Ab of the present invention by conjugation with free amino groups in lysines after reaction of the antibody with Traut's reagent is shown in Figure 2.

### Compositions Comprising the Drug Antibody Conjugate of the Invention and Uses Thereof

There is also provided a pharmaceutical composition comprising a drug conjugate according to the present invention and a pharmaceutically acceptable carrier. Examples of the administration form of a drug conjugate having the general formula [D-(X)_{b}-(AA)_{w}-(T)_{g}-(L)-]ₙ-Ab of the present invention include without limitation oral, topical, parenteral, sublingual, rectal, vaginal, ocular, and intranasal. Parenteral administration includes subcutaneous injections, intravenous, intramuscular, intrasternal injection or infusion techniques. Preferably, the compositions are administered parenterally. Pharmaceutical compositions of the invention can be formulated so as to allow a drug conjugate of the present invention to be bioavailable upon administration of the composition to an animal, preferably human. Compositions can take the form of one or more dosage units, where for example, a tablet can be a single dosage unit, and a container of a drug antibody conjugate of the present invention in aerosol form can hold a plurality of dosage units.

The pharmaceutically acceptable carrier or vehicle can be particulate, so that the compositions are, for example, in tablet or powder form. The carrier(s) can be liquid, with the compositions being, for example, an oral syrup or injectable liquid. In addition, the carrier(s) can be gaseous, so as to provide an aerosol composition useful in, for example, inhalatory administration. The term "carrier" refers to a diluent, adjuvant or excipient, with which a drug antibody conjugate of the present invention is administered. Such pharmaceutical carriers can be liquids, such as water and oils, including those of petroleum, animal, vegetable or synthetic origin, such as peanut oil, soybean oil, mineral oil, sesame oil and the like. The carriers can be saline, gum acacia, gelatin, starch paste, talc, keratin, colloidal silica, urea, and the like. In addition, auxiliary, stabilizing, thickening, lubricating and coloring agents can be used. In one embodiment, when administered to an animal, the drug antibody conjugates of the present invention or compositions and pharmaceutically acceptable carriers are sterile. Water is a preferred carrier when the drug antibody conjugates of the present invention are administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or pH buffering agents.

When intended for oral administration, the composition is preferably in solid or liquid form, where semi-solid, semi-liquid, suspension and gel forms are included within the forms considered herein as either solid or liquid.

As a solid composition for oral administration, the composition can be formulated into a powder, granule, compressed tablet, pill, capsule, chewing gum, wafer or the like form. Such a solid composition typically contains one or more inert diluents. In addition, one or more of the following can be present: binders such as carboxymethylcellulose, ethyl cellulose, microcrystalline cellulose, or gelatin; excipients such as starch, lactose or dextrins, disintegrating agents such as alginic acid, sodium alginate, corn starch and the like; lubricants such as magnesium stearate; glidants such as colloidal silicon dioxide; sweetening agents such as sucrose or saccharin; a flavoring agent such as peppermint, methyl salicylate or orange flavoring; and a coloring agent.

When the composition is in the form of a capsule (e. g. a gelatin capsule), it can contain, in addition to materials of the above type, a liquid carrier such as polyethylene glycol, cyclodextrin or a fatty oil.

The composition can be in the form of a liquid, e. g. an elixir, syrup, solution, emulsion or suspension. The liquid can be useful for oral administration or for delivery by injection. When intended for oral administration, a composition can comprise one or more of a sweetening agent, preservatives, dye/colorant and flavor enhancer. In a composition for administration by injection, one or more of a surfactant, preservative, wetting agent, dispersing agent, suspending agent, buffer, stabilizer and isotonic agent can also be included.

The preferred route of administration is parenteral administration including, but not limited to, intradermal, intramuscular, intraperitoneal, intravenous, subcutaneous, intranasal, epidural, intranasal, intracerebral, intraventricular, intrathecal, intravaginal or transdermal. The preferred mode of administration is left to the discretion of the practitioner, and will depend in part upon the site of the medical condition (such as the site of cancer). In a more preferred embodiment, the present drug antibody conjugates of the present invention are administered intravenously.

The liquid compositions of the invention, whether they are solutions, suspensions or other like form, can also include one or more of the following: sterile diluents such as water for injection, saline solution, preferably physiological saline, Ringer's solution, isotonic sodium chloride, fixed oils such as synthetic mono or digylcerides, polyethylene glycols, glycerin, or other solvents; antibacterial agents such as benzyl alcohol or methyl paraben; and agents for the adjustment of tonicity such as sodium chloride or dextrose. A parenteral composition can be enclosed in an ampoule, a disposable syringe or a multiple-dose vial made of glass, plastic or other material. Physiological saline is a preferred adjuvant.

The amount of the drug conjugate of the present invention that is effective in the treatment of a particular disorder or condition will depend on the nature of the disorder or condition, and can be determined by standard clinical techniques. In addition, *in vitro* or *in vivo* assays can optionally be employed to help identify optimal dosage ranges. The precise dose to be employed in the compositions will also depend on the route of administration, and the seriousness of the disease or disorder, and should be decided according to the judgment of the practitioner and each patient's circumstances.

The compositions comprise an effective amount of a drug conjugate of the present invention such that a suitable dosage will be obtained. The correct dosage of the compounds will vary according to the particular formulation, the mode of application, and its particular site, host and the diease being treated, e.g. cancer and, if so, what type of tumor. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

The drug conjugate of the present invention or compositions can be administered by any convenient route, for example by infusion or bolus injection, by absorption through epithelial or mucocutaneous linings.

In specific embodiments, it can be desirable to administer one or more drug conjugates of the present invention or compositions locally to the area in need of treatment. In one embodiment, administration can be by direct injection at the site (or former site) of a cancer, tumor or neoplastic or pre-neoplastic tissue. In another embodiment, administration can be by direct injection at the site (or former site) of a manifestation of an autoimmune disease.

Pulmonary administration can also be employed, e. g. by use of an inhaler or nebulizer, and formulation with an aerosolizing agent, or via perfusion in a fluorocarbon or synthetic pulmonary surfactant. In certain embodiments, the drug antibody conjugate of the present invention or compositions can be formulated as a suppository, with traditional binders and carriers such as triglycerides.

The present compositions can take the form of solutions, suspensions, emulsion, tablets, pills, pellets, capsules, capsules containing liquids, powders, sustained- release formulations, suppositories, emulsions, aerosols, sprays, suspensions, or any other form suitable for use. Other examples of suitable pharmaceutical carriers are described in "Remington's Pharmaceutical Sciences" by E. W. Martin.

The pharmaceutical compositions can be prepared using methodology well known in the pharmaceutical art. For example, a composition intended to be administered by injection can be prepared by combining a drug conjugate of the present invention with water so as to form a solution. A surfactant can be added to facilitate the formation of a homogeneous solution or suspension.

We have found that the drug conjugates and compositions of the present invention are particularly effective in the treatment of cancer.

Thus, as described earlier, the present invention provides a drug conjugate according to the present invention for use in the treatment of cancer, preferably a cancer selected from lung cancer, including NSCLC, gastric cancer, colorectal cancer, breast cancer, pancreas carcinoma, endometrial cancer, bladder cancer, cervical cancer, esophageal cancer, gallbladder cancer, uterine cancer, salivary duct cancer, ovarian cancer, kidney cancer, leukaemia, multiple myeloma, and lymphoma, wherein the cancer is preferably a HER2 positive cancer, wherein the HER2 positive cancers include HER2 positive lung cancer including HER2 positive NSCLC, HER2 positive gastric cancer, HER2 positive colorectal cancer, HER2 positive breast cancer, HER2 positive pancreas carcinoma, HER2 positive endometrial cancer, HER2 positive bladder cancer, HER2 positive cervical cancer, HER2 positive esophageal cancer, HER2 positive gallbladder cancer, HER2 positive uterine cancer, HER2 positive salivary duct cancer and HER2 positive ovarian cancer, more preferably HER2 positive breast cancer, HER2 positive ovarian cancer and HER2 positive gastric cancer, most preferably HER2 positive breast cancer.

The drug conjugates and compositions of the present invention are useful for inhibiting the multiplication of a tumor cell or cancer cell, or for treating cancer in an animal. The drug conjugates and compositions of the present invention can be used accordingly in a variety of settings for the treatment of animal cancers. The conjugates of the invention comprising Drug -Linker-Moiety comprising at least one antigen binding site can be used to deliver a Drug or Drug unit to a tumor cell or cancer cell. Without being bound by theory, in one embodiment, the Moiety comprising at least one antigen binding site of a drug conjugate of the present invention binds to or associates with a cancer-cell or a tumor-cell-associated antigen, and the drug conjugate of the present invention can be taken up inside a tumor cell or cancer cell through receptor-mediated endocytosis. The antigen can be attached to a tumor cell or cancer cell or can be an extracellular matrix protein associated with the tumor cell or cancer cell. Once inside the cell, one or more specific sequences within the Linker unit are hydrolytically cleaved by one or more tumor-cell or cancer-cell-associated proteases or hydrolases, resulting in release of a Drug or a Drug-Linker Compound. The released Drug or Drug-Linker Compound is then free to migrate in the cell and induce cytotoxic activities. In an alternative embodiment, the Drug or Drug unit is cleaved from the drug conjugate of the present invention outside the tumor cell or cancer cell, and the Drug or Drug-Linker Compound subsequently penetrates the cell.

In one embodiment, the Moiety comprising at least one antigen binding site binds to the tumor cell or cancer cell. In another embodiment, the Moiety comprising at least one antigen binding site binds to a tumor cell or cancer cell antigen which is on the surface of the tumor cell or cancer cell. In yet another embodiment, the Moiety comprising at least one antigen binding site binds to a tumor cell or cancer cell antigen which is an extracellular matrix protein associated with the tumor cell or cancer cell.

The specificity of the Moiety comprising at least one antigen binding site for a particular tumor cell or cancer cell can be important for determining those tumors or cancers that are most effectively treated. For example, drug conjugates of the present invention having a Trastuzumab unit can be useful for treating antigen positive carcinomas including leukaemias, lung cancer, colon cancer, lymphomas (e.g. Hodgkin's disease, non-Hodgkin's Lymphoma), solid tumors such as, sarcoma and carcinomas, Multiple myeloma, kidney cancer and melanoma. The cancer may preferably be lung cancer, colorectal cancer, breast cancer, pancreas carcinoma, kidney cancer, leukaemia, multiple myeloma, lymphoma or ovarian cancer. For example, drug conjugates of the present invention having a Rituximab unit can be useful for treating CD-20 expressing tumors such as haematological cancers including leukemias and lymphomas. For example, drug conjugates of the present invention having an anti-CD4 antibody unit can be useful for treating CD-4 expressing tumors such as haematological cancers including lymphomas. For example, drug conjugates of the present invention having an anti-CD5 antibody unit can be useful for treating CD-5 expressing tumors such as haematological cancers including leukemias and lymphomas.

Other particular types of cancers that can be treated with drug conjugates of the present invention include, but are not limited to: blood-borne cancers including all forms of leukemia; lymphomas, such as Hodgkin's disease, non-Hodgkin's Lymphoma and Multiple myeloma.

In particular, the drug conjugates and compositions of the present invention show excellent activity in the treatment of breast cancer.

Drug conjugates and compositions of the present invention provide conjugation specific tumor or cancer targeting, thus reducing general toxicity of these conjugates. The Linker units stabilize the drug antibody conjugates in blood, yet are cleavable by tumor-specific proteases and hydrolases within the cell, liberating a Drug.

The drug conjugates and compositions of the present invention can be administered to an animal that has also undergone surgery as treatment for the cancer. In one embodiment of the present invention, the additional treatment is radiation therapy.

In a specific embodiment of the present invention, the drug conjugate or composition of the present invention may be administered with radiotherapy. Radiotherapy may be administered at the same time, prior to or after treatment with the drug conjugate or composition of the present invention. In an embodiment, the drug conjugate or composition of the present invention is administered concurrently with radiation therapy. In another specific embodiment, the radiation therapy is administered prior or subsequent to administration of a drug conjugate or composition of the present invention, preferably at least an hour, five hours, 12 hours, a day, a week, a month, more preferably several months (e. g. up to three months), prior or subsequent to administration of a drug antibody conjugate or composition of the present invention.

With respect to radiation, any radiation therapy protocol can be used depending upon the type of cancer to be treated. For example, but not by way of limitation, x-ray radiation can be administered; in particular, high-energy megavoltage (radiation of greater that 1 MeV energy) can be used for deep tumors, and electron beam and orthovoltage x-ray radiation can be used for skin cancers. Gamma-ray emitting radioisotopes, such as radioactive isotopes of radium, cobalt and other elements, can also be administered.

In the present invention, there is provided a kit comprising a therapeutically effective amount of a drug conjugate according to the present invention and a pharmaceutically acceptable carrier. In an embodiment, there is provided a kit comprising a composition according to the present invention and, optionally, instructions for use in the treatment of cancer, preferably a cancer selected from lung cancer, including NSCLC, gastric cancer, colorectal cancer, breast cancer, pancreas carcinoma, endometrial cancer, bladder cancer, cervical cancer, esophageal cancer, gallbladder cancer, uterine cancer, salivary duct cancer, ovarian cancer, kidney cancer, leukaemia, multiple myeloma, and lymphoma, wherein the cancer is preferably a HER2 positive cancer, wherein the HER2 positive cancers include HER2 positive lung cancer including HER2 positive NSCLC, HER2 positive gastric cancer, HER2 positive colorectal cancer, HER2 positive breast cancer, HER2 positive pancreas carcinoma, HER2 positive endometrial cancer, HER2 positive bladder cancer, HER2 positive cervical cancer, HER2 positive esophageal cancer, HER2 positive gallbladder cancer, HER2 positive uterine cancer, HER2 positive salivary duct cancer and HER2 positive ovarian cancer, more preferably HER2 positive breast cancer, HER2 positive ovarian cancer and HER2 positive gastric cancer, most preferably HER2 positive breast cancer.

In one embodiment, the kit according to this aspect is for use in the treatment of cancer, preferably a cancer selected from lung cancer, including NSCLC, gastric cancer, colorectal cancer, breast cancer, pancreas carcinoma, endometrial cancer, bladder cancer, cervical cancer, esophageal cancer, gallbladder cancer, uterine cancer, salivary duct cancer, ovarian cancer, kidney cancer, leukaemia, multiple myeloma, and lymphoma, wherein the cancer is preferably a HER2 positive cancer, wherein the HER2 positive cancers include HER2 positive lung cancer including HER2 positive NSCLC, HER2 positive gastric cancer, HER2 positive colorectal cancer, HER2 positive breast cancer, HER2 positive pancreas carcinoma, HER2 positive endometrial cancer, HER2 positive bladder cancer, HER2 positive cervical cancer, HER2 positive esophageal cancer, HER2 positive gallbladder cancer, HER2 positive uterine cancer, HER2 positive salivary duct cancer and HER2 positive ovarian cancer, more preferably HER2 positive breast cancer, HER2 positive ovarian cancer and HER2 positive gastric cancer, most preferably HER2 positive breast cancer. Most preferred kit is for use in the treatment of HER2 positive breast cancer.

### Brief Description of the Drawings

The invention is diagrammatically illustrated, by way of example, in the accompanying drawings in which:
Figure 1 is a schematic illustration of one process according to the present invention wherein conjugation to the antibody is via free thiol groups;
Figure 2 is a schematic illustration of one process according to the present invention wherein conjugation to the antibody is via free amino groups.
Figure 3. Tumor volume evaluation of BT-474 tumors in mice treated with placebo, Herceptin, Kadcyla (both at 30 mg/kg) and ADC3 (at 4 mg/kg).

### Examples

The present invention is further illustrated by way of the following, non-limiting examples. In the examples, the following abbreviations are used:
CDI, 1,1'-carbonyldiimidazole
DIPEA, diisopropylethylamine
Hex, hexane
EtOAc, ethyl acetate
DCM, dichloromethane
NMP, N-methyl-2-pyrrolidone
DMF, dimethylformamide
EDC, *N*-(3-dimethylaminopropyl)-*N'-*ethylcarbodiimide hydrochloride
EDTA, ethylenediaminetetraacetic acid
MeOH, methanol
DTT, dithiothreitol
Py, pyridine
THF, tetrahydrofuran
TCEP, Tris[2-carboxyethyl]phosphine hydrochloride
MC, 6-maleimidocaproyl
Fmoc, 9-fluorenylmethoxycarbonyl
Cit, citrulline
Val, valine
DMSO, dimethylsulfoxide
Trt, triphenylmethyl
HOBt, 1-hydroxybenzotriazole
DIPCDI, *N,N*'-diisopropylcarbodiimide
TFA, trifluoroacetic acid
PABOH, 4-aminobenzyl alcohol
bis-PNP, bis(4-nitrophenyl) carbonate
NAC, N-Acetylcysteine
SEC, size-exclusion chromatography
HPLC, high performance liquid chromatography
ADC, antibody drug conjugate
ATCC, American Type Culture Collection
DMEM, Dulbecco's Modified Eagle's Medium
RPMI, Rosmell Park Memorial Institute medium
ITS, Insulin-transferrin-sodium selenite media supplement
FCS, Fetal Calf Serum
SRB, sulforhodamine B
PBS, phosphate buffered saline
DR, dose-response
UV, ultraviolet
SMCC, Succinimidyl-4-(*N*-maleimidomethyl)cyclohexane-1-carboxylate
LAR, Linker to Antibody Ratio

### Synthesis of intermediates

Compounds 1 and 2 were obtained following the procedures described in WO2018167270. 6-Maleimidohexanoic acid N-hydroxysuccinimide ester was obtained following the procedure described in Org. Biomol. Chem. 2009, 7, 3400-3406.

### Intermediate 7

a) 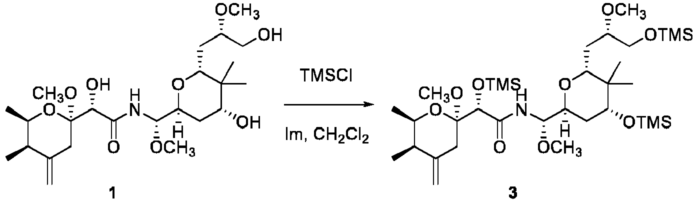 To a solution of 1 (19.2 mg, 0.039 mmol) and imidazole (10 eq, 0.39 mmol) in CH₂Cl₂ (3 mL) was added chlorotrimethylsilane (6 eq., 0.234 mmol) at 23 °C. After being stirred at 23 °C for 1h, the reaction mixture was concentrated under vacuum. The residue obtained was purified in a system for flash chromatography (SiO₂, Hex:EtOAc, from 90:10 to 70:30) to afford pure 3 (22 mg, 80% yield) as colourless oil.
   ¹H NMR (400 MHz, (CD₃)₂CO): δ 7.45 (d, *J* = 10.0 Hz, 1H), 5.33 (dd, *J* = 10.0, 6.8 Hz, 1H), 4.77 (t, *J* = 2.2 Hz, 1H), 4.61 (t, *J* = 2.2 Hz, 1H), 4.37 (d, *J* = 0.7 Hz, 1H), 3.90-3.80 (m, 2H), 3.74-3.69 (m, 2H), 3.53 (dd, *J* = 11.3, 5.8 Hz, 1H), 3.36 (s, 3H), 3.35-3.31 (m, 2H), 3.32 (s, 3H), 3.29 (s, 3H), 2.59 (d, *J* = 14.7 Hz, 1H), 2.51 (d, *J* = 14.7 Hz, 1H), 2.23 (qd, *J* = 7.0, 2.5 Hz, 1H), 1.97-1.92 (m, 1H), 1.82-1.77 (m, 1H), 1.71-1.53 (m, 2H), 1.20 (d, *J* = 6.6 Hz, 3H), 1.03 (d, *J* = 7.0, 3H), 0.92 (s, 3H), 0.85 (s, 3H), 0.24 (s, 9H), 0.14 (s, 9H), 0.11 (s, 9H).
   ¹³C NMR (100 MHz, (CD₃)₂CO): δ 170.8, 147.7, 108.7, 99.2, 78.9 (x3), 78.3, 76.0, 72.5, 69.0, 63.5, 55.9, 55.0, 49.6, 41.3, 38.2, 36.3, 30.5, 29.7, 23.7, 19.0, 17.4, 11.4, -0.2, -0.6, -0.9.
   (+)-HRESI-TOFMS m/z: 728.4108 [M + Na]⁺ (calcd. for C₃₃H₆₇NO₉Si₃Na: 728.4021).
b) 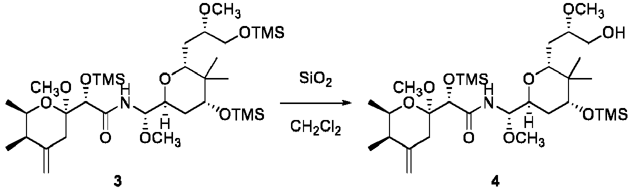 To a solution of 3 (20 mg, 0.028 mmol) in CH₂Cl₂ (30 mL) was added SiO₂ (2.5 g) at 23 °C. After being stirred at 23 °C for 2.5h, an extra amount of SiO₂ was added again (1.5 g) and stirred for 3 h. The solid was filtered and washed with EtOAc (100 mL). The filtrate was concentrated under reduced pressure and then purified in a system for flash chromatography (SiO₂, Hex:EtOAc, from 80:20 to 60:40) to afford pure 4 (15 mg, 85% yield) as colourless oil.
   ¹H NMR (400 MHz, (CD₃)₂CO): δ 7.50 (d, *J* = 10.0 Hz, 1H), 5.41-5.33 (m, 1H), 4.80-4.74 (m, 1H), 4.61-4.60 (m, 1H), 4.45 (s, 1H), 3.90-3.80 (m, 2H), 3.73 (dd, *J* = 10.1, 4.2 Hz, 1H), 3.67-3.56 (m, 1H), 3.43-3.27 (m, 4H), 3.37 (m, 3H), 3.32 (s, 3H), 3.29 (s, 3H), 2.58-2.48 (m, 2H), 2.27-2.17 (m, 1H), 2.01-1.92 (m, 1H), 1.81-1.61 (m, 2H), 1.51 (ddd, *J* = 14.2, 9.6, 1.8 Hz, 1H), 1.20 (d, *J* = 6.6 Hz, 3H), 1.02 (d, *J* = 7.0 Hz, 3H), 0.91 (s, 3H), 0.84 (s, 3H), 0.24 (s, 9H), 0.14 (s, 9H).
   ¹³C NMR (100 MHz, (CD₃)₂CO): δ 171.4, 147.7, 108.6, 99.2, 79.6, 78.8, 78.1, 75.6, 72.3, 69.1, 63.5, 59.6, 55.7, 55.0, 49.5, 41.3, 38.3, 36.2, 30.4, 23.3, 19.9, 17.4, 13.6, 11.3, -0.3, - 0.6.
   (+)-HRESI-TOFMS m/z: 656.3718 [M + Na]⁺ (calcd. for C₃₀H₅₉NO₉Si₂Na: 656.3626).
c) 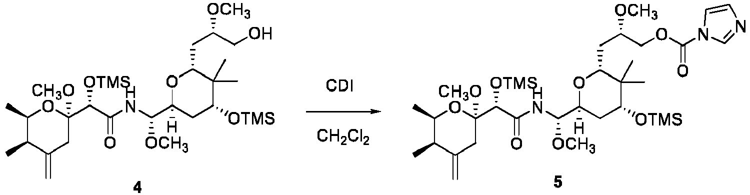 To a solution of 4 (18 mg, 0.028 mmol) in CH₂Cl₂ (3 mL) was added 1,1'-carbonyldiimidazole (6 eq, 0.168 mmol) at 23 °C. After being stirred overnight at 23 °C, the reaction mixture was concentrated under vacuum. The residue obtained was purified in a system for flash chromatography (SiO₂, Hex:EtOAc, from 80:20 to 50:50) to yield pure 5 (17 mg, 85% yield) as colourless oil.
   ¹H NMR (400 MHz, (CD₃)₂CO): δ 8.14 (s, 1H), 7.52 (s, 1H), 7.45 (d, *J* = 10.0 Hz, 1H), 7.05 (s, 1H), 5.35 (dd, *J* = 9.9, 6.8 Hz, 1H), 4.74 (d, *J* = 2.4 Hz, 1H), 4.66 (dd, *J* = 11.8, 2.4 Hz, 1H), 4.59-4.57 (m, 1H), 4.37 (ddd, *J* = 11.8, 5.9, 1.3 Hz, 1H), 4.28 (s, 1H), 3.90 (q, *J* = 5.4 Hz, 1H), 3.86-3.78 (m, 1H), 3.76 (dd, *J* = 9.7, 4.1 Hz, 1H), 3.64 (td, *J* = 6.4, 2.9 Hz, 1H), 3.55-3.48 (m, 1H), 3.36 (s, 6H), 3.30 (s, 3H), 2.45 (d, *J* = 14.5 Hz, 1H), 2.33 (d, *J* = 14.7 Hz, 1H), 2.19 (dd, *J* = 10.1, 5.0 Hz, 1H), 2.02-1.88 (m, 2H), 1.79-1.60 (m, 2H), 1.18 (d, *J* = 6.5, 3H), 1.05-0.93 (m, 6H), 0.88 (d, *J* = 1.2 Hz, 3H), 0.23 (s, 9H), 0.14 (s, 9H).
   ¹³C NMR (100 MHz, (CD₃)₂CO): δ 170.9, 148.6, 147.6, 136.8, 130.5, 117.1, 108.7, 99.1, 79.0, 78.0, 76.2 75.5, 72.2, 69.1, 68.2, 59.6, 56.1, 54.9, 49.4, 41.3, 38.2, 35.8, 30.3, 29.7, 23.4, 17.3, 13.6, 11.4, -0.4, -0.6.
   (+)-HRESI-TOFMS m/z: 750.3975 [M + Na]⁺ (calcd. for C₃₄H₆₁N₃O₁₀Si₂Na: 750.3793).
d) 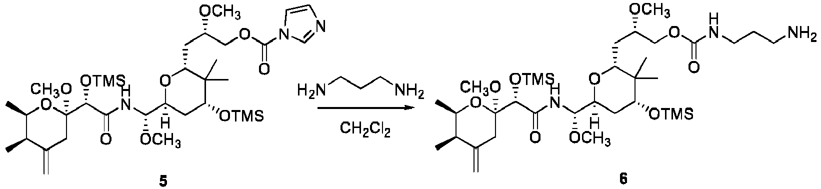 To a solution of 5 (17 mg, 0.023 mmol) in CH₂Cl₂ (2 mL) was added propane 1,3-diamine (16.6 eq, 32 µL) at 23 °C. The reaction mixture was stirred at 23 °C overnight and concentrated under vacuum. The residue obtained was purified in a system for flash chromatography (Si-NH₂, CH₂Cl₂:CH₃OH, from 100:0 to 90:10) to obtain pure 6 (16 mg, 94% yield) as colourless oil.
   ¹H NMR (400 MHz, CD₃OD): δ 5.20 (d, *J* = 4.4 Hz, 1H), 4.84-4.77 (m, 1H), 4.65-4.62 (m, 1H), 4.35 (s, 1H), 4.31-4.21 (m, 1H), 4.04 (dd, *J =* 12.0, 5.1 Hz, 1H), 3.96-3.92 (m, 1H), 3.84 (qd, *J* = 6.5, 2.5 Hz, 1H), 3.71 (dd, *J* = 8.2, 3.9 Hz, 1H), 3.55-3.48 (m, 2H), 3.39 (d, *J* = 0.7 Hz, 3H), 3.35 (s, 3H), 3.29 (s, 3H), 3.17 (t, *J* = 6.7 Hz, 2H), 2.76 (t, *J* = 7.1 Hz, 2H), 2.61 (d, *J* = 14.6 Hz, 1H), 2.36 (d, *J* = 14.6 Hz, 1H), 2.25-2.13 (m, 1H), 2.06-2.03 (m, 1H), 2.00-1.86 (m, 2H), 1.73-1.55 (m, 3H), 1.16 (d, *J* = 6.6 Hz, 3H), 0.98 (d, *J* = 7.0 Hz, 3H), 0.94 (s, 3H), 0.85 (s, 3H), 0.20 (s, 9H), 0.13 (s, 9H).
   ¹³C NMR (100 MHz, CD₃OD): δ 172.4, 157.8, 147.1, 108.6, 99.7, 81.7, 77.2, 76.3, 75.7, 72.8, 70.8, 69.5, 64.1, 55.7, 55.2, 48.6, 41.4, 37.7, 37.6, 37.4, 34.3, 30.8, 30.0, 29.1, 23.9, 16.9, 15.6, 10.9, -1.1, -1.2.
   (+)-HRESI-TOFMS m/z: 734.4526 [M + H]⁺ (calcd. for C₃₄H₆₇N₃O₁₀Si₂: 733.4365).
e) 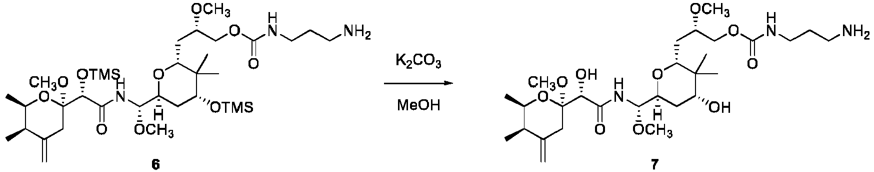 To a solution of 6 (16 mg, 0.022 mmol) in MeOH (4 mL) was added 2.4 mL of 0.033 M potassium carbonate solution in methanol (3.65 eq, 0.08 mmol) at 23 °C. The reaction mixture was stirred at 23 °C overnight and concentrated under vacuum. The residue obtained was purified in a system for flash chromatography (Si-NH₂, CH₂Cl₂:CH₃OH, from 100:0 to 80:20) to afford pure 7 (12.8 mg, 99% yield) as colourless oil.
   ¹H NMR (400 MHz, CD₃OD): δ 5.49 (d, *J* = 1.0 Hz, 1H), 5.30 (d, *J* = 6.7 Hz, 1H), 4.83-4.79 (m, 1H), 4.67-4.63 (m, 1H), 4.28 (d, *J* = 0.8 Hz, 1H), 4.20 (dd, *J* = 11.8, 3.0 Hz, 1H), 4.03 (dd, *J* = 11.8, 5.1 Hz, 1H), 3.94-3.83 (m, 2H), 3.61 (dd, *J* = 10.5, 4.6 Hz, 1H), 3.57-3.51 (m, 1H), 3.39 (s, 3H), 3.33 (s, 3H), 3.28 (d, *J* = 0.8 Hz, 3H), 3.17 (t, *J* = 6.7 Hz, 2H), 2.70 (t, *J* = 7.0 Hz, 2H), 2.47 (d, *J* = 14.3 Hz, 1H), 2.35 (d, *J* = 14.3 Hz, 1H), 2.27-2.13 (m, 1H), 2.04-1.94 (m, 1H), 1.78-1.60 (m, 5H), 1.18 (dd, *J* = 6.6, 0.8 Hz, 3H), 0.98 (dd, *J* = 7.0, 0.8 Hz, 3H), 0.93 (s, 3H), 0.86 (s, 3H).
   ¹³C NMR (100 MHz, CD₃OD): δ 172.8, 157.8, 146.8, 108.8, 99.8, 80.3, 77.0, 75.7, 72.7, 71.8, 71.1, 69.3, 64.4, 55.7, 55.3, 53.4, 41.5, 38.0, 37.5, 33.5, 31.7, 29.9, 29.0, 22.5, 16.7, 13.0, 12.8, 11.1.
   (+)-HRESI-TOFMS m/z: 590.3687 [M + H]⁺ (calcd. For C₂₈H₅₁N₃O₁₀: 589.3574).

### Intermediate 12

a) 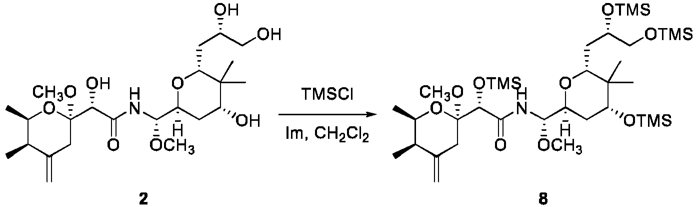 To a solution of 2 (26 mg, 0.055 mmol) and imidazole (13 eq, 0.715 mmol) in CH₂Cl₂ (3 mL) was added chlorotrimethylsilane (8 eq., 0.44 mmol) at 23 °C. After being stirred at 23 °C for 1 h, the reaction mixture was concentrated under vacuum. The residue obtained was purified in a system for flash chromatography (SiO₂, Hex:EtOAc, from 90:10 to 70:30) ) to afford pure 8 (26 mg, 60% yield) as colourless oil.
   ¹H NMR (400 MHz, (CD₃)₂CO): δ 7.46 (d, *J* = 9.9 Hz, 1H), 5.21 (dd, *J* = 9.9, 4.4 Hz, 1H), 4.78-4.76 (m, 1H), 4.61-4.59 (m, 1H), 4.35 (d, *J* = 0.6 Hz, 1H), 4.01-3.92 (m, 2H), 3.88-3.82 (m, 1H), 3.72 (dd, *J* = 7.3, 3.6 Hz, 1H), 3.68-3.61 (m, 1H), 3.56-3.45 (m, 2H), 3.35 (s, 3H), 3.31 (s, 3H), 2.62-2.49 (m, 2H), 2.22 (qd, *J =* 7.0, 2.6 Hz, 1H), 2.10-2.02 (m, 1H), 1.91 (ddd, *J* = 13.5, 7.0, 3.7 Hz, 1H), 1.67 (ddd, *J* = 14.1, 9.9, 2.7 Hz, 1H), 1.59-1.47 (m, 1H), 1.20 (dd, *J* = 6.6, 0.7 Hz, 3H), 1.02 (dd, *J* = 7.0, 0.7 Hz, 3H), 0.97 (s, 3H), 0.85 (s, 3H), 0.23 (s ,9H), 0.13 (s ,9H), 0.12 (s ,9H), 0.11 (s ,9H).
   ¹³C NMR (100 MHz, (CD₃)₂CO): δ 170.7, 147.7, 108.6, 99.3, 80.3, 77.8, 76.6, 72.9, 70.5, 69.1, 65.8, 55.0, 49.4, 41.3, 37.5, 35.9, 33.2, 30.9, 24.9, 17.4, 11.3, -0.2, -0.3, -0.6, -0.9.
b) 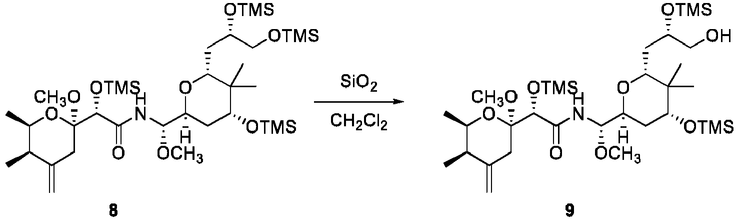 To a solution of 8 (40 mg, 0.052 mmol) in CH₂Cl₂ (30 mL) was added SiO₂ (2.5 g) at 23 °C. After being stirred at 23 °C for 2.5 h, an extra amount of SiO₂ was added again (1.5 g) and stirred for 3 h. The solid was filtered and washed with EtOAc (100 mL). The filtrate was concentrated under reduced pressure and then purified in a system for flash chromatography (SiO₂, Hex:EtOAc, from 80:20 to 60:40) to afford pure 9 (16 mg, 44% yield) as colourless oil.
   ¹H NMR (400 MHz, (CD₃)₂CO): δ 7.52 (d, *J* = 9.9 Hz, 1H), 5.26 (dd, *J* = 9.9, 5.4 Hz, 1H), 4.76-4.78 (m, 1H), 4.59-4.61 (m, 1H), 4.39 (s, 1H), 4.01-3.90 (m, 2H), 3.88-3.82 (m, 1H), 3.71 (dd, *J* = 8.1, 3.8 Hz, 1H), 3.63-3.49 (m, 2H), 3.39-3.28 (m, 2H), 3.36 (s, 3H), 3.31 (s, 3H), 2.62-2.54 (m, 2H), 2.25-2.19 (m, 1H), 1.97-1.91 (m, 2H), 1.69-1.50 (m, 2H), 1.20 (d, *J* = 6.6 Hz, 3H), 1.01 (d, *J* = 7.0 Hz, 3H), 0.96 (s, 3H), 0.85 (s, 3H), 0.23 (s, 9H), 0.14 (s, 9H), 0.11 (s, 9H).
   ESI-MS *m*/*z:* 714.4 (M+Na)⁺.
c) 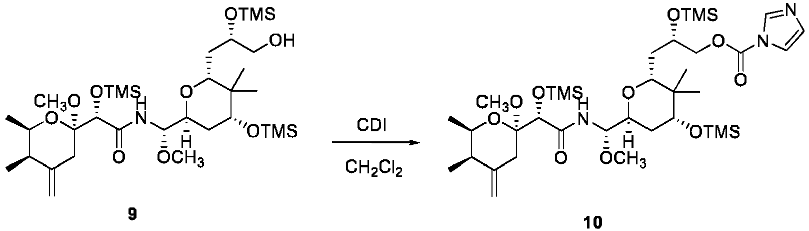 To a solution of 9 (16 mg, 0.023 mmol) in CH₂Cl₂ (2 mL) was added 1,1'-carbonyldiimidazole (6 eq, 0.138 mmol) at 23 °C. After being stirred overnight at 23 °C, the reaction mixture was concentrated under vacuum. The residue obtained was purified in a system for flash chromatography (SiO₂, Hex:EtOAc, from 80:20 to 50:50) to yield pure 10 (12.4 mg, 68% yield) as colourless oil.
   ¹H NMR (500 MHz, (CD₃)₂CO): δ 8.14 (d, *J* = 1.0 Hz, 1H), 7.53 (t, *J* = 1.4 Hz, 1H), 7.48 (d, *J* = 9.8 Hz, 1H), 7.06 (dd, *J* = 1.6, 0.8 Hz, 1H), 5.27 (dd, *J* = 9.8, 4.8 Hz, 1H), 4.76 (m, 1H), 4.61-4.53 (m, 2H), 4.39 (dd, *J* = 11.1, 6.6 Hz, 1H), 4.31 (s, 1H), 4.29 (m, 1H), 4.02 (dt, *J* = 5.6, 3.1 Hz, 1H), 3.84 (qd, *J* = 6.6, 2.6 Hz, 1H), 3.76 (dd, *J* = 7.8, 3.6 Hz, 1H), 3.64 (dd, *J* = 11.5, 2.3 Hz, 1H), 3.36 (s, 3H), 3.31 (s, 3H), 2.46-2.43 (m, 2H), 2.25-2.17 (m, 2H), 2.00-1.95 (m, 1H), 1.80-1.75 (m, 1H), 1.6-1.55 (m, 1H), 1.19 (d, *J* = 6.6 Hz, 3H), 1.02 (s, 3H), 0.98 (d, *J* = 7.0 Hz, 3H), 0.89 (s, 3H), 0.23 (s, 9H), 0.15 (s, 9H), 0.14 (m, 9H).
   ¹³C NMR (100 MHz, (CD₃)₂CO): δ 170.8, 148.6, 147.6, 136.8, 130.5, 117.1, 108.6, 99.3, 80.0, 77.7, 76.0, 72.7, 70.8, 69.2, 67.5, 55.0, 49.4, 41.3, 37.6, 35.6, 33.5, 30.7, 24.5, 17.3, 11.2, - 0.4, -0.5, -0.6.
   ESI-MS *m*/*z:* 808.4 (M+Na)⁺.
d) 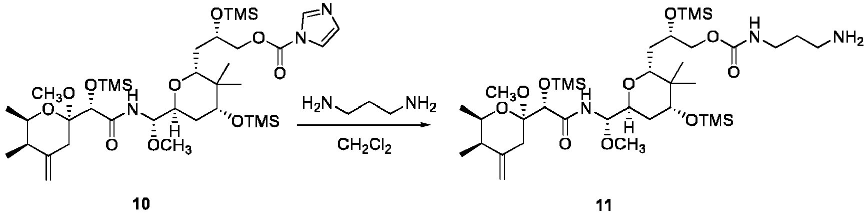 To a solution of **10** (12.5 mg, 0.016 mmol) in CH₂Cl₂ (2.5 mL) was added propane 1,3-diamine (6 eq, 7 µL) at 23 °C. The reaction mixture was stirred at 23 °C overnight, and concentrated under vacuum. The residue obtained was purified in a system for flash chromatography (Si-NH₂, CH₂Cl₂:CH₃OH, from 100:0 to 90:10) to obtain pure **11** (5 mg, 39% yield) as colourless oil.
   ¹H NMR (400 MHz, (CD₃)₂CO): δ 7.47 (d, *J* = 9.7 Hz, 1H), 6.36-6.28 (m, 1H), 5.20 (dd, *J* = 9.8, 4.2 Hz, 1H), 4.77-4.75 (m, 1H), 4.61-4.59 (m, 1H), 4.38 (s, 1H), 4.12-3.95 (m, 4H), 3.89-3.79 (m, 1H), 3.73 (dd, *J* = 7.1, 3.6 Hz, 1H), 3.53 (d, *J* = 10.7 Hz, 1H), 3.36 (s, 3H), 3.30 (s, 3H), 3.26-3.21 (m, 2H), 2.64 (d, *J* = 14.7 Hz, 1H), 2.47 (d, *J* = 14.7 Hz, 1H), 2.26-2.16 (m, 1H), 1.97-1.90 (m, 4H), 1.81-1.65 (m, 5H), 1.50-1.56 (m, 1H), 1.19 (d, *J* = 6.5 Hz, 3H), 1.00 (d, *J* = 7.0 Hz, 3H), 0.97 (s, 3H), 0.85 (s, 3H), 0.23 (s, 9H), 0.13 (s, 9H), 0.12 (s, 9H).
   ¹³C NMR (100 MHz, (CD₃)₂CO): δ 170.8, 156.4, 147.9, 108.5, 99.5, 80.8, 77.2, 76.4, 72.9, 69.7, 69.1, 68.3, 66.9, 55.1, 49.2, 48.9, 41.4, 39.6, 37.4, 35.4, 33.4, 30.8, 30.7, 24.9, 17.4, 11.3, -0.4 (x2), -0.6.
   (+)-HRESI-TOFMS m/z: 792.4403 [M + H]⁺ (calcd. for C₃₆H₇₃N₃O₁₀Si₃: 791.4604).
e) 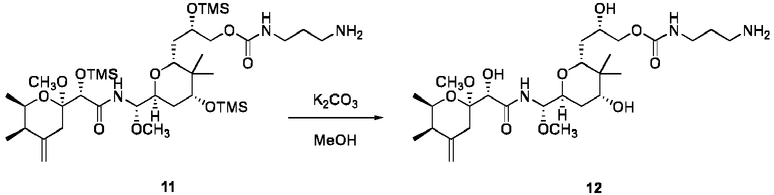 To a solution of 11 (6 mg, 0.0076 mmol) in MeOH (4 mL) was added 2.4 mL of 0.033 M potassium carbonate solution in methanol (10.5 eq, 0.08 mmol). The reaction mixture was stirred at 23 °C overnight and concentrated under vacuum. The residue obtained was purified in a system for flash chromatography (Si-NH₂, CH₂Cl₂:CH₃OH, from 100:0 to 70:30) to afford 12 (4.3 mg, 99% yield) as colourless oil.
   ¹H NMR (400 MHz, CD₃OD): δ 5.33 (m, 1H), 4.80 (m, 1H), 4.65 (m, 1H), 4.28 (s, 1H), 4.06-3.83 (m, 3H), 3.61-3.46 (m, 3H), 3.47-3.36 (m, 1H), 3.39 (s, 3H), 3.28 (s, 3H), 3.20-3.14 (m, 2H), 2.72-2.65 (m, 2H), 2.53-2.48 (m, 1H), 2.38-2.34 (m, 1H), 2.20 (dt, *J* = 8.1, 4.0 Hz, 1H), 2.01-1.95 (m, 2H), 1.78-1.61 (m, 4H), 1.17 (d, *J* = 6.6 Hz, 3H), 0.98 (d, *J* = 7.0 Hz, 3H), 0.94 (s, 3H), 0.87 (s, 3H).
   (+)-HRESI-TOFMS m/z: 576.3494 [M + H]⁺ (calcd. for C₂₇H₄₉N₃O₁₀: 575.3418).

### Intermediate 13

a) 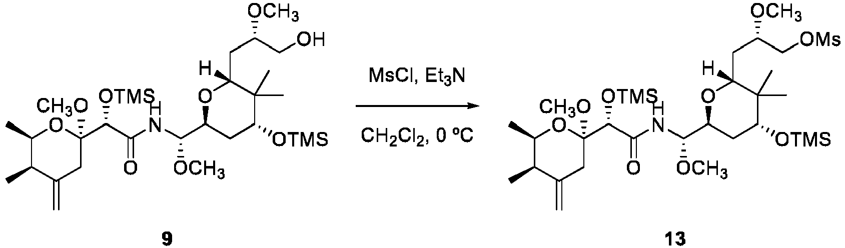 To a solution of 9 (80 mg, 0.126 mmol) and Et₃N (6 equiv., 0.756 mmol) in CH₂Cl₂ (5 mL) was dropwise added methanesulfonyl chloride (6 equiv., 0.756 mmol) at 0 °C. After being stirred at 0 °C for 30 min., the reaction mixture was concentrated under vacuum. The residue obtained was purified in an automatic system for flash chromatography (SiO₂, Hex:EtOAc, from 90:10 to 70:30) to afford pure 13 (88 mg, 99% yield) as colourless oil.
   ¹H NMR (500 MHz, (CD₃)₂CO): δ 7.50 (d, *J* = 10.0 Hz, 1H), 5.37 (dd, *J* = 10.0, 7.0 Hz, 1H), 4.80-4.75 (m, 1H), 4.63-4.48 (m, 1H), 4.45 (s, 1H), 4.32 (dd, *J* = 11.1, 2.0 Hz, 1H), 4.22 (dd, *J* = 11.1, 6.6 Hz, 1H), 3.92-3.73 (m, 2H), 3.75 (ddd, *J* = 9.7, 4.2, 0.5 Hz, 1H), 3.63-3.55 (m, 1H), 3.47-3.40 (m, 1H), 3.38 (s, 3H), 3.34 (s, 3H), 3.33 (s, 3H), 3.13 (s, 3H), 2.64-2.56 (m, 1H), 2.52 (dt, *J* = 14.5, 0.7 Hz, 1H), 2.26-2.18 (m, 1H), 2.03-1.90 (m, 2H), 1.70-1.60 (m, 2H), 1.20 (d, *J* = 6.6 Hz, 3H), 1.03 (d, *J* = 7.0 Hz, 3H), 0.96 (s, 3H), 0.87 (s, 3H), 0.25 (s, 9H), 0.15 (s, 9H).
   ¹³C NMR (125 MHz, (CD₃)₂CO): δ 172.1, 148.7, 109.4, 100.1, 79.7, 78.8, 77.1, 76.2, 73.1, 72.2, 70.0, 56.7, 55.9, 50.3, 42.2, 39.1, 37.2, 36.8, 31.2, 24.3, 18.2, 15.1, 12.1, 0.5, 0.3.
   ESI-MS m/z: 734.3 [M+Na]⁺.

### Synthesis of linkers

### Preparation of LIN 1: MC-Val-Cit-PABC-PNP

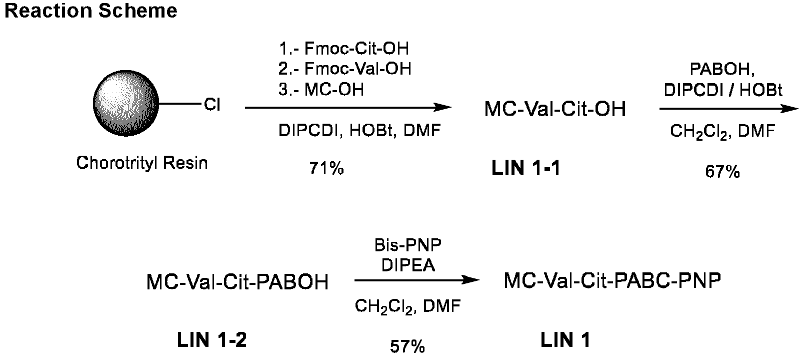

### (a) Preparation of LIN 1-1: MC-Val-Cit-OH

### LIN 1-1

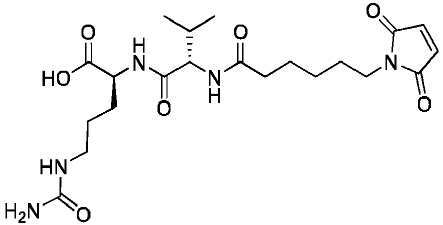

C!-TrtC!-resin (20 g, 1.49 mmol/g) (Iris Biotech, Ref.: BR-1065, 2-Chlorotrityl chloride resin (200-400 mesh, 1% DVB, 1.0-1.6 mmol/g), CAS 42074-68-0) was placed in a filter plate. 100 mL of DCM was added to the resin and the mixture was stirred for 1 h. The solvent was eliminated by filtration under vacuum. A solution of Fmoc-Cit-OH (11.83 g, 29.78 mmol) and DIPEA (17.15 mL, 98.45 mmol) in DCM (80 mL) was added and the mixture was stirred for 10 min. After that DIPEA (34.82 mmol, 199.98 mmol) was added and the mixture was stirred for 1 h. The reaction was terminated by addition of MeOH (30 mL) after stirring for 15 minutes. The Fmoc-Cit-O-TrtCl-resin produced as a result was subjected to the following washing/treatments: DCM (5 x 50 mL x 0.5 min), DMF (5 x 50 mL x 0.5 min), piperidine:DMF (1:4, 1 x 1 min, 2 x 10 min), DMF (5 x 50 mL x 0.5 min), DCM (5 x 50 mL x 0.5 min). The final piperidine wash gave NH₂-Cit-O-TrtCl-resin. The loading was calculated: 1.15 mmol/g.

The NH₂-Cit-O-TrtCl-resin produced above was washed with DMF (5 x 50 mL x 0.5 min) and a solution of Fmoc-Val-OH (31.22 g, 91.98 mmol), HOBt (11.23 g, 91.98 mmol) in DMF (100 mL) was added to the NH₂-Cit-O-TrtCl-resin, stirred and DIPCDI (14.24 mL, 91.98 mmol) was added and the mixture was stirred for 1.5 h. The reaction was terminated by washing with DMF (5 x 50 mL x 0.5 min). The Fmoc-Val-Cit-O-TrtCI-resin thus produced was treated with piperidine:DMF (1:4, 1 x 1 min, 2 x 10 min) and washed with DMF (5 x 50 mL x 0.5 min). The final piperidine wash gave NH₂-Val-Cit-O-TrtCl-resin.

A solution of 6-maleimidocaproic acid (MC-OH) (9.7 g, 45.92 mmol), HOBt (6.21 g, 45.92 mmol) in DMF (100 mL) was added to the NH₂-Val-Cit-O-TrtCl-resin produced above, stirred and DIPCDI (7.12 mL, 45.92 mmol) was added and the mixture was stirred for 1.5 h. The reaction was terminated by washing with DMF (5 x 50 mL x 0.5 min) and DCM (5 x 50 mL x 0.5 min).

The peptide was cleaved from the resin by treatments with TFA:DCM (1:99, 5 x 100 mL). The resin was washed with DCM (7 x 50 mL x 0.5 min). The combined filtrates were evaporated to dryness under reduced pressure and the solid obtained was triturated with Et₂O and filtrated to obtain **LIN 1-1** (7.60 g, 71%) as a white solid.

¹H NMR (500 MHz, DMSO-*d*₆): δ 12.47 (s, 1H), 8.13 (d, *J* = 7.3 Hz, 1H), 7.74 (d, *J* = 9.0 Hz, 1H), 6.99 (s, 2H), 5.93 (s, 1H), 5.35 (s, 2H), 4.20 (dd, *J* = 9.0, 6.8 Hz, 1H), 4.15-4.07 (m, 1H), 3.36 (t, *J* = 7.0 Hz, 2H), 3.00-2.88 (m, 2H), 2.21-2.12 (m, 1H), 2.11-2.03 (m, 1H), 1.98-1.86 (m, 1H), 1.74-1.62 (m, 1H), 1.61-1.50 (m, 1H), 1.50-1.31 (m, 6H), 1.21-1.11 (m, 2H), 0.84 (d, *J* = 6.8 Hz, 3H), 0.80 (d, *J* = 6.8 Hz, 3H).

ESI-MS m/z: 468.3 (M+H)⁺.

### (b) Preparation of LIN 1-2: MC-Val-Cit-PABOH

### LIN 1-2

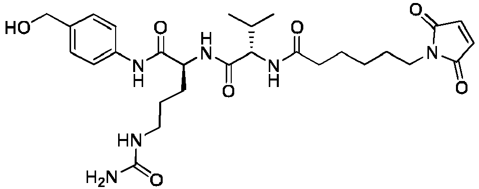

To a solution of **LIN 1-1** (1.6 g, 3.42 mmol) and 4-aminobenzyl alcohol (PABOH) (0.84 g, 6.84 mmol) in DCM (60 mL) was added a solution of HOBt (0.92 g, 6.84 mmol) in DMF (5 mL). DIPCDI (1.05 mL, 6.84 mmol) was added, the reaction mixture was stirred for 2 h at 23 °C, Et₂O (150 mL) was added, and the solid obtained was filtrated in a filter plate under vacuum to obtain **LIN 1-2** (1.31 g, 67%).

¹H NMR (500 MHz, DMSO-*d*₆): δ 9.88 (s, 1H), 8.03 (d, *J* = 7.6 Hz, 1H), 7.77 (dd, *J* = 12.2, 8.5 Hz, 1H), 7.53 (d, *J* = 8.2 Hz, 2H), 7.21 (d, *J* = 8.2 Hz, 2H), 6.99 (s, 3H), 6.01-5.92 (m, 1H), 5.39 (s, 2H), 5.07 (s, 1H), 4.41 (s, 2H), 4.39-4.31 (m, 1H), 4.23-4.12 (m, 1H), 3.36 (t, *J* = 7.0 Hz, 2H), 3.06-2.97 (m, 1H), 2.96-2.90 (m, 1H), 2.22-2.03 (m, 2H), 2.01-1.88 (m, 1H), 1.76-1.62 (m, 1H), 1.63-1.28 (m, 6H), 1.25-1.11 (m, 2H), 0.84 (d, *J* = 6.9 Hz, 3H), 0.81 (d, *J* = 6.8 Hz, 3H).

ESI-MS m/z: 573.3 (M+H)⁺.

### (c) Preparation of LIN 1: MC-Val-Cit-PAB-PNP

### LIN 1

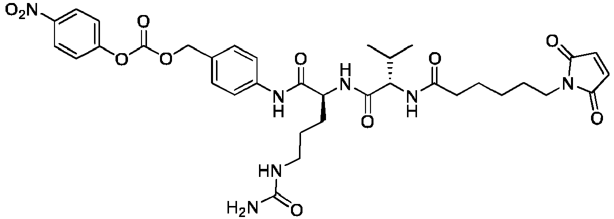

To a solution of **LIN 1-2** (500 mg, 0.87 mmol) and bis(4-nitrophenyl) carbonate (bis-PNP) (2.64 g, 8.72 mmol) in DCM:DMF (8:2, 25 mL) was added DIPEA (0.45 mL, 2.61 mmol). The reaction mixture was stirred for 20 h at 23 °C and poured onto a silica gel column (DCM:CH₃OH, from 50:1 to 10:1) to afford pure target **LIN** 1 (364 mg, 57%).

R_{f}= 0.40 (CH₂Cl₂:CH₃OH, 9:1).

¹H NMR (400 MHz, CDCl₃/CD₃OD): δ 9.45 (s, 1H), 8.23 (d, *J* = 8.3 Hz, 2H), 7.59 (d, *J* = 8.5 Hz, 2H), 7.35 (d, *J* = 8.3 Hz, 2H), 7.34 (d, *J* = 8.5 Hz, 2H), 6.65 (s, 2H), 5.20 (s, 2H), 4.56 (dt, *J* = 10.5, 5.4 Hz, 1H), 4.15 (d, *J* = 7.2 Hz, 1H), 3.46 (dd, *J* = 8.0, 6.4 Hz, 2H), 3.16-2.89 (m, 2H), 2.21 (dd, *J* = 8.3, 6.6 Hz, 2H), 2.06-1.97 (m, 1H), 1.90-1.83 (m, 1H), 1.73-1.46 (m, 7H), 1.34-1.20 (m, 2H), 0.91 (d, *J* = 6.7 Hz, 3H), 0.90 (d, *J* = 6.7 Hz, 3H).

¹³C NMR (125 MHz, CDCl₃/CD₃OD) δ 174.4, 172.4, 171.1, 170.6, 160.5, 155.5, 152.5, 145.3, 138.7, 134.1, 129.9, 129.5, 125.2, 121.8, 120.0, 70.6, 59.0, 53.2, 37.5, 35.8, 30.6, 29.6, 29.3, 28.1, 26.2, 26.2, 25.1, 19.1, 18.1.

ESI-MS m/z: 738.3 (M+H)⁺.

### Example 1: Synthesis of compounds of formula D-X-(AA)_{w-}(T)_{g}-L₁

### Preparation of Compound DL-1

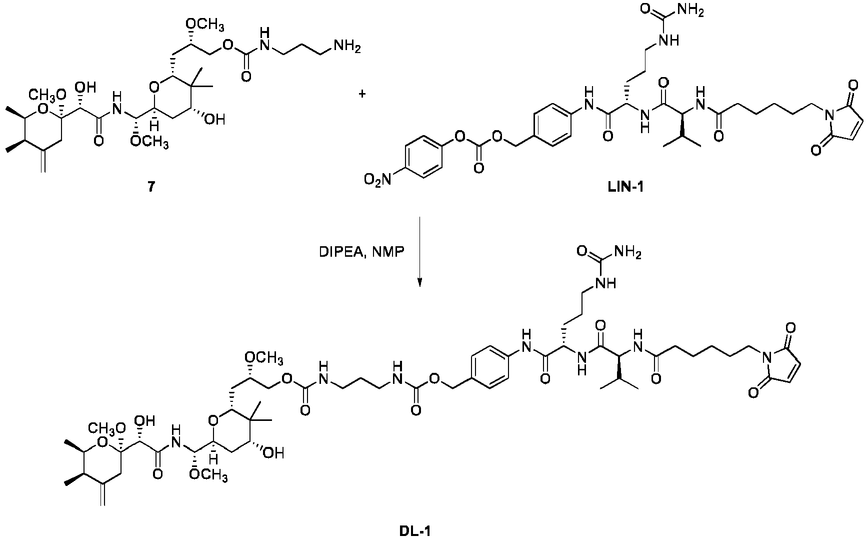

To a solution of 7 (13 mg, 0.022 mmol) and **LIN-1** (24.4 mg, 0.033 mmol) in NMR (3 mL) was added DIPEA (10.5 µL, 0.06 mmol) at 23 °C. After 15 h at 23 °C the reaction mixture was diluted with H₂O and extracted with EtOAc. The combined organic layers were dried over anhydrous Na₂SO₄, filtered, and concentrated under vacuum. The residue obtained was purified by semipreparative HPLC (sunfireC18, 10x150mm. CH₃CN:H₂O, flow 4 mL/min, UV detection) to obtain pure DL-1 (14.5 mg, 54% yield) as a white solid.

¹H NMR (500 MHz, CD₃CN:D₂O 2:1): δ 7.53 (d, *J* = 8.5 Hz, 2H), 7.31 (d, *J* = 8.3 Hz, 2H), 6.77 (m, 2H), 5.25 (d, *J* = 7.6 Hz, 1H), 4.99 (s, 2H), 4.81 (t, *J* = 2.1 Hz, 1H), 4.67 (t, *J* = 2.1 Hz, 1H), 4.39 (dd, *J* = 9.1, 5.1 Hz, 1H), 4.24 (s, 1H), 4.11-4.04 (m, 2H), 3.98 (dd, J= 11.9, 5.0 Hz, 1H), 3.83 (ddd, *J* = 11.3, 5.5, 3.4 Hz, 2H), 3.57 (dd, *J* = 10.7, 4.5 Hz, 1H), 3.43-3.40 (m, 3H), 3.33 (s, 3H), 3.25 (s, 3H), 3.20 (s, 3H), 3.19-3.18 (m, 1H), 3.10-3.01 (m, 6H), 2.38-2.28 (m, 2H), 2.28-2.17 (m, 3H), 2.06-1.97 (m, 1H), 1.89 (ddd, *J* = 13.7, 4.6, 3.1 Hz, 1H), 1.82 (s, 1H), 1.75-1.44 (m, 10H), 1.30-1.17 (m, 4H), 1.12 (d, *J* = 6.6 Hz, 3H), 0.92 (d, *J* = 7.0 Hz, 3H), 0.89 (d, *J* = 3.0 Hz, 3H), 0.88 (d, *J* = 3.0 Hz, 3H), 0.85 (s, 3H), 0.78 (s, 3H).

¹³C NMR (125 MHz, CD₃CN:D₂O 2:1): δ 175.3, 172.6, 172.5, 172.1, 171.1, 158.3, 158.2, 146.8, 137.7, 134.3, 133.1, 128.6, 120.2, 119.5, 109.7, 99.9, 79.8, 76.8, 75.7, 72.4 (x2), 70.8, 69.6, 65.9, 64.2, 63.9, 59.1, 56.1, 55.9, 53.7, 48.1, 41.1, 38.1, 37.8, 37.4, 35.3, 33.5, 32.1, 30.2, 29.8, 29.4, 28.9, 28.6, 27.8, 25.9, 25.0, 22.6, 21.9, 18.6, 17.6, 17.1, 11.5, 0.9.

(+)-HRESI-TOFMS m/z: 1210.6301 [M + Na]⁺ (calcd. for C₅₇H₈₉N₉O₁₈Na: 1210.6223).

### Preparation of compound DL-2

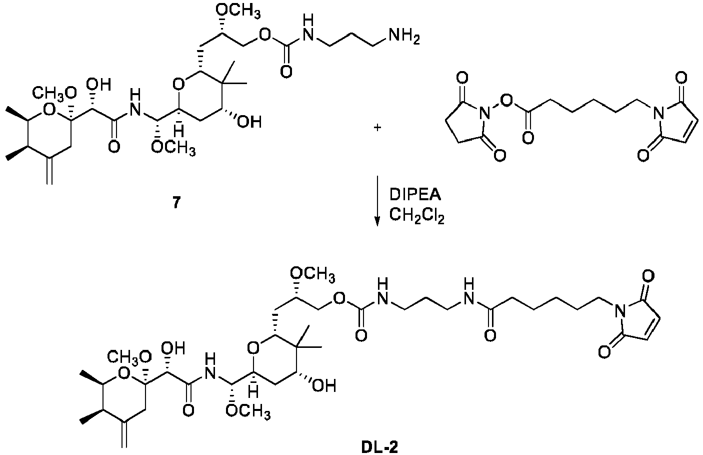

To a solution of 7 (17.8 mg, 0.030 mmol) and 6-Maleimidohexanoic acid N-hydroxysuccinimide ester (0.060 mmol, 19 mg) in CH₂Cl₂ (3 mL), was added DIPEA (16 µL, 0.091 mmol) at 23 °C. The reaction mixture was stirred at 23 °C overnight and concentrated under vacuum. The residue obtained was purified in a system for flash chromatography (SiO₂, CH₂Cl₂:CH₃OH, from 100:0 to 80:20) to afford pure DL-2 (17.5 mg, 74% yield).

¹H NMR (400 MHz, (CD₃)₂CO): δ 7.42 (d, *J* = 9.6 Hz, 1H), 7.14-7.12 (m, 1H), 6.86-6.84 (m, 2H), 6.39-6.36 (m, 1H), 5.35 (dd, *J* = 9.6, 7.5 Hz, 1H), 4.82-4.76 (m, 1H), 4.64-4.59 (m, 2H), 4.34-4.32 (m, 1H), 4.15 (dd, *J* = 11.7, 3.3 Hz, 1H), 4.00 (dd, *J* = 11.7, 5.1 Hz, 1H), 3.96-3.87 (m, 1H), 3.90-3.80 (m, 2H), 3.65-3.60 (m, 1H), 3.48-3.44 (m, 3H), 3.36 (s, 3H), 3.34-3.31 (m, 1H), 3.28-3.21 (m, 8H), 3.16-3.11 (m, 2H), 2.42-2.39 (m, 2H), 2.32-2.11 (m, 3H), 2.09-1.94 (m, 1H), 1.79-1.52 (m, 9H), 1.39-1.23 (m, 2H), 1.16 (d, *J* = 6.6 Hz, 3H), 1.01 (d, *J* = 7.0 Hz, 3H), 0.94 (s, 3H), 0.86 (s, 3H).

¹³C NMR (100 MHz, (CD₃)₂CO): δ 172.3, 171.7, 170.8, 156.6, 147.2, 134.2, 109.0, 99.7, 79.8, 76.6, 75.5, 73.3, 72.1, 70.7, 69.0, 64.4, 55.9, 55.4, 48.2, 41.4, 38.2, 37.7, 37.2, 35.9, 35.6, 34.2, 30.8, 30.0, 29.8, 28.1, 26.1, 25.0, 23.0, 17.2, 13.2, 11.6.

(+)-HRESI-TOFMS m/z: 805.4233 [M + Na]⁺ (calcd. for C₃₈H₆₂N₄O₁₃Na: 805.4211).

### Preparation of compound DL 3

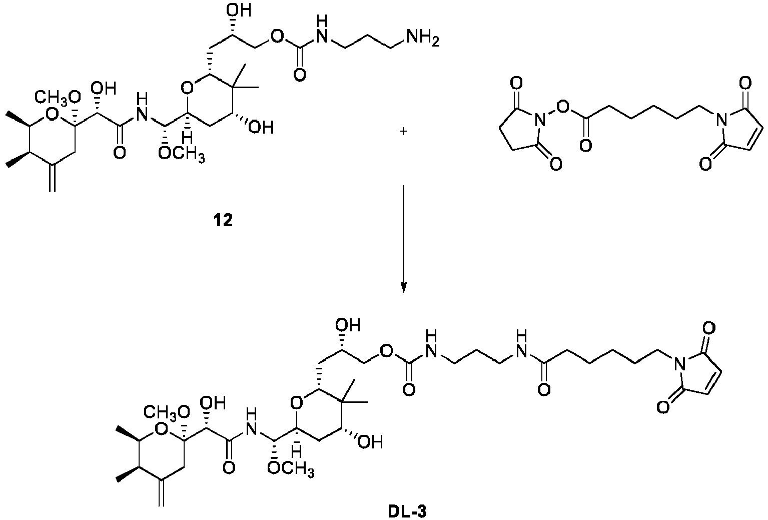

To a solution of 12 (4.5 mg, 0.0078 mmol) and 6-Maleimidohexanoic acid N-hydroxysuccinimide ester (2 eq., 4.8 mg) in CH₂Cl₂ (2 mL), was added DIPEA (4.1 µL, 3 eq.) at 23 °C. The reaction mixture was stirred at 23 °C overnight and concentrated under vacuum. The residue obtained was purified in a system for flash chromatography (SiO₂, CH₂Cl₂:CH₃OH, from 100:0 to 80:20) to afford DL-3 (3 mg, 50% yield).

¹H NMR (500 MHz, (CD₃)₂CO): δ 7.58 (d, *J* = 9.7 Hz, 1H), 7.11-7.09 (m, 1H), 6.86 (s, 2H), 6.38-6.34 (m, 1H), 5.45-5.35 (m, 1H), 4.85-4.72 (m, 2H), 4.64-4.62 (m, 1H), 4.35-4.30 (m, 1H), 4.09-3.87 (m, 7H), 3.70-3.58 (m, 2H), 3.51-3.45 (m, 2H), 3.37 (s, 3H), 3.29 (s, 3H), 3.27-3.20 (m, 2H), 3.17-3.12 (m, 2H), 2.55-2.47 (m, 1H), 2.42-2.38 (m, 1H), 2.26-2.19 (m, 1H), 2.18-2.13 (m, 2H), 2.02-1.95 (m, 1H), 1.79-1.54 (m, 11H), 1.15 (d, *J* = 6.6 Hz, 3H), 1.00 (d, *J* = 7.0 Hz, 3H), 0.97 (s, 3H), 0.89 (s, 3H).

¹³C NMR (125 MHz, (CD₃)₂CO): δ 173.6, 172.6, 171.4, 157.3, 148.2, 134.9, 109.3, 100.4, 80.2, 79.9, 74.6, 72.3, 71.2, 70.2, 69.5, 68.5, 56.0, 49.0, 42.1, 38.8, 38.3, 37.8, 36.5 (x2), 34.7, 33.3, 30.6, 30.5, 28.7, 26.8, 25.7, 23.8, 17.9, 14.5, 12.1.

(+)-HRESI-TOFMS m/z: 791.3830 [M + Na]⁺ (calcd. for C₃₇H₆₀N₄O₁₃Na: 791.4055).

### Example 2: Preparation of Antibody-Drug Conjugates (ADCs)

In this Example, syntheses of antibody-drug conjugates of the present invention are described. It should be noted that these syntheses are exemplary and that the processes described can be applied to all the compounds and antibodies described herein.

### Preparation of Antibody-Drug Conjugate ADC-1 with Trastuzumab and Compound DL-1

### (a) Preparation of Trastuzumab

Trastuzumab (Trastuzumab purchased from Roche as a white lyophilized powder for the preparation of a concentrated solution for infusion) was dissolved in 5 mL of phosphate buffer (50 mM, pH 8.0) and purified by desalting using Sephadex G25 PD-10 columns into phosphate buffer (50 mM, pH 8.0). Concentration of Trastuzumab (16.1 mg/mL) was determined by measuring the absorbance at 280 nm.

### (b) Reaction of Trastuzumab with 2-iminothiolane (Traut's reagent) to give thiol-activated Trastuzumab

Trastuzumab solution (0.75 mL, 12 mg, 72.7 nmol) was diluted to a concentration of 10 mg/mL using phosphate buffer (50 mM phosphate, 2 mM EDTA, pH 8). Traut's reagent was added (72.6 µL, 872 nmol, 12 eq.), and the reaction stirred for 2 h at 20 °C. The mixture was buffer exchanged using two Sephadex G25 NAP-5 columns into PBS buffer, and concentrated to a volume of 1.3 mL (9.3 mg/mL). Immediately after, an Ellman assay was performed to give a Free Thiol to Antibody ratio (FTAR) of 4.2.

### (c) Preparation of ADC-1

To the solution of thiol-activated Trastuzumab (200 µL, 1.86 mg, 12.4 nmol), DMA was added (37 µL) followed by addition of a freshly prepared solution of DL-1 (10 mM in DMA, 12.3 µL, 123 nmol, 10 eq.). The conjugation reaction was stirred for 2 h at 25 °C and purified by desalting using a Sephadex G25 NAP-5 column into PBS buffer. The final target product **ADC-1** was concentrated to a final concentration of 6.32 mg/mL as determined by UV and 210 µL (1.32 mg, 8.8 nmol, 71%) ADC solution was obtained.

### Preparation of Antibody-Drug Conjugate ADC-2 with Trastuzumab and Compound DL-2

### (a) Preparation of Trastuzumab

Trastuzumab (Trastuzumab purchased from Roche as a white lyophilized powder for the preparation of a concentrated solution for infusion) was dissolved in 5 mL of phosphate buffer (50 mM, pH 8.0) and purified by desalting using Sephadex G25 PD-10 columns into phosphate buffer (50 mM, pH 8.0). Concentration of Trastuzumab (16.1 mg/mL) was determined by measuring the absorbance at 280 nm.

### (b) Reaction of Trastuzumab with 2-iminothiolane (Traut's reagent) to give thiol-activated Trastuzumab

Trastuzumab solution (0.75 mL, 12 mg, 72.7 nmol) was diluted to a concentration of 10 mg/mL using phosphate buffer (50 mM phosphate, 2 mM EDTA, pH 8). Traut's reagent was added (72.6 µL, 872 nmol, 12 eq.), and the reaction stirred for 2 h at 20 °C. The mixture was buffer exchanged using two Sephadex G25 NAP-5 columns into PBS buffer, and concentrated to a volume of 1.3 mL (9.3 mg/mL). Immediately after, an Ellman assay was performed to give a Free Thiol to Antibody ratio (FTAR) of 4.2.

### (c) Preparation of ADC-2

To the solution of thiol-activated Trastuzumab (200 µL, 1.86 mg, 12.4 nmol), DMA was added (37 µL) followed by addition of a freshly prepared solution of DL-2 (10 mM in DMA, 12.3 µL, 123 nmol, 10 eq.). The conjugation reaction was stirred for 2 h at 25 °C and purified by desalting using a Sephadex G25 NAP-5 column into PBS buffer. The final target product **ADC-2** was concentrated to a final concentration of 7.11 mg/mL as determined by UV and 220 µL (1.56 mg, 10.4 nmol, 84%) ADC solution was obtained.

### Preparation of Antibody-Drug Conjugate ADC-3 with Trastuzumab and Compound DL-2

### (a) Preparation of Trastuzumab

Trastuzumab (Trastuzumab purchased from Roche as a white lyophilized powder for the preparation of a concentrated solution for infusion) was dissolved in 5 mL of phosphate buffer (50 mM, pH 8.0) and purified by desalting using Sephadex G25 PD-10 columns into phosphate buffer (50 mM, pH 8.0). Concentration of Trastuzumab (17.6 mg/mL) was determined by measuring the absorbance at 280 nm.

### (b) Partial reduction of Trastuzumab to give Partially Reduced Trastuzumab

Trastuzumab solution (0.55 mL, 9.7 mg, 64.5 nmol) was diluted to a concentration of 10 mg/mL with phosphate buffer (50 mM, pH 8). Partial reduction of the disulfide bonds in the antibody was performed by the addition of a 5.0 mM tris[2-carboxyethyl]phosphine hydrochloride (TCEP) solution (29.2 µL, 145.8 nmol, 2.2 eq.). The reduction reaction was left to stir for 90 min at 20 °C. Immediately after the reduction, an Ellman assay was performed to give a Free Thiol to Antibody ratio (FTAR) of 3.4.

### (c) Preparation of ADC-3

To the solution of partially reduced Trastuzumab (0.14 mL, 1.78 mg, 11.9 nmol), DMA was added (28.2 µL) followed by addition of a freshly prepared solution of DL-2 (10 mM in DMA, 6.8 µL, 68 nmol, 5.6 eq.). The conjugation reaction was stirred for 30 min at 20 °C and the excess of drug was quenched by addition of N-acetylcysteine (NAC) (10 mM, 6.8 µL, 68 nmol) followed by stirring the solution for 20 min. The quenched conjugation reaction was purified by desalting using Sephadex G25 NAP-5 columns into PBS buffer. The final target product **ADC-3** was concentrated to a final concentration of 6.73 mg/mL as determined by UV and 188 µL (1.26 mg, 8.4 nmol, 70%) ADC solution was obtained. HIC HPLC runs were performed to determine the percentage of conjugation reaction (91%).

### Preparation of Antibody-Drug Conjugate ADC-4 with Trastuzumab and Compound DL-1

### (a) Preparation of Trastuzumab

Trastuzumab (Trastuzumab purchased from Roche as a white lyophilized powder for the preparation of a concentrated solution for infusion) was dissolved in 5 mL of phosphate buffer (50 mM, pH 8.0) and purified by desalting using Sephadex G25 PD-10 columns into phosphate buffer (50 mM, pH 8.0). Concentration of Trastuzumab (17.1 mg/mL) was determined by measuring the absorbance at 280 nm.

### (b) Partial reduction of Trastuzumab to give Partially Reduced Trastuzumab

Trastuzumab solution (0.25 mL, 4.27 mg, 28.5 nmol) was diluted to a concentration of 10 mg/mL with phosphate buffer (50 mM, pH 8). Partial reduction of the disulfide bonds in the antibody was performed by the addition of a 5.0 mM tris[2-carboxyethyl]phosphine hydrochloride (TCEP) solution (16.5 µL, 81.6 µmol, 3 eq.). The reduction reaction was left to stir for 90 min at 20 °C. Immediately after the reduction, an Ellman assay was performed to give a Free Thiol to Antibody ratio (FTAR) of 6.4.

### (c) Preparation of ADC-4

To the solution of partially reduced Trastuzumab (125 µL, 1.24 mg, 8.3 nmol), DMA was added (19.3 µL) followed by addition of a freshly prepared solution of DL-1 (10 mM in DMA, 7.1 µL, 71 nmol, 8.5 eq.). The conjugation reaction was stirred for 2 h at 25 °C. The excess of drug was quenched by addition of N-acetylcysteine (NAC) (10 mM, 7.1 µL, 71 nmol) followed by stirring the solution for 20 min. The quenched conjugation reaction was purified by desalting using Sephadex G25 NAP-5 columns into PBS buffer. The final target product ADC-4 was concentrated to a final concentration of 4.58 mg/mL as determined by UV and 270 µL (1.24 mg, 8.26 nmol, 99 %) ADC solution was obtained. HIC HPLC runs were performed to determine the percentage of conjugation reaction (96%).

### Preparation of Antibody-Drug Conjugate ADC-5 with Trastuzumab and Compound DL-3

### (a) Preparation of Trastuzumab

Trastuzumab (Trastuzumab purchased from Roche as a white lyophilized powder for the preparation of a concentrated solution for infusion) was dissolved in 5 mL of phosphate buffer (50 mM, pH 8.0) and purified by desalting using Sephadex G25 PD-10 columns into phosphate buffer (50 mM, pH 8.0). Concentration of Trastuzumab (16.1 mg/mL) was determined by measuring the absorbance at 280 nm.

### (b) Partial reduction of Trastuzumab to give Partially Reduced Trastuzumab

Trastuzumab solution (0.3 mL, 4.8 mg, 32.2 nmol) was diluted to a concentration of 10 mg/mL with phosphate buffer (50 mM, pH 8). Partial reduction of the disulfide bonds in the antibody was performed by the addition of a 5.0 mM tris[2-carboxyethyl]phosphine hydrochloride (TCEP) solution (19.3 µL, 96.6 µmol, 3 eq.). The reduction reaction was left to stir for 90 min at 20 °C. Immediately after the reduction, an Ellman assay was performed to give a Free Thiol to Antibody ratio (FTAR) of 4.3.

### (c) Preparation of ADC-5

To the solution of partially reduced Trastuzumab (150 µL, 1.5 mg, 10 nmol), DMA was added (29.5 µL) followed by addition of a freshly prepared solution of DL-3 (10 mM in DMA, 8 µL, 80 nmol, 8 eq.). The conjugation reaction was stirred for 30 min at 20 °C. The excess of drug was quenched by addition of N-acetylcysteine (NAC) (10 mM, 8 µL, 80 nmol) followed by stirring the solution for 20 min. The quenched conjugation reaction was purified by desalting using Sephadex G25 NAP-5 columns into PBS buffer. The final target product **ADC-5** was concentrated to a final concentration of 6.1 mg/mL as determined by UV and 180 µL (1.1 mg, 7.3 nmol, 73%) ADC solution was obtained. HIC HPLC runs were performed to determine the percentage of conjugation reaction (96%).

### Example 3: Demonstrating the Cytotoxicity of the Antibody-Drug Conjugates of the Present Invention

### Bioassays for the detection of antitumor activity

The aim of the assay was to evaluate the *in vitro* cytostatic (ability to delay or arrest tumor cell growth) or cytotoxic (ability to kill tumor cells) activity of the samples being tested.

### Cell lines and cell culture

The following human tumor cell lines were obtained from the American Type Culture Collection (ATCC): SK-BR-3 (ATCC HB-30), HCC-1954 (ATCC CRL-2338) (both being breast cancer, HER2+), MDA-MB-231 (ATCC HTB-26), MCF-7 (ATCC HTB-22) (both being breast cancer, HER2-). Cells were maintained at 37 °C, 5% CO₂ and 95% humidity either in Dulbecco's Modified Eagle's Medium (DMEM) (for SK-BR-3, MDA-MB-231 and MCF-7 cells), or RPMI-1640 (for HCC-1954 cell line), all media supplemented with 10% Fetal Calf Serum (FCS), 2 mM L-glutamine and 100 units/mL of penicillin and streptomycin.

### Cytotoxicity Assay

For SK-BR-3, HCC-1954, MDA-MB-231 and MCF-7 cells, a colorimetric assay using Sulforhodamine B (SRB) was adapted for quantitative measurement of cell growth and cytotoxicity, as described by V. Vichai and K. Kirtikara. In "Sulforhodamine B colorimetric assay for cytotoxicity screening", Nature Protocols, 2006, 1: 1112-1116. Briefly, cells were seeded in 96-well microtiter plates and allowed to stand for 24 hours in drug-free medium before treatment with vehicle alone or with the tested substances for 72 hours. For quantification, cells were washed twice with phosphate buffered saline (PBS), fixed for 15 min in 1% glutaraldehyde solution, rinsed twice with PBS, stained in 0.4% (w/v) SRB with 1% (v/v) acetic acid solution for 30 min, rinsed several times with 1% acetic acid solution and air-dried. SRB was then extracted in 10 mM Trizma base solution and the optical density measured at 490 nm in a microplate spectrophotometer.

Cell survival was expressed in all cases as percentage of control, untreated cell survival. All evaluations were performed in triplicate and the resulting data were fitted by nonlinear regression to a four-parameters logistic equation using the Prism 5.0 statistical software (GraphPad, La Jolla, CA, USA), from which the IC₅₀ value (the concentration of compound causing 50% effect on cell growth as compared to the untreated control) was calculated. The data presented here correspond to the geometric mean of three independent experiments performed in triplicate.

The *in vitro* cytotoxicity of the ADCs along with the parent cytotoxic compounds 1, 2 and DL-2 and Trastuzumab were evaluated against four different human breast cancer cell lines over-expressing or not the HER2 receptor, including SK-BR-3, HCC-1954 (HER2-positive cells) as well as MDA-MB-231 and MCF-7 (HER2-negative cells). Standard dose-response (DR) curves for 72 hours incubation with the tested substances were performed.

Assays *with HER2 positive* /*negative cell lines.*

### Cytotoxicity of Trastuzumab

The *in vitro* cytotoxicty of Trastuzumab was evaluated against the different tumor cell lines by performing triplicate 10-points, 2.5-fold dilution DR curves ranging from 50 to 0.01 µg/mL (3.33E-07 - 8.74E-11 M). Trastuzumab was completely inactive, not reaching the IC₅₀ in any of the cell lines tested, independently of their HER2 status as shown in Table 3 where results corresponding to the geometric mean of the IC₅₀ values obtained in three independent experiments are presented.

**Table 3. Summary of the in vitro cytotoxicity of Trastuzumab.**

| | **HER2 positive** | | **HER2 negative** | |
|---|---|---|---|---|
| | **SK-BR-3** | **HCC-1954** | **MDA-MB-231** | **MCF-7** |
| IC₅₀, µg/mL | > 50 | > 50 | > 50 | > 50 |
| IC₅₀, M | > 3.4E-07 | > 3.4E-07 | > 3.4E-07 | > 3.4E-07 |

### Cytotoxicity of 1

The cytotoxicity of the intermediate compound 1 was evaluated against the different tumor cell lines by performing triplicate 10-points, 2.5-fold dilution DR curves ranging from 100 to 0.03 ng/mL (2.04E-07 - 6.12E-11 M).

As shown in Table 4, where results corresponding to the geometric mean of the IC₅₀ values obtained in three independent experiments are presented, the cytotoxicity of this compound was similar in all the tumor cell lines regardless of their HER2 expression, with IC₅₀ values in the nanomolar range.

**Table 4. Summary of the in vitro cytotoxicity of 1**

| | **HER2 positive** | | **HER2 negative** | |
|---|---|---|---|---|
| | **SK-BR-3** | **HCC-1954** | **MDA-MB-231** | **MCF-7** |
| IC₅₀, µg/mL | 9.49E-04 | 6.66E-04 | 5.96E-04 | 8.37E-04 |
| IC₅₀, M | 1.94E-09 | 1.36E-09 | 1.22E-09 | 1.71E-09 |

### Cytotoxicity of 2

The cytotoxicity of the intermediate compound 2 was evaluated against the different tumor cell lines by performing triplicate 10-points, 2.5-fold dilution DR curves ranging from 1 µg/mL to 0.3 ng/mL (2.10E-06 - 6.30E-10 M)

As shown in Table 5, the cytotoxicity of this compound was similar in all the tumor cell-lines regardless of the HER2 expression, with IC₅₀ values in the nanomolar range.

**Table 5. Summary of the in vitro cytotoxicity of 2**

| | **HER2 positive** | | **HER2 negative** | |
|---|---|---|---|---|
| | **SK-BR-3** | **HCC-1954** | **MDA-MB-231** | **MCF-7** |
| IC₅₀, µg/mL | 1.30E-02 | 6.18E-03 | 5.61E-03 | 1.20E-02 |
| IC₅₀, M | 2.73E-08 | 1.30E-08 | 1.18E-08 | 2.52E-08 |

### Cytotoxicity of DL-2

The cytotoxicity of the intermediate compound **DL-2** was evaluated against the different tumor cell lines by performing triplicate 10-points, 2.5-fold dilution DR curves ranging from 10 µg/mL to 2.6 ng/mL (1.28E-05 - 3.32E-09 M).

As shown in Table 6, the cytotoxicity of this compound was similar in all the tumor cell lines regardless of their HER2 expression, with IC₅₀ values in the high nanomolar range.

**Table 6. Summary of the in vitro cytotoxicity of DL-2**

| | **HER2 positive** | | **HER2 negative** | |
|---|---|---|---|---|
| | **SK-BR-3** | **HCC-1954** | **MDA-MB-231** | **MCF-7** |
| IC₅₀, µg/mL | 1.08E-01 | 7.60E-02 | 6.52E-02 | 7.59E-02 |
| IC₅₀, M | 1.38E-07 | 9.71E-08 | 8.33E-08 | 9.70E-08 |

### Cytotoxicity of ADC-3

The cytotoxicity of **ADC-3** was evaluated against the different tumor cell lines by performing triplicate 10-points, 2.5-fold dilution DR curves ranging from 100 µg/mL to 26 ng/mL (6.67E-07 - 1.75E-10 M). Table 7 summarizes the results corresponding to the geometric mean of the IC₅₀ values obtained. **ADC-3** showed a cytotoxicity which is similar to that shown by the parent drug 1 only in HER2-positive cells. However, in HER2-negative cells such toxicity is significantly lower: more than 500-fold lower according to the selectivity ratio obtained by dividing the mean IC₅₀ value in HER2-negative cells between that in HER2-positive cells. This selectivity leads us to conclude that the conjugate is acting through the interaction of the antibody with the membrane associated HER2 receptor on the tumor cells, followed by intracellular delivery of the cytotoxic drug.

**Table 1. Summary of the in vitro cytotoxicity of ADC-3**

| | **HER2 positive** | | **HER2 negative** | | **IC₅₀ in HER2+ (geom. mean)** | **IC₅₀ in HER2-(geom. mean** | **Selec. ratio** |
|---|---|---|---|---|---|---|---|
| | **SK-BR-3** | **HCC-1954** | **MDA-MB-231** | **MCF-7** | | | |
| IC₅₀ (µg/mL) | 1.61E-01 | 2.10E-01 | > 1.00E+02 | > 1.00E+02 | 1.84E-01 | > 1.00E+02 | >546.7 |
| IC₅₀ (M) | 1.07E-09 | 1.40E-09 | > 6.67E-07 | > 6.67E-07 | 1.22E-09 | >6.67E-07 | |

### Cytotoxicity of ADC-2

The cytotoxicity of the **ADC-2** was evaluated against the different tumor cell lines by performing triplicate 10-points, 2.5-fold dilution DR curves ranging from 100 µg/mL to 26 ng/mL (6.67E-07 - 1.75E-10 M). Table 8 summarizes the results corresponding to the geometric mean of the IC₅₀ values obtained. **ADC-2** showed a cytotoxicity which is similar to that shown by the parent drug 1 only in HER2-positive cells. However, in HER2-negative cells such toxicity is lower, nearly 7-fold lower according to the selectivity ratio obtained by dividing the mean IC₅₀ value in HER2-negative cells between that in HER2-positive cells. This selectivity leads us to conclude that the conjugate is acting through the interaction of the antibody with the membrane associated HER2 receptor on the tumor cells, followed by intracellular delivery of the cytotoxic drug.

**Table 8. Summary of the in vitro cytotoxicity of ADC-2**

| | **HER2 p ositive** | | **HER2 negative** | | **IC₅₀ in HER2+ (geom. mean)** | **IC₅₀ in HER2-(geom. mean** | **Selec. Ratio** |
|---|---|---|---|---|---|---|---|
| | **SK-BR-3** | **HCC-1954** | **MDA-MB-231** | **MCF-7** | | | |
| IC₅₀ (µg/mL) | 3.30E-01 | 5.00E-01 | 1.91E00 | 4.20E00 | 4.06E-01 | 2.83E00 | 6.97 |
| IC₅₀ (M) | 2.20E-09 | 3.33E-09 | 1.27E-08 | 2.80E-08 | 2.71E-09 | 1.89E-08 | |

### Cytotoxicity of ADC-1

The cytotoxicity of the ADC-1 was evaluated against the different tumor cell lines by performing triplicate 10-points, 2.5-fold dilution DR curves ranging from 100 µg/mL to 26 ng/mL (6.67E-07 - 1.75E-10 M). Table 9 summarizes the results corresponding to the geometric mean of the IC₅₀ values obtained. ADC-1 showed a cytotoxicity which is similar to that shown by the parent drug 1 only in HER2-positive cells. However, in HER2-negative cells such toxicity is lower, more than 400-fold lower according to the selectivity ratio obtained by dividing the mean IC₅₀ value in HER2-negative cells between that in HER2-positive cells. This selectivity leads us to conclude that the conjugate is acting through the interaction of the antibody with the membrane associated HER2 receptor on the tumor cells, followed by intracellular delivery of the cytotoxic drug.

**Table 9. Summary of the in vitro cytotoxicity of ADC-1**

| | **HER2 positive** | | **HER2 ne gative** | | **IC₅₀ in HER2+ (geom. mean)** | **IC₅₀ in HER2-(geom. mean** | **Selec. Ratio** |
|---|---|---|---|---|---|---|---|
| | **SK-BR-3** | **HCC-1954** | **MDA-MB-231** | **MCF-7** | | | |
| IC₅₀ (µg/mL) | 1.50E-01 | 3.41E-01 | >1.00E+02 | >1.00E+02 | 2.27E-01 | >1.00E+02 | >440 |
| IC₅₀ (M) | 1.00E-09 | 2.27E-09 | > 6.67E-07 | > 6.67E-07 | 1.51E-09 | >6.67E-07 | |

### Cytotoxicity of ADC-4

The cytotoxicity of the ADC-4 was evaluated against the different tumor cell lines by performing triplicate 10-points, 2.5-fold dilution DR curves ranging from 100 µg/mL to 26 ng/mL (6.67E-07 - 1.75E-10 M). Table 10 summarizes the results corresponding to the geometric mean of the IC₅₀ vales obtained. ADC-4 showed a cytotoxicity which is similar to that shown by the parent drug 1 only in HER2-positive cells. However, in HER2-negative cells such toxicity is lower, more than 1200-fold lower according to the selectivity ratio obtained by dividing the mean IC₅₀ value in HER2-negative cells between that in HER2-positive cells. This selectivity lead us to conclude that the conjugate is acting through the intereaction of the antibody with the membrane associated HER2 receptor on the tumor cells, followed by intracellular delivery of the cytotoxic drug.

**Table 10. Summary of the in vitro cytotoxicity of ADC-4**

| | **HER2 positive** | | **HER2 ne gative** | | **IC₅₀ in HER2+ (geom. mean)** | **IC₅₀ in HER2-(geom. mean** | **Selec. Ratio** |
|---|---|---|---|---|---|---|---|
| | **SK-BR-3** | **HCC-1954** | **MDA-MB-231** | **MCF-7** | | | |
| IC₅₀ (µg/mL) | <2.60E-02 | 7.80E-02 | >1.00E+02 | >1.00E+02 | <7.80E-02 | >1.00E+02 | >1280 |
| IC₅₀ (M) | < 1.73E-10 | 5.20E-10 | >6.67E-07 | >6.67E-07 | <5.20E-10 | >6.67E-07 | |

### Cytotoxicity of ADC-5

The cytotoxicity of the ADC-5 was evaluated against the different tumor cell lines by performing triplicate 10-points, 2.5-fold dilution DR curves ranging from 100 µg/mL to 26 ng/mL (6.67E-07 - 1.75E-10 M). Table 11 summarizes the results corresponding to the geometric mean of the IC₅₀ values obtained. This conjugate selectively impairs growth of HER2-expressing cell lines, with potencies that exceed those shown by the corresponding drug, hence suggesting that the conjugation is favouring cellular uptake of the drug. This remarkable activity in HER2-expressing cell lines is responsible for the large selectivity observed with regard to HER2-negative cells.

**Table 11. Summary of the in vitro cytotoxicity of ADC-5**

| | **HER2 positive** | | **HER2 n egative** | | **IC₅₀ in HER2+ (geom. mean)** | **IC₅₀ in HER2-(geom. mean** | **Selec. Ratio** |
|---|---|---|---|---|---|---|---|
| | **SK-BR-3** | **HCC-1954** | **MDA-MB-231** | **MCF-7** | | | |
| IC₅₀ (µg/mL) | >7.61E-02 | 1.70E-01 | >1.00E+02 | >1.00E+02 | 1.13E-01 | >1.00E+02 | >880 |
| IC₅₀ (M) | > 5.07E-10 | 1.13E-09 | > 6.67E-07 | > 6.67E-07 | 7.57E-10 | > 6.67E-07 | |

It is also seen from WO 2018/167270 that drug moieties D of the present invention demonstrate efficacy in the following cell lines, the data of which is hereby incorporated by reference.

| Name | No ATCC | Species | Tissue | Characteristics |
|---|---|---|---|---|
| A549 | CCL-185 | human | lung | lung carcinoma (NSCLC) |
| HT29 | HTB-38 | human | colon | colorectal adenocarcinoma |
| MDA-MB-231 | HTB-26 | human | breast | breast adenocarcinoma |
| PSN1 | CRM-CRL-3211 | human | pancreas | pancreas adenocarcinoma |

### Example 4. MTD and MTMD of compound 1

### Maximum Tolerated Dose of 1 in mice

CB17/SCID female mice (Envigo) were used for this study. Animals were randomly allocated to dose groups and received a single intravenous administration.

After the administration, animals were observed for clinical signs at ficed intervals, up to 14 days after dosing. Mortality was recorded daily.

The Maximum Tolerated Dose (MTD) was defined as the dose level with no mortality recorded. Results are summarized in Table 12.

**Table 12. MTD of compound 1**

| **Dose Levels (mg/kg)** | **MTD (mg/kg)** |
|---|---|
| 50.0 | 0.1 |
| 25.0 | |
| 15.0 | |
| 10.0 | |
| 5.0 | |
| 2.5 | |
| 1.0 | |
| 0.5 | |
| 0.25 | |
| 0.1 | |
| Placebo | |

### Maximum Tolerated Multiple Dose of 1 in mice

Athymic and CB17/SCID female mice (Envigo) were used for these studies. Animals were randomly allocated to dose groups and received weekly intravenous administration of 1 for 5 consecutive days (q7dx5).

After administration, animals were observed fro clinical signs at fixed intervals, up to 14 days after the last dose. Mortality was recorded daily.

The Maximum Tolerated Multiple Dose (MTMD) was defined as the dose level with no mortality recorded. Results are summarized in Table 13.

**Table 13. MTMD of compound 1.**

| **Animals** | **Dose Levels (mg/kg)** | **MTMD (mg/kg)** |
|---|---|---|
| Athymmic | 2.00 | 0.1 * |
| | 1.00 | |
| | 0.10 | |
| | 0.075 | |
| | 0.025 | |
| | Placebo | |
| CB17/SCID | 0.10 | 0.075* |
| | 0.075 | |
| | 0.025 | |
| | Placebo | |

| | | |
|---|---|---|
| *Preliminary data, animals still on the follow-up period. | | |

### Example 5. In vivo activity of ADC 3

ADC-3 has been evaluated for in vivo antitumor activity in BT474 cell line, a HER2 positive breast tumor model.

### Experimental Design

Briefly, 4 to 6 week-old SCID mice were subcutaneously implanted with tumor fragments previously generated in donor mice. Before the initiation of each experiment, a blinded, independent determination of C-erb2 (oncogene related with HER-2 expression) by IHC was performed.

The animals were implanted as described above and when tumors reached ca. 150-200 mm3, tumor bearing animals (N = 10/group) were randomly allocated into treatment groups according to the following experimental design:

| **Tumor** | **Group** | **Dose (mg/kg)** |
|---|---|---|
| BT-474 | Control | 0.0 |
| | Herceptin | 30.0 |
| | Kadcyla | 30.0 |
| | ADC3 | 4.0 |

Treatments were administered intravenously on days 0.7.14, 21 and 28 (q7dx5).

Tumor dimensions and body weights were recorded 3 times per week starting from the first day of treatment (Day 0). Treatments producing >20% lethality and/or 20% net body weight loss were considered toxic. Tumor volume was calculated using the equation (a·b²)/2, where a and b were the longest and shortest diameters, respectively. Animals were euthanized when their tumors reached ca. 2000 mm³ and/or severe necrosis was seen. Median was calculated for tumor volume on each measurement day. Complete tumor regression (CR) was defined when tumor volume < 63 mm³ for 2 or more consecutive measurements.

### Results

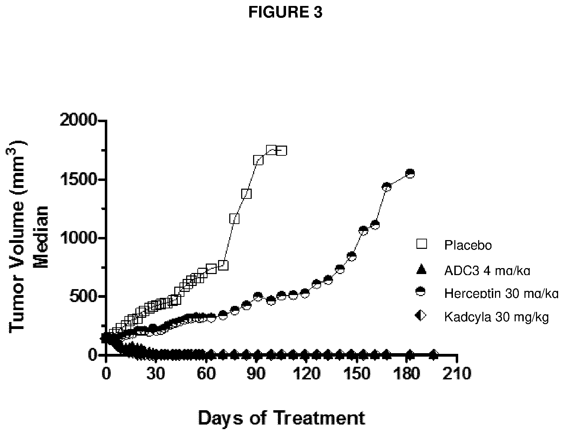

### All the treatments administered to mice bearing BT-474 tumors resulted in antitumor activity (Figure 3)

ADC3 administered at 4 mg/kg and Kadcyla (30 mg/kg) induced tumor regressions. ADC3 at 4.0 mg/kg, 7 out of 7 tumor regressions from day 26 to day 190 and Kadcyla at 30.0 mg/kg, 7 out of 7 tumor regressions from day 14 to day 190. Since no tumor regrowth has yet been observed (on Day 190), this experiment is considered to be still on going.

Herceptin (30 mg/Kg) treatment also induced marked antitumor activity that was statistically significant, p < 0.038 from day 12 until the last experimental data registered day, although complete tumor regressions were not achieved.

## Claims

1. A drug conjugate comprising a drug moiety covalently attached to the rest of the drug conjugate, the drug conjugate having formula [D-(X)_{b}-(AA)_{w}-(T)_{g}-(L)-]ₙ-Ab wherein:
D is a drug moiety having the following formula (I) or a pharmaceutically acceptable salt or ester thereof, 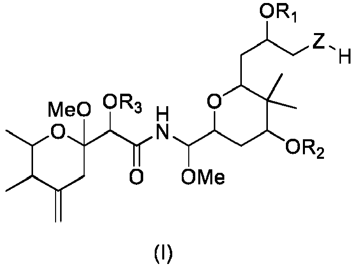 wherein:
D is covalently attached via a hydroxy group at OR_{1,} OR₃ or ZH or via a thiol group at ZH, to (X)_{b} if any, or (AA)_{w} if any, or to (T)_{g} if any, or (L);
R₁ is selected from hydrogen, substituted or unsubstituted C₁-C₁₂ alkyl, substituted or unsubstituted C₂-C₁₂ alkenyl, substituted or unsubstituted C₂-C₁₂ alkynyl, -C(=O)Rₐ, -C(=O)OR_{b} and -C(=O)NR_{c}R_{d};
R₂ is hydrogen;
R₃ is selected from hydrogen, -C(=O)Rₐ, -C(=O)OR_{b}, and -C(=O)NR_{c}R_{d};
Z is selected from -O- and -S-;
Rₐ is selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group;
R_{b} is selected from substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group;
R_{c} and R_{d} are independently selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl and substituted or unsubstituted heterocyclic group;
X and T are extending groups that may be the same or different;
each AA is independently an amino acid unit;
L is a linker group;
w is an integer ranging from 0 to 12;
b is an integer of 0 or 1;
g is an integer of 0 or 1;
Ab is a moiety comprising at least one antigen binding site; and
n is the ratio of the group [D-(X)_{b}-(AA)_{w}-(T)_{g}-(L)-] to the moiety comprising at least one antigen binding site and is in the range from 1 to 20.

2. The drug conjugate according to claim 1, wherein D is also a compound of formula (Ia), or a pharmaceutically acceptable salt or ester thereof: 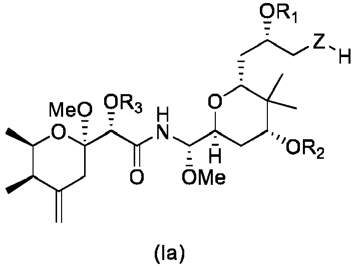 wherein R₁, R₂, R₃ and Z are as defined in formula (I) in claim 1.

3. The drug conjugate according to claim 1, wherein D is a compound of formula: 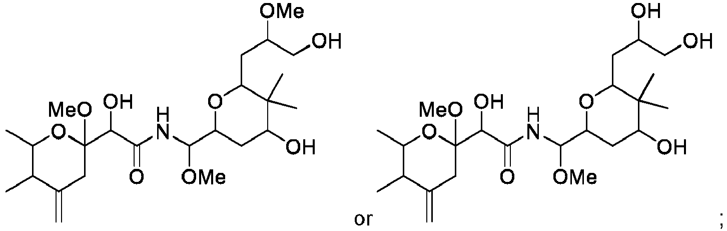 or a pharmaceutically acceptable salt or ester thereof.

4. The drug conjugate according to claim 2, wherein D is a compound of formula: 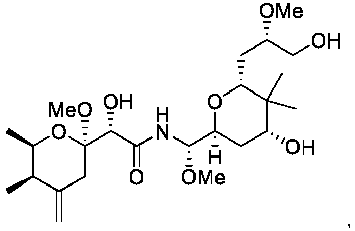 or a pharmaceutically acceptable salt or ester thereof.

5. The drug conjugate according to claim 2, wherein D is a compound of formula: 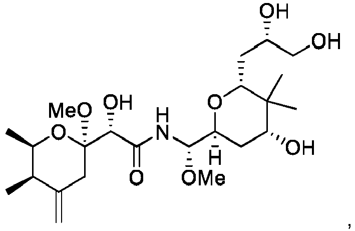 or a pharmaceutically acceptable salt or ester thereof.

6. A drug conjugate comprising a drug moiety covalently attached to the rest of the drug conjugate, the drug conjugate having formula [D-(X)_{b}-(AA)_{w}-(T)_{g}-(L)-]ₙ-Ab wherein:
D is a drug moiety having the following formula (II) or a pharmaceutically acceptable salt or ester thereof: 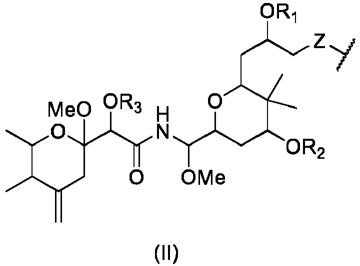 wherein:
the wavy line indicates the point of covalent attachment to (X)_{b} if any, or (AA)_{w} if any, or to (T)_{g} if any, or (L);
R₁ is selected from hydrogen, substituted or unsubstituted C₁-C₁₂ alkyl, substituted or unsubstituted C₂-C₁₂ alkenyl, substituted or unsubstituted C₂-C₁₂ alkynyl, -C(=O)Rₐ, -C(=O)OR_{b} and -C(=O)NR_{c}R_{d}; wherein the optional substituents are one or more substituents Rₓ;
R₂ is hydrogen;
R₃ is selected from hydrogen, -C(=O)Rₐ, -C(=O)OR_{b}, and -C(=O)NR_{c}R_{d};
Z is -O- or -S-;
Rₐ is selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group; wherein the optional substituents are one or more substituents Rₓ;
R_{b} is selected from substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group; wherein the optional substituents are one or more substituents Rₓ;
R_{c} and R_{d} are independently selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl and substituted or unsubstituted heterocyclic group; wherein the optional substituents are one or more substituents Rₓ;
substituents Rₓ are selected from the group consisting of C₁-C₁₂ alkyl groups which may be optionally substituted with at least one group R_{y}, C₂-C₁₂ alkenyl groups which may be optionally substituted with at least one group R_{y}, C₂-C₁₂ alkynyl groups which may be optionally substituted with at least one group R_{y}, halogen atoms, oxo groups, thio groups, cyano groups, nitro groups, OR_{y}, OCOR_{y}, OCOOR_{y}, COR_{y}, COOR_{y}, OCONR_{y}R_{z}, CONR_{y}R_{z}, SR_{y}, S(=O)R_{y}, SO₂R_{y}, SSR_{y}, P(O)(R_{y})OR_{z}, NR_{y}R_{z}, NR_{y}COR_{z}, NR_{y}C(=O)NR_{y}R_{z}, NR_{y}C(=NR_{y})NR_{y}R_{z}, aryl groups having from 6 to 18 carbon atoms in one or more rings which may optionally be substituted with one or more substituents which may be the same or different selected from the group consisting of R_{y}, OR_{y}, OCOR_{y}, OCOOR_{y}, NR_{y}R_{z}, NR_{y}COR_{z}, and NR_{y}C(=NR_{y})NR_{y}R_{z}, aralkyl groups comprising an alkyl group having from 1 to 12 carbon atoms substituted with an optionally substituted aryl group as defined above, aralkyloxy groups comprising an alkoxy group having from 1 to 12 carbon atoms substituted with an optionally substituted aryl group as defined above, and a 5- to 14- membered saturated or unsaturated heterocyclic group having one or more rings and comprising at least one oxygen, nitrogen or sulphur atom in said ring(s), said heterocyclic group optionally being substituted with one or more substituents R_{y}, and where there is more than one optional substituents on any given group the optional substituents R_{y} may be the same or different;
each R_{y} and R_{z} is independently selected from the group consisting of hydrogen, C₁-C₁₂ alkyl groups, C₁-C₁₂ alkyl groups that are substituted with at least one halogen atom, aralkyl groups comprising a C₁-C₁₂ alkyl group that is substituted with an aryl group having from 6 to 18 carbon atoms in one or more rings and heterocycloalkyl groups comprising a C₁-C₁₂ alkyl group that is substituted with a 5- to 14- membered saturated or unsaturated heterocyclic group having one or more rings and comprising at least one oxygen, nitrogen or sulphur atom in said ring(s);
X and T are extending groups that may be the same or different;
each AA is independently an amino acid unit;
L is a linker group;
w is an integer ranging from 0 to 12;
b is an integer of 0 or 1;
g is an integer of 0 or 1;
where b+g+w is optionally not 0;
Ab is a moiety comprising at least one antigen binding site; and
n is the ratio of the group [D-(X)_{b}-(AA)_{w}-(T)_{g}-(L)-] to the moiety comprising at least one antigen binding site and is in the range from 1 to 20.

7. The drug conjugate according to claim 6, or a pharmaceutically acceptable salt or ester thereof, wherein D is a drug moiety of formula (Ila): 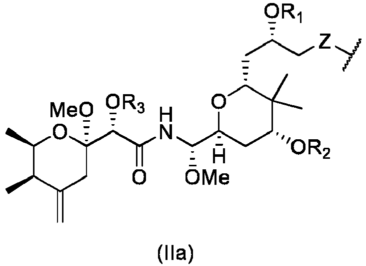 where the wavy line, R₁, R₂, R₃ and Z are as defined for formula (II).

8. The drug conjugate according to any one of claims 1 to 7, wherein the salt is selected from hydrochloride, hydrobromide, hydroiodide, sulfate, nitrate, phosphate, acetate, trifluoroacetate, maleate, fumarate, citrate, oxalate, succinate, tartrate, malate, mandelate, methanesulfonate, p-toluenesulfonate, sodium, potassium, calcium, ammonium, ethylenediamine, ethanolamine, N,N-dialkylenethanolamine, triethanolamine and basic aminoacids.

9. The drug conjugate according to any one of the preceding claims, wherein L is a linker group selected from the group consisting of: 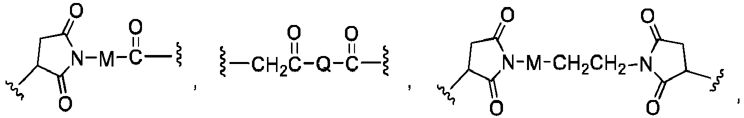 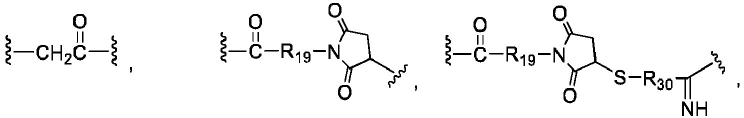 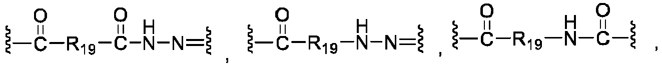 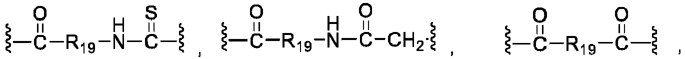 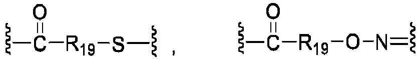 wherein
the wavy lines indicate the point of covalent attachments to an Ab (the wavy line to the right) and to (T)_{g} if any, or (AA)_{w} if any, or (X)_{b} if any, or to D (the wavy line to the left);
R₁₉ is selected from -C₁-C₁₂ alkylene-, -C₃-C₈ carbocyclo-, -O-(C₁-C₁₂ alkylene)-, -C₆-C₁₈ arylene- in one or more rings which may optionally be substituted with one or more substituents Rₓ, -C₁-C₁₂ alkylene-C₆-C₁₈ arylene- wherein the arylene group is in one or more rings which may optionally be substituted with one or more substituents Rₓ, -C₆-C₁₈ arylene-C₁-C₁₂ alkylene-wherein the arylene group is in one or more rings which may optionally be substituted with one or more substituents Rₓ, -C₁-C₁₂ alkylene-(C₃-C₈ carbocyclo)-, -(C₃-C₈ carbocyclo)-C₁-C₁₂ alkylene-, -C₅-C₁₄ heterocyclo- wherein said heterocyclo group may be a saturated or unsaturated group having one or more rings and comprising at least one oxygen, nitrogen or sulphur atom in said ring(s), said group optionally being substituted with one or more substituents Rₓ, -C₁-C₁₂ alkylene-(C₅-C₁₄ heterocyclo)- wherein said heterocyclo group may be a saturated or unsaturated group having one or more rings and comprising at least one oxygen, nitrogen or sulphur atom in said ring(s), said group optionally being substituted with one or more substituents Rₓ, -(C₅-C₁₄ heterocyclo)-C₁-C₁₂ alkylene- wherein said heterocyclo group may be a saturated or unsaturated group having one or more rings and comprising at least one oxygen, nitrogen or sulphur atom in said ring(s), said group optionally being substituted with one or more substituents Rₓ, -(OCH₂CH₂)ᵣ- and -CH₂-(OCH₂CH₂)ᵣ-, wherein each of the above alkylene substituents whether alone or attached to another moiety in the carbon chain may optionally be substituted by one or more substituents Rₓ;
R₃₀ is a -C₁-C₆ alkylene- group;
M is selected from the group consisting of -C₁-C₆ alkylene-, -C₁-C₆ alkylene-(C₃-C₈ carbocyclo)-, -(CH₂CH₂O)ₛ-, -C₁-C₆ alkylene-(C₃-C₈ carbocyclo)-CON(H or C₁-C₆ alkyl)-C₁-C₆ alkylene-, - phenylene- which may optionally be substituted with one or more substituents Rₓ, -phenylene-C₁-C₆ alkylene- wherein the phenylene moiety may optionally be substituted with one or more substituents Rₓ and -C₁-C₆ alkylene-CON(H or C₁-C₆ alkyl)C₁-C₆ alkylene-;
Q is selected from the group consisting of -N(H or C₁-C₆ alkyl)phenylene- and -N(H or C₁-C₆ alkyl)-(CH₂)ₛ;
r is an integer ranging from 1 to 10; and
s is an integer ranging from 1 to 10;
or
wherein L is a linker group selected from the group consisting of: 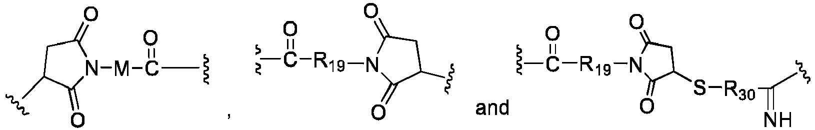 wherein:
the wavy lines indicate the point of covalent attachments to an Ab (the wavy line to the right) and to (T)_{g} if any, or (AA)_{w} if any, or (X)_{b}, if any, or to D (the wavy line to the left);
R₁₉ is selected from -C₁-C₁₂ alkylene-, -O-(C₁-C₁₂ alkylene), -C₆-C₁₂ arylene- in one or more rings which may optionally be substituted with one or more substituents Rₓ, -C₁-C₁₂ alkylene-C₆-C₁₂ arylene- wherein the arylene group is in one or more rings which may optionally be substituted with one or more substituents Rₓ, -C₆-C₁₂ arylene-C₁-C₁₂ alkylene- wherein the arylene group is in one or more rings which may optionally be substituted with one or more substituents Rₓ, -C₅-C₁₂ heterocyclo- wherein said heterocyclo group may be a saturated or unsaturated group having one or more rings and comprising at least one oxygen, nitrogen or sulphur atom in said ring(s), said group optionally being substituted with one or more substituents Rₓ, -C₁-C₁₂ alkylene-(C₅-C₁₂ heterocyclo)- wherein said heterocyclo group may be a saturated or unsaturated group having one or more rings and comprising at least one oxygen, nitrogen or sulphur atom in said ring(s), said group optionally being substituted with one or more substituents Rₓ, -(C₅-C₁₂ heterocyclo)-C₁-C₁₂ alkylene- wherein said heterocyclo group may be a saturated or unsaturated group having one or more rings and comprising at least one oxygen, nitrogen or sulphur atom in said ring(s), said group optionally being substituted with one or more substituents Rₓ, -(OCH₂CH₂)ᵣ- and -CH₂-(OCH₂CH₂)ᵣ-, wherein each of the above alkylene substituents whether alone or attached to another moiety in the carbon chain may optionally be substituted by one or more substituents Rₓ;
R₃₀ is a -C₁-C₆ alkylene- group;
M is selected from the group consisting of -C₁-C₆ alkylene-, -C₁-C₆ alkylene-(C₃-C₈ carbocyclo)- and -phenylene- which may optionally be substituted with one or more substituents Rₓ; and
r is an integer ranging from 1-6.

10. The drug conjugate according to any one of claims 1 to 9, selected from the formulas (V), (VI) and (VII): 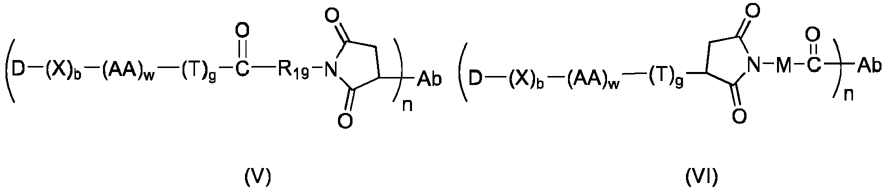 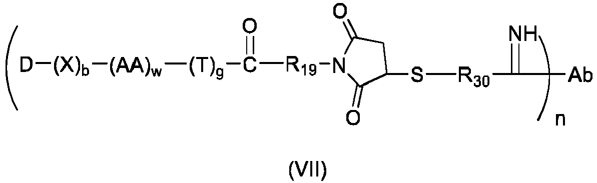 wherein:
X and T are extending groups that may be the same or different;
each AA is independently an amino acid unit;
w is an integer ranging from 0 to 12;
b is an integer of 0 or 1;
g is an integer of 0 or 1;
D is a drug moiety;
Ab is a moiety comprising at least one antigen binding site;
n is the ratio of the group [D-(X)_{b}-(AA)_{w}-(T)_{g}-(L)-] wherein L is as defined in formula (V), (VI) or (VII) to the moiety comprising at least one antigen binding site and is in the range from 1 to 20;
R₁₉ is selected from -C₁-C₈ alkylene-, -O-(C₁-C₈ alkylene)-, -C₁-C₈ alkylene-C₆-C₁₂ arylene-wherein the arylene group is in one or more rings which may optionally be substituted with one or more substituents Rₓ, and -C₆-C₁₂ arylene-C₁-C₈ alkylene- wherein the arylene group is in one or more rings which may optionally be substituted with one or more substituents Rₓ, wherein each of the above alkylene substituents whether alone or attached to another moiety in the carbon chain may optionally be substituted by one or more substituents Rₓ;
R₃₀ is a -C₂-C₄ alkylene- group; and
M is selected from the group consisting of -C₁-C₃ alkylene- and -C₁-C₃ alkylene-(C₅-C₇ carbocyclo)-; or
selected from the formulas (V), (VI) and (VII): 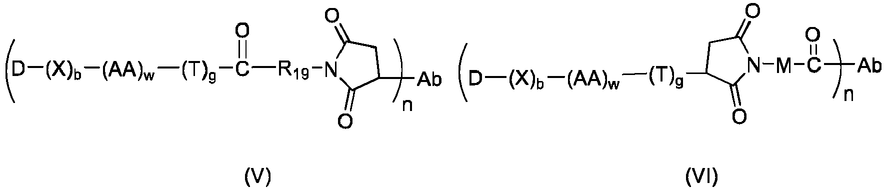 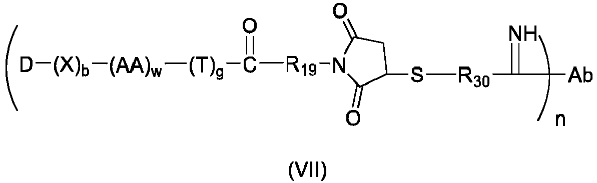 wherein:
X and T are extending groups that may be the same or different;
each AA is independently an amino acid unit;
w is an integer ranging from 0 to 12;
b is an integer of 0 or 1;
g is an integer of 0 or 1;
D is a drug moiety;
Ab is a moiety comprising at least one antigen binding site;
n is the ratio of the group [D-(X)_{b}-(AA)_{w}-(T)_{g}-(L)-] wherein L is as defined in (V), (VI) or (VII) to the moiety comprising at least one antigen binding site and is in the range from 1 to 20;
R₁₉ is selected from -C₁-C₆ alkylene-, -phenylene-C₁-C₆ alkylene- wherein the phenylene group may optionally be substituted with one or more substituents Rₓ selected from the group consisting of alkyl groups having from 1 to 6 carbon atoms, alkoxy groups having from 1 to 6 carbon atoms, halogen atoms, nitro groups and cyano groups, wherein each of the above alkylene substituents whether alone or attached to another moiety in the carbon chain may optionally be substituted by one or more substituents Rₓ selected from the group consisting of alkyl groups having from 1 to 6 carbon atoms, alkoxy groups having from 1 to 6 carbon atoms,
aryl groups having from 6 to 12 carbon atoms, halogen atoms, nitro groups and cyano groups,
and preferably R₁₉ is a -C₁-C₆ alkylene- group;
R₃₀ is a -C₂-C₄ alkylene- group; and
M is -C₁-C₃ alkylene-(C₅-C₇ carbocyclo)-.

11. The drug conjugate according to any one of claims 1 to 10, wherein (AA)_{w} is of formula (III): 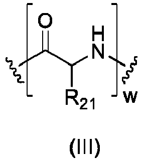
wherein the wavy lines indicate the point of covalent attachments to (X)_{b} if any, or to the drug moiety (the wavy line to the left) and to (T)_{g} if any, or to the linker (the wavy line to the right); and
R₂₁ is, at each occurrence, selected from the group consisting of hydrogen, methyl, isopropyl, isobutyl, sec-butyl, benzyl, p-hydroxybenzyl, -CH₂OH, -CH(OH)CH₃, -CH₂CH₂SCH₃, - CH₂CONH₂, -CH₂COOH, -CH₂CH₂CONH₂, -CH₂CH₂COOH, -(CH₂)₃NHC(=NH)NH₂, - (CH₂)₃NH₂, -(CH₂)₃NHCOCH₃, -(CH₂)₃NHCHO, -(CH₂)₄NHC(=NH)NH₂, -(CH₂)₄NH₂, - (CH₂)₄NHCOCH₃, -(CH₂)₄NHCHO, -(CH₂)₃NHCONH₂, -(CH₂)₄NHCONH₂, - CH₂CH₂CH(OH)CH₂NH₂, 2-pyridylmethyl-, 3-pyridylmethyl-, 4-pyridylmethyl-, phenyl, cyclohexyl, 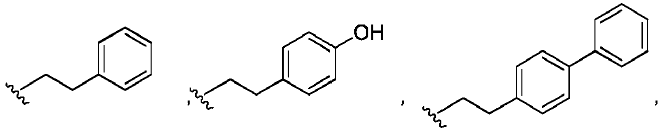 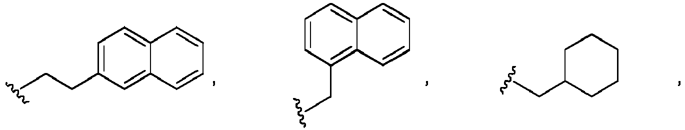 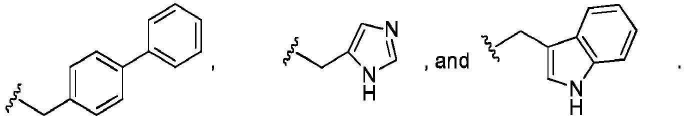
and w is an integer ranging from 1 to 12;
or
wherein (AA)_{w} is of formula (III) wherein:
R₂₁ is selected, at each occurrence, from the group consisting of hydrogen, methyl, isopropyl, sec-butyl, benzyl, indolylmethyl, -(CH₂)₃NHCONH₂, -(CH₂)₄NH₂, -(CH₂)₃NHC(=NH)NH₂ and - (CH₂)₄NHC(=NH)NH₂; and
w is an integer ranging from 0 to 6;
or
wherein w is 0 or 2, and where w is 2, then (AA)_{w} is of formula (IV): 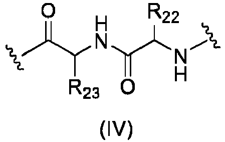 wherein:
the wavy lines indicate the point of covalent attachments to (X)_{b} if any, or to the drug moiety (the wavy line to the left) and to (T)_{g} if any, or to the linker (the wavy line to the right);
R₂₂ is selected from methyl, benzyl, isopropyl, sec-butyl and indolylmethyl; and
R₂₃ is selected from methyl, -(CH₂)₄NH₂, -(CH₂)₃NHCONH₂ and -(CH₂)₃NHC(=NH)NH₂.

12. The drug conjugate according to any one of claims 1 to 11, wherein X is an extending group selected from:
-CONH-(C₁-C₆ alkylene)NH-;
-COO-CH₂-(phenylene which may optionally be substituted with one or more substituents Rₓ)-NH-;
-CONH-(C₁-C₆ alkylene)NH-COO-CH₂-(phenylene which may optionally be substituted with one or more substituents Rₓ)-NH-;
-COCH₂NH-COCH₂-NH-;
-COCH₂NH-;
-CONH-(C₁-C₆ alkylene)S-;
-CONH-(C₁-C₆ alkylene)NHCO(C₁-C₆ alkylene)S-; and
b is 0 or 1, preferably 1;
or
wherein X is an extending group selected from the group consisting of:
-CONH-(C₂-C₄ alkylene)NH-;
-COO-CH₂-phenylene-NH-, wherein said phenylene group may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups;
-CONH-(C₂-C₄ alkylene)NH-COO-CH₂-(phenylene which may optionally be substituted with from one to four substituents Rₓ selected from the group consisting of alkyl groups having from 1 to 6 carbon atoms, alkoxy groups having from 1 to 6 carbon atoms, halogen atoms, nitro groups and cyano groups)-NH-;
-COCH₂NH-COCH₂-NH-;
-CONH-(C₂-C₄ alkylene)S-;
-CONH-(C₂-C₄ alkylene)NHCO(C₁-C₃ alkylene)S-; and
b is 0 or 1, preferably 1; or
wherein X is an extending group selected from the group consisting of:
-COO-CH₂-phenylene-NH-;
-CONH(CH₂)₃NHCOOCH₂-phenylene-NH-;
-CONH(CH₂)₃NH-;
-CONH(CH₂)₃-S-;
-CONH(CH₂)₃NHCO(CH₂)₂S-; and
b is 0 or 1, preferably 1.

13. The drug conjugate according to any one of claims 1 to 12, wherein T is an extending group selected from the group consisting of -CO-(C₁-C₆ alkylene)-NH-,-CO-(C₁-C₆ alkylene)-[O-(C₂-C₆ alkylene)]ⱼ-NH-, -COO-(C₁-C₆, alkylene)-[O-(C₂-C₆ alkylene)]ⱼ-NH-; where j is an integer from 1 to 25, and g is 0 or 1;
or
wherein T is an extending group selected from the group consisting of -CO-(C₁-C₄ alkylene)NH-, -CO-(C₁-C₄ alkylene)-[O-(C₂-C₄ alkylene)]ⱼ-NH-, -COO-(C₁-C₄ alkylene)-[O-(C₂-C₄ alkylene)]ⱼ-NH-,where j is an integer from 1 to 10; and g is 0 or 1;
or
wherein T is an extending group selected from the group consisting of -CO-(C₁-C₄ alkylene)NH-, -CO-(C₁-C₄ alkylene)-[O-(C₂-C₄ alkylene)]ⱼ-NH-, -COO-(C₁-C₄ alkylene)-[O-(C₂-C₄ alkylene)]ⱼ-NH-; where j is an integer from 1 to 5; and g is 0 or 1

14. The drug conjugate according to any one of claims 6 to 13, wherein D is a drug moiety of formula (II) or formula (Ila) or a pharmaceutically acceptable salt or ester thereof, wherein:
R₁ is hydrogen or substituted or unsubstituted C₁-C₆ alkyl, wherein the optional substituents are one or more substituents Rₓ;
R₂ is hydrogen;
R₃ is hydrogen or -C(=O)Rₐ, wherein Rₐ is substituted or unsubstituted C₁-C₆ alkyl, wherein the optional substituents are one or more substituents Rₓ; and
Z is -O-;
or
wherein D is a drug moiety of formula (II) or formula (Ila) or a pharmaceutically acceptable salt or ester thereof, wherein:
R₁ is hydrogen or methyl;
R₂ is hydrogen;
R₃ is hydrogen; and
Z is -O-;
or
wherein D is a drug moiety of formula (II) or formula (Ila), or a pharmaceutically acceptable salt or ester thereof wherein:
R₁ is methyl;
R₂ is hydrogen;
R₃ is hydrogen; and
Z is -O-.

15. The drug conjugate according to any one of claims 1, 3, 6 to 14, wherein D is selected from: 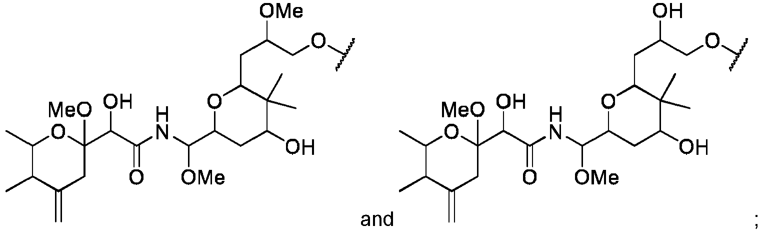
or a pharmaceutically acceptable salt or ester thereof, wherein the wavy line indicates the point of covalent attachment to (X)_{b} if any, or (AA)_{w} if any, or to (T)_{g} if any, or to (L);
or
wherein D is selected from: 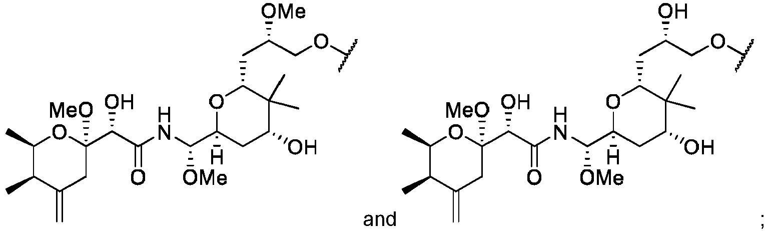 or a pharmaceutically acceptable salt or ester thereof, wherein the wavy line indicates the point of covalent attachment to (X)_{b} if any, or (AA)_{w} if any, or to (T)_{g} if any, or to (L).

16. The drug conjugate according to any one of claims 1 to 15, wherein the moiety Ab comprising at least one antigen binding site is an antigen-binding peptide;
optionally
wherein the moiety Ab comprising at least one antigen binding site is an antibody, a single domain antibody or an antigen-binding fragment thereof;
optionally
wherein the moiety Ab comprising at least one antigen binding site is a monoclonal antibody, polyclonal antibody or bispecific antibody and wherein the antibody or an antigen-binding fragment thereof is derived from any species, preferably a human, mouse or rabbit;
optionally
wherein the antibody or antigen-binding fragment thereof is selected from the group consisting of a human antibody, an antigen-binding fragment of a human antibody, a humanized antibody, an antigen-binding fragment of a humanized antibody, a chimeric antibody, an antigen-binding fragment of a chimeric antibody, a glycosylated antibody and a glycosylated antigen binding fragment;
optionally
wherein the antibody or antigen-binding fragment thereof is an antigen-binding fragment selected from the group consisting of an Fab fragment, an Fab' fragment, an F(ab')2 fragment and an Fv fragment;
optionally
wherein the antibody or antigen-binding fragment thereof is a monoclonal antibody which immunospecifically binds to cancer cell antigens, viral antigens, antigens of cells that produce autoimmune antibodies associated with autoimmune disease, microbial antigens, and preferably a monoclonal antibody which immunospecifically binds to cancer cell antigens;
optionally
wherein the moiety Ab comprising at least one antigen binding site is an antibody selected from the group consisting of Abciximab, Alemtuzumab, Anetumab, Atezolizumab, Avelumab, Basiliximab, Bevacizumab, Blinatomumab, Brentuximab, Catumaxomab, Cetuximab, Coltuximab, Daclizumab, Daratumumab, Denintuzumab, Denosumab, Depatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labetuzumab, Ladiratuzumab, Laprituximab, Lifastuzumab, Lorvotuzumab, Milatuzumab, Mirvetuximab, Naratuximab, Necitumumab, Nimotuzumab, Nivolumab, Obinutuzumab, Ofatumumab, Olaratumab, Omalizumab, Palivizumab, Panitumumab, Pembrolizumab, Pertuzumab, Pinatuzumab, Polatuzumab, Ramucirumab, Rovalpituzumab, Sacituzumab, Siltuximab, Sirtratumab, Sofituzumab, Vadastuximab, Vorsetuzumab, an anti-HER2 antibody such as Trastuzumab, an anti-CD4 antibody, an anti-CD5 antibody, and an anti-CD30 antibody, or an antigen-binding fragment or an immunologically active portion thereof;
optionally
wherein the moiety Ab comprising at least one antigen binding site is an antibody selected from the group consisting of Abciximab, Alemtuzumab, Anetumab, Atezolizumab, Avelumab, Basiliximab, Bevacizumab, Blinatomumab, Brentuximab, Catumaxomab, Cetuximab, Daclizumab, Daratumumab, Denintuzumab, Denosumab, Depatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labetuzumab, Ladiratuzumab, Laprituximab, Mirvetuximab, Naratuximab, Necitumumab, Nimotuzumab, Nivolumab, Obinutuzumab, Ofatumumab, Olaratumab, Omalizumab, Palivizumab, Panitumumab, Pembrolizumab, Pertuzumab, Polatuzumab, Ramucirumab, Rovalpituzumab, Sacituzumab, Siltuximab, Sirtratumab, Vadastuximab, Vorsetuzumab, an anti-HER2 antibody such as Trastuzumab, an anti-CD4 antibody, an anti-CD5 antibody, and an anti-CD30 antibody, or an antigen-binding fragment or an immunologically active portion thereof;
optionally
wherein the moiety Ab comprising at least one antigen binding site is an antibody selected from the group consisting of Abciximab, Alemtuzumab, Atezolizumab, Avelumab, Basiliximab, Bevacizumab, Blinatomumab, Brentuximab, Catumaxomab, Cetuximab, Daclizumab, Daratumumab, Denosumab, Dinutuximab, Durvalumab, Elotuzumab, Gemtuzumab, Ibritumomab, Inotuzumab, Ipilimumab, Labetuzumab, Necitumumab, Nimotuzumab, Nivolumab, Obinutuzumab, Ofatumumab, Olaratumab, Omalizumab, Palivizumab, Panitumumab, Pembrolizumab, Pertuzumab, Ramucirumab, Rovalpituzumab, Siltuximab, an anti-HER2 antibody such as Trastuzumab, an anti-CD4 antibody, an anti-CD5 antibody, and an anti-CD30 antibody, or an antigen-binding fragment or an immunologically active portion thereof, more preferably Brentuximab, Gemtuzumab, Inozutumab, Rovalpituzumab, an anti-HER2 antibody such as Trastuzumab, an anti-CD4 antibody, an anti-CD5 antibody, and an anti-CD30 antibody, or an antigen-binding fragment or an immunologicallly active portion thereof, preferably an anti-HER2 antibody such as Trastuzumab or an antigen-binding fragment or an immunologically active portion thereof, more preferably Trastuzumab or an antigen-binding fragment or an immunologically active portion thereof.

17. The antibody drug conjugate according to claim 1, selected from the group consisting of: 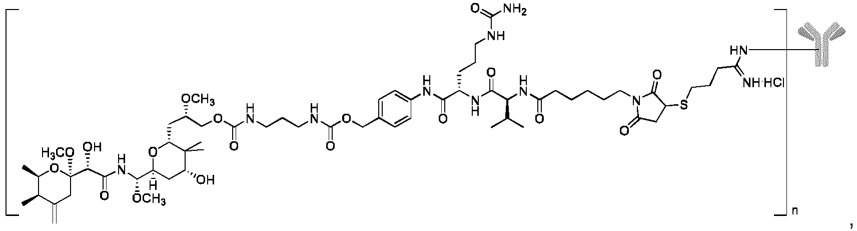 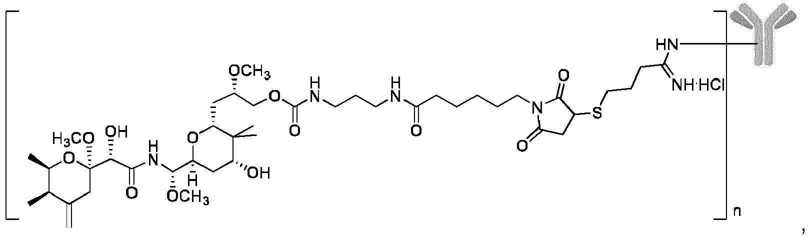 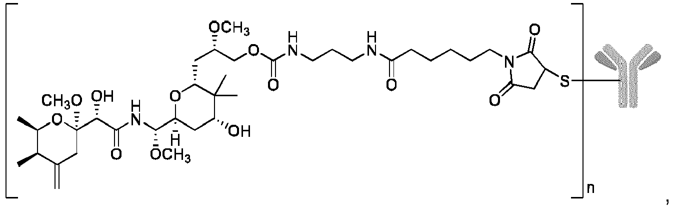 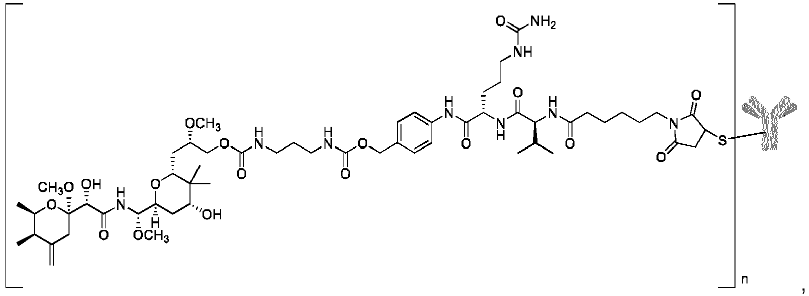 and 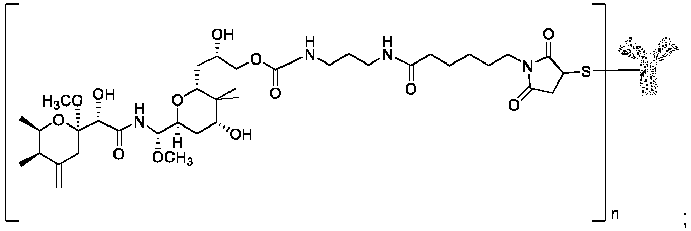 wherein n is from 2 to 6, more preferably 3, 4, or 5 and each 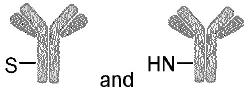 is independently selected from Brentuximab, Gemtuzumab, Inozutumab, Rovalpituzumab, an anti-HER2 antibody such as Trastuzumab, an anti-CD4 antibody, an anti-CD5 antibody, and an anti-CD30 antibody, or an antigen-binding fragment or an immunologically active portion thereof, and more preferably it is an anti-HER2 antibody such as Trastuzumab or an antigen-binding fragment or an immunologically active portion thereof, particularly Trastuzumab or an antigen-binding fragment or an immunologically active portion thereof.

18. The drug conjugate according to claim 17, wherein the moiety Ab comprising at least one antigen binding site is an anti-HER2 antibody such as Trastuzumab or an antigen-binding fragment or an immunologically active portion thereof.

19. The drug conjugate according to claim 17, wherein the moiety Ab comprising at least one antigen binding site is selected from Trastuzumab or an antigen-binding fragment or an immunologically active portion thereof.

20. The antibody drug conjugate according to any one of claims 1 to 19 in isolated or purified form.

21. A compound of formula D-(X)_{b}-(AA)_{w}-(T)_{g}-L₁ or of formula D-(X)_{b}-(AA)_{w}-(T)_{g}-H, wherein: L₁ is a linker selected from the group of formulas consisting of: 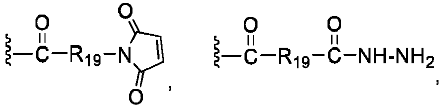 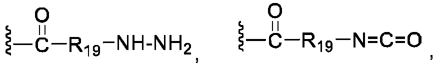 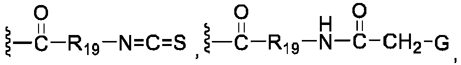 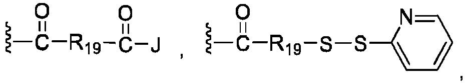 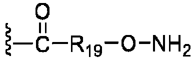
wherein each of the the wavy lines indicates the point of covalent attachment to (T)_{g} if any, or (AA)_{w} if any, or to (X)_{b} if any, or to D;
G is selected from halo, -O-mesyl and -O-tosyl;
J is selected from halo, hydroxy, -N-succinimidoxy, -O-(4-nitrophenyl), -O-pentafluorophenyl, - O-tetrafluorophenyl and -O-C(O)-OR₂₀;
R₁₉ is selected from -C₁-C₁₂ alkylene-, -C₃-C₈ carbocyclo-, -O-(C₁-C₁₂ alkylene)-, -C₆-C₁₈ arylene- in one or more rings which may optionally be substituted with one or more substituents Rₓ, -C₁-C₁₂ alkylene-C₆-C₁₈ arylene- wherein the arylene group is in one or more rings which may optionally be substituted with one or more substituents Rₓ, - C₆-C₁₈ arylene-C₁-C₁₂ alkylene- wherein the arylene group is in one or more rings which may optionally be substituted with one or more substituents Rₓ, -C₁-C₁₂ alkylene-(C₃-C₈ carbocyclo)-, -(C₃-C₈ carbocyclo)-C₁-C₁₂ alkylene-, -C₅-C₁₄ heterocyclo- wherein said heterocyclo group may be a saturated or unsaturated group having one or more rings and comprising at least one oxygen, nitrogen or sulphur atom in said ring(s), said group optionally being substituted with one or more substituents Rₓ, -C₁-C₁₂ alkylene-(C₅-C₁₄ heterocyclo)- wherein said heterocyclo group may be a saturated or unsaturated group having one or more rings and comprising at least one oxygen, nitrogen or sulphur atom in said ring(s), said group optionally being substituted with one or more substituents Rₓ, -(C₅-C₁₄ heterocyclo)-C₁-C₁₂ alkylene-, wherein said heterocyclo group may be a saturated or unsaturated group having one or more rings and comprising at least one oxygen, nitrogen or sulphur atom in said ring(s), said group optionally being substituted with one or more substituents Rₓ, -(OCH₂CH₂)ᵣ- and -CH₂-(OCH₂CH₂)ᵣ-, wherein each of the above alkylene substituents whether alone or attached to another moiety in the carbon chain may optionally be substituted by one or more substituents Rₓ;
R₂₀ is a C₁-C₁₂ alkyl or an aryl group having from 6 to 18 carbon atoms in one or more aromatic rings, said aryl groups optionally being substituted with one or more substituents Rₓ;
substituents Rₓ are selected from the group consisting of C₁-C₁₂ alkyl groups which may be optionally substituted with at least one group R_{y}, C₂-C₁₂ alkenyl groups which may be optionally substituted with at least one group R_{y}, C₂-C₁₂ alkynyl groups which may be optionally substituted with at least one group R_{y}, halogen atoms, oxo groups, thio groups, cyano groups, nitro groups, OR_{y}, OCOR_{y}, OCOOR_{y}, COR_{y}, COOR_{y}, OCONR_{y}R_{z}, CONR_{y}R_{z}, SR_{y}, S(=O)R_{y}, SO₂R_{y}, SSR_{y}, P(O)(R_{y})OR_{z}, NR_{y}R_{z}, NR_{y}COR_{z}, NR_{y}C(=O)NR_{y}R_{z}, NR_{y}C(=NR_{y})NR_{y}R_{z}, aryl groups having from 6 to 18 carbon atoms in one or more rings which may optionally be substituted with one or more substituents which may be the same or different selected from the group consisting of R_{y}, OR_{y}, OCOR_{y}, OCOOR_{y}, NR_{y}R_{z}, NR_{y}COR_{z}, and NR_{y}C(=NR_{y})NR_{y}R_{z}, aralkyl groups comprising an alkyl group having from 1 to 12 carbon atoms substituted with an optionally substituted aryl group as defined above, aralkyloxy groups comprising an alkoxy group having from 1 to 12 carbon atoms substituted with an optionally substituted aryl group as defined above, and a 5- to 14- membered saturated or unsaturated heterocyclic group having one or more rings and comprising at least one oxygen, nitrogen or sulphur atom in said ring(s), said heterocyclic group optionally being substituted with one or more substituents R_{y}, and where there is more than one optional substituents on any given group the optional substituents R_{y} may be the same or different;
each R_{y} and R_{z} is independently selected from the group consisting of hydrogen, C₁-C₁₂ alkyl groups, C₁-C₁₂ alkyl groups that are substituted with at least one halogen atom, aralkyl groups comprising a C₁-C₁₂ alkyl group that is substituted with an aryl group having from 6 to 18 carbon atoms in one or more rings and heterocycloalkyl groups comprising a C₁-C₁₂ alkyl group that is substituted with a 5- to 14- membered saturated or unsaturated heterocyclic group having one or more rings and comprising at least one oxygen, nitrogen or sulphur atom in said ring(s);
r is an integer ranging from 1-10;
b is an integer of 0 or 1;
g is an integer of 0 or 1;
w is an integer ranging from 0 to 12;
each of D, X, T, and AA is as defined in any one of claims 1 to 15;
wherein for compounds of formula D-(X)_{b}-(AA)_{w}-(T)_{g}-H, b+w+g is not 0;
optionally
wherein the compound of formula D-(X)_{b}-(AA)_{w}-(T)_{g}-L₁ is selected from: 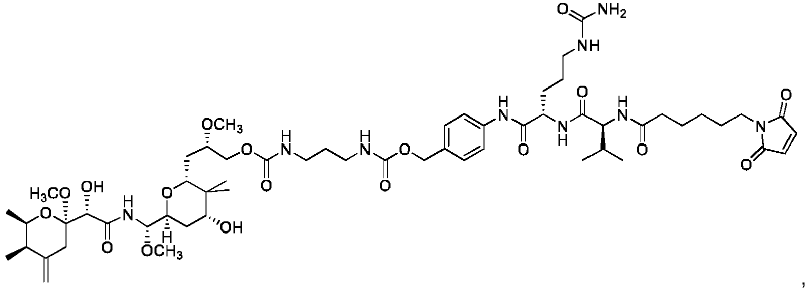 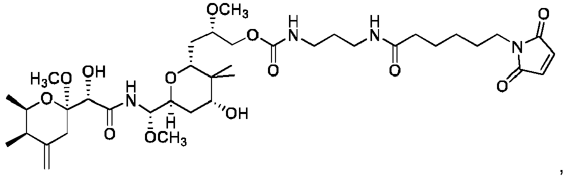 and

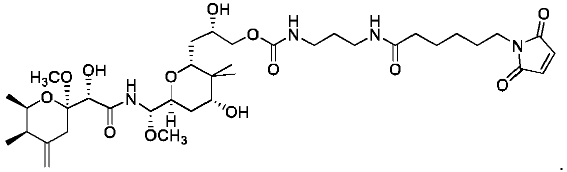

22. A compound of formula D-(X)_{b}-(AA)_{w}-(T)_{g}-L₁ or of formula D-(X)_{b}-(AA)_{w}-(T)_{g}-H, wherein each of D, X, AA, T, b, g and w are as defined in any one of claims 1 to 15 and L₁ is as defined in claim 21; but further wherein if the compound is a compound of formula D-(X)_{b}-(AA)_{w}-(T)_{g}-H then b+w+g≠0.

23. The drug conjugate or compound according to any one of claims 1 to 22, wherein b+g+w is not 0;
or
wherein b+w is not 0;
or
wherein when w is not 0, then b is 1.

24. a drug moiety as described in any one of claims 1 to 20, for use as a payload in an antibody drug conjugate.

25. Use of a drug moiety as described in any one of claims 1 to 20, in the manufacture of an antibody drug conjugate.

26. A drug conjugate according to any one of claims 1 to 20, for use as a medicament.

27. A drug conjugate according to any one of claims 1 to 20 for use in the treatment of cancer, preferably a cancer selected from lung cancer, including NSCLC, gastric cancer, colorectal cancer, breast cancer, pancreas carcinoma, endometrial cancer, bladder cancer, cervical cancer, esophageal cancer, gallbladder cancer, uterine cancer, salivary duct cancer, ovarian cancer, kidney cancer, leukaemia, multiple myeloma, and lymphoma.

28. The drug conjugate for use according to claim 27, wherein the cancer is a HER2 positive cancer, preferably HER2 positive lung cancer including HER2 positive NSCLC, HER2 positive gastric cancer, HER2 positive colorectal cancer, HER2 positive breast cancer, HER2 positive pancreas carcinoma, HER2 positive endometrial cancer, HER2 positive bladder cancer, HER2 positive cervical cancer, HER2 positive esophageal cancer, HER2 positive gallbladder cancer, HER2 positive uterine cancer, HER2 positive salivary duct cancer and HER2 positive ovarian cancer.

29. A pharmaceutical composition comprising a drug conjugate according to any one of claims 1 to 20 and a pharmaceutically acceptable carrier.

30. A kit comprising a therapeutically effective amount of a drug conjugate according to any one of claims 1 to 20 and a pharmaceutically acceptable carrier.

31. The kit according to claim 30 for use in the treatment of cancer, and preferably a cancer selected from lung cancer, including NSCLC, gastric cancer, colorectal cancer, breast cancer, pancreas carcinoma, endometrial cancer, bladder cancer, cervical cancer, esophageal cancer, gallbladder cancer, uterine cancer, salivary duct cancer, ovarian cancer, kidney cancer, leukaemia, multiple myeloma, and lymphoma.

32. A process for the preparation of a drug antibody conjugate according to any one of claims 1 to 20 comprising conjugating a moiety Ab comprising at least one antigen binding site and a drug D, Ab and D being as defined in any one of claims 1 to 20;
optionally wherein
the preparation of a drug antibody conjugate of formula (G), (G') or (G"): 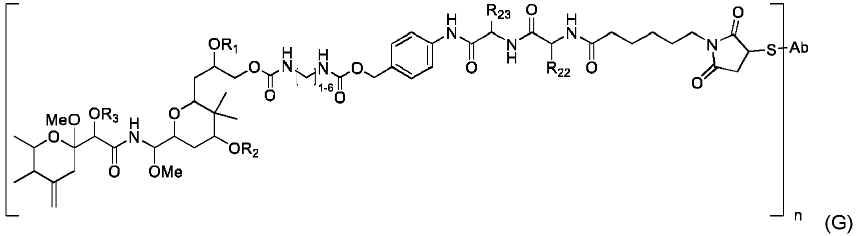 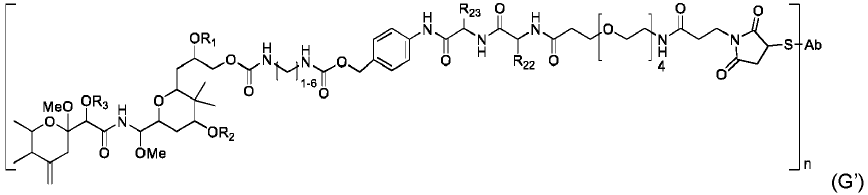 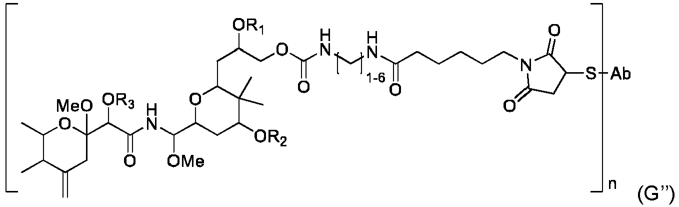 comprising the following steps:
(i) either:
(a) reacting a compound of formula (Ib): 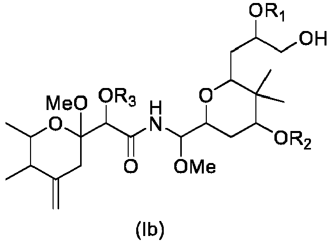 wherein
R₁ is selected from a protecting group for OH, substituted or unsubstituted C₁-C₁₂ alkyl, substituted or unsubstituted C₂-C₁₂ alkenyl, substituted or unsubstituted C₂-C₁₂ alkynyl, - C(=O)Rₐ, -C(=O)OR_{b} and -C(=O)NR_{c}R_{d};
R₂ is hydrogen;
R₃ is selected from a protecting group for OH, -C(=O)Rₐ, -C(=O)OR_{b}, and -C(=O)NR_{c}R_{d};
Rₐ is selected from hydrogen, substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group;
R_{b} is selected from substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted heterocyclic group;
R_{c} and R_{d} are independently selected from hydrogen, substituted or unsubstituted C₁-C₁₂ alkyl, substituted or unsubstituted C₂-C₁₂ alkenyl, substituted or unsubstituted C₂-C₁₂ alkynyl, and substituted or unsubstituted aryl and substituted or unsubstituted heterocyclic group;
with a compound of formula X₂-C(=O)-X₁ wherein X₁ and X₂ are leaving groups to give a compound of formula (B): 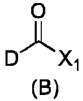
and the point of attachment of the -C(=O)X₁ moiety is the free primary -OH group of the compound of formula (Ib), or
(b) reacting said compound of formula (Ib) as defined above with 4-nitro-phenylchloroformate to give a compound of formula (J): 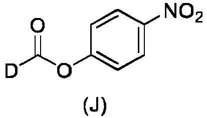 wherein the point of attachment of the (4-nitrophenyl)-O-CO- group is the same as that for the X₁(C=O) moiety in (a) above; or
(c) reacting a compound of formula (Ib) as defined above with an isocyanate of formula O=C=N-(CH₂)₁₋₆NHProt^{NH} wherein Prot^{NH} is a protecting group for amino suitable to be deprotected under basic conditions to give a compound of formula 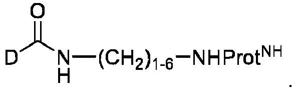
(ii) either
(a) reacting the compound of formula (B) or (J) produced in step (i)(a) or (i)(b) with an amino compound of formula NH₂-(CH₂)₁₋₆NH₂ to give a compound of formula (C): 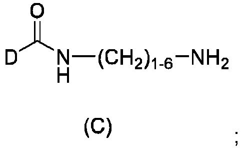 or
(b) deprotecting the compound of formula 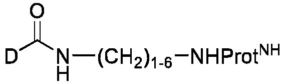 obtained in step (i)(c) to give a compound of formula (C).
(iii) reacting the compound of formula (C) with a compound of formula (D'), (E) or (E'): 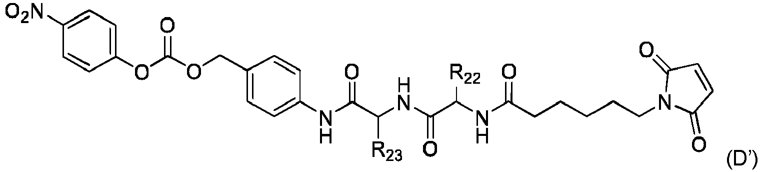 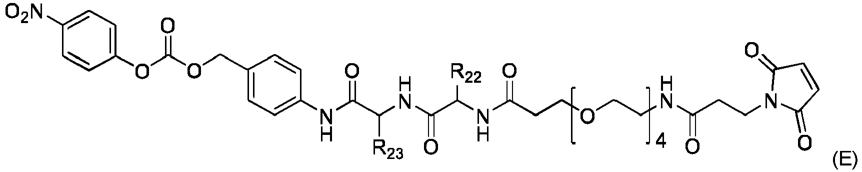 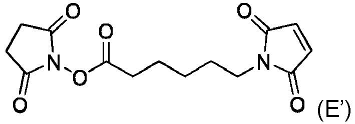
wherein R₂₂ and R₂₃ are as defined above in the definitions of AA groups;
to give a compound of formula (F), (F') or (F"), respectively: 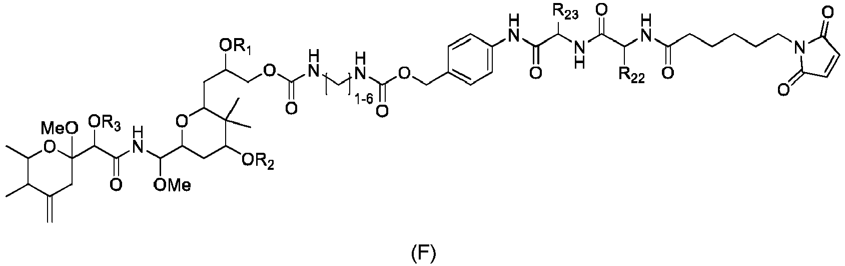 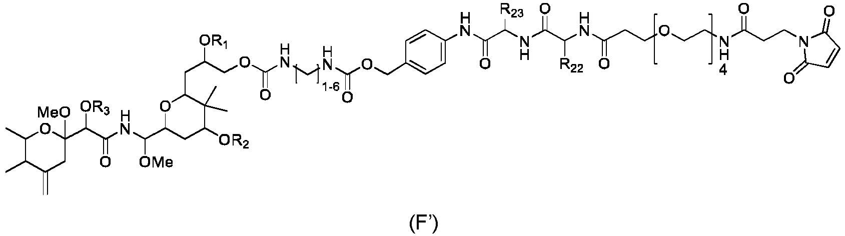 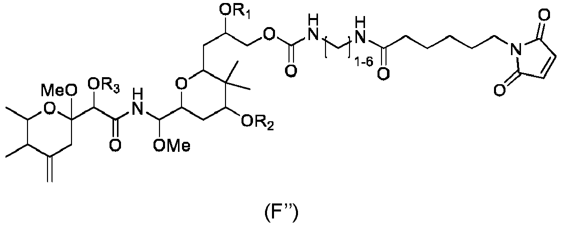
wherein R₁, R₂ and R₃ are as defined above for the compounds of formula (Ib) and R₂₂ and R₂₃ are as defined above in the definitions of AA groups.
(iv) if protecting groups for the OH are present in the compounds of formula (F), (F') or (F"), removing such protecting groups to give compounds of formula (F), (F') or (F") wherein R₁, R₂ and R₃ are as defined above for the compounds of formula (II).
(v) partial reduction of one or more disulfide bonds in the antibody to be conjugated to give a reduced antibody Ab-SH having free thiol groups: 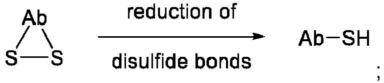 and
(vi) reacting the partially reduced antibody Ab-SH having free thiol groups with the compound of formula (F), (F') or (F") produced in step (iv) to give the desired drug antibody conjugate of formula (G), (G') or (G") respectively:

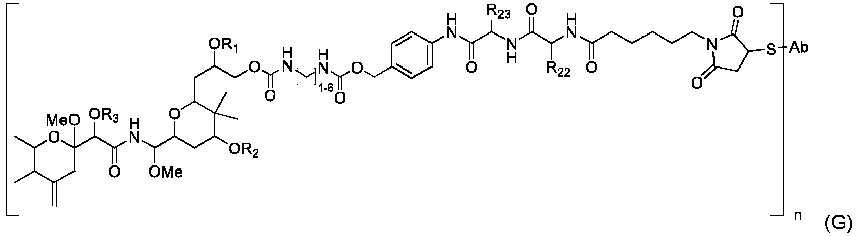

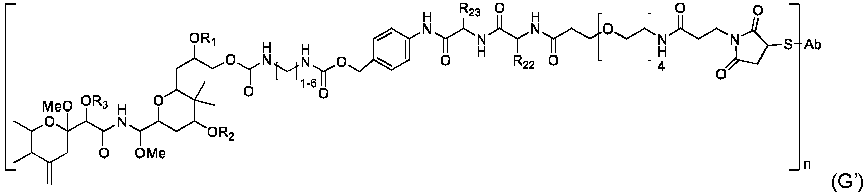

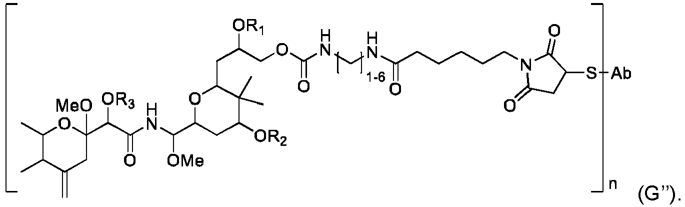

## Patentansprüche

1. Wirkstoffkonjugat, umfassend einen Wirkstoffteil, der kovalent an den Rest des Wirkstoffkonjugats angelagert ist, wobei das Wirkstoffkonjugat die Formel [D-(X)_{b}-(AA)_{w}-(T)_{g}-(L)-]ₙ-Ab aufweist, wobei:
D ein Wirkstoffteil mit der folgenden Formel (I) oder ein pharmazeutisch unbedenkliches Salz oder ein pharmazeutisch unbedenklicher Ester davon ist, 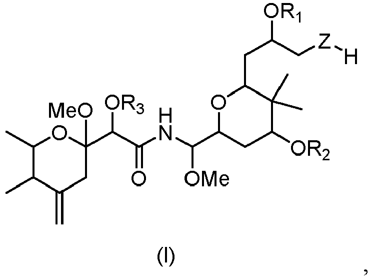 wobei:
D kovalent mittels einer Hydroxygruppe an OR₁, OR₃ oder ZH oder mittels einer Thiolgruppe an ZH, an (X)_{b}, falls vorhanden, oder (AA)_{w}, falls vorhanden, oder an (T)_{g}, falls vorhanden, oder (L) angelagert ist;
R₁ aus Wasserstoff, substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, -C(=O)Rₐ, -C(=O)OR_{b} und -C(=O)NR_{c}R_{d} ausgewählt ist;
R₂ Wasserstoff ist;
R₃ aus Wasserstoff, -C(=O)Rₐ, -C(=O)OR_{b} und -C(=O)NR_{c}R_{d} ausgewählt ist;
Z aus -O- und -S- ausgewählt ist;
Rₐ aus Wasserstoff, substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, substituiertem oder unsubstituiertem Aryl und einer substituierten oder unsubstituierten heterocyclischen Gruppe ausgewählt ist;
R_{b} aus substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, substituiertem oder unsubstituiertem Aryl und einer substituierten oder unsubstituierten heterocyclischen Gruppe ausgewählt ist;
R_{c} und R_{d} unabhängig aus Wasserstoff, substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, substituiertem oder unsubstituiertem Aryl und einer substituierten oder unsubstituierten heterocyclischen Gruppe ausgewählt sind;
X und T Verlängerungsgruppen sind, die gleich oder verschieden sein können;
jedes AA unabhängig eine Aminosäureeinheit ist;
L eine Linkergruppe ist;
w eine ganze Zahl ist, die von 0 bis 12 reicht;
b eine ganze Zahl von 0 oder 1 ist;
g eine ganze Zahl von 0 oder 1 ist;
Ab ein Teil ist, der mindestens eine Antigenbindungsstelle umfasst; und
n das Verhältnis der Gruppe [D-(X)_{b}-(AA)_{w}-(T)_{g}-(L)-] zu dem Teil, der mindestens eine Antigenbindungsstelle umfasst, ist und im Bereich von 1 bis 20 liegt.

2. Wirkstoffkonjugat nach Anspruch 1, wobei D außerdem eine Verbindung der Formel (Ia) oder ein pharmazeutisch unbedenkliches Salz oder ein pharmazeutisch unbedenklicher Ester davon ist: 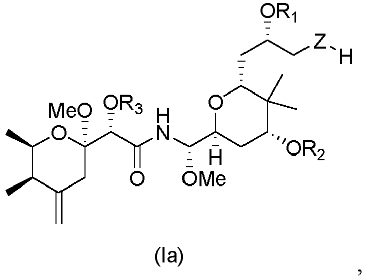 wobei R₁, R₂, R₃ und Z wie in Formel (I) in Anspruch 1 definiert sind.

3. Wirkstoffkonjugat nach Anspruch 1, wobei D eine Verbindung der Formel: 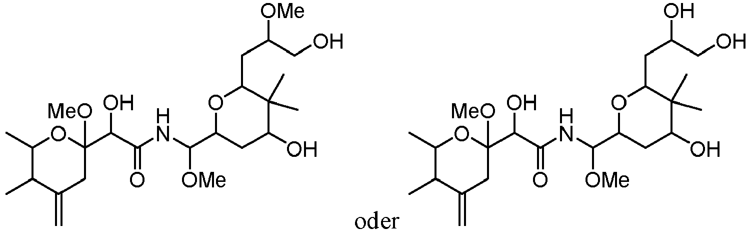 oder ein pharmazeutisch unbedenkliches Salz oder ein pharmazeutisch unbedenklicher Ester davon ist.

4. Wirkstoffkonjugat nach Anspruch 2, wobei D eine Verbindung der Formel: 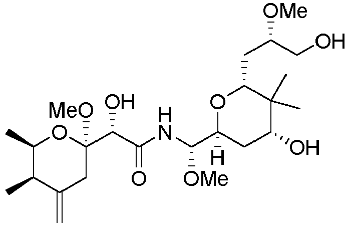 oder ein pharmazeutisch unbedenkliches Salz oder ein pharmazeutisch unbedenklicher Ester davon ist.

5. Wirkstoffkonjugat nach Anspruch 2, wobei D eine Verbindung der Formel: 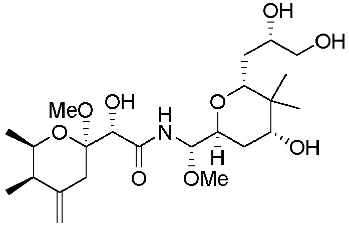 oder ein pharmazeutisch unbedenkliches Salz oder ein pharmazeutisch unbedenklicher Ester davon ist.

6. Wirkstoffkonjugat, umfassend einen Wirkstoffteil, der kovalent an den Rest des Wirkstoffkonjugats angelagert ist, wobei das Wirkstoffkonjugat die Formel [D-(X)_{b}-(AA)_{w}-(T)_{g}-(L)-]ₙ-Ab aufweist, wobei:
D ein Wirkstoffteil mit der folgenden Formel (II) oder ein pharmazeutisch unbedenkliches Salz oder ein pharmazeutisch unbedenklicher Ester davon ist: 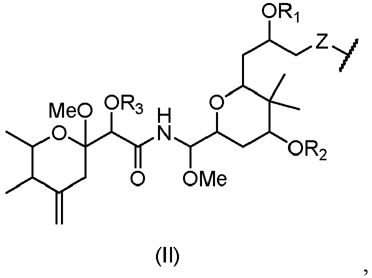 wobei:
die Wellenlinie den Punkt der kovalenten Anlagerung an (X)_{b}, falls vorhanden, oder (AA)_{w}, falls vorhanden, oder an (T)_{g}, falls vorhanden, oder (L) angibt;
R₁ aus Wasserstoff, substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, -C(=O)Rₐ, -C(=O)OR_{b} und -C(=O)NR_{c}R_{d} ausgewählt ist; wobei die optionalen Substituenten ein oder mehrere Substituenten Rₓ sind;
R₂ Wasserstoff ist;
R₃ aus Wasserstoff, -C(=O)Rₐ, -C(=O)OR_{b} und -C(=O)NR_{c}R_{d} ausgewählt ist;
Z -O- oder -S- ist;
Rₐ aus Wasserstoff, substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, substituiertem oder unsubstituiertem Aryl und einer substituierten oder unsubstituierten heterocyclischen Gruppe ausgewählt ist; wobei die optionalen Substituenten ein oder mehrere Substituenten Rₓ sind;
R_{b} aus substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, substituiertem oder unsubstituiertem Aryl und einer substituierten oder unsubstituierten heterocyclischen Gruppe ausgewählt ist; wobei die optionalen Substituenten ein oder mehrere Substituenten Rₓ sind;
R_{c} und R_{d} unabhängig aus Wasserstoff, substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, substituiertem oder unsubstituiertem Aryl und einer substituierten oder unsubstituierten heterocyclischen Gruppe ausgewählt sind; wobei die optionalen Substituenten ein oder mehrere Substituenten Rₓ sind;
Substituenten Rₓ aus der Gruppe bestehend aus C₁-C₁₂-Alkylgruppen, die optional mit mindestens einer Gruppe R_{y} substituiert sein können, C₂-C₁₂-Alkenylgruppen, die optional mit mindestens einer Gruppe R_{y} substituiert sein können, C₂-C₁₂-Alkinylgruppen, die optional mit mindestens einer Gruppe R_{y} substituiert sein können, Halogenatomen, Oxogruppen, Thiogruppen, Cyanogruppen, Nitrogruppen, OR_{y}, OCOR_{y}, OCOOR_{y}, COR_{y}, COOR_{y}, OCONR_{y}R_{z}, CONR_{y}R_{z}, SR_{y}, S(=O)R_{y}, SO₂R_{y}, SSR_{y}, P(O)(R_{y})OR_{z}, NR_{y}R_{z}, NR_{y}COR_{z}, NR_{y}C(=O)NR_{y}R_{z}, NR_{y}C(=NR_{y})NR_{y}R_{z}, Arylgruppen mit von 6 bis 18 Kohlenstoffatomen in einem oder mehreren Ringen, die optional mit einem oder mehreren Substituenten substituiert sein können, die gleich oder verschieden sein können, ausgewählt aus der Gruppe bestehend aus R_{y}, OR_{y}, OCOR_{y}, OCOOR_{y}, NR_{y}R_{z}, NR_{y}COR_{z} und NR_{y}C(=NR_{y})NR_{y}R_{z}, Aralkylgruppen, umfassend eine Alkylgruppe mit 1 bis 12 Kohlenstoffatomen, die mit einer wie oben definierten optional substituierten Arylgruppe substituiert ist, Aralkyloxygruppen, umfassend eine Alkoxygruppe mit 1 bis 12 Kohlenstoffatomen, die mit einer wie oben definierten optional substituierten Arylgruppe substituiert ist, und einer 5- bis 14-gliedrigen gesättigten oder ungesättigten heterocyclischen Gruppe mit einem oder mehreren Ringen und umfassend mindestens ein Sauerstoff-, Stickstoff- oder Schwefelatom in dem bzw. den Ringen ausgewählt sind, wobei die heterocyclische Gruppe optional mit einem oder mehreren Substituenten R_{y} substituiert ist und, wenn mehr als ein optionaler Substituent an einer beliebigen gegebenen Gruppe vorliegt, die optionalen Substituenten R_{y} gleich oder verschieden sein können;
jedes R_{y} und R_{z} unabhängig aus der Gruppe bestehend aus Wasserstoff, C₁-C₁₂-Alkylgruppen, C₁-C₁₂-Alkylgruppen, die mit mindestens einem Halogenatom substituiert sind, Aralkylgruppen, umfassend eine C₁-C₁₂-Alkylgruppe, die mit einer Arylgruppe mit 6 bis 18 Kohlenstoffatomen in einem oder mehreren Ringen substituiert ist, und Heterocycloalkylgruppen, umfassend eine C₁-C₁₂-Alkylgruppe, die mit einer 5- bis 14-gliedrigen gesättigten oder ungesättigten heterocyclischen Gruppe mit einem oder mehreren Ringen substituiert ist und umfassend mindestens ein Sauerstoff-, Stickstoff- oder Schwefelatom in dem bzw. den Ringen, ausgewählt ist;
X und T Verlängerungsgruppen sind, die gleich oder verschieden sein können;
jedes AA unabhängig eine Aminosäureeinheit ist;
L eine Linkergruppe ist;
w eine ganze Zahl ist, die von 0 bis 12 reicht;
b eine ganze Zahl von 0 oder 1 ist;
g eine ganze Zahl von 0 oder 1 ist;
wobei b + g + w optional nicht 0 ist;
Ab ein Teil ist, der mindestens eine Antigenbindungsstelle umfasst; und
n das Verhältnis der Gruppe [D-(X)_{b}-(AA)_{w}-(T)_{g}-(L)-] zu dem Teil, der mindestens eine Antigenbindungsstelle umfasst, ist und im Bereich von 1 bis 20 liegt.

7. Wirkstoffkonjugat nach Anspruch 6 oder ein pharmazeutisch unbedenkliches Salz davon oder ein pharmazeutisch unbedenklicher Ester davon, wobei D ein Wirkstoffteil der Formel (IIa) ist: 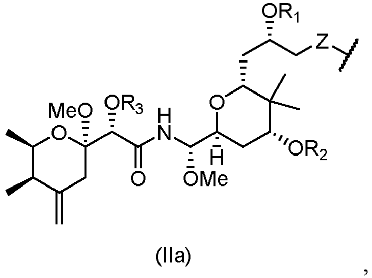 wobei die Wellenlinie, R₁, R₂, R₃ und Z wie für Formel (II) definiert sind.

8. Wirkstoffkonjugat nach einem der Ansprüche 1 bis 7, wobei das Salz aus Hydrochlorid, Hydrobromid, Hydroiodid, Sulfat, Nitrat, Phosphat, Acetat, Trifluoracetat, Maleat, Fumarat, Citrat, Oxalat, Succinat, Tartrat, Malat, Mandelat, Methansulfonat, p-Toluolsulfonat, Natrium, Kalium, Calcium, Ammonium, Ethylendiamin, Ethanolamin, N,N-Dialkylenethanolamin, Triethanolamin und basischen Aminosäuren ausgewählt ist.

9. Wirkstoffkonjugat nach einem der vorhergehenden Ansprüche, wobei L eine Linkergruppe ist, die ausgewählt ist aus der Gruppe bestehend aus: 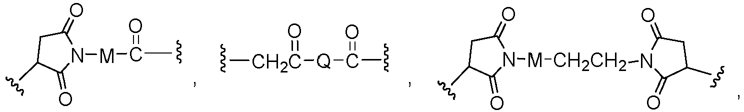 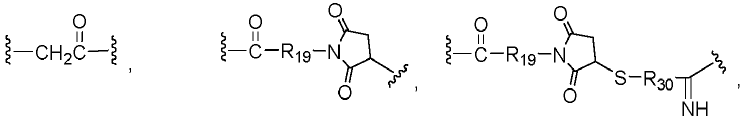 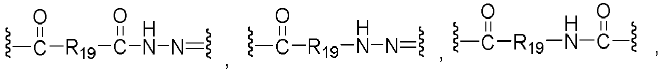 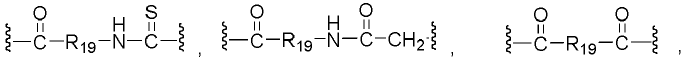 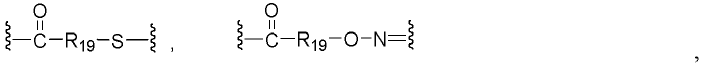 wobei
die Wellenlinien den Punkt von kovalenten Anlagerungen an ein Ab (die Wellenlinie rechts) und an (T)_{g}, falls vorhanden, oder (AA)_{w}, falls vorhanden, oder (X)_{b}, falls vorhanden, oder an D (die Wellenlinie links) angeben;
R₁₉ aus -C₁-C₁₂-Alkylen-, -C₃-C₈-Carbocyclo-, -O-(C₁-C₁₂-Alkylen)-, -C₆-C₁₈-Arylen- in einem oder mehreren Ringen, die optional mit einem oder mehreren Substituenten Rₓ substituiert sein können, -C₁-C₁₂-Alkylen-C₆-C₁₈-arylen-, wobei die Arylengruppe in einem oder mehreren Ringen ist, die optional mit einem oder mehreren Substituenten Rₓ substituiert sein können, -C₆-C₁₈-Arylen-C₁-C₁₂-alkylen-, wobei die Arylengruppe in einem oder mehreren Ringen ist, die optional mit einem oder mehreren Substituenten Rₓ substituiert sein können, -C₁-C₁₂-Alkylen-(C₃-C₈-carbocyclo)-, -(C₃-C₈-Carbocyclo)-C₁-C₁₂-alkylen-, -C₅-C₁₄-Heterocyclo-, wobei die Heterocyclogruppe eine gesättigte oder ungesättigte Gruppe mit einem oder mehreren Ringen sein kann und umfassend mindestens ein Sauerstoff-, Stickstoff- oder Schwefelatom in dem bzw. den Ringen, wobei die Gruppe optional mit einem oder mehreren Substituenten Rₓ substituiert ist, -C₁-C₁₂-Alkylen-(C₅-C₁₄-heterocyclo)-, wobei die Heterocyclogruppe eine gesättigte oder ungesättigte Gruppe mit einem oder mehreren Ringen sein kann und umfassend mindestens ein Sauerstoff-, Stickstoff- oder Schwefelatom in dem bzw. den Ringen, wobei die Gruppe optional mit einem oder mehreren Substituenten Rₓ substituiert ist, -(C₅-C₁₄-Heterocyclo)-C₁-C₁₂-alkylen-, wobei die Heterocyclogruppe eine gesättigte oder ungesättigte Gruppe mit einem oder mehreren Ringen sein kann und umfassend mindestens ein Sauerstoff-, Stickstoff- oder Schwefelatom in dem bzw. den Ringen, wobei die Gruppe optional mit einem oder mehreren Substituenten Rₓ substituiert ist, -(OCH₂CH₂)ᵣ- und -CH₂-(OCH₂CH₂)ᵣ- ausgewählt ist, wobei jeder der obigen Alkylensubstituenten, ob allein oder an einen anderen Teil in der Kohlenstoffkette angelagert, optional durch einen oder mehrere Substituenten Rₓ substituiert sein kann;
R₃₀ eine -C₁-C₆-Alkylen-Gruppe ist;
M aus der Gruppe bestehend aus -C₁-C₆-Alkylen-, -C₁-C₆-Alkylen-(C₃-C₈-carbocyclo)-, -(CH₂CH₂O)ₛ-, -C₁-C₆-Alkylen-(C₃-C₈-carbocyclo)-CON(H oder C₁-C₆-alkyl)-C₁-C₆-alkylen-, -Phenylen-, das optional mit einem oder mehreren Substituenten Rₓ sein kann, -Phenylen-C₁-C₆-alkylen-, wobei der Phenylenteil optional mit einem oder mehreren Substituenten Rₓ sein kann, und -C₁-C₆-Alkylen-CON(H oder C₁-C₆-alkyl)-C₁-C₆-alkylen- ausgewählt ist;
Q aus der Gruppe bestehend aus -N(H oder C₁-C₆-Alkyl)phenylen- und -N(H oder C₁-C₆-Alkyl)-(CH₂)ₛ ausgewählt ist;
r eine ganze Zahl ist, die von 1 bis 10 reicht; und
s eine ganze Zahl ist, die von 1 bis 10 reicht;
oder
wobei L eine Linkergruppe ist, die ausgewählt ist aus der Gruppe bestehend aus: 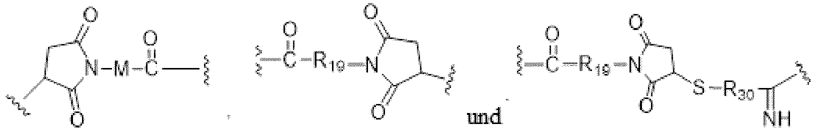 wobei:
die Wellenlinien den Punkt von kovalenten Anlagerungen an ein Ab (die Wellenlinie rechts) und an (T)_{g}, falls vorhanden, oder (AA)_{w}, falls vorhanden, oder (X)_{b}, falls vorhanden, oder an D (die Wellenlinie links) angeben;
R₁₉ aus -C₁-C₁₂-Alkylen-, -O-(C₁-C₁₂-Alkylen), -C₆-C₁₂-Arylen- in einem oder mehreren Ringen, die optional mit einem oder mehreren Substituenten Rₓ substituiert sein können, -C₁-C₁₂-Alkylen-C₆-C₁₂-arylen-, wobei die Arylengruppe in einem oder mehreren Ringen ist, die optional mit einem oder mehreren Substituenten Rₓ substituiert sein können, -C₆-C₁₂-Arylen-C₁-C₁₂-alkylen-, wobei die Arylengruppe in einem oder mehreren Ringen ist, die optional mit einem oder mehreren Substituenten Rₓ substituiert sein können, -C₅-C₁₂-Heterocyclo-, wobei die Heterocyclogruppe eine gesättigte oder ungesättigte Gruppe mit einem oder mehreren Ringen sein kann und umfassend mindestens ein Sauerstoff-, Stickstoff- oder Schwefelatom in dem bzw. den Ringen, wobei die Gruppe optional mit einem oder mehreren Substituenten Rₓ substituiert ist, -C₁-C₁₂-Alkylen-(C₅-C₁₂-heterocyclo)-, wobei die Heterocyclogruppe eine gesättigte oder ungesättigte Gruppe mit einem oder mehreren Ringen sein kann und umfassend mindestens ein Sauerstoff-, Stickstoff- oder Schwefelatom in dem bzw. den Ringen, wobei die Gruppe optional mit einem oder mehreren Substituenten Rₓ substituiert ist, -(C₅-C₁₂-Heterocyclo)-C₁-C₁₂-alkylen-, wobei die Heterocyclogruppe eine gesättigte oder ungesättigte Gruppe mit einem oder mehreren Ringen sein kann und umfassend mindestens ein Sauerstoff-, Stickstoff- oder Schwefelatom in dem bzw. den Ringen, wobei die Gruppe optional mit einem oder mehreren Substituenten Rₓ substituiert ist, -(OCH₂CH₂)ᵣ- und -CH₂-(OCH₂CH₂)ᵣ-ausgewählt ist, wobei jeder der obigen Alkylensubstituenten, ob allein oder an einen anderen Teil in der Kohlenstoffkette angelagert, optional durch einen oder mehrere Substituenten Rₓ substituiert sein kann;
R₃₀ eine -C₁-C₆-Alkylen-Gruppe ist;
M aus der Gruppe bestehend aus -C₁-C₆-Alkylen-, -C₁-C₆-Alkylen-(C₃-C₈-carbocyclo)- und -Phenylen-, das optional mit einem oder mehreren Substituenten Rₓ substituiert sein kann, ausgewählt ist und
r eine ganze Zahl ist, die von 1 bis 6 reicht.

10. Wirkstoffkonjugat nach einem der Ansprüche 1 bis 9, ausgewählt aus den Formeln (V), (VI) und (VII): 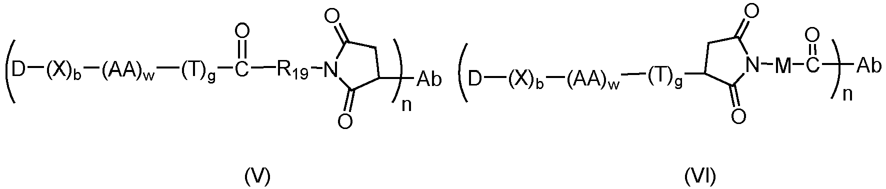 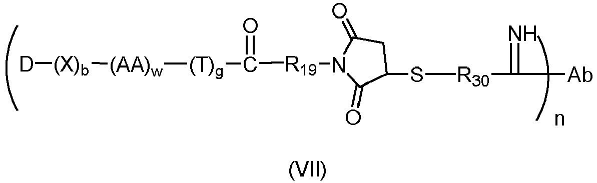 wobei:
X und T Verlängerungsgruppen sind, die gleich oder verschieden sein können;
jedes AA unabhängig eine Aminosäureeinheit ist;
w eine ganze Zahl ist, die von 0 bis 12 reicht;
b eine ganze Zahl von 0 oder 1 ist;
g eine ganze Zahl von 0 oder 1 ist;
D ein Wirkstoffteil ist;
Ab ein Teil ist, der mindestens eine Antigenbindungsstelle umfasst;
n das Verhältnis der Gruppe [D-(X)_{b}-(AA)_{w}-(T)_{g}-(L)-], wobei L wie in Formel (V), (VI) oder (VII) definiert ist, zu dem Teil, der mindestens eine Antigenbindungsstelle umfasst, ist und im Bereich von 1 bis 20 liegt;
R₁₉ aus -C₁-C₈-Alkylen-, -O-(C₁-C₈-Alkylen)-, -C₁-C₈-Alkylen-C₆-C₁₂-arylen-, wobei die Arylengruppe in einem oder mehreren Ringen ist, die optional mit einem oder mehreren Substituenten Rₓ substituiert sein können, und -C₆-C₁₂-Arylen-C₁-C₈-alkylen-, wobei die Arylengruppe in einem oder mehreren Ringen ist, die optional mit einem oder mehreren Substituenten Rₓ substituiert sein können, wobei jeder der obigen Alkylensubstituenten, ob allein oder an einen anderen Teil in der Kohlenstoffkette angelagert, optional durch einen oder mehrere Substituenten Rₓ substituiert sein kann;
R₃₀ eine -C₂-C₄-Alkylen-Gruppe ist und
M aus der Gruppe bestehend aus -C₁-C₃-Alkylen- und -C₁-C₃-Alkylen-(C₅-C₇-carbocyclo)- ausgewählt ist; oder
ausgewählt aus den Formeln (V), (VI) und (VII): 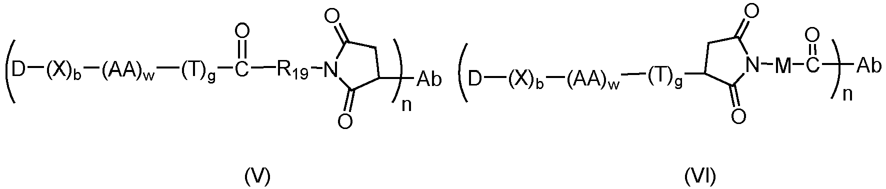 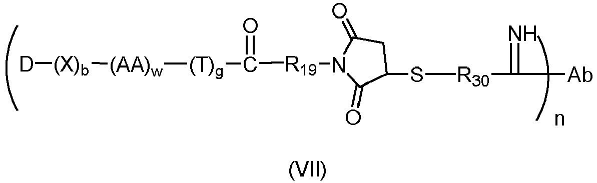 wobei:
X und T Verlängerungsgruppen sind, die gleich oder verschieden sein können;
jedes AA unabhängig eine Aminosäureeinheit ist;
w eine ganze Zahl ist, die von 0 bis 12 reicht;
b eine ganze Zahl von 0 oder 1 ist;
g eine ganze Zahl von 0 oder 1 ist;
D ein Wirkstoffteil ist;
Ab ein Teil ist, der mindestens eine Antigenbindungsstelle umfasst;
n das Verhältnis der Gruppe [D-(X)_{b}-(AA)_{w}-(T)_{g}-(L)-], wobei L wie in (V), (VI) oder (VII) definiert ist, zu dem Teil, der mindestens eine Antigenbindungsstelle umfasst, ist und im Bereich von 1 bis 20 liegt;
R₁₉ aus -C₁-C₆-Alkylen-, -Phenylen-C₁-C₆-alkylen- ausgewählt ist, wobei die Phenylengruppe optional mit einem oder mehreren Substituenten Rₓ substituiert sein kann, ausgewählt aus der Gruppe bestehend aus Alkylgruppen mit von 1 bis 6 Kohlenstoffatomen, Alkoxygruppen mit von 1 bis 6 Kohlenstoffatomen, Halogenatomen, Nitrogruppen und Cyanogruppen, wobei jeder der obigen Alkylensubstituenten, ob allein oder an einen anderen Teil in der Kohlenstoffkette angelagert, optional durch einen oder mehrere Substituenten Rₓ substituiert sein kann, ausgewählt aus der Gruppe bestehend aus Alkylgruppen mit von 1 bis 6 Kohlenstoffatomen, Alkoxygruppen mit von 1 bis 6 Kohlenstoffatomen, Arylgruppen mit von 6 bis 12 Kohlenstoffatomen, Halogenatomen, Nitrogruppen und Cyanogruppen, und vorzugsweise R₁₉ eine -C₁-C₆-Alkylen-Gruppe ist;
R₃₀ eine -C₂-C₄-Alkylen-Gruppe ist und
M -C₁-C₃-Alkylen-(C₅-C₇-carbocyclo)- ist.

11. Wirkstoffkonjugat nach einem der Ansprüche 1 bis 10, wobei (AA)_{w} die Formel (III) aufweist: 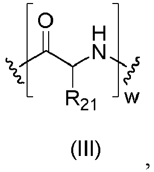
wobei die Wellenlinien den Punkt von kovalenten Anlagerungen an (X)_{b}, falls vorhanden, oder an den Wirkstoffteil (die Wellenlinie links) und an (T)_{g}, falls vorhanden, oder an den Linker (die Wellenlinie rechts) angeben und
R₂₁ bei jedem Auftreten aus der Gruppe bestehend aus Wasserstoff, Methyl, Isopropyl, Isobutyl, *sek*.-Butyl, Benzyl, *p*-Hydroxybenzyl, -CH₂OH, -CH(OH)CH₃, -CH₂CH₂SCH₃, -CH₂CONH₂, -CH₂COOH, -CH₂CH₂CONH₂, -CH₂CH₂COOH, -(CH₂)₃NHC(=NH)NH₂, -(CH₂)₃NH₂, -(CH₂)₃NHCOCH₃, -(CH₂)₃NHCHO, -(CH₂)₄NHC(=NH)NH₂, -(CH₂)₄NH₂, -(CH₂)₄NHCOCH₃, -(CH₂)₄NHCHO, -(CH₂)₃NHCONH₂, -(CH₂)₄NHCONH₂, -CH₂CH₂CH(OH)CH₂NH₂, 2-Pyridylmethyl-, 3-Pyridylmethyl-, 4-Pyridylmethyl-, Phenyl, Cyclohexyl, 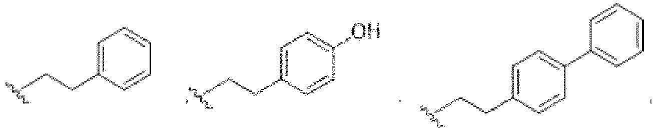 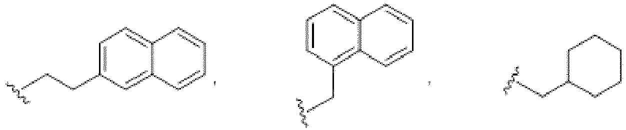 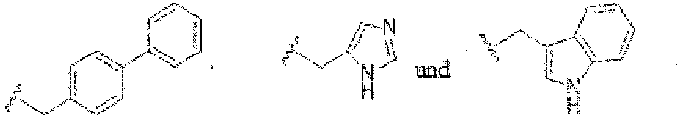 ausgewählt ist;
und w eine ganze Zahl ist, die von 1 bis 12 reicht;
oder
wobei (AA)_{w} die Formel (III) aufweist, wobei:
R₂₁ bei jedem Auftreten aus der Gruppe bestehend aus Wasserstoff, Methyl, Isopropyl, *sek*.-Butyl, Benzyl, Indolylmethyl, -(CH₂)₃NHCONH₂, -(CH₂)₄NH₂, -(CH₂)₃NHC(=NH)NH₂ und -(CH₂)₄NHC(=NH)NH₂ ausgewählt ist und
w eine ganze Zahl ist, die von 0 bis 6 reicht;
oder
wobei w 0 oder 2 ist und, wenn w 2 ist, (AA)_{w} die Formel (IV) aufweist: 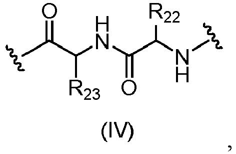 wobei:
die Wellenlinien den Punkt von kovalenten Anlagerungen an (X)_{b}, falls vorhanden, oder an den Wirkstoffteil (die Wellenlinie links) und an (T)_{g}, falls vorhanden, oder an den Linker (die Wellenlinie rechts) angeben;
R₂₂ aus Methyl, Benzyl, Isopropyl, *sek.* -Butyl und Indolylmethyl ausgewählt ist und
R₂₃ aus Methyl, -(CH₂)₄NH₂, -(CH₂)₃NHCONH₂ und -(CH₂)₃NHC(=NH)NH₂ ausgewählt ist.

12. Wirkstoffkonjugat nach einem der Ansprüche 1 bis 11, wobei X eine Verlängerungsgruppe ist, die ausgewählt ist aus:
-CONH-(C₁-C₆-Alkylen)-NH-;
-COO-CH₂-(Phenylen, das optional mit einem oder mehreren Substituenten Rₓ substituiert sein kann)-NH-;
-CONH-C₁-C₆-Alkylen)-NH-COO-CH₂-(phenylen, das optional mit einem oder mehreren Substituenten Rₓ substituiert sein kann)-NH-;
-COCH₂NH-COCH₂-NH-;
-COCH₂NH-;
-CONH-(C₁-C₆-Alkylen)-S-;
-CONH-(C₁-C₆-Alkylen)-NHCO(C₁-C₆-alkylen)-S-; und
b 0 oder 1, vorzugsweise 1 ist;
oder
wobei X eine Verlängerungsgruppe ist, die ausgewählt ist aus der Gruppe bestehend aus:
-CONH-(C₂-C₄-Alkylen)-NH-;
-COO-CH₂-Phenylen-NH-, wobei die Phenylengruppe optional mit von einem bis vier Substituenten Rₓ substituiert sein kann, ausgewählt aus der Gruppe bestehend aus Alkylgruppen mit von 1 bis 6 Kohlenstoffatomen, Alkoxygruppen mit von 1 bis 6 Kohlenstoffatomen, Halogenatomen, Nitrogruppen und Cyanogruppen;
-CONH-(C₂-C₄-Alkylen)-NH-COO-CH₂-phenylen-, das optional mit von einem bis vier Substituenten Rₓ substituiert sein kann, ausgewählt aus der Gruppe bestehend aus Alkylgruppen mit von 1 bis 6 Kohlenstoffatomen, Alkoxygruppen mit von 1 bis 6 Kohlenstoffatomen, Halogenatomen, Nitrogruppen und Cyanogruppen)-NH;
-COCH₂NH-COCH₂-NH-;
-CONH-(C₂-C₄-Alkylen)-S-;
-CONH-(C₂-C₄-Alkylen)-NHCO(C₁-C₃-alkylen)-S-; und
b 0 oder 1, vorzugsweise 1 ist;
oder
wobei X eine Verlängerungsgruppe ist, die ausgewählt ist aus der Gruppe bestehend aus:
-COO-CH₂-Phenylen-NH-;
-CONH(CH₂)₃NHCOOCH₂-Phenylen-NH-;
-CONH(CH₂)₃NH-;
-CONH(CH₂)₃-S-;
-CONH(CH₂)₃NHCO(CH₂)₂S-; und
b 0 oder 1, vorzugsweise 1 ist.

13. Wirkstoffkonjugat nach einem der Ansprüche 1 bis 12, wobei T eine Verlängerungsgruppe ist, die aus der Gruppe bestehend aus -CO-(C₁-C₆-Alkylen)-NH-,
-CO-(C₁-C₆-Alkylen)-[O-(C₂-C₆-alkylen)]ⱼ-NH-, -COO-(C₁-C₆-Alkylen)-[O-(C₂-C₆-alkylen)]ⱼ-NH- ausgewählt ist; wobei j eine ganze Zahl von 1 bis 25 ist und g 0 oder 1 ist;
oder
wobei T eine Verlängerungsgruppe ist, die aus der Gruppe bestehend aus -CO-(C₁-C₄-Alkylen)-NH-, -CO-(C₁-C₄-Alkylen)-[O-(C₂-C₄-alkylen)]ⱼ-NH-, -COO-(C₁-C₄-Alkylen)-[O-(C₂-C₄-alkylen)]ⱼ-NH- ausgewählt ist, wobei j eine ganze Zahl von 1 bis 10 ist und g 0 oder 1 ist;
oder
wobei T eine Verlängerungsgruppe ist, die aus der Gruppe bestehend aus -CO-(C₁-C₄-Alkylen)-NH-, -CO-(C₁-C₄-Alkylen)-[O-(C₂-C₄-alkylen)]ⱼ-NH-, -COO-(C₁-C₄-Alkylen)-[O-(C₂-C₄-alkylen)]ⱼ-NH- ausgewählt ist, wobei j eine ganze Zahl von 1 bis 5 ist und g 0 oder 1 ist.

14. Wirkstoffkonjugat nach einem der Ansprüche 6 bis 13, wobei D ein Wirkstoffteil der Formel (II) oder der Formel (IIa) oder ein pharmazeutisch unbedenkliches Salz oder ein pharmazeutisch unbedenklicher Ester davon ist, wobei:
R₁ Wasserstoff oder substituiertes oder unsubstituiertes C₁-C₆-Alkyl ist, wobei es sich bei den optionalen Substituenten um einen oder mehrere Substituenten Rₓ handelt;
R₂ Wasserstoff ist;
R₃ Wasserstoff oder -C(=O)Rₐ ist, wobei Rₐ substituiertes oder unsubstituiertes C₁-C₆-Alkyl ist, wobei es sich bei den optionalen Substituenten um einen oder mehrere Substituenten Rₓ handelt; und
Z -O- ist;
oder
wobei D ein Wirkstoffteil der Formel (II) oder der Formel (IIa) oder ein pharmazeutisch unbedenkliches Salz oder ein pharmazeutisch unbedenklicher Ester davon ist, wobei:
R₁ Wasserstoff oder Methyl ist;
R₂ Wasserstoff ist;
R₃ Wasserstoff ist und
Z -O- ist;
oder
wobei D ein Wirkstoffteil der Formel (II) oder der Formel (IIa) oder ein pharmazeutisch unbedenkliches Salz oder ein pharmazeutisch unbedenklicher Ester davon ist, wobei:
R₁ Methyl ist;
R₂ Wasserstoff ist;
R₃ Wasserstoff ist und
Z -O- ist.

15. Wirkstoffkonjugat nach einem der Ansprüche 1, 3, 6 bis 14, wobei D ausgewählt ist aus: 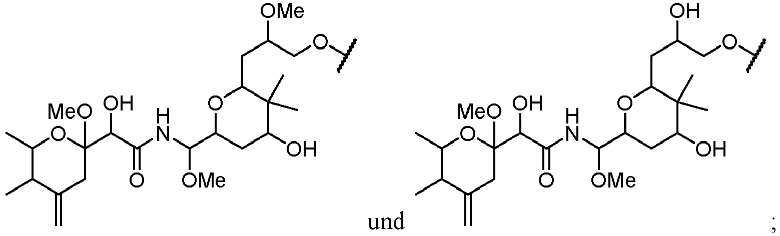
oder ein pharmazeutisch unbedenkliches Salz oder ein pharmazeutisch unbedenklicher Ester davon, wobei die Wellenlinie den Punkt der kovalenten Anlagerung an (X)_{b}, falls vorhanden, oder (AA)_{w}, falls vorhanden, oder an (T)_{g}, falls vorhanden, oder an (L) angibt;
oder
wobei D ausgewählt ist aus: 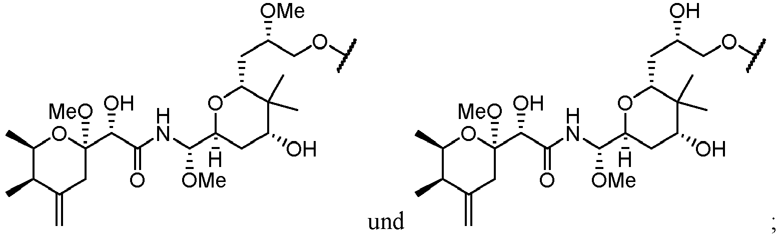
oder ein pharmazeutisch unbedenkliches Salz oder ein pharmazeutisch unbedenklicher Ester davon, wobei die Wellenlinie den Punkt der kovalenten Anlagerung an (X)_{b}, falls vorhanden, oder (AA)_{w}, falls vorhanden, oder an (T)_{g}, falls vorhanden, oder an (L) angibt.

16. Wirkstoffkonjugat nach einem der Ansprüche 1 bis 15, wobei der Teil Ab, der mindestens eine Antigenbindungsstelle umfasst, ein antigenbindendes Peptid ist;
optional
wobei der Teil Ab, der mindestens eine Antigenbindungsstelle umfasst, ein Antikörper, ein Einzeldomänenantikörper oder ein antigenbindendes Fragment davon ist;
optional
wobei der Teil Ab, der mindestens eine Antigenbindungsstelle umfasst, ein monoklonaler Antikörper, polyklonaler Antikörper oder bispezifischer Antikörper ist und wobei der Antikörper oder ein antigenbindendes Fragment davon von einer beliebigen Spezies, vorzugsweise einem Menschen, einer Maus oder einem Kaninchen abgeleitet ist;
optional
wobei der Antikörper oder ein antigenbindendes Fragment davon aus der Gruppe bestehend aus einem humanen Antikörper, einem antigenbindenden Fragment eines humanen Antikörpers, einem humanisierten Antikörper, einem antigenbindenden Fragment eines humanisierten Antikörpers, einem chimären Antikörper, einem antigenbindenden Fragment eines chimären Antikörpers, einem glykosylierten Antikörper und einem glykosylierten antigenbindenden Fragment ausgewählt ist;
optional
wobei der Antikörper oder ein antigenbindendes Fragment davon ein antigenbindendes Fragment ist, das aus der Gruppe bestehend aus einem Fab-Fragment, einem Fab'-Fragment, einem F(ab')2-Fragment und einem Fv-Fragment ausgewählt ist;
optional
wobei der Antikörper oder ein antigenbindendes Fragment davon ein monoklonaler Antikörper ist, der immunspezifisch an Krebszellantigene, virale Antigene, Antigene von Zellen, die autoimmune Antikörper produzieren, die mit einer Autoimmunkrankheit assoziiert sind, mikrobielle Antigene bindet, und vorzugsweise ein monoklonaler Antikörper ist, der immunspezifisch an Krebszellantigene bindet;
optional
wobei der Teil Ab, der mindestens eine Antigenbindungsstelle umfasst, ein Antikörper ist, der aus der Gruppe bestehend aus Abciximab, Alemtuzumab, Anetumab, Atezolizumab, Avelumab, Basiliximab, Bevacizumab, Blinatomumab, Brentuximab, Catumaxomab, Cetuximab, Coltuximab, Daclizumab, Daratumumab, Denintuzumab, Denosumab, Depatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labetuzumab, Ladiratuzumab, Laprituximab, Lifastuzumab, Lorvotuzumab, Milatuzumab, Mirvetuximab, Naratuximab, Necitumumab, Nimotuzumab, Nivolumab, Obinutuzumab, Ofatumumab, Olaratumab, Omalizumab, Palivizumab, Panitumumab, Pembrolizumab, Pertuzumab, Pinatuzumab, Polatuzumab, Ramucirumab, Rovalpituzumab, Sacituzumab, Siltuximab, Sirtratumab, Sofituzumab, Vadastuximab, Vorsetuzumab, einem Anti-HER2-Antikörper wie Trastuzumab, einem Anti-CD4-Antikörper, einem Anti-CD5-Antikörper und einem Anti-CD30-Antikörper oder einem antigenbindenden Fragment oder einem immunologisch aktiven Abschnitt davon ausgewählt ist;
optional
wobei der Teil Ab, der mindestens eine Antigenbindungsstelle umfasst, ein Antikörper ist, der aus der Gruppe bestehend aus Abciximab, Alemtuzumab, Anetumab, Atezolizumab, Avelumab, Basiliximab, Bevacizumab, Blinatomumab, Brentuximab, Catumaxomab, Cetuximab, Daclizumab, Daratumumab, Denintuzumab, Denosumab, Depatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labetuzumab, Ladiratuzumab, Laprituximab, Mirvetuximab, Naratuximab, Necitumumab, Nimotuzumab, Nivolumab, Obinutuzumab, Ofatumumab, Olaratumab, Omalizumab, Palivizumab, Panitumumab, Pembrolizumab, Pertuzumab, Polatuzumab, Ramucirumab, Rovalpituzumab, Sacituzumab, Siltuximab, Sirtratumab, Vadastuximab, Vorsetuzumab, einem Anti-HER2-Antikörper wie Trastuzumab, einem Anti-CD4-Antikörper, einem Anti-CD5-Antikörper und einem Anti-CD30-Antikörper oder einem antigenbindenden Fragment oder einem immunologisch aktiven Abschnitt davon ausgewählt ist;
optional
wobei der Teil Ab, der mindestens eine Antigenbindungsstelle umfasst, ein Antikörper ist, der aus der Gruppe bestehend aus Abciximab, Alemtuzumab, Atezolizumab, Avelumab, Basiliximab, Bevacizumab, Blinatomumab, Brentuximab, Catumaxomab, Cetuximab, Daclizumab, Daratumumab, Denosumab, Dinutuximab, Durvalumab, Elotuzumab, Gemtuzumab, Ibritumomab, Inotuzumab, Ipilimumab, Labetuzumab, Necitumumab, Nimotuzumab, Nivolumab, Obinutuzumab, Ofatumumab, Olaratumab, Omalizumab, Palivizumab, Panitumumab, Pembrolizumab, Pertuzumab, Ramucirumab, Rovalpituzumab, Siltuximab, einem Anti-HER2-Antikörper wie Trastuzumab, einem Anti-CD4-Antikörper, einem Anti-CD5-Antikörper und einem Anti-CD30-Antikörper oder einem antigenbindenden Fragment oder einem immunologisch aktiven Abschnitt davon, mehr bevorzugt Brentuximab, Gemtuzumab, Inozutumab, Rovalpituzumab, einem Anti-HER2-Antikörper wie Trastuzumab, einem Anti-CD4-Antikörper, einem Anti-CD5-Antikörper und einem Anti-CD30-Antikörper oder einem antigenbindenden Fragment davon oder einem immunologisch aktiven Abschnitt davon, vorzugsweise einem Anti-HER2-Antikörper wie Trastuzumab oder einem antigenbindenden Fragment oder einem immunologisch aktiven Abschnitt davon, mehr bevorzugt Trastuzumab oder einem antigenbindenden Fragment oder einem immunologisch aktiven Abschnitt davon ausgewählt ist.

17. Antikörper-Wirkstoffkonjugat nach Anspruch 1, ausgewählt aus der Gruppe bestehend aus: 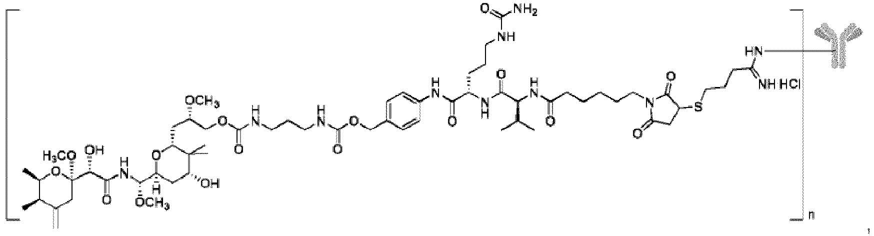 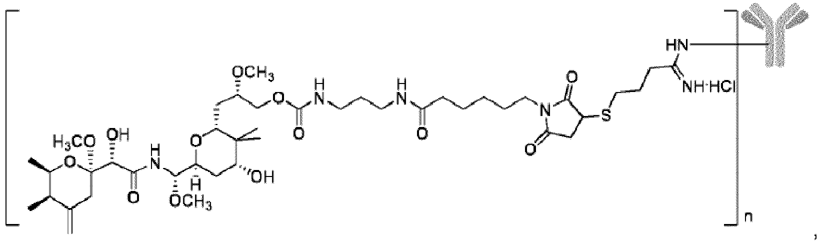 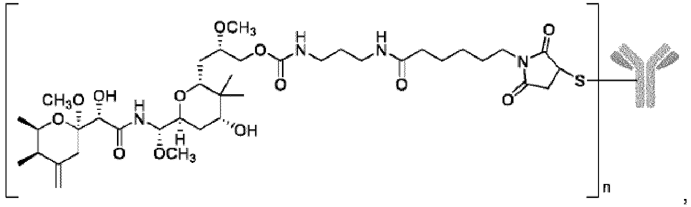 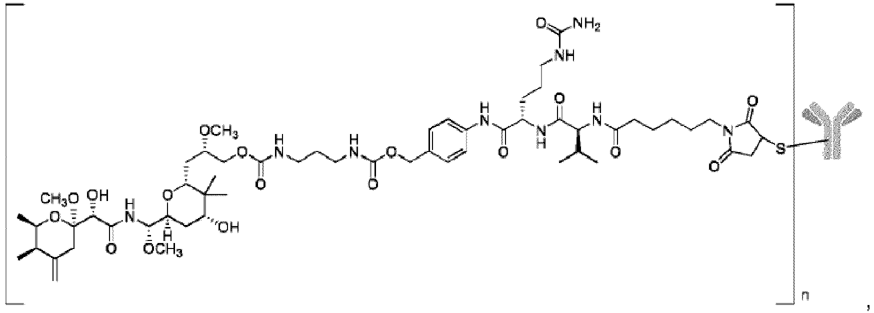 und 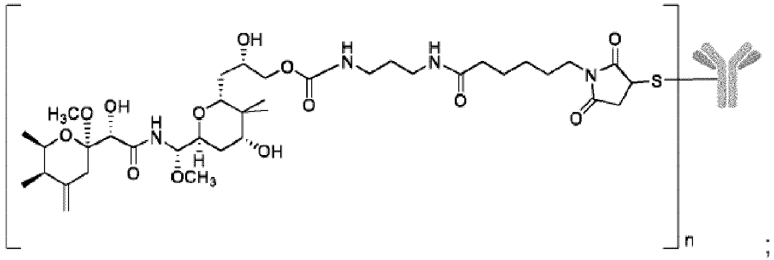 wobei n von 2 bis 6, mehr bevorzugt 3, 4 oder 5 ist und jedes 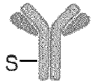 und 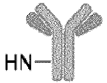 unabhängig aus Brentuximab, Gemtuzumab, Inozutumab, Rovalpituzumab, einem Anti-HER2-Antikörper wie Trastuzumab, einem Anti-CD4-Antikörper, einem Anti-CD5-Antikörper und einem Anti-CD30-Antikörper oder einem antigenbindenden Fragment davon oder einem immunologisch aktiven Abschnitt davon ausgewählt ist und mehr bevorzugt ein Anti-HER2-Antikörper wie Trastuzumab oder ein antigenbindendes Fragment oder ein immunologisch aktiver Abschnitt davon, insbesondere Trastuzumab oder ein antigenbindendes Fragment oder ein immunologisch aktiver Abschnitt davon ist.

18. Wirkstoffkonjugat nach Anspruch 17, wobei Teil Ab, der mindestens eine Antigenbindungsstelle umfasst, ein Anti-HER2-Antikörper wie Trastuzumab oder ein antigenbindendes Fragment oder ein immunologisch aktiver Abschnitt davon ist.

19. Wirkstoffkonjugat nach Anspruch 17, wobei Teil Ab, der mindestens eine Antigenbindungsstelle umfasst, aus Trastuzumab oder einem antigenbindenden Fragment oder einem immunologisch aktiven Abschnitt davon ausgewählt ist.

20. Antikörper-Wirkstoffkonjugat nach einem der Ansprüche 1 bis 19 in isolierter oder aufgereinigter Form.

21. Verbindung der Formel D-(X)_{b}-(AA)_{w}-(T)_{g}-L₁ oder der Formel D-(X)_{b}-(AA)_{w}-(T)_{g}-H, wobei:
L₁ ein Linker ist, der ausgewählt ist aus der Gruppe von Formeln bestehend aus: 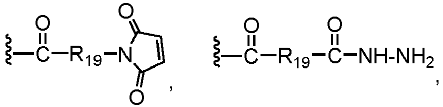 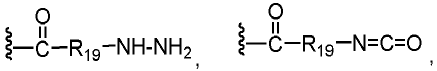 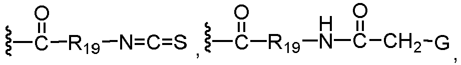 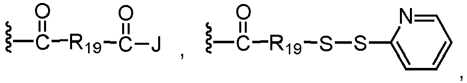 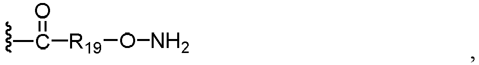
wobei jede der Wellenlinien den Punkt der kovalenten Anlagerung an (T)_{g}, falls vorhanden, oder (AA)_{w}, falls vorhanden, oder an (X)_{b}, falls vorhanden, oder an (D) angibt;
G aus Halo, -O-Mesyl und -O-Tosyl ausgewählt ist;
J aus Halo, Hydroxy, -N-Succinimidoxy, -O-(4-Nitrophenyl), -O-Pentafluorphenyl, -O-Tetrafluorphenyl und -O-C(O)-OR₂₀ ausgewählt ist;
R₁₉ aus -C₁-C₁₂-Alkylen-, -C₃-C₈-Carbocyclo-, -O-(C₁-C₁₂-Alkylen)-, -C₆-C₁₈-Arylen- in einem oder mehreren Ringen, die optional mit einem oder mehreren Substituenten Rₓ substituiert sein können, -C₁-C₁₂-Alkylen-C₆-C₁₈-arylen-, wobei die Arylengruppe in einem oder mehreren Ringen ist, die optional mit einem oder mehreren Substituenten Rₓ substituiert sein können, -C₆-C₁₈-Arylen-C₁-C₁₂-alkylen-, wobei die Arylengruppe in einem oder mehreren Ringen ist, die optional mit einem oder mehreren Substituenten Rₓ substituiert sein können, -C₁-C₁₂-Alkylen-(C₃-C₈-carbocyclo)-, -(C₃-C₈-Carbocyclo)-C₁-C₁₂-alkylen-, -C₅-C₁₄-Heterocyclo-, wobei die Heterocyclogruppe eine gesättigte oder ungesättigte Gruppe mit einem oder mehreren Ringen sein kann und umfassend mindestens ein Sauerstoff-, Stickstoff- oder Schwefelatom in dem bzw. den Ringen, wobei die Gruppe optional mit einem oder mehreren Substituenten Rₓ substituiert ist, -C₁-C₁₂-Alkylen-(C₅-C₁₄-heterocyclo)-, wobei die Heterocyclogruppe eine gesättigte oder ungesättigte Gruppe mit einem oder mehreren Ringen sein kann und umfassend mindestens ein Sauerstoff-, Stickstoff- oder Schwefelatom in dem bzw. den Ringen, wobei die Gruppe optional mit einem oder mehreren Substituenten Rₓ substituiert ist, -(C₅-C₁₄-Heterocyclo)-C₁-C₁₂-alkylen-, wobei die Heterocyclogruppe eine gesättigte oder ungesättigte Gruppe mit einem oder mehreren Ringen sein kann und umfassend mindestens ein Sauerstoff-, Stickstoff- oder Schwefelatom in dem bzw. den Ringen, wobei die Gruppe optional mit einem oder mehreren Substituenten Rₓ substituiert ist, -(OCH₂CH₂)ᵣ- und - CH₂-(OCH₂CH₂)ᵣ- ausgewählt ist, wobei jeder der obigen Alkylensubstituenten, ob allein oder an einen anderen Teil in der Kohlenstoffkette angelagert, optional durch einen oder mehrere Substituenten Rₓ substituiert sein kann;
R₂₀ ein C₁-C₁₂-Alkyl oder eine Arylgruppe mit von 6 bis 18 Kohlenstoffatomen in einem oder mehreren aromatischen Ringen ist, wobei die Arylgruppen optional mit einem oder mehreren Substituenten Rₓ substituiert sind;
Substituenten Rₓ aus der Gruppe bestehend aus C₁-C₁₂-Alkylgruppen, die optional mit mindestens einer Gruppe R_{y} substituiert sein können, C₂-C₁₂-Alkenylgruppen, die optional mit mindestens einer Gruppe R_{y} substituiert sein können, C₂-C₁₂-Alkinylgruppen, die optional mit mindestens einer Gruppe R_{y} substituiert sein können, Halogenatomen, Oxogruppen, Thiogruppen, Cyanogruppen, Nitrogruppen, OR_{y}, OCOR_{y}, OCOOR_{y}, COR_{y}, COOR_{y}, OCONR_{y}R_{z}, CONR_{y}R_{z}, SR_{y}, S(=O)R_{y}, SO₂R_{y}, SSR_{y}, P(O)(R_{y})OR_{z}, NR_{y}R_{z}, NR_{y}COR_{z}, NR_{y}C(=O)NR_{y}R_{z}, NR_{y}C(=NR_{y})NR_{y}R_{z}, Arylgruppen mit von 6 bis 18 Kohlenstoffatomen in einem oder mehreren Ringen, die optional mit einem oder mehreren Substituenten substituiert sein können, die gleich oder verschieden sein können, ausgewählt aus der Gruppe bestehend aus R_{y}, OR_{y}, OCOR_{y}, OCOOR_{y}, NR_{y}R_{z}, NR_{y}COR_{z} und NR_{y}C(=NR_{y})NR_{y}R_{z}, Aralkylgruppen, umfassend eine Alkylgruppe mit 1 bis 12 Kohlenstoffatomen, die mit einer wie oben definierten optional substituierten Arylgruppe substituiert ist, Aralkyloxygruppen, umfassend eine Alkoxygruppe mit 1 bis 12 Kohlenstoffatomen, die mit einer wie oben definierten optional substituierten Arylgruppe substituiert ist, und einer 5- bis 14-gliedrigen gesättigten oder ungesättigten heterocyclischen Gruppe mit einem oder mehreren Ringen und umfassend mindestens ein Sauerstoff-, Stickstoff- oder Schwefelatom in dem bzw. den Ringen ausgewählt sind, wobei die heterocyclische Gruppe optional mit einem oder mehreren Substituenten R_{y} substituiert ist und, wenn mehr als ein optionaler Substituent an einer beliebigen gegebenen Gruppe vorliegt, die optionalen Substituenten R_{y} gleich oder verschieden sein können;
jedes R_{y} und R_{z} unabhängig aus der Gruppe bestehend aus Wasserstoff, C₁-C₁₂-Alkylgruppen, C₁-C₁₂-Alkylgruppen, die mit mindestens einem Halogenatom substituiert sind, Aralkylgruppen, umfassend eine C₁-C₁₂-Alkylgruppe, die mit einer Arylgruppe mit 6 bis 18 Kohlenstoffatomen in einem oder mehreren Ringen substituiert ist, und Heterocycloalkylgruppen, umfassend eine C₁-C₁₂-Alkylgruppe, die mit einer 5- bis 14-gliedrigen gesättigten oder ungesättigten heterocyclischen Gruppe mit einem oder mehreren Ringen substituiert ist und umfassend mindestens ein Sauerstoff-, Stickstoff- oder Schwefelatom in dem bzw. den Ringen, ausgewählt ist;
r eine ganze Zahl ist, die von 1 bis 10 reicht;
b eine ganze Zahl von 0 oder 1 ist;
g eine ganze Zahl von 0 oder 1 ist;
w eine ganze Zahl ist, die von 0 bis 12 reicht;
jedes von D, X, T und AA wie in einem der Ansprüche 1 bis 15 definiert ist; wobei für Verbindungen der Formel D-(X)_{b}-(AA)_{w}-(T)_{g}-H b + w + g nicht 0 ist; optional
wobei die Verbindung der Formel D-(X)_{b}-(AA)_{w}-(T)_{g}-L₁ ausgewählt ist aus: 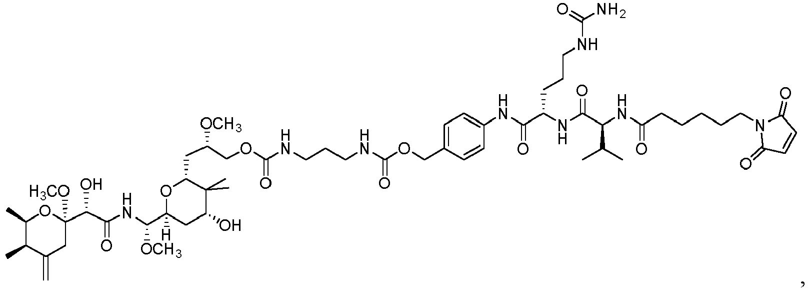 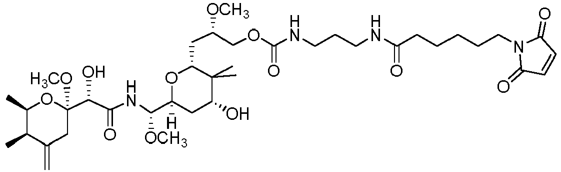 und

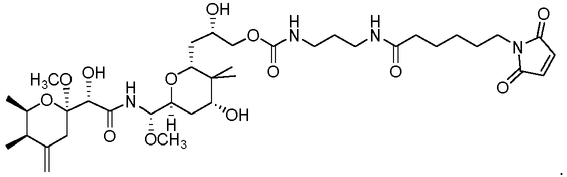

22. Verbindung der Formel D-(X)_{b}-(AA)_{w}-(T)_{g}-L₁ oder der Formel D-(X)_{b}-(AA)_{w}-(T)_{g}-H, wobei jedes von D, X, AA, T, b, g und w wie in einem der Ansprüche 1 bis 15 definiert ist und L₁ wie in Anspruch 21 definiert ist; jedoch ferner wobei, wenn die Verbindung eine Verbindung der Formel D-(X)_{b}-(AA)_{w}-(T)_{g}-H ist, b + w + g ≠ 0.

23. Wirkstoffkonjugat oder Verbindung nach einem der Ansprüche 1 bis 22, wobei b + w + g nicht 0 ist;
oder
wobei b + w nicht 0 ist;
oder
wobei, wenn w nicht 0 ist, b 1 ist.

24. Wirkstoffkonjugat nach einem der Ansprüche 1 bis 20 zur Verwendung als eine Nutzlast in einem Antikörper-Wirkstoffkonjugat.

25. Verwendung eines Wirkstoffteils nach einem der Ansprüche 1 bis 20 in der Herstellung eines Antikörper-Wirkstoffkonjugats.

26. Wirkstoffkonjugat nach einem der Ansprüche 1 bis 20 zur Verwendung als ein Arzneimittel.

27. Wirkstoffkonjugat nach einem der Ansprüche 1 bis 20 zur Verwendung bei der Behandlung einer Krebserkrankung, vorzugsweise einer Krebserkrankung, die aus Lungenkrebs, einschließlich NSCLC, Magenkrebs, kolorektalem Karzinom, Brustkrebs, Pankreaskarzinom, Endometriumkarzinom, Blasenkrebs, Gebärmutterhalskrebs, Speiseröhrenkrebs, Gallenblasenkrebs, Gebärmutterkrebs, Speichelkanalkrebs, Eierstockkrebs, Nierenkrebs, Leukämie, multiplem Myelom und Lymphom ausgewählt ist.

28. Wirkstoffkonjugat zur Verwendung nach Anspruch 27, wobei die Krebserkrankung eine HER2-positive Krebserkrankung, vorzugsweise HER2-positiver Lungenkrebs, einschließlich HER2-positivem NSCLC, HER2-positiver Magenkrebs, HER2-positives kolorektales Karzinom, HER2-positiver Brustkrebs, HER2-positives Pankreaskarzinom, HER2-positives Endometriumkarzinom, HER2-positiver Blasenkrebs, HER2-positiver Gebärmutterhalskrebs, HER2-positiver Speiseröhrenkrebs, HER2-positiver Gallenblasenkrebs, HER2-positiver Gebärmutterkrebs, HER2-positiver Speichelkanalkrebs und HER2-positiver Eierstockkrebs ist.

29. Pharmazeutische Zusammensetzung, umfassend ein Wirkstoffkonjugat nach einem der Ansprüche 1 bis 20 und einen pharmazeutisch unbedenklichen Trägerstoff.

30. Kit, umfassend eine therapeutisch wirksame Menge eines Wirkstoffkonjugats nach einem der Ansprüche 1 bis 20 und einen pharmazeutisch unbedenklichen Trägerstoff.

31. Kit nach Anspruch 30 zur Verwendung bei der Behandlung einer Krebserkrankung und vorzugsweise einer Krebserkrankung, die aus Lungenkrebs, einschließlich NSCLC, Magenkrebs, kolorektalem Karzinom, Brustkrebs, Pankreaskarzinom, Endometriumkarzinom, Blasenkrebs, Gebärmutterhalskrebs, Speiseröhrenkrebs, Gallenblasenkrebs, Gebärmutterkrebs, Speichelkanalkrebs, Eierstockkrebs, Nierenkrebs, Leukämie, multiplem Myelom und Lymphom ausgewählt ist.

32. Verfahren zur Herstellung eines Wirkstoff-Antikörperkonjugats nach einem der Ansprüche 1 bis 20, umfassend ein Konjugieren eines Teils Ab, der mindestens eine Antigenbindungsstelle umfasst, und eines Wirkstoffs D, wobei Ab und D wie in einem der Ansprüche 1 bis 20 definiert sind;
optional wobei
die Herstellung eines Wirkstoff-Antikörperkonjugats der Formel (G), (G') oder (G"): 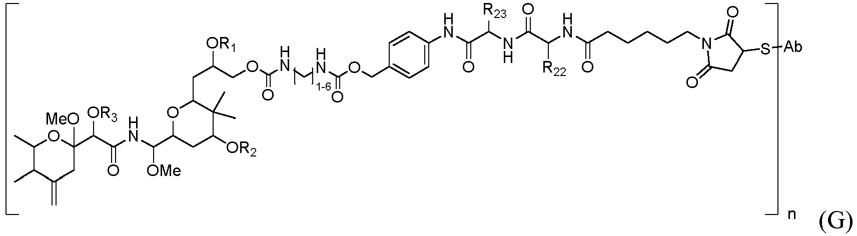 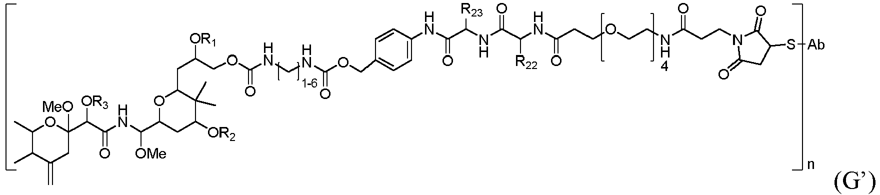 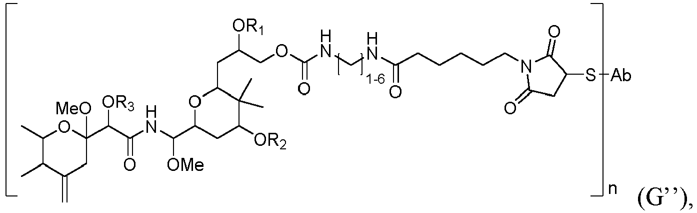
umfassend die folgenden Schritte:
(i) entweder:
(a) das Umsetzen einer Verbindung der Formel (Ib): 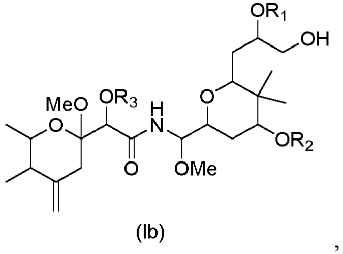 wobei
R₁ aus einer Schutzgruppe für OH, substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, -C(=O)Rₐ, -C(=O)OR_{b} und -C(=O)NR_{c}R_{d} ausgewählt ist;
R₂ Wasserstoff ist;
R₃ aus einer Schutzgruppe für OH, -C(=O)Rₐ, -C(=O)OR_{b} und -C(=O)NR_{c}R_{d} ausgewählt ist;
Rₐ aus Wasserstoff, substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, substituiertem oder unsubstituiertem Aryl und einer substituierten oder unsubstituierten heterocyclischen Gruppe ausgewählt ist;
R_{b} aus substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl, substituiertem oder unsubstituiertem Aryl und einer substituierten oder unsubstituierten heterocyclischen Gruppe ausgewählt ist;
R_{c} und R_{d} unabhängig aus Wasserstoff, substituiertem oder unsubstituiertem C₁-C₁₂-Alkyl, substituiertem oder unsubstituiertem C₂-C₁₂-Alkenyl, substituiertem oder unsubstituiertem C₂-C₁₂-Alkinyl und substituiertem oder unsubstituiertem Aryl und einer substituierten oder unsubstituierten heterocyclischen Gruppe ausgewählt sind;
mit einer Verbindung der Formel X₂-C(=O)-X₁, wobei X₁ und X₂ Abgangsgruppen sind, um eine Verbindung der Formel (B) zu ergeben: 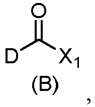
und der Punkt der Anlagerung des -C(=O)X₁-Teils die freie primäre -OH-Gruppe der Verbindung der Formel (Ib) ist, oder
(b) das Umsetzen der wie oben definierten Verbindung der Formel (Ib) mit 4-Nitrophenylchloroformiat, um eine Verbindung der Formel (J) zu ergeben: 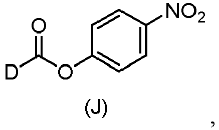 wobei der Punkt der Anlagerung der (4-Nitrophenyl)-O-CO-Gruppe identisch mit dem des X₁(C=O)-Teils in (a) oben ist; oder
(c) das Umsetzen einer wie oben definierten Verbindung der Formel (Ib) mit einem Isocyanat der Formel O=C=N-(CH₂)₁₋₆NHProt^{NH}, wobei Prot^{NH} eine Schutzgruppe für Amino ist, die dazu geeignet ist, unter basischen Bedingungen entschützt zu werden, um eine Verbindung der Formel zu ergeben: 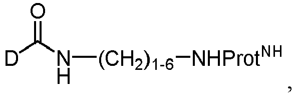
(ii) entweder
(a) das Umsetzen der in Schritt (i)(a) oder (i)(b) produzierten Verbindung der Formel (B) oder (J) mit einer Aminoverbindung der Formel NH₂-(CH₂)₁₋₆NH₂, um eine Verbindung der Formel (C) zu ergeben: 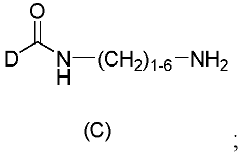 oder
(b) das Entschützen der Verbindung der Formel: 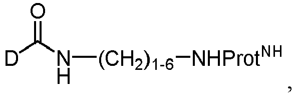
die in Schritt (i)(c) erhalten wurde, um eine Verbindung der Formel (C) zu ergeben,
(iii) das Umsetzen der Verbindung der Formel (C) mit einer Verbindung der Formel (D'), (E) oder (E'): 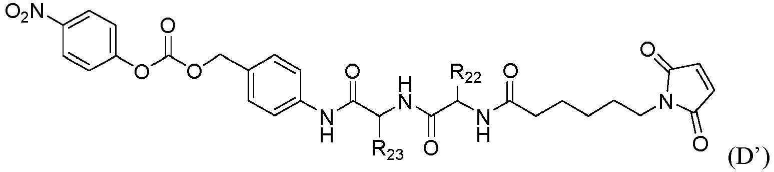 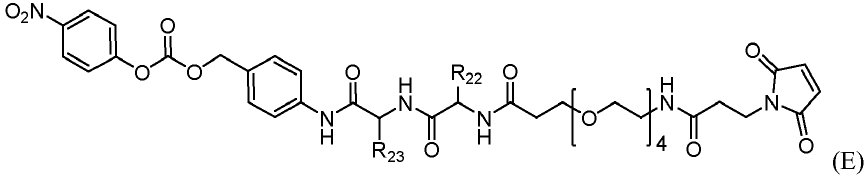 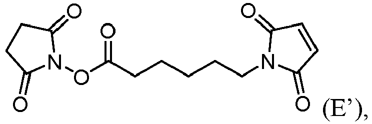
wobei R₂₂ und R₂₃ wie oben in den Definitionen von AA-Gruppen definiert sind; um eine Verbindung der Formel (F), (F') beziehungsweise (F") zu ergeben: 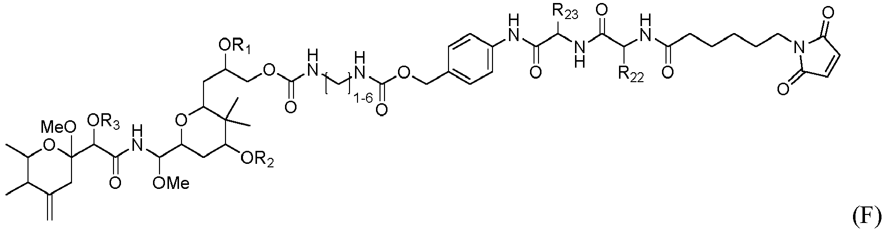 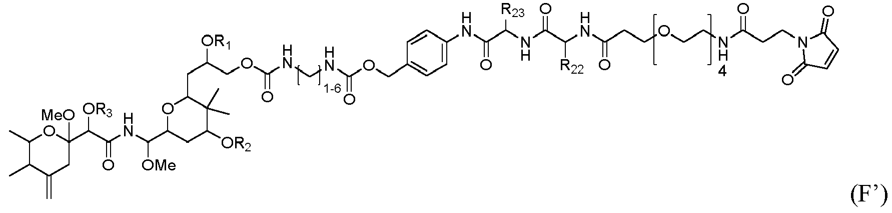 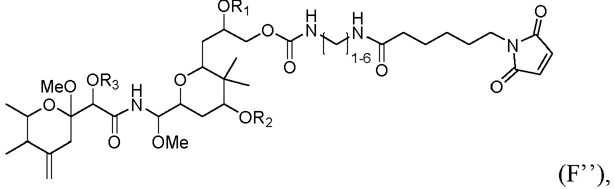
wobei R₁, R₂ und R₃ wie oben für die Verbindungen der Formel (Ib) definiert sind und R₂₂ und R₂₃ wie oben in den Definitionen von AA-Gruppen definiert sind,
(iv) wenn Schutzgruppen für die OH in den Verbindungen der Formel (F), (F') oder (F") vorliegen, das Entfernen derartiger Schutzgruppen, um Verbindungen der Formel (F), (F') oder (F") zu ergeben, wobei R₁, R₂ und R₃ wie oben für die Verbindungen der Formel (II) definiert sind,
(v) das partielle Reduzieren einer oder mehrerer Disulfidbindungen in dem zu konjugierenden Antikörper, um einen reduzierten Antikörper Ab-SH mit freien Thiolgruppen zu ergeben; 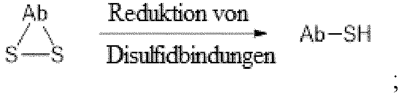 und
(vi) das Umsetzen des partiell reduzierten Antikörpers Ab-SH mit freien Thiolgruppen mit der in Schritt (iv) produzierten Verbindung der Formel (F), (F') oder (F"), um das gewünschte Wirkstoff-Antikörperkonjugat der Formel (G), (G') beziehungsweise (G") zu ergeben:

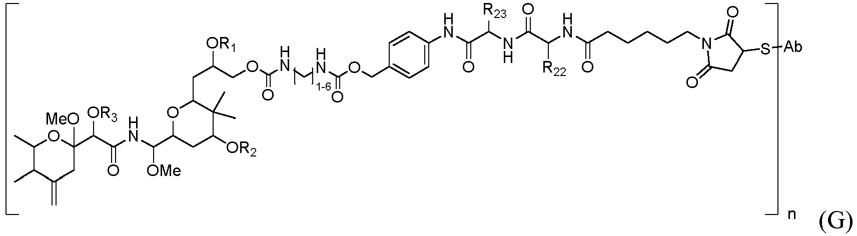

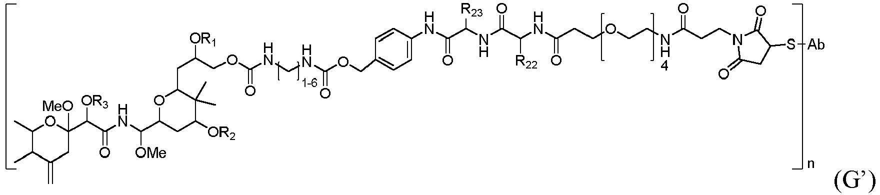

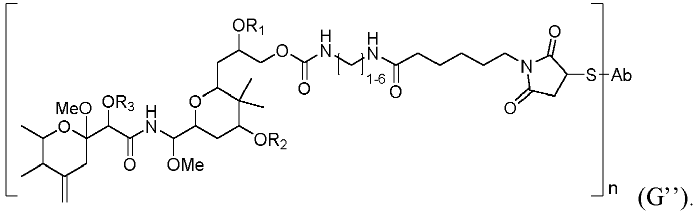

## Revendications

1. Conjugué de médicament comprenant une fraction de médicament liée de manière covalente au reste du conjugué de médicament, le conjugué de médicament présentant la formule [D-(X)_{b}-(AA)_{w}-(T)_{g}-(L)-]ₙ-Ab, dans lequel :
D est une fraction de médicament présentant la formule (I) suivante ou un sel ou un ester pharmaceutiquement acceptables de celle-ci, 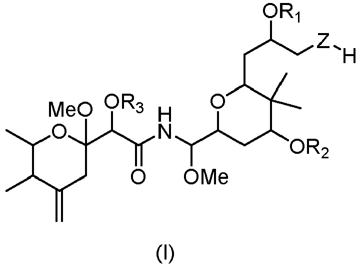 dans laquelle :
D est lié de manière covalente via un groupe hydroxy à OR₁, OR₃ ou ZH ou via un groupe thiol à ZH, à (X)_{b} s'il existe ou (AA)_{w} s'il existe ou à (T)_{g} s'il existe ou (L) ;
R₁ est choisi parmi : hydrogène, C₁-C₁₂ alkyle substitué ou non substitué, C₂-C₁₂ alcényle substitué ou non substitué, C₂-C₁₂ alcynyle substitué ou non substitué, -C(=O)Rₐ, -C(=O)OR_{b} et -C(=O)NR_{c}R_{d} ;
R₂ est un hydrogène ;
R₃ est choisi parmi : hydrogène, -C(=O)Rₐ, -C(=O)OR_{b} et -C(=O)NR_{c}R_{d} ;
Z est choisi parmi : -O- et -S- ;
Rₐ est choisi parmi : hydrogène, C₁-C₁₂ alkyle substitué ou non substitué, C₂-C₁₂ alcényle substitué ou non substitué, C₂-C₁₂ alcynyle substitué ou non substitué, aryle substitué ou non substitué et groupe hétérocyclique substitué ou non substitué ;
R_{b} est choisi parmi : C₁-C₁₂ alkyle substitué ou non substitué, C₂-C₁₂ alcényle substitué ou non substitué, C₂-C₁₂ alcynyle substitué ou non substitué, aryle substitué ou non substitué et groupe hétérocyclique substitué ou non substitué ;
R_{c} et R_{d} sont indépendamment choisis parmi : hydrogène, C₁-C₁₂ alkyle substitué ou non substitué, C₂-C₁₂ alcényle substitué ou non substitué, C₂-C₁₂ alcynyle substitué ou non substitué, aryle substitué ou non substitué et groupe hétérocyclique substitué ou non substitué ;
X et T sont des groupes d'extension qui peuvent être identiques ou différents ;
chaque AA est indépendamment une unité d'acide aminé ;
L est un groupe lieur ;
w est un nombre entier variant de 0 à 12 ;
b est un nombre entier de 0 ou 1 ;
g est un nombre entier de 0 ou 1 ;
Ab est une fraction comprenant au moins un site de liaison d'antigène ; et
n est le rapport du groupe [D-(X)_{b}-(AA)_{w}-(T)_{g}-(L)-] sur la fraction comprenant au moins un site de liaison d'antigène et il est dans l'intervalle de 1 à 20.

2. Conjugué de médicament selon la revendication 1, dans lequel D est aussi un composé de formule (Ia), ou un sel ou un ester pharmaceutiquement acceptables de celui-ci : 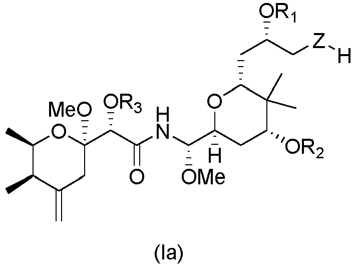 dans laquelle R₁, R₂, R₃ et Z sont tels que définis dans la formule (I) dans la revendication 1.

3. Conjugué de médicament selon la revendication 1, dans lequel D est un composé de formule : 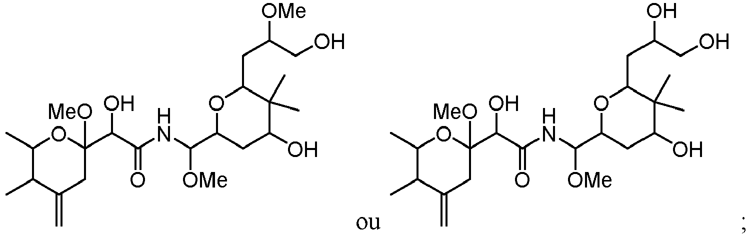 ou un sel ou un ester pharmaceutiquement acceptables de celui-ci.

4. Conjugué de médicament selon la revendication 2, dans lequel D est un composé de formule : 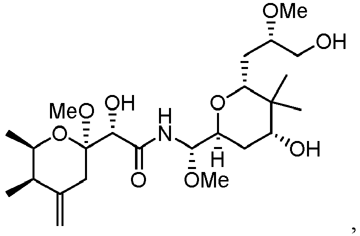 ou un sel ou un ester pharmaceutiquement acceptables de celui-ci.

5. Conjugué de médicament selon la revendication 2, dans lequel D est un composé de formule : 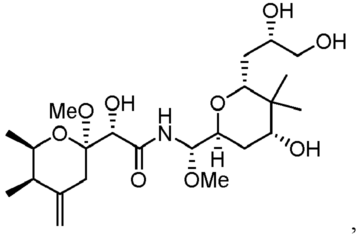 ou un sel ou un ester pharmaceutiquement acceptables de celui-ci.

6. Conjugué de médicament comprenant une fraction de médicament liée de manière covalente au reste du conjugué de médicament, le conjugué de médicament présentant la formule [D-(X)_{b}-(AA)_{w}-(T)_{g}-(L)-]ₙ-Ab, dans laquelle :
D est une fraction de médicament présentant la formula (II) suivante ou un sel ou un ester pharmaceutiquement acceptables de celle-ci : 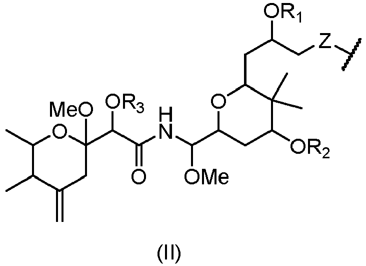 dans laquelle :
la ligne ondulée indique le point de liaison covalente à (X)_{b} s'il existe ou (AA)_{w} s'il existe ou à (T)_{g} s'il existe ou (L) ;
R₁ est choisi parmi : hydrogène, C₁-C₁₂ alkyle substitué ou non substitué, C₂-C₁₂ alcényle substitué ou non substitué, C₂-C₁₂ alcynyle substitué ou non substitué, -C(=O)Rₐ, -C(=O)OR_{b} et -C(=O)NR_{c}R_{d} ; dans lequel les substituants optionnels sont un ou plusieurs substituants Rₓ ;
R₂ est un hydrogène ;
R₃ est choisi parmi : hydrogène, -C(=O)Rₐ, -C(=O)OR_{b} et -C(=O)NR_{c}R_{d} ;
Z est -O- ou -S- ;
Rₐ est choisi parmi : hydrogène, C₁-C₁₂ alkyle substitué ou non substitué, C₂-C₁₂ alcényle substitué ou non substitué, C₂-C₁₂ alcynyle substitué ou non substitué, aryle substitué ou non substitué et groupe hétérocyclique substitué ou non substitué ; les substituants optionnels étant un ou plusieurs substituants Rₓ ;
R_{b} est choisi parmi : C₁-C₁₂ alkyle substitué ou non substitué, C₂-C₁₂ alcényle substitué ou non substitué, C₂-C₁₂ alcynyle substitué ou non substitué, aryle substitué ou non substitué et groupe hétérocyclique substitué ou non substitué ; dans lequel les substituants optionnels sont un ou plusieurs substituants Rₓ ;
R_{c} et R_{d} sont indépendamment choisis parmi : hydrogène, C₁-C₁₂ alkyle substitué ou non substitué, C₂-C₁₂ alcényle substitué ou non substitué, C₂-C₁₂ alcynyle substitué ou non substitué, aryle substitué ou non substitué et groupe hétérocyclique substitué ou non substitué ; dans lequel les substituants optionnels sont un ou plusieurs substituants Rₓ ;
les substituants Rₓ sont choisis dans le groupe constitué de : groupes C₁-C₁₂ alkyles qui peuvent être optionnellement substitués par au moins un groupe R_{y}, groupes C₂-C₁₂ alcényles qui peuvent être optionnellement substitués par au moins un groupe R_{y}, groupes C₂-C₁₂ alcynyles qui peuvent être optionnellement substitués par au moins un groupe R_{y}, atomes d'halogène, groupes oxo, groupes thio, groupes cyano, groupes nitro, OR_{y}, OCOR_{y}, OCOOR_{y}, COR_{y}, COOR_{y}, OCONR_{y}R_{z}, CONR_{y}R_{z}, SR_{y}, S(=O)R_{y}, SO₂R_{y}, SSR_{y}, P(O)(R_{y})OR_{z}, NR_{y}R_{z}, NR_{y}COR_{z}, NR_{y}C(=O)NR_{y}R_{z}, NR_{y}C(=NR_{y})NR_{y}R_{z}, groupes aryles possédant 6 à 18 atomes de carbone dans un ou plusieurs cycles qui peuvent optionnellement être substitués par un ou plusieurs substituants qui peuvent être identiques ou différents choisis dans le groupe constitué de : R_{y}, OR_{y}, OCOR_{y}, OCOOR_{y}, NR_{y}R_{z}, NR_{y}COR_{z} et NR_{y}C(=NR_{y})NR_{y}R_{z}, groupes aralkyles comprenant un groupe alkyle possédant 1 à 12 atomes de carbone substitué par un groupe aryle optionnellement substitué tel que défini ci-dessus, groupes aralkyloxy comprenant un groupe alcoxy possédant 1 à 12 atomes de carbone substitué par un groupe aryle optionnellement substitué tel que défini ci-dessus et un groupe hétérocyclique à 5 à 14 chaînons saturé ou insaturé possédant un ou plusieurs cycles et comprenant au moins un atome d'oxygène, d'azote ou de soufre dans le(s)dit(s) cycle(s), ledit groupe hétérocyclique étant optionnellement substitué par un ou plusieurs substituants R_{y} et lorsqu'il y a plus d'un substituant optionnel sur un quelconque groupe donné, les substituants optionnels R_{y} peuvent être identiques ou différents ;
chaque R_{y} et R_{z} est indépendamment choisi dans le groupe constitué de : hydrogène, groupes C₁-C₁₂ alkyles, groupes C₁-C₁₂ alkyles qui sont substitués par au moins un atome d'halogène, groupes aralkyles comprenant un groupe C₁-C₁₂ alkyle qui est substitué par un groupe aryle possédant 6 à 18 atomes de carbone dans un ou plusieurs cycles et groupes hétérocycloalkyles comprenant un groupe C₁-C₁₂ alkyle qui est substitué par un groupe hétérocyclique à 5 à 14 chaînons saturé ou insaturé possédant un ou plusieurs cycles et comprenant au moins un atome d'oxygène, d'azote ou de soufre dans le(s)dit(s) cycles(s) ;
X et T sont des groupes d'extension qui peuvent être identiques ou différents ;
chaque AA est indépendamment une unité d'acide aminé ;
L est un groupe lieur ;
w est un nombre entier variant de 0 à 12 ;
b est un nombre entier de 0 ou 1 ;
g est un nombre entier de 0 ou 1 ;
où b + g + w n'est optionnellement pas 0 ;
Ab est une fraction comprenant au moins un site de liaison d'antigène ; et
n est le rapport du groupe [D-(X)_{b}-(AA)_{w}-(T)_{g}-(L)-] sur la fraction comprenant au moins un site de liaison d'antigène et il est dans l'intervalle de 1 à 20.

7. Conjugué de médicament selon la revendication 6, ou un sel ou un ester pharmaceutiquement acceptables de celui-ci, dans lequel D est une fraction de médicament de formule (IIa) : 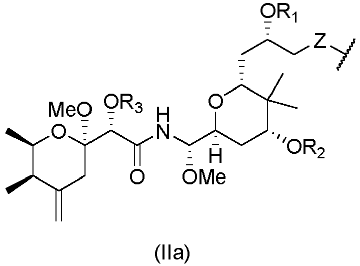 dans laquelle la ligne ondulée, R₁, R₂, R₃ et Z sont tels que définis pour la formule (II).

8. Conjugué de médicament selon l'une quelconque des revendications 1 à 7, dans lequel le sel est choisi parmi : chlorhydrate, bromhydrate, iodhydrate, sulfate, nitrate, phosphate, acétate, trifluoroacétate, maléate, fumarate, citrate, oxalate, succinate, tartrate, malate, mandélate, méthanesulfonate, p-toluènesulfonate, sodium, potassium, calcium, ammonium, éthylènediamine, éthanolamine, N,N-dialkylénéthanolamine, triéthanolamine et aminoacides basiques.

9. Conjugué de médicament selon l'une quelconque des revendications précédentes, dans lequel L est un groupe lieur choisi dans le groupe constitué de : 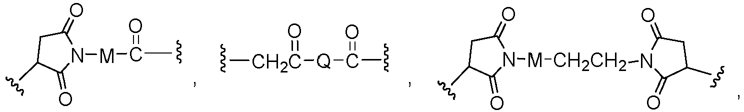 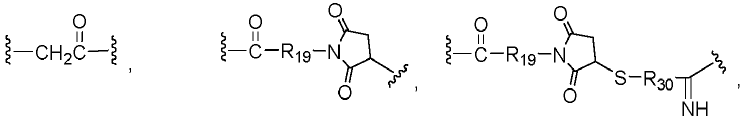 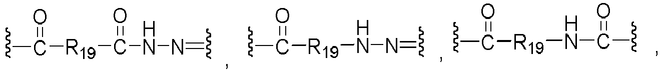 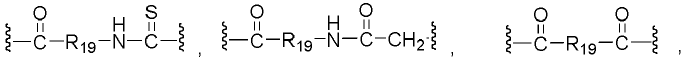 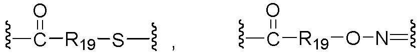 où :
les lignes ondulées indiquent le point de liaisons covalentes à un Ab (la ligne ondulée à droite) et à (T)_{g} s'il existe, ou (AA)_{w} s'il existe, ou (X)_{b} s'il existe, ou à D (la ligne ondulée à gauche) ;
R₁₉ est choisi parmi : -C₁-C₁₂ alkylène-, -C₃-C₈ carbocyclo-, -O-(C₁-C₁₂ alkylène)-, -C₆-C₁₈ arylène- dans un ou plusieurs cycles qui peuvent optionnellement être substitués par un ou plusieurs substituants Rₓ, -C₁-C₁₂ alkylène-C₆-C₁₈ arylène- dans lequel le groupe arylène est dans un ou plusieurs cycles qui peuvent optionnellement être substitués par un ou plusieurs substituants Rₓ, -C₆-C₁₈ arylène-C₁-C₁₂ alkylène- dans lequel le groupe arylène est dans un ou plusieurs cycles qui peuvent optionnellement être substitués par un ou plusieurs substituants Rₓ, -C₁-C₁₂ alkylène-(C₃-C₈ carbocyclo)-, -(C₃-C₈ carbocyclo)-C₁-C₁₂ alkylène-, -C₅-C₁₄ hétérocyclo- dans lequel ledit groupe hétérocyclo peut être un groupe saturé ou insaturé possédant un ou plusieurs cycles et comprenant au moins un atome d'oxygène, d'azote ou de soufre dans le(s)dit(s) cycle(s), ledit groupe étant optionnellement substitué par un ou plusieurs substituants Rₓ, -C₁-C₁₂ alkylène-(C₅-C₁₄ hétérocyclo)- dans lequel ledit groupe hétérocyclo peut être un groupe saturé ou insaturé possédant un ou plusieurs cycles et comprenant au moins un atome d'oxygène, d'azote ou de soufre dans le(s)dit(s) cycle(s), ledit groupe étant optionnellement substitué par un ou plusieurs substituants Rₓ, -(C₅-C₁₄ hétérocyclo)-C₁-C₁₂ alkylène- dans lequel ledit groupe hétérocyclo peut être un groupe saturé ou insaturé possédant un ou plusieurs cycles et comprenant au moins un atome d'oxygène, d'azote ou de soufre dans le(s)dit(s) cycle(s), ledit groupe étant optionnellement substitué par un ou plusieurs substituants Rₓ, -(OCH₂CH₂)ᵣ- et -CH₂-(OCH₂CH₂)ᵣ-, dans lequel chacun des substituants alkylènes ci-dessus, soit seul, soit lié à une autre fraction dans la chaîne carbonée, peut optionnellement être substitué par un ou plusieurs substituants Rₓ ;
R₃₀ est un groupe -C₁-C₆ alkylène- ;
M est choisi dans le groupe constitué de : -C₁-C₆ alkylène-, -C₁-C₆ alkylène-(C₃-C₈ carbocyclo)-, -(CH₂CH₂O)ₛ-, -C₁-C₆ alkylène-(C₃-C₈ carbocyclo)-CON(H ou C₁-C₆ alkyl)-C₁-C₆ alkylène-, -phénylène- qui peut optionnellement être substitué par un ou plusieurs substituants Rₓ, -phénylène-C₁-C₆ alkylène- dans lequel la fraction phénylène peut optionnellement être substituée par un ou plusieurs substituants Rₓ et -C₁-C₆ alkylène-CON(H ou C₁-C₆ alkyl)C₁-C₆ alkylène- ;
Q est choisi dans le groupe constitué de : -N(H ou C₁-C₆ alkyl)phénylène- et -N(H ou C₁-C₆ alkyl)-(CH₂)ₛ ;
r est un nombre entier variant de 1 à 10 ; et
s est un nombre entier variant de 1 à 10 ;
ou
dans lequel L est un groupe lieur choisi dans le groupe constitué de : 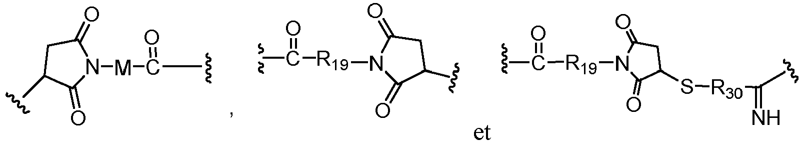 où :
les lignes ondulées indiquent le point de liaisons covalentes à un Ab (la ligne ondulée à droite) et à (T)_{g} s'il existe, ou (AA)_{w} s'il existe, ou (X)_{b} s'il existe, ou à D (la ligne ondulée à gauche) ;
R₁₉ est choisi parmi : -C₁-C₁₂ alkylène-, -O-(C₁-C₁₂ alkylène), -C₆-C₁₂ arylène-dans un ou plusieurs cycles qui peuvent optionnellement être substitués par un ou plusieurs substituants Rₓ, -C₁-C₁₂ alkylène-C₆-C₁₂ arylène- dans lequel le groupe arylène est dans un ou plusieurs cycles qui peuvent optionnellement être substitués par un ou plusieurs substituants Rₓ, -C₆-C₁₂ arylène-C₁-C₁₂ alkylène- dans lequel le groupe arylène est dans un ou plusieurs cycles qui peuvent optionnellement être substitués par un ou plusieurs substituants Rₓ, -C₅-C₁₂ hétérocyclo- dans lequel ledit groupe hétérocyclo peut être un groupe saturé ou insaturé possédant un ou plusieurs cycles et comprenant au moins un atome d'oxygène, d'azote ou de soufre dans le(s)dit(s) cycle(s), ledit groupe étant optionnellement substitué par un ou plusieurs substituants Rₓ, -C₁-C₁₂ alkylène-(C₅-C₁₂ hétérocyclo)- dans lequel ledit groupe hétérocyclo peut être un groupe saturé ou insaturé possédant un ou plusieurs cycles et comprenant au moins un atome d'oxygène, d'azote ou de soufre dans le(s)dit(s) cycle(s), ledit groupe étant optionnellement substitué par un ou plusieurs substituants Rₓ, -(C₅-C₁₂ hétérocyclo)-C₁-C₁₂ alkylène- dans lequel ledit groupe hétérocyclo peut être un groupe saturé ou insaturé possédant un ou plusieurs cycles et comprenant au moins un atome d'oxygène, d'azote ou de soufre dans le(s)dit(s) cycle(s), ledit groupe étant optionnellement substitué par un ou plusieurs substituants Rₓ, -(OCH₂CH₂)ᵣ- et -CH₂-(OCH₂CH₂)ᵣ-, dans lequel chacun des substituants alkylènes ci-dessus, soit seul, soit lié à une autre fraction dans la chaîne carbonée, peut optionnellement être substitué par un ou plusieurs substituants Rₓ ;
R₃₀ est un groupe -C₁-C₆ alkylène- ;
M est choisi dans le groupe constitué de : -C₁-C₆ alkylène-, -C₁-C₆ alkylène-(C₃-C₈ carbocyclo)- et -phénylène- qui peut optionnellement être substitué par un ou plusieurs substituants Rₓ ; et
r est un nombre entier variant de 1 à 6.

10. Conjugué de médicament selon l'une quelconque des revendications 1 à 9, choisi parmi les formules (V), (VI) et (VII) : 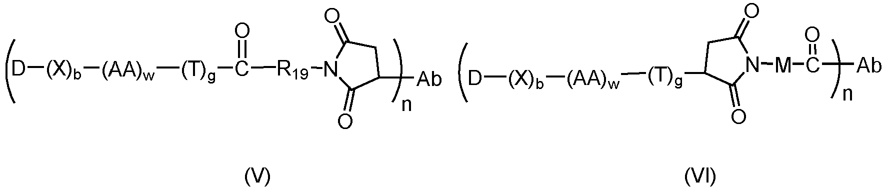 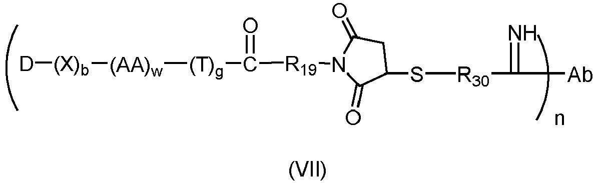 dans lesquelles :
X et T sont des groupes d'extension qui peuvent être identiques ou différents ;
chaque AA est indépendamment une unité d'acide aminé ;
w est un nombre entier variant de 0 à 12 ;
b est un nombre entier de 0 ou 1 ;
g est un nombre entier de 0 ou 1 ;
D est une fraction de médicament ;
Ab est une fraction comprenant au moins un site de liaison d'antigène ;
n est le rapport du groupe [D-(X)_{b}-(AA)_{w}-(T)_{g}-(L)-] dans lequel L est tel que défini dans la formule (V), (VI) ou (VII) sur la fraction comprenant au moins un site de liaison d'antigène et il est dans l'intervalle de 1 à 20 ;
R₁₉ est choisi parmi : -Ci-Cs alkylène-, -O-(C₁-C₈ alkylène)-, -Ci-Cs alkylène-C₆-C₁₂ arylène- dans lequel le groupe arylène est dans un ou plusieurs cycles qui peuvent optionnellement être substitués par un ou plusieurs substituants Rₓ et -C₆-C₁₂ arylène-C₁-C₈ alkylène- dans lequel le groupe arylène est dans un ou plusieurs cycles qui peuvent optionnellement être substitués par un ou plusieurs substituants Rₓ, dans lequel chacun des substituants alkylènes ci-dessus, soit seul, soit lié à une autre fraction dans la chaîne carbonée, peut optionnellement être substitué par un ou plusieurs substituants Rₓ ;
R₃₀ est un groupe -C₂-C₄ alkylène- ; et
M est choisi dans le groupe constitué de : -C₁-C₃ alkylène- et -C₁-C₃ alkylène-(C₅-C₇ carbocyclo)- ; ou
choisi parmi les formules (V), (VI) et (VII) : 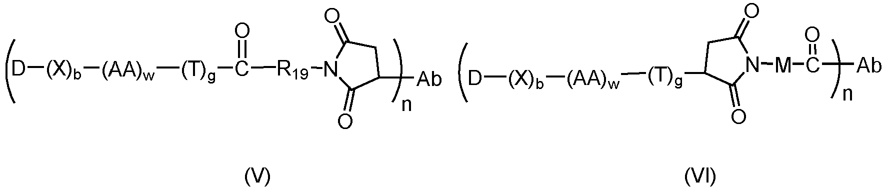 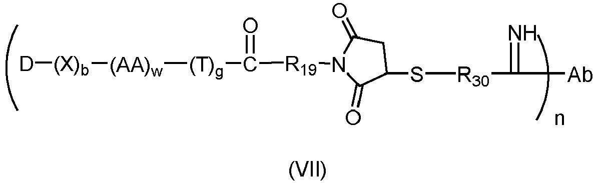 dans lesquelles :
X et T sont des groupes d'extension qui peuvent être identiques ou différents ;
chaque AA est indépendamment une unité d'acide aminé ;
w est un nombre entier variant de 0 à 12 ;
b est un nombre entier de 0 ou 1 ;
g est un nombre entier de 0 ou 1 ;
D est une fraction de médicament ;
Ab est une fraction comprenant au moins un site de liaison d'antigène ;
n est le rapport du groupe [D-(X)_{b}-(AA)_{w}-(T)_{g}-(L)-] dans lequel L est tel que défini dans (V), (VI) ou (VII) sur la fraction comprenant au moins un site de liaison d'antigène et il est dans l'intervalle de 1 à 20 ;
R₁₉ est choisi parmi : -C₁-C₆ alkylène-, -phénylène-C₁-C₆ alkylène- dans lequel le groupe phénylène peut optionnellement être substitué par un ou plusieurs substituants Rₓ choisis dans le groupe constitué de : groupes alkyles possédant 1 à 6 atomes de carbone, groupes alcoxy possédant 1 à 6 atomes de carbone, atomes d'halogène, groupes nitro et groupes cyano, dans lequel chacun des substituants alkylènes ci-dessus, soit seul, soit lié à une autre fraction dans la chaîne carbonée, peut optionnellement être substitué par un ou plusieurs substituants Rₓ choisis dans le groupe constitué de : groupes alkyles possédant 1 à 6 atomes de carbone, groupes alcoxy possédant 1 à 6 atomes de carbone, groupes aryles possédant 6 à 12 atomes de carbone, atomes d'halogène, groupes nitro et groupes cyano et de préférence R₁₉ est un groupe -C₁-C₆ alkylène- ;
R₃₀ est un groupe -C₂-C₄ alkylène- ; et
M est un -C₁-C₃ alkylène-(C₅-C₇ carbocyclo)-.

11. Conjugué de médicament selon l'une quelconque des revendications 1 à 10, dans lequel (AA)_{w} est de formule (III) : 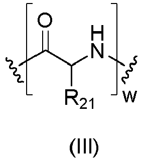
dans laquelle les lignes ondulées indiquent le point de liaisons covalentes à (X)_{b} s'il existe ou à la fraction de médicament (la ligne ondulée à gauche) et à (T)_{g} s'il existe, ou au lieur (la ligne ondulée à droite) ; et
R₂₁ est, à chaque occurrence, choisi dans le groupe constitué de : hydrogène, méthyle, isopropyle, isobutyle, sec-butyle, benzyle, p-hydroxybenzyle, -CH₂OH, -CH(OH)CH₃, -CH₂CH₂SCH₃, -CH₂CONH₂, -CH₂COOH, -CH₂CH₂CONH₂, -CH₂CH₂COOH, -(CH₂)₃NHC(=NH)NH₂, -(CH₂)₃NH₂, -(CH₂)₃NHCOCH₃, -(CH₂)₃NHCHO, -(CH₂)₄NHC(=NH)NH₂, -(CH₂)₄NH₂, -(CH₂)₄NHCOCH₃, -(CH₂)₄NHCHO, -(CH₂)₃NHCONH₂, -(CH₂)₄NHCONH₂, -CH₂CH₂CH(OH)CH₂NH₂, 2-pyridylméthyl-, 3-pyridylméthyl-, 4-pyridylméthyl-, phényle, cyclohexyle, 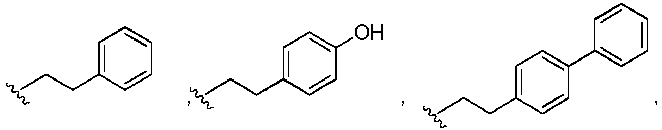 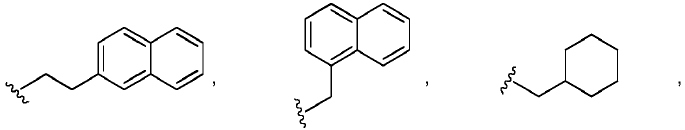 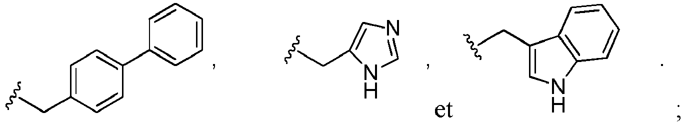
et w est un nombre entier variant de 1 à 12 ;
ou
dans lequel (AA)_{w} est de formule (III) dans laquelle :
R₂₁ est choisi, à chaque occurrence, dans le groupe constitué de : hydrogène, méthyle, isopropyle, sec-butyle, benzyle, indolylméthyle, -(CH₂)₃NHCONH₂, -(CH₂)₄NH₂, -(CH₂)₃NHC(=NH)NH₂ et -(CH₂)₄NHC(=NH)NH₂ ; et
w est un nombre entier variant de 0 à 6 ;
ou
dans lequel w est 0 ou 2 et lorsque w est 2, alors (AA)_{w} est de formule (IV) : 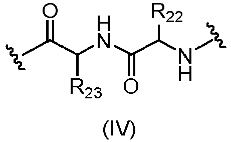 dans laquelle :
les lignes ondulées indiquent le point de liaisons covalentes à (X)_{b} s'il existe, ou à la fraction de médicament (la ligne ondulée à gauche) et à (T)_{g} s'il existe, ou au lieur (la ligne ondulée à droite) ;
R₂₂ est choisi parmi : méthyle, benzyle, isopropyle, sec-butyle et indolylméthyle ; et
R₂₃ est choisi parmi : méthyle, -(CH₂)₄NH₂, -(CH₂)₃NHCONH₂ et -(CH₂)₃NHC(=NH)NH₂.

12. Conjugué de médicament selon l'une quelconque des revendications 1 à 11, dans lequel X est un groupe d'extension choisi parmi :
-CONH-(C₁-C₆ alkylène)NH- ;
-COO-CH₂-(phénylène qui peut optionnellement être substitué par un ou plusieurs substituants Rₓ)-NH- ;
-CONH-(C₁-C₆ alkylène)NH-COO-CH₂-(phénylène qui peut optionnellement être substitué par un ou plusieurs substituants Rₓ)-NH- ;
-COCH₂NH-COCH₂-NH- ;
-COCH₂NH- ;
-CONH-(C₁-C₆ alkylène)S- ;
-CONH-(C₁-C₆ alkylène)NHCO(C₁-C₆ alkylène)S- ; et
b est 0 ou 1, de préférence 1 ;
ou
dans lequel X est un groupe d'extension choisi dans le groupe constitué de :
-CONH-(C₂-C₄ alkylène)NH- ;
-COO-CH₂-phénylène-NH-, dans lequel ledit groupe phénylène peut optionnellement être substitué par un à quatre substituants Rₓ choisis dans le groupe constitué de : groupes alkyles possédant 1 à 6 atomes de carbone, groupes alcoxy possédant 1 à 6 atomes de carbone, atomes d'halogène, groupes nitro et groupes cyano ;
-CONH-(C₂-C₄ alkylène)NH-COO-CH₂-(phénylène qui peut optionnellement être substitué par un à quatre substituants Rₓ choisis dans le groupe constitué de : groupes alkyles possédant 1 à 6 atomes de carbone, groupes alcoxy possédant 1 à 6 atomes de carbone, atomes d'halogène, groupes nitro et groupes cyano)-NH- ;
-COCH₂NH-COCH₂-NH- ;
-CONH-(C₂-C₄ alkylène)S- ;
-CONH-(C₂-C₄ alkylène)NHCO(C₁-C₃ alkylène)S- ; et
b est 0 ou 1, de préférence 1 ;
ou
dans lequel X est un groupe d'extension choisi dans le groupe constitué de :
-COO-CH₂-phénylène-NH- ;
-CONH(CH₂)₃NHCOOCH₂-phénylène-NH- ;
-CONH(CH₂)₃NH- ;
-CONH(CH₂)₃-S- ;
-CONH(CH₂)₃NHCO(CH₂)₂S- ; et
b est 0 ou 1, de préférence 1.

13. Conjugué de médicament selon l'une quelconque des revendications 1 à 12, dans lequel T est un groupe d'extension choisi dans le groupe constitué de : -CO-(C₁-C₆ alkylène)-NH-, -CO-(C₁-C₆ alkylène)-[O-(C₂-C₆ alkylène)]ⱼ-NH-, -COO-(C₁-C₆ alkylène)-[O-(C₂-C₆ alkylène)]ⱼ-NH- ; j étant un nombre entier de 1 à 25 et g étant 0 ou 1 ;
ou
dans lequel T est un groupe d'extension choisi dans le groupe constitué de : -CO-(C₁-C₄ alkylène)NH-, -CO-(C₁-C₄ alkylène)-[O-(C₂-C₄ alkylène)]ⱼ-NH-, -COO-(C₁-C₄ alkylène)-[O-(C₂-C₄ alkylène)]ⱼ-NH-, j étant un nombre entier de 1 à 10 ; et g étant 0 ou 1 ;
ou
dans lequel T est un groupe d'extension choisi dans le groupe constitué de : -CO-(C₁-C₄ alkylène)NH-, -CO-(C₁-C₄ alkylène)-[O-(C₂-C₄ alkylène)]ⱼ-NH-, -COO-(C₁-C₄ alkylène)-[O-(C₂-C₄ alkylène)]ⱼ-NH- ; j étant un nombre entier de 1 à 5 ; et g étant 0 ou 1.

14. Conjugué de médicament selon l'une quelconque des revendications 6 à 13, dans lequel D est une fraction de médicament de formule (II) ou de formule (IIa) ou un sel ou un ester pharmaceutiquement acceptables de celle-ci, dans lequel :
R₁ est un hydrogène ou un C₁-C₆ alkyle substitué ou non substitué, dans lequel les substituants optionnels sont un ou plusieurs substituants Rₓ ;
R₂ est un hydrogène ;
R₃ est un hydrogène ou -C(=O)Rₐ, dans lequel Rₐ est un C₁-C₆ alkyle substitué ou non substitué, dans lequel les substituants optionnels sont un ou plusieurs substituants Rₓ ; et
Z est -O- ;
ou
dans lequel D est une fraction de médicament de formule (II) ou de formule (IIa) ou un sel ou un ester pharmaceutiquement acceptables de celle-ci, dans lequel :
R₁ est un hydrogène ou un méthyle ;
R₂ est un hydrogène ;
R₃ est un hydrogène ; et
Z est -O- ;
ou
dans lequel D est une fraction de médicament de formule (II) ou de formule (IIa) ou un sel ou un ester pharmaceutiquement acceptables de celle-ci dans lequel :
R₁ est un méthyle ;
R₂ est un hydrogène ;
R₃ est un hydrogène ; et
Z est -O-.

15. Conjugué de médicament selon l'une quelconque des revendications 1, 3, 6 à 14, dans lequel D est choisi parmi : 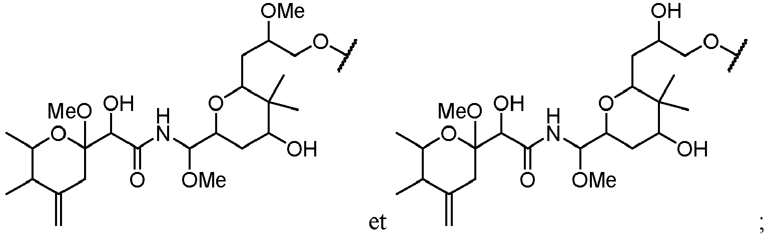
ou un sel ou un ester pharmaceutiquement acceptables de celui-ci, la ligne ondulée indiquant le point de liaison covalente à (X)_{b} s'il existe, ou (AA)_{w} s'il existe, ou à (T)_{g} s'il existe, ou à (L) ;
ou
dans lequel D est choisi parmi : 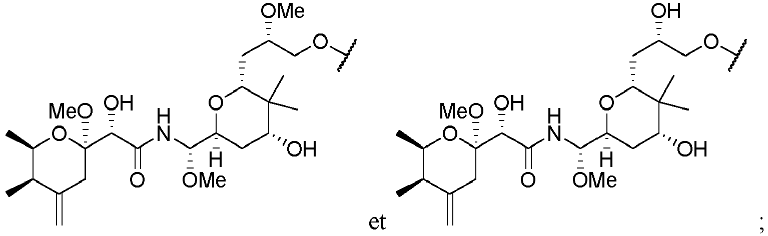
ou un sel ou un ester pharmaceutiquement acceptables de celui-ci, la ligne ondulée indiquant le point de liaison covalente à (X)_{b} s'il existe, ou (AA)_{w} s'il existe, ou à (T)_{g} s'il existe, ou à (L).

16. Conjugué de médicament selon l'une quelconque des revendications 1 à 15, dans lequel la fraction Ab comprenant au moins un site de liaison d'antigène est un peptide de liaison d'antigène ;
optionnellement
dans lequel la fraction Ab comprenant au moins un site de liaison d'antigène est un anticorps, un anticorps à domaine unique ou un fragment de liaison d'antigène de celui-ci ;
optionnellement
dans lequel la fraction Ab comprenant au moins un site de liaison d'antigène est un anticorps monoclonal, un anticorps polyclonal ou un anticorps bispécifique et dans lequel l'anticorps ou un fragment de liaison d'antigène de celui-ci est dérivé de n'importe quelle espèce, de préférence un humain, une souris ou un lapin ;
optionnellement
dans lequel l'anticorps ou un fragment de liaison d'antigène de celui-ci est choisi dans le groupe constitué de : un anticorps humain, un fragment de liaison d'antigène d'un anticorps humain, un anticorps humanisé, un fragment de liaison d'antigène d'un anticorps humanisé, un anticorps chimérique, un fragment de liaison d'antigène d'un anticorps chimérique, un anticorps glycosylé et un fragment de liaison d'antigène glycosylé ;
optionnellement
dans lequel l'anticorps ou un fragment de liaison d'antigène de celui-ci est un fragment de liaison d'antigène choisi dans le groupe constitué de : un fragment Fab, un fragment Fab', un fragment F(ab')2 et un fragment Fv ;
optionnellement
dans lequel l'anticorps ou un fragment de liaison d'antigène de celui-ci est un anticorps monoclonal qui se lie immuno-spécifiquement à des antigènes de cellules cancéreuses, des antigènes viraux, des antigènes de cellules qui produisent des anticorps auto-immuns associés à une maladie auto-immune, des antigènes microbiens et de préférence un anticorps monoclonal qui se lie immuno-spécifiquement à des antigènes de cellules cancéreuses ;
optionnellement
dans lequel la fraction Ab comprenant au moins un site de liaison d'antigène est un anticorps choisi dans le groupe constitué de : Abciximab, Alemtuzumab, Anétumab, Atézolizumab, Avélumab, Basiliximab, Bévacizumab, Blinatomumab, Brentuximab, Catumaxomab, Cétuximab, Coltuximab, Daclizumab, Daratumumab, Dénintuzumab, Dénosumab, Dépatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labétuzumab, Ladiratuzumab, Laprituximab, Lifastuzumab, Lorvotuzumab, Milatuzumab, Mirvétuximab, Naratuximab, Nécitumumab, Nimotuzumab, Nivolumab, Obinutuzumab, Ofatumumab, Olaratumab, Omalizumab, Palivizumab, Panitumumab, Pembrolizumab, Pertuzumab, Pinatuzumab, Polatuzumab, Ramucirumab, Rovalpituzumab, Sacituzumab, Siltuximab, Sirtratumab, Sofituzumab, Vadastuximab, Vorsétuzumab, un anticorps anti-HER2 tel que Trastuzumab, un anticorps anti-CD4, un anticorps anti-CD5 et un anticorps anti-CD30 ou un fragment de liaison d'antigène ou une portion immunologiquement active de ceux-ci ;
optionnellement
dans lequel la fraction Ab comprenant au moins un site de liaison d'antigène est un anticorps choisi dans le groupe constitué de : Abciximab, Alemtuzumab, Anétumab, Atézolizumab, Avélumab, Basiliximab, Bévacizumab, Blinatomumab, Brentuximab, Catumaxomab, Cétuximab, Daclizumab, Daratumumab, Dénintuzumab, Dénosumab, Dépatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labétuzumab, Ladiratuzumab, Laprituximab, Mirvétuximab, Naratuximab, Nécitumumab, Nimotuzumab, Nivolumab, Obinutuzumab, Ofatumumab, Olaratumab, Omalizumab, Palivizumab, Panitumumab, Pembrolizumab, Pertuzumab, Polatuzumab, Ramucirumab, Rovalpituzumab, Sacituzumab, Siltuximab, Sirtratumab, Vadastuximab, Vorsétuzumab, un anticorps anti-HER2 tel que Trastuzumab, un anticorps anti-CD4, un anticorps anti-CD5 et un anticorps anti-CD30 ou un fragment de liaison d'antigène ou une portion immunologiquement active de ceux-ci ;
optionnellement
dans lequel la fraction Ab comprenant au moins un site de liaison d'antigène est un anticorps choisi dans le groupe constitué de : Abciximab, Alemtuzumab, Atézolizumab, Avélumab, Basiliximab, Bévacizumab, Blinatomumab, Brentuximab, Catumaxomab, Cétuximab, Daclizumab, Daratumumab, Dénosumab, Dinutuximab, Durvalumab, Elotuzumab, Gemtuzumab, Ibritumomab, Inotuzumab, Ipilimumab, Labétuzumab, Nécitumumab, Nimotuzumab, Nivolumab, Obinutuzumab, Ofatumumab, Olaratumab, Omalizumab, Palivizumab, Panitumumab, Pembrolizumab, Pertuzumab, Ramucirumab, Rovalpituzumab, Siltuximab, un anticorps anti-HER2 tel que Trastuzumab, un anticorps anti-CD4, un anticorps anti-CD5 et un anticorps anti-CD30 ou un fragment de liaison d'antigène ou une portion immunologiquement active de ceux-ci, plus préférablement Brentuximab, Gemtuzumab, Inozutumab, Rovalpituzumab, un anticorps anti-HER2 tel que Trastuzumab, un anticorps anti-CD4, un anticorps anti-CD5 et un anticorps anti-CD30 ou un fragment de liaison d'antigène ou une portion immunologiquement active de ceux-ci, de préférence un anticorps anti-HER2 tel que Trastuzumab ou un fragment de liaison d'antigène ou une portion immunologiquement active de celui-ci, plus préférablement Trastuzumab ou un fragment de liaison d'antigène ou une portion immunologiquement active de celui-ci.

17. Conjugué anticorps-médicament selon la revendication 1, choisi dans le groupe constitué de : 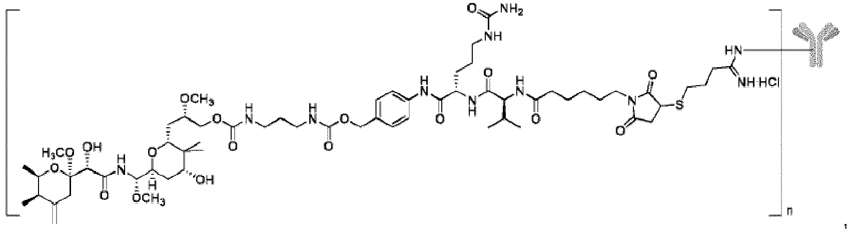 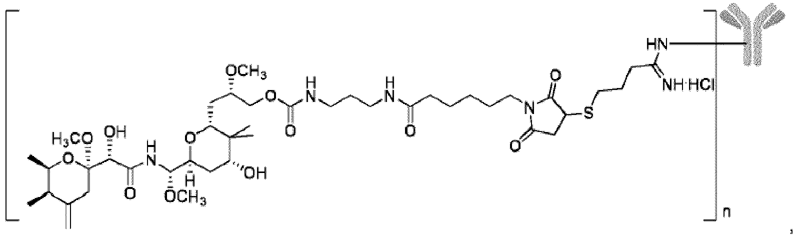 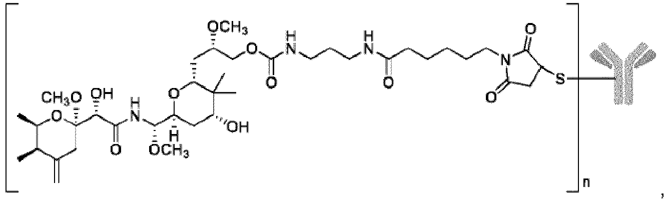 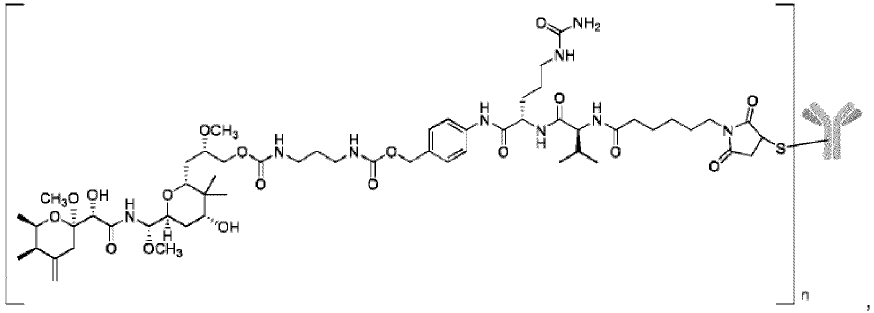 et 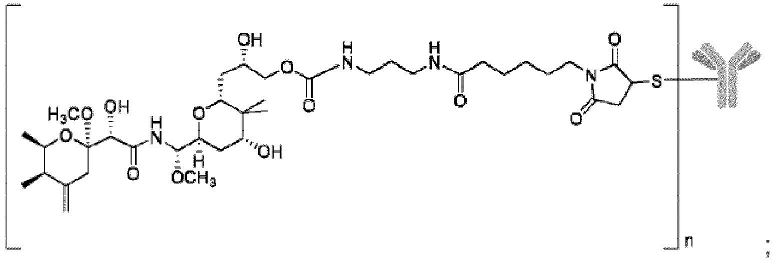 où n vaut 2 à 6, plus préférablement 3, 4 ou 5 et chaque 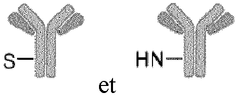 est indépendamment choisi parmi : Brentuximab, Gemtuzumab, Inozutumab, Rovalpituzumab, un anticorps anti-HER2 tel que Trastuzumab, un anticorps anti-CD4, un anticorps anti-CD5 et un anticorps anti-CD30 ou un fragment de liaison d'antigène ou une portion immunologiquement active de ceux-ci et plus préférablement un anticorps anti-HER2 tel que Trastuzumab ou un fragment de liaison d'antigène ou une portion immunologiquement active de celui-ci, particulièrement Trastuzumab ou un fragment de liaison d'antigène ou une portion immunologiquement active de celui-ci.

18. Conjugué de médicament selon la revendication 17, dans lequel la fraction Ab comprenant au moins un site de liaison d'antigène est un anticorps anti-HER2 tel que Trastuzumab ou un fragment de liaison d'antigène ou une portion immunologiquement active de celui-ci.

19. Conjugué de médicament selon la revendication 17, dans lequel la fraction Ab comprenant au moins un site de liaison d'antigène est choisie parmi Trastuzumab ou un fragment de liaison d'antigène ou une portion immunologiquement active de celui-ci.

20. Conjugué anticorps-médicament selon l'une quelconque des revendications 1 à 19 sous une forme isolée ou purifiée.

21. Composé de formule D-(X)_{b}-(AA)_{w}-(T)_{g}-L₁ ou de formule D-(X)_{b}-(AA)_{w}-(T)_{g}-H, dans lequel :
L₁ est un lieur choisi dans le groupe de formules constitué de : 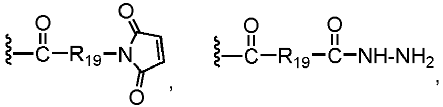 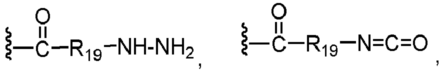 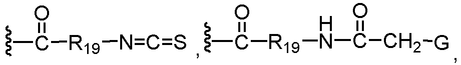 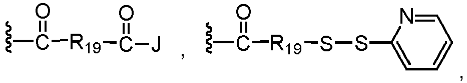 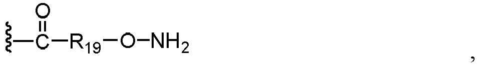
dans lequel chacune des lignes ondulées indique le point de liaison covalente à (T)_{g} s'il existe, ou (AA)_{w} s'il existe, ou à (X)_{b} s'il existe, ou à D ;
G est choisi parmi : halo, -O-mésyle et -O-tosyle ;
J est choisi parmi : halo, hydroxy, -N-succinimidoxy, -O-(4-nitrophényle), -O-pentafluorophényle, -O-tetrafluorophényle et -O-C(O)-OR₂₀ ;
R₁₉ est choisi parmi : -C₁-C₁₂ alkylène-, -C₃-C₈ carbocyclo-, -O-(C₁-C₁₂ alkylène)-, -C₆-C₁₈ arylène- dans un ou plusieurs cycles qui peuvent optionnellement être substitués par un ou plusieurs substituants Rₓ, -C₁-C₁₂ alkylène-C₆-C₁₈ arylène- dans lequel le groupe arylène est dans un ou plusieurs cycles qui peuvent optionnellement être substitués par un ou plusieurs substituants Rₓ, -C₆-C₁₈ arylène-C₁-C₁₂ alkylène- dans lequel le groupe arylène est dans un ou plusieurs cycles qui peuvent optionnellement être substitués par un ou plusieurs substituants Rₓ, -C₁-C₁₂ alkylène-(C₃-C₈ carbocyclo)-, -(C₃-C₈ carbocyclo)-C₁-C₁₂ alkylène-, -C₅-C₁₄ hétérocyclo- dans lequel ledit groupe hétérocyclo peut être un groupe saturé ou insaturé possédant un ou plusieurs cycles et comprenant au moins un atome d'oxygène, d'azote ou de soufre dans le(s)dit(s) cycle(s), ledit groupe étant optionnellement substitué par un ou plusieurs substituants Rₓ, -C₁-C₁₂ alkylène-(C₅-C₁₄ hétérocyclo)- dans lequel ledit groupe hétérocyclo peut être un groupe saturé ou insaturé possédant un ou plusieurs cycles et comprenant au moins un atome d'oxygène, d'azote ou de soufre dans le(s)dit(s) cycle(s), ledit groupe étant optionnellement substitué par un ou plusieurs substituants Rₓ, -(C₅-C₁₄ hétérocyclo)-C₁-C₁₂ alkylène-, dans lequel ledit groupe hétérocyclo peut être un groupe saturé ou insaturé possédant un ou plusieurs cycles et comprenant au moins un atome d'oxygène, d'azote ou de soufre dans le(s)dit(s) cycle(s), ledit groupe étant optionnellement substitué par un ou plusieurs substituants Rₓ, -(OCH₂CH₂)ᵣ- et -CH₂-(OCH₂CH₂)ᵣ-, dans lequel chacun des substituants alkylènes ci-dessus, soit seul, soit lié à une autre fraction dans la chaîne carbonée, peut optionnellement être substitué par un ou plusieurs substituants Rₓ ;
R₂₀ est un C₁-C₁₂ alkyle ou un groupe aryle possédant 6 à 18 atomes de carbone dans un ou plusieurs cycles aromatiques, lesdits groupes aryles étant optionnellement substitués par un ou plusieurs substituants Rₓ ;
les substituants Rₓ sont choisis dans le groupe constitué de : groupes C₁-C₁₂ alkyles qui peuvent être optionnellement substitués par au moins un groupe R_{y}, groupes C₂-C₁₂ alcényles qui peuvent être optionnellement substitués par au moins un groupe R_{y}, groupes C₂-C₁₂ alcynyles qui peuvent être optionnellement substitués par au moins un groupe R_{y}, atomes d'halogène, groupes oxo, groupes thio, groupes cyano, groupes nitro, OR_{y}, OCOR_{y}, OCOOR_{y}, COR_{y}, COOR_{y}, OCONR_{y}R_{z}, CONR_{y}R_{z}, SR_{y}, S(=O)R_{y}, SO₂R_{y}, SSR_{y}, P(O)(R_{y})OR_{z}, NR_{y}R_{z}, NR_{y}COR_{z}, NR_{y}C(=O)NR_{y}R_{z}, NR_{y}C(=NR_{y})NR_{y}R_{z}, groupes aryles possédant 6 à 18 atomes de carbone dans un ou plusieurs cycles qui peuvent optionnellement être substitués par un ou plusieurs substituants qui peuvent être identiques ou différents choisis dans le groupe constitué de : R_{y}, OR_{y}, OCOR_{y}, OCOOR_{y}, NR_{y}R_{z}, NR_{y}COR_{z} et NR_{y}C(=NR_{y})NR_{y}R_{z}, groupes aralkyles comprenant un groupe alkyle possédant 1 à 12 atomes de carbone substitué par un groupe aryle optionnellement substitué tel que défini ci-dessus, groupes aralkyloxy comprenant un groupe alcoxy possédant 1 à 12 atomes de carbone substitué par un groupe aryle optionnellement substitué tel que défini ci-dessus et un groupe hétérocyclique à 5 à 14 chaînons saturé ou insaturé possédant un ou plusieurs cycles et comprenant au moins un atome d'oxygène, d'azote ou de soufre dans le(s)dit(s) cycle(s), ledit groupe hétérocyclique étant optionnellement substitué par un ou plusieurs substituants R_{y} et lorsqu'il y a plus d'un substituent optionnel sur un quelconque groupe donné, les substituants optionnels R_{y} peuvent être identiques ou différents ;
chaque R_{y} et R_{z} est indépendamment choisi dans le groupe constitué de : hydrogène, groupes C₁-C₁₂ alkyles, groupes C₁-C₁₂ alkyles qui sont substitués par au moins un atome d'halogène, groupes aralkyles comprenant un groupe C₁-C₁₂ alkyle qui est substitué par un groupe aryle possédant 6 à 18 atomes de carbone dans un ou plusieurs cycles et groupes hétérocycloalkyles comprenant un groupe C₁-C₁₂ alkyle qui est substitué par un groupe hétérocyclique à 5 à 14 chaînons saturé ou insaturé possédant un ou plusieurs cycles et comprenant au moins un atome d'oxygène, d'azote ou de soufre dans le(s)dit(s) cycle(s) ;
r est un nombre entier variant de 1 à 10 ;
b est un nombre entier de 0 ou 1 ;
g est un nombre entier de 0 ou 1 ;
w est un nombre entier variant de 0 à 12 ;
chacun de D, X, T et AA est tel que défini dans l'une quelconque des revendications 1 à 15 ;
dans lequel, pour les composés de formule D-(X)_{b}-(AA)_{w}-(T)_{g}-H, b + w + g n'est pas 0 ;
optionnellement
dans lequel le composé de formule D-(X)_{b}-(AA)_{w}-(T)_{g}-L₁ est choisi parmi : 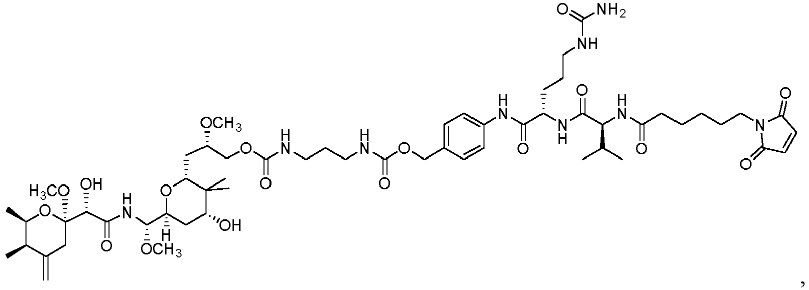 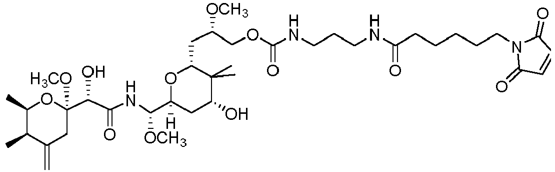 et

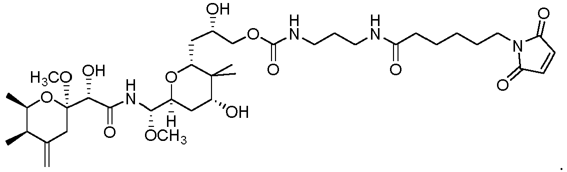

22. Composé de formule D-(X)_{b}-(AA)_{w}-(T)_{g}-L₁ ou de formule D-(X)_{b}-(AA)_{w}-(T)_{g}-H, dans lequel chacun de D, X, AA, T, b, g et w est tel que défini dans l'une quelconque des revendications 1 à 15 et L₁ est tel que défini dans la revendication 21 ; mais en outre dans lequel si le composé est un composé de formule D-(X)_{b}-(AA)_{w}-(T)_{g}-H, alors b + w + g ≠ 0.

23. Conjugué de médicament ou composé selon l'une quelconque des revendications 1 à 22, dans lequel b + w + g n'est pas 0 ;
ou
dans lequel b + w n'est pas 0 ;
ou
dans lequel lorsque w n'est pas 0, alors b est 1.

24. Fraction de médicament selon l'une quelconque des revendications 1 à 20 pour une utilisation comme charge utile dans un conjugué anticorps-médicament.

25. Utilisation d'une fraction de médicament selon l'une quelconque des revendications 1 à 20, dans la fabrication d'un conjugué anticorps-médicament.

26. Conjugué de médicament selon l'une quelconque des revendications 1 à 20, pour utilisation en tant que médicament.

27. Conjugué de médicament selon l'une quelconque des revendications 1 à 20 pour utilisation dans le traitement d'un cancer, de préférence un cancer choisi parmi : cancer du poumon, incluant NSCLC, cancer gastrique, cancer colorectal, cancer du sein, carcinome du pancréas, cancer de l'endomètre, cancer de la vessie, cancer du col de l'utérus, cancer oesophagien, cancer de la vésicule biliaire, cancer utérin, cancer du canal salivaire, cancer ovarien, cancer du rein, leucémie, myélome multiple et lymphome.

28. Conjugué de médicament pour utilisation selon la revendication 27, où le cancer est un cancer HER2-positif, de préférence cancer du poumon HER2-positif incluant NSCLC HER2-positif, cancer gastrique HER2-positif, cancer colorectal HER2-positif, cancer du sein HER2-positif, carcinome du pancréas HER2-positif, cancer de l'endomètre HER2-positif, cancer de la vessie HER2-positif, cancer du col de l'utérus HER2-positif, cancer oesophagien HER2-positif, cancer de la vésicule biliaire HER2-positif, cancer utérin HER2-positif, cancer du canal salivaire HER2-positif et cancer ovarien HER2-positif.

29. Composition pharmaceutique comprenant un conjugué de médicament selon l'une quelconque des revendications 1 à 20 et un véhicule pharmaceutiquement acceptable.

30. Kit comprenant une quantité thérapeutiquement efficace d'un conjugué de médicament selon l'une quelconque des revendications 1 à 20 et un véhicule pharmaceutiquement acceptable.

31. Kit selon la revendication 30 pour utilisation dans le traitement d'un cancer et de préférence un cancer choisi parmi : cancer du poumon, incluant NSCLC, cancer gastrique, cancer colorectal, cancer du sein, carcinome du pancréas, cancer de l'endomètre, cancer de la vessie, cancer du col de l'utérus, cancer oesophagien, cancer de la vésicule biliaire, cancer utérin, cancer du canal salivaire, cancer ovarien, cancer du rein, leucémie, myélome multiple et lymphome.

32. Procédé de préparation d'un conjugué médicament-anticorps selon l'une quelconque des revendications 1 à 20 comprenant la conjugaison d'une fraction Ab comprenant au moins un site de liaison d'antigène et d'un médicament D, Ab et D étant tels que définis dans l'une quelconque des revendications 1 à 20 ;
optionnellement dans lequel :
la préparation d'un conjugué médicament-anticorps de formule (G), (G') ou (G") : 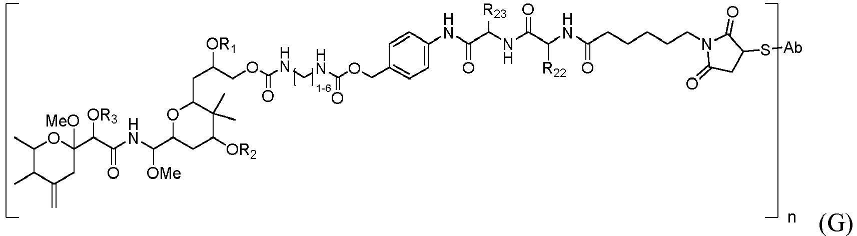 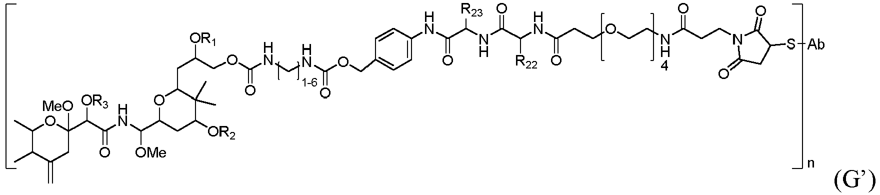 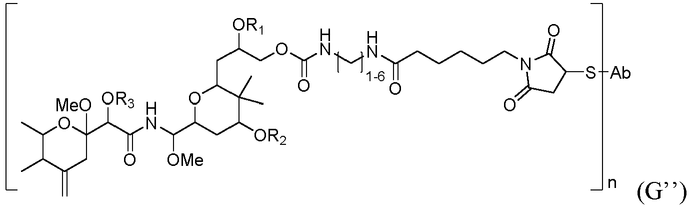
comprenant les étapes suivantes :
(i) soit :
(a) la mise en réaction d'un composé de formule (Ib) : 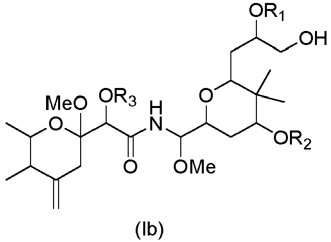 dans laquelle :
R₁ est choisi parmi un groupe protecteur pour OH, un C₁-C₁₂ alkyle substitué ou non substitué, un C₂-C₁₂ alcényle substitué ou non substitué, un C₂-C₁₂ alcynyle substitué ou non substitué, -C(=O)Rₐ, -C(=O)OR_{b} et -C(=O)NR_{c}R_{d} ;
R₂ est un hydrogène ;
R₃ est choisi parmi un groupe protecteur pour OH, -C(=O)Rₐ, -C(=O)OR_{b} et -C(=O)NR_{c}R_{d} ;
Rₐ est choisi parmi : hydrogène, C₁-C₁₂ alkyle substitué ou non substitué, C₂-C₁₂ alcényle substitué ou non substitué, C₂-C₁₂ alcynyle substitué ou non substitué, aryle substitué ou non substitué et groupe hétérocyclique substitué ou non substitué ;
R_{b} est choisi parmi : C₁-C₁₂ alkyle substitué ou non substitué, C₂-C₁₂ alcényle substitué ou non substitué, C₂-C₁₂ alcynyle substitué ou non substitué, aryle substitué ou non substitué et groupe hétérocyclique substitué ou non substitué ;
R_{c} et R_{d} sont indépendamment choisis parmi : hydrogène, C₁-C₁₂ alkyle substitué ou non substitué, C₂-C₁₂ alcényle substitué ou non substitué, C₂-C₁₂ alcynyle substitué ou non substitué et aryle substitué ou non substitué et groupe hétérocyclique substitué ou non substitué ;
avec un composé de formule X₂-C(=O)-X₁ dans lequel X₁ et X₂ sont des groupes partants pour donner un composé de formule (B) : 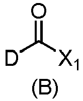
et le point de liaison de la fraction -C(=O)X₁ est le groupe -OH primaire libre du composé de formule (Ib), ou
(b) la mise en réaction dudit composé de formule (Ib) tel que défini ci-dessus avec du 4-nitro-phénylchloroformate pour donner un composé de formule (J) : 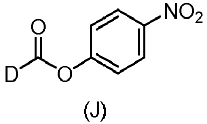 dans lequel le point de liaison du groupe (4-nitrophényl)-O-CO- est le même que celui pour la fraction X₁(C=O) dans (a) ci-dessus ; ou
(c) la mise en réaction d'un composé de formule (Ib) tel que défini ci-dessus avec un isocyanate de formule O=C=N-(CH₂)₁₋₆NHProt^{NH} dans lequel Prot^{NH} est un groupe protecteur pour amino approprié pour être déprotégé dans des conditions basiques pour donner un composé de formule : 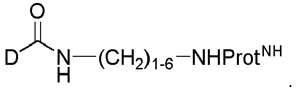
(ii) soit :
(a) la mise en réaction du composé de formule (B) ou (J) produit dans l'étape (i)(a) ou (i)(b) avec un composé amino de formule NH₂-(CH₂)₁₋₆NH₂ pour donner un composé de formule (C) : 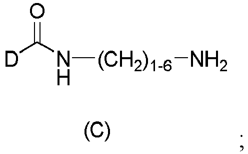 ou
(b) la déprotection du composé de formule : 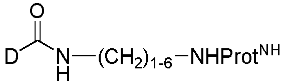 obtenu dans l'étape (i)(c) pour donner un composé de formule (C).
(iii) la mise en réaction du composé de formule (C) avec un composé de formule (D'), (E) ou (E') : 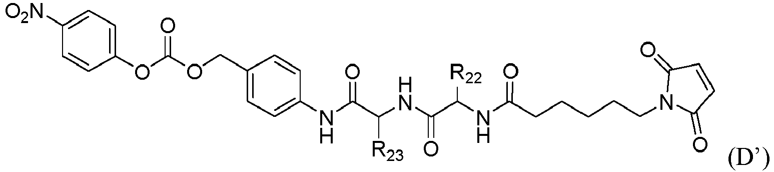 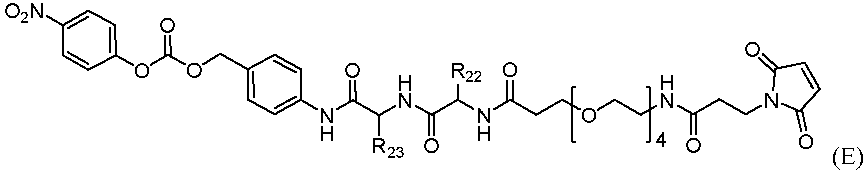 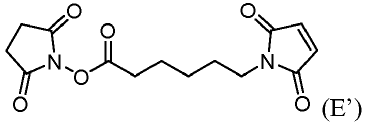
où R₂₂ et R₂₃ sont tels que définis ci-dessus dans les définitions des groupes AA ;
pour donner un composé de formule (F), (F') ou (F"), respectivement : 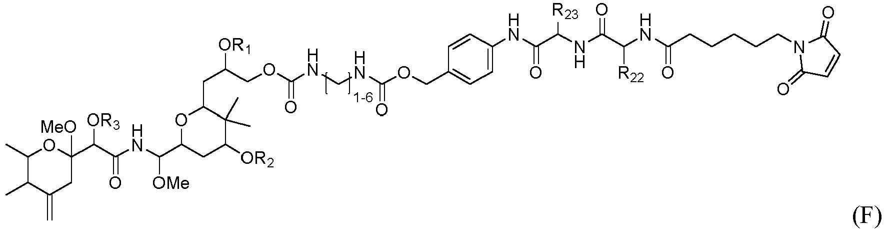 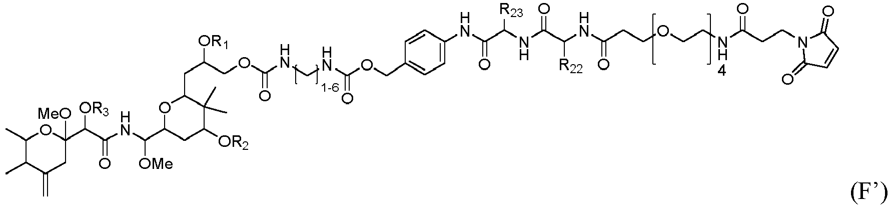 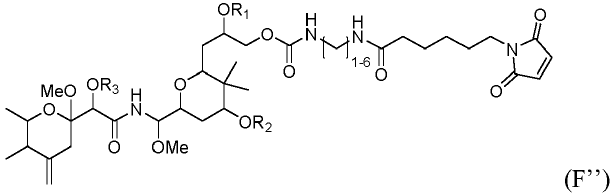
où R₁, R₂ et R₃ sont tels que définis ci-dessus pour les composés de formule (Ib) et R₂₂ et R₂₃ sont tels que définis ci-dessus dans les définitions des groupes AA,
(iv) si des groupes protecteurs pour OH sont présents dans les composés de formule (F), (F') ou (F"), le retrait de ces groupes protecteurs pour donner des composés de formule (F), (F') ou (F") où R₁, R₂ et R₃ sont tels que définis ci-dessus pour les composés de formule (II),
(v) réduction partielle d'une ou de plusieurs liaisons disulfures dans l'anticorps à conjuguer pour donner un anticorps réduit Ab-SH possédant des groupes thiols libres : 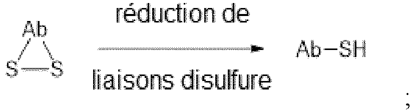 et
(vi) la mise en réaction de l'anticorps partiellement réduit Ab-SH possédant des groupes thiols libres avec le composé de formule (F), (F') ou (F") produit dans l'étape (iv) pour donner le conjugué médicament-anticorps désiré de formule (G), (G') ou (G") respectivement :
